# EUROPEAN PATENT APPLICATION

(11) **EP 4 621 029 A1**
(43) Date of publication of application: **24.09.2025**
(21) Application number: 25164260.9
(22) Date of filing: 17.03.2025
(51) Int. Cl.: C09K 11/06, H10K 50/15, H10K 85/60, C07C 211/61

(54) **LIGHT EMITTING ELEMENT, AMINE COMPOUND FOR THE SAME, AND DISPLAY DEVICE INCLUDING THE SAME**

(30) Priority: 22.03.2024 KR 20240039578
(71) Applicant: Samsung Display Co., Ltd, Yongin-si, Gyeonggi-do (KR)
(72) Inventor: JEONG, Eunjae, Yongin-si (KR); YOO, Byeongwook, Yongin-si (KR); LEE, Jeong Min, Yongin-si (KR); JO, Sohee, Yongin-si (KR)
(74) Representative: Marks & Clerk LLP

(57) **Abstract**

Embodiments provide an amine compound, a light emitting element that includes the amine compound, and a display device that includes the light emitting element. The light emitting element includes a first electrode, a hole transport region disposed on the first electrode, an emission layer disposed on the hole transport region, an electron transport region disposed on the emission layer, and a second electrode disposed on the electron transport region, wherein the hole transport region includes the amine compound. The amine compound is represented by Formula 1, which is explained in the specification.

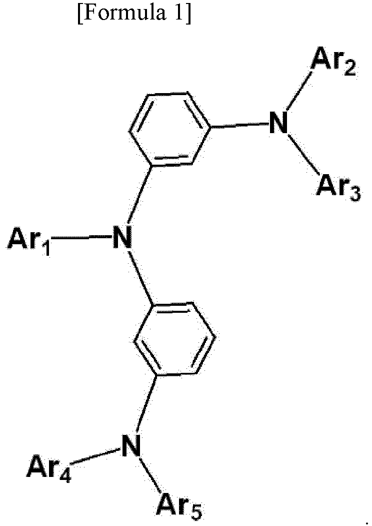

## Description

### BACKGROUND

### 1. Technical Field

The disclosure relates to a light emitting element, an amine compound used therein, and a display device including the light emitting element.

### 2. Description of the Related Art

Ongoing development continues for organic electroluminescence display devices and the like as image display devices. Organic electroluminescence display devices and the like include so-called self-luminescent light emitting elements in which holes and electrons respectively injected from a first electrode and a second electrode recombine in an emission layer, so that a luminescent material in the emission layer emits light to achieve display.

In the application of light emitting elements to display devices, there is a persistent demand for greater luminous efficiency and service life. Continuous development is required on materials for light emitting elements that are capable of stably achieving such characteristics.

It is to be understood that this background of the technology section is, in part, intended to provide useful background for understanding the technology. However, this background of the technology section may also include ideas, concepts, or recognitions that were not part of what was known or appreciated by those skilled in the pertinent art prior to a corresponding effective filing date of the subject matter disclosed herein.

### SUMMARY

The disclosure provides a light emitting element having increased luminous efficiency and lifetime, and a display device including the same.

The disclosure also provides an amine compound as a material for a light emitting element, which improves luminous efficiency and service life.

According to embodiment, a light emitting element includes a first electrode, a hole transport region disposed on the first electrode, an emission layer disposed on the hole transport region, an electron transport region disposed on the emission layer, and a second electrode disposed on the electron transport region, wherein the hole transport region includes an amine compound represented by Formula 1.

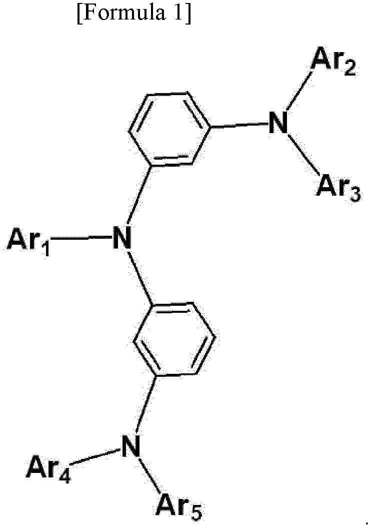

In Formula 1, at least one of Ar₁ to Ar₅ is each independently a group represented by Formula 2; and the remainder of Ar₁ to Ar₅ are each independently a substituted or unsubstituted aryl group having 6 to 30 ring-forming carbon atoms or a substituted or unsubstituted heteroaryl group having 2 to 30 ring-forming carbon atoms.

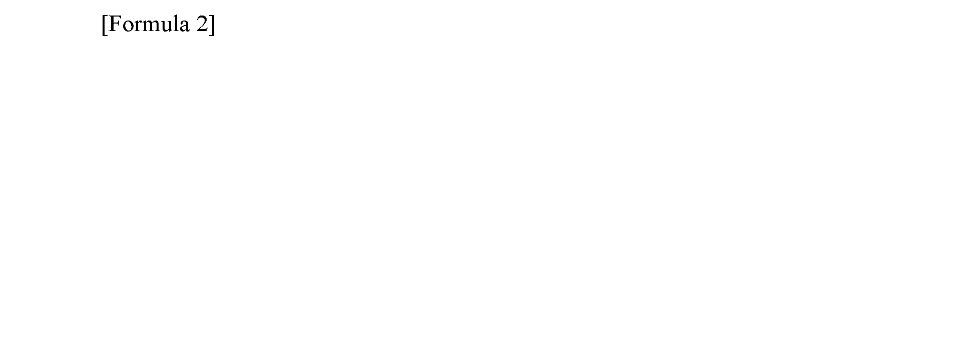

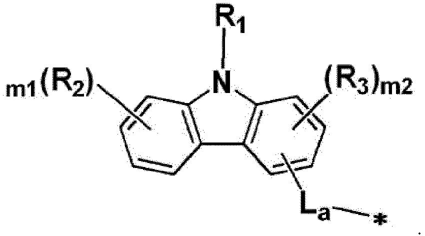

In Formula 2, m1 is an integer from 0 to 4; m2 is an integer from 0 to 3; R₁ to R₃ are each independently a hydrogen atom, a deuterium atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms; and Lₐ is a direct linkage, a substituted or unsubstituted arylene group having 6 to 30 ring-forming carbon atoms, or a substituted or unsubstituted heteroarylene group having 2 to 30 ring-forming carbon atoms.

In an embodiment, the amine compound may be represented by one of Formula 1-A to Formula 1-C.

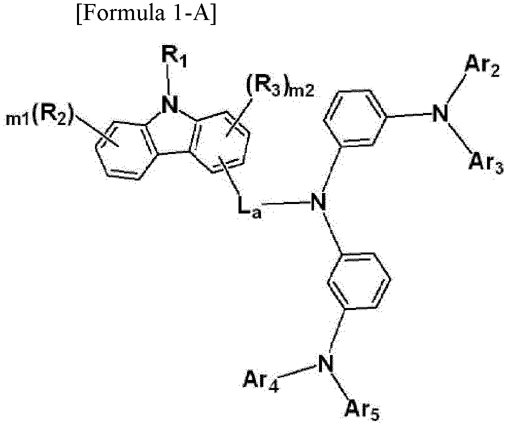

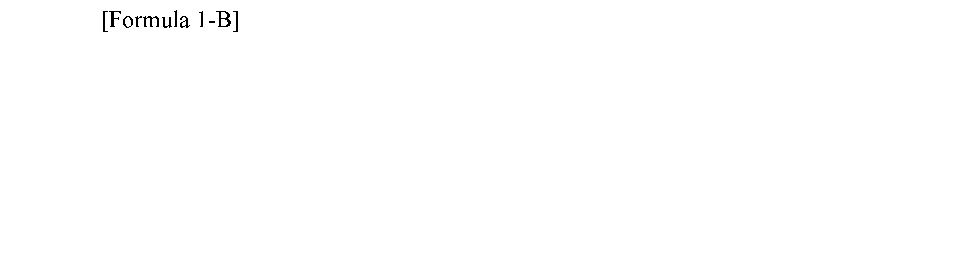

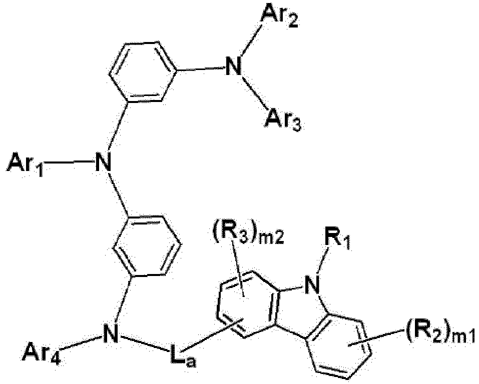

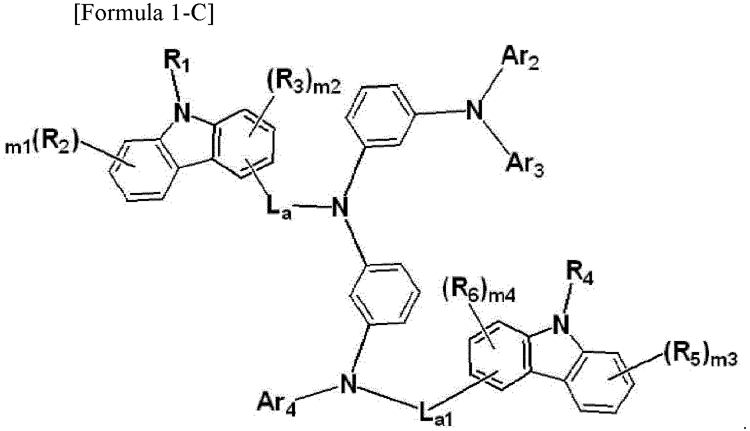

In Formula 1-C, m3 is an integer from 0 to 4; m4 is an integer from 0 to 3; R₄ to R₆ are each independently a hydrogen atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms; and Lₐ₁ is a direct linkage, a substituted or unsubstituted arylene group having 6 to 30 ring-forming carbon atoms, or a substituted or unsubstituted heteroarylene group having 2 to 30 ring-forming carbon atoms. In Formulas 1-A to 1-C, m1, m2, Ar₁ to Ar₅, R₁ to R₃, and Lₐ are the same as defined in Formulas 1 and 2.

In an embodiment, the amine compound may be represented by one of Formula 1-A1 to Formula 1-A4.

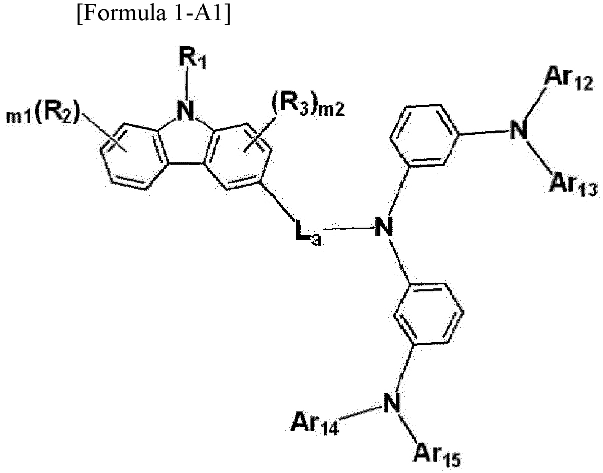

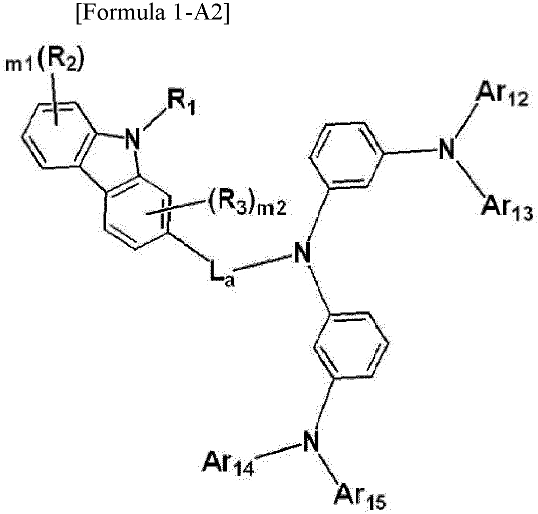

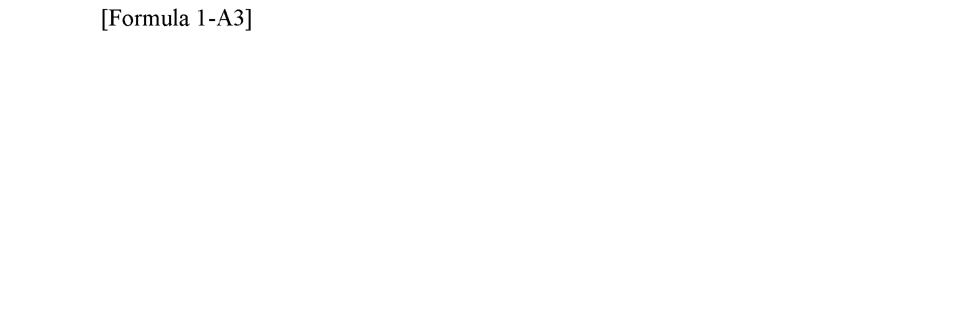

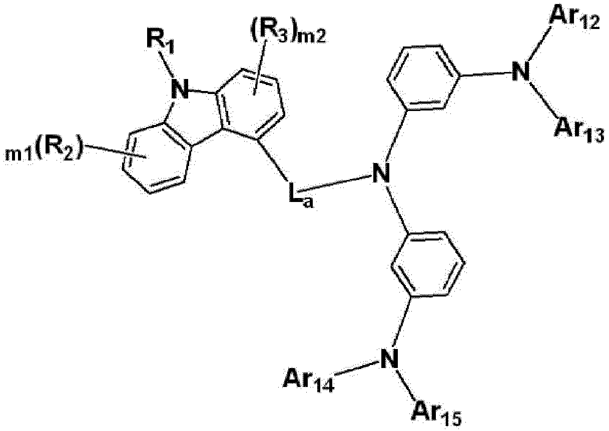

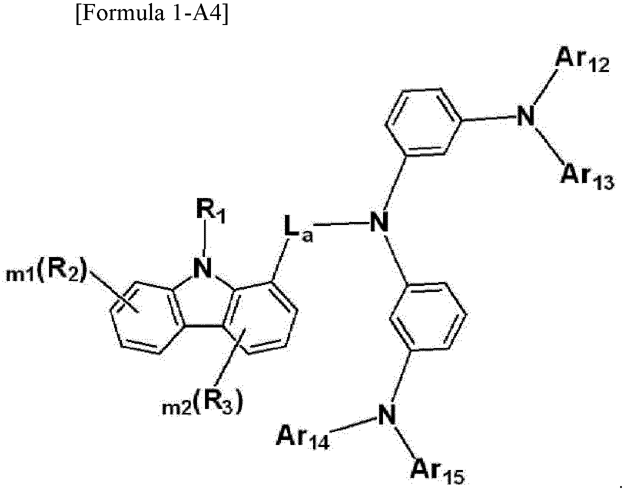

In Formulas 1-A1 to 1-A4, Ar₁₂ to Ar₁₅ are each independently a substituted or unsubstituted aryl group having 6 to 30 ring-forming carbon atoms; and m1, m2, R₁ to R₃, and Lₐ are the same as defined in Formulas 1 and 2.

In an embodiment, in Formula 1, the remainder of Ar₁ to Ar₅ that are not a group represented by Formula 2 may each independently be a substituted or unsubstituted phenyl group, an unsubstituted biphenyl group, an unsubstituted naphthyl group, or a substituted or unsubstituted terphenyl group.

In an embodiment, in Formula 1, the remainder of Ar₁ to Ar₅ that are not a group represented by Formula 2 may each independently be a group represented by one of Formula AR1-1 to Formula AR1-20.

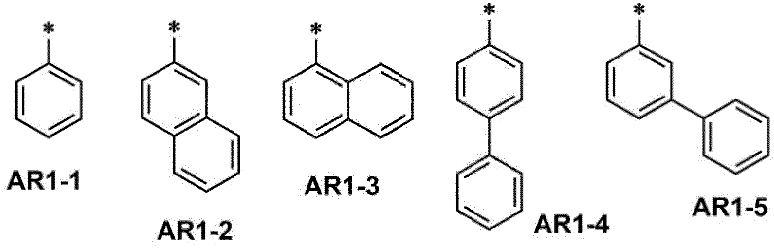

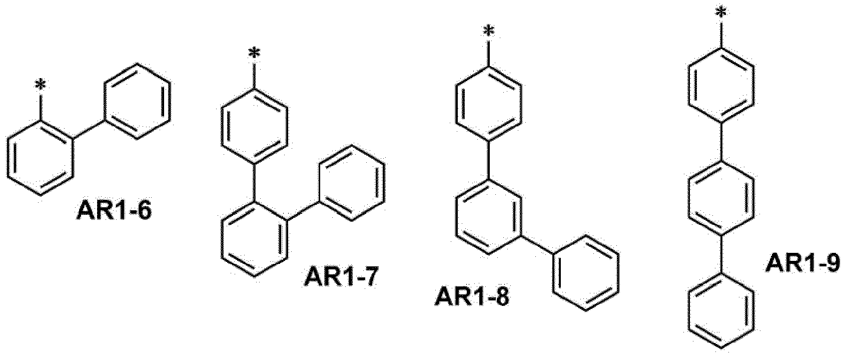

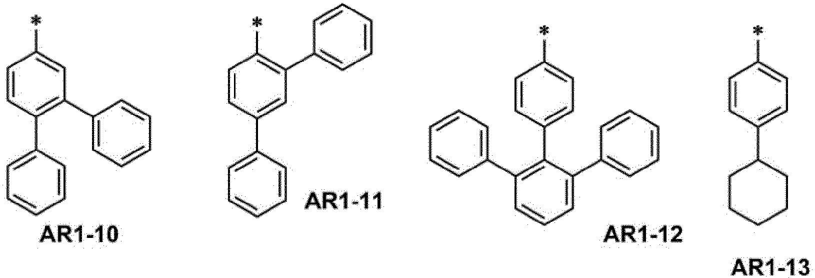

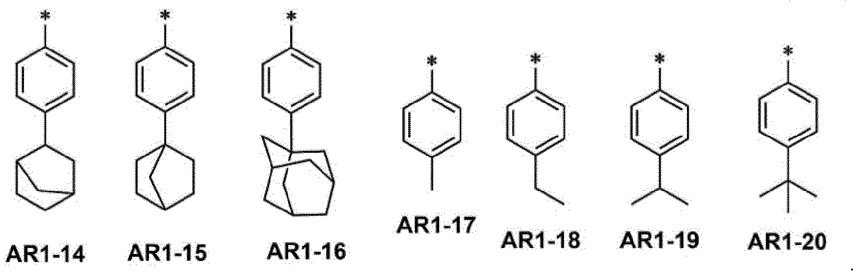

In an embodiment, in Formula 2, R₁ may be an unsubstituted methyl group, an unsubstituted ethyl group, an unsubstituted isopropyl group, an unsubstituted t-butyl group, an unsubstituted phenyl group, or a group represented by one of Formula R1-1 to Formula R1-7.

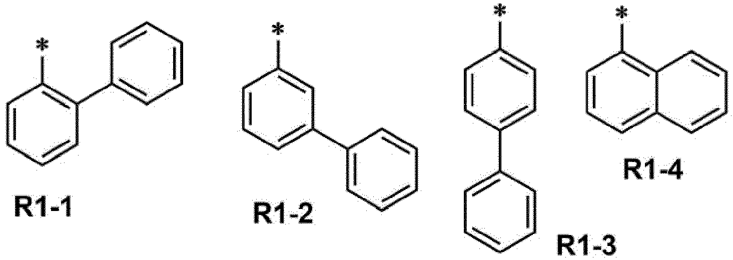

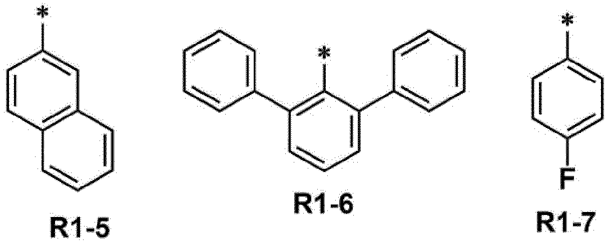

In an embodiment, the group represented by Formula 2 may be a group represented by one of Formula 2-1 to Formula 2-10.

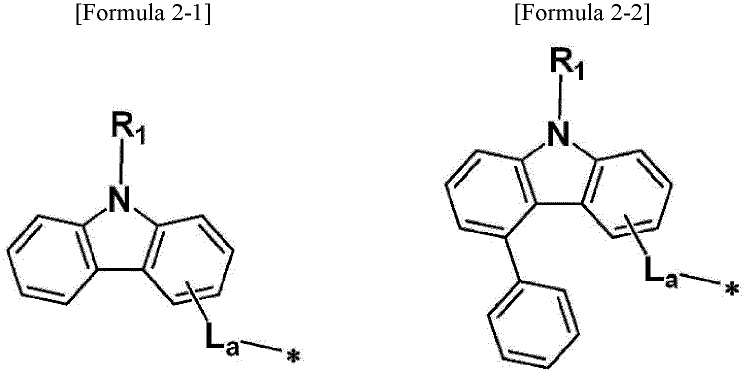

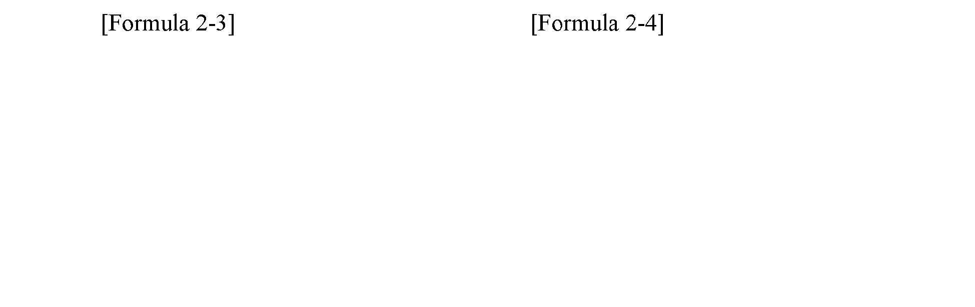

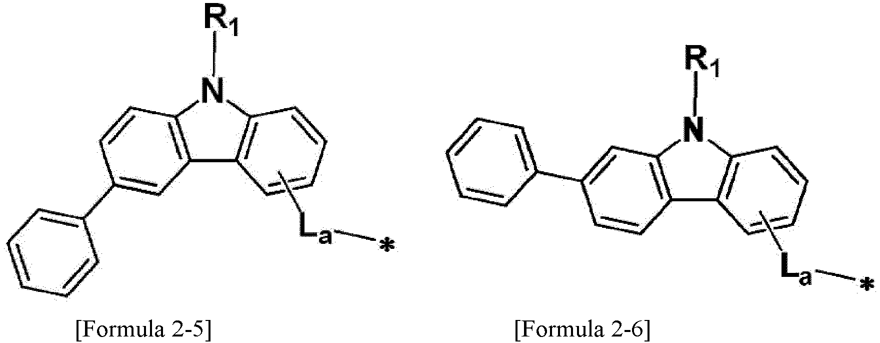

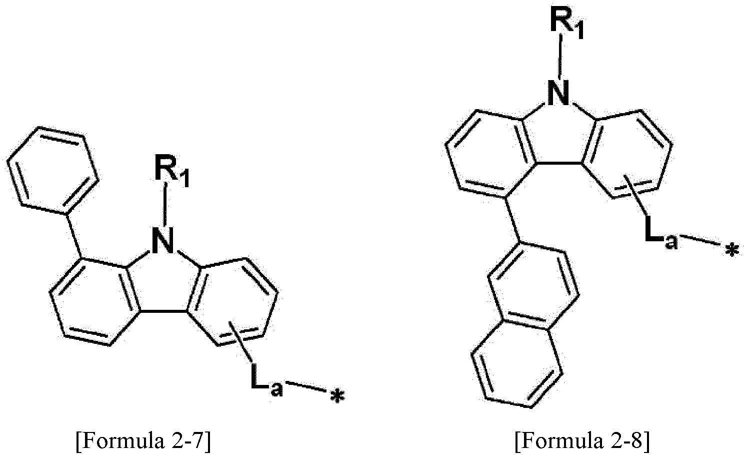

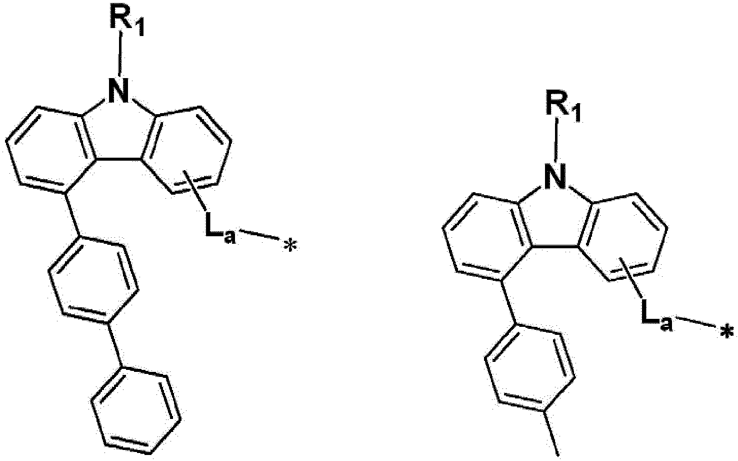

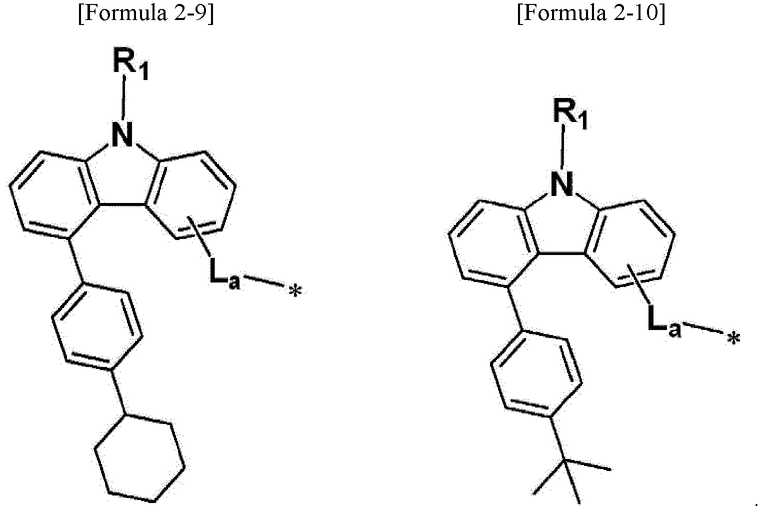

In Formulas 2-1 to 2-10, R₁ and Lₐ are the same as defined in Formula 2.

In an embodiment, in Formula 2, Lₐ may be a direct linkage or an unsubstituted phenylene group.

In an embodiment, the emission layer may include at least one of a first compound represented by Formula HT-1, a second compound represented by Formula ET-1, and a third compound represented by Formula D-1.

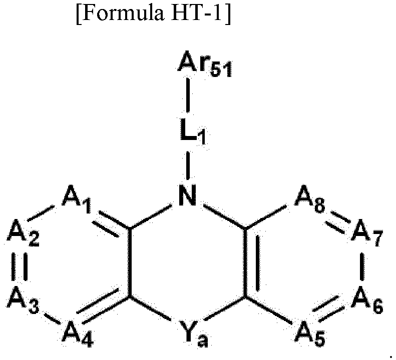

In Formula HT-1, A₁ to As are each independently N or C(R₅₁); L₁ is a direct linkage, a substituted or unsubstituted arylene group having 6 to 30 ring-forming carbon atoms, or a substituted or unsubstituted heteroarylene group having 2 to 30 ring-forming carbon atoms; Yₐ is a direct linkage, C(R₅₂)(R₅₃), or Si(R₅₄)(R₅₅); Ar₅₁ is a substituted or unsubstituted aryl group having 6 to 30 ring-forming carbon atoms or a substituted or unsubstituted heteroaryl group having 2 to 30 ring-forming carbon atoms; and R₅₁ to R₅₅ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms, or bonded to an adjacent group to form a ring.

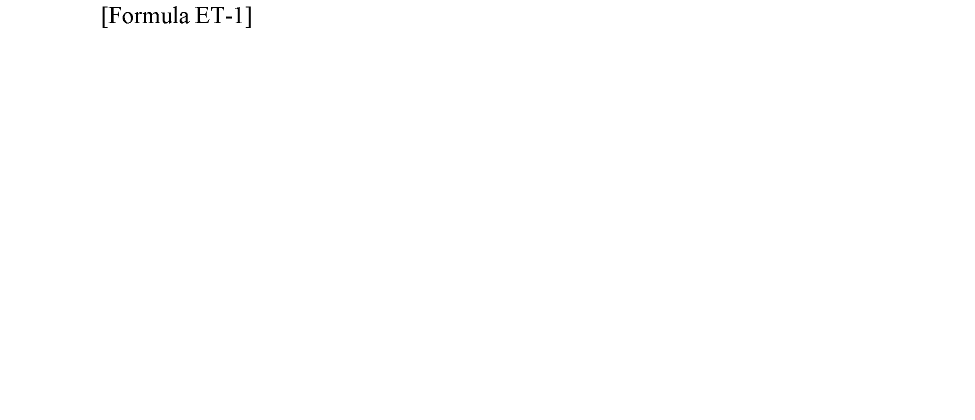

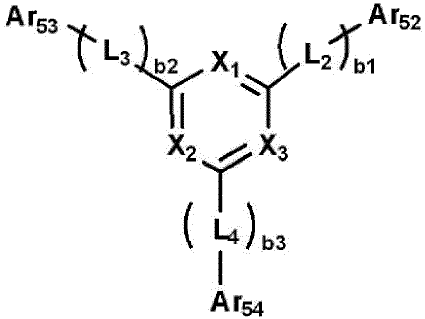

In Formula ET-1, at least one of X₁ to X₃ is N; the remainder of X₁ to X₃ are each independently C(R₅₆); R₅₆ is a hydrogen atom, a deuterium atom, a substituted or unsubstituted alkyl group having 1 to 20 carbon atoms, a substituted or unsubstituted aryl group having 6 to 60 ring-forming carbon atoms, or a substituted or unsubstituted heteroaryl group having 2 to 60 ring-forming carbon atoms; b1 to b3 are each independently an integer from 0 to 10; Ar₅₂ to Ar₅₄ are each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms; and L₂ to L₄ are each independently a direct linkage, a substituted or unsubstituted arylene group having 6 to 30 ring-forming carbon atoms, or a substituted or unsubstituted heteroarylene group having 2 to 30 ring-forming carbon atoms.

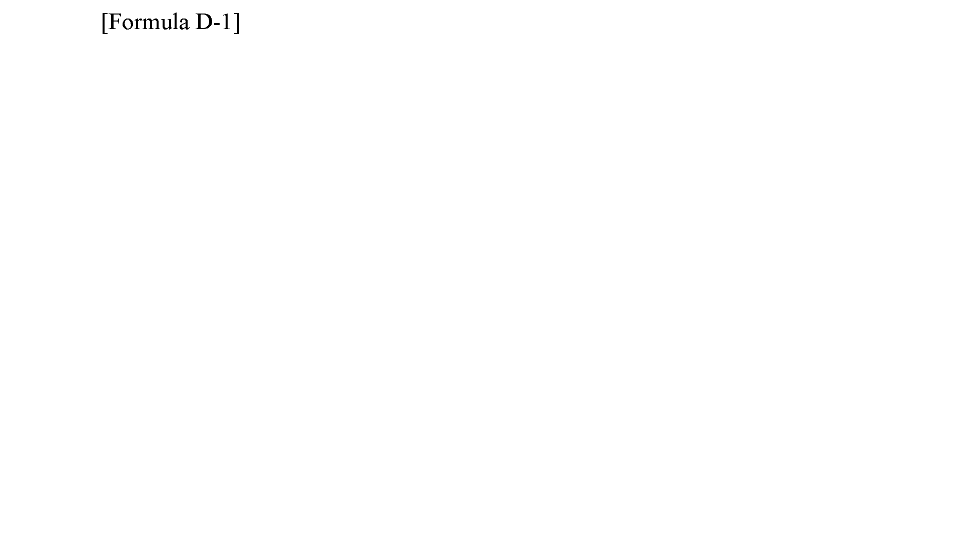

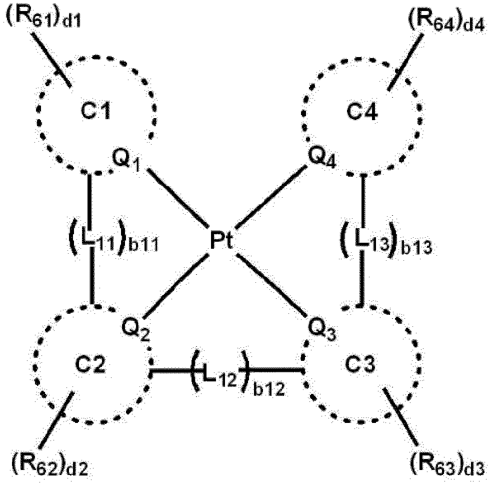

In Formula D-1, Q₁ to Q₄ are each independently C or N; C1 to C4 are each independently a substituted or unsubstituted hydrocarbon ring having 5 to 30 ring-forming carbon atoms, a substituted or unsubstituted aryl group having 6 to 30 ring-forming carbon atoms, or a substituted or unsubstituted heterocycle having 2 to 30 ring-forming carbon atoms; L₁₁ to L₁₃ are each independently a direct linkage, 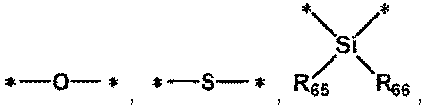 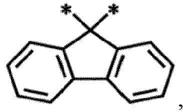 a substituted or unsubstituted alkylene group having 1 to 20 carbon atoms, a substituted or unsubstituted arylene group having 6 to 30 ring-forming carbon atoms, or a substituted or unsubstituted heteroarylene group having 2 to 30 ring-forming carbon atoms; b11 to b13 are each independently 0 or 1; R₆₁ to R₆₆ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 ring-forming carbon atoms, a substituted or unsubstituted aryl group having 6 to 60 ring-forming carbon atoms, or a substituted or unsubstituted heteroaryl group having 2 to 60 ring-forming carbon atoms; and d1 to d4 are each independently an integer from 0 to 4.

In an embodiment, the amine compound may include at least one compound selected from Compound Group 1, which is explained below.

According to an embodiment, an amine compound is represented by Formula 1, which is explained herein.

In an embodiment, the amine compound may be selected from Compound Group 1, which is explained below.

According to an embodiment, a display device includes a circuit layer disposed on a base layer, and a display element layer disposed on the circuit layer and including a light emitting element, wherein
the light emitting element includes a first electrode, a hole transport region disposed on the first electrode, an emission layer disposed on the hole transport region, an electron transport region disposed on the emission layer, and a second electrode disposed on the electron transport region, and
the hole transport region includes an amine compound represented by Formula 1, which is explained herein.

In an embodiment, the display device may further include at least one of a light control layer and a color filter layer, wherein the light control layer may include a quantum dot, and the color filter layer may include a pigment or a dye.

In an embodiment, the display device may be a television set, a monitor, a billboard, a personal computer, a laptop computer, a personal digital terminal, a display device for a vehicle, a game console, a portable electronic device, or a camera.

At least some of the above and other features of the invention are set out in the claims.

It is to be understood that the embodiments above are described in a generic and explanatory sense only and not for the purposes of limitation, and the disclosure is not limited to the embodiments described above.

### BRIEF DESCRIPTION OF THE DRAWINGS

The accompanying drawings are included to provide a further understanding of the embodiments, and are incorporated in and constitute a part of this specification. The drawings illustrate embodiments of the disclosure and principles thereof. The above and other aspects and features of the disclosure will become more apparent by describing in detail embodiments thereof with reference to the accompanying drawings, in which:
FIG. 1 is a schematic plan view of a display device according to an embodiment;
FIG. 2 is a schematic cross-sectional view of a portion of a display device corresponding to virtual line I-I' of FIG. 1;
FIG. 3 is a schematic cross-sectional view of a light emitting element according to an embodiment;
FIG. 4 is a schematic cross-sectional view of a light emitting element according to an embodiment;
FIG. 5 is a schematic cross-sectional view of a light emitting element according to an embodiment;
FIG. 6 is a schematic cross-sectional view of a light emitting element according to an embodiment;
FIG. 7 is a schematic cross-sectional view of a light emitting element according to an embodiment;
FIG. 8 is a schematic cross-sectional view of a display device according to an embodiment;
FIG. 9 is a schematic cross-sectional view of a display device according to an embodiment;
FIG. 10 is a schematic cross-sectional view of a display device according to an embodiment;
FIG. 11 is a schematic cross-sectional view of a display device according to an embodiment; and
FIG. 12 is a schematic diagram of an interior of a vehicle in which a display device according to an embodiment is disposed.

### DETAILED DESCRIPTION OF THE EMBODIMENTS

The disclosure will now be described more fully hereinafter with reference to the accompanying drawings, in which embodiments are shown. This disclosure may, however, be embodied in different forms and should not be construed as limited to the embodiments set forth herein. Rather, these embodiments are provided so that this disclosure will be thorough and complete, and will fully convey the scope of the disclosure to those skilled in the art.

In the drawings, the sizes, thicknesses, ratios, and dimensions of the elements may be exaggerated for ease of description and for clarity. Like reference numbers and reference characters refer to like elements throughout.

In the specification, it will be understood that when an element (or region, layer, part, etc.) is referred to as being "on", "connected to", or "coupled to" another element, it can be directly on, connected to, or coupled to the other element, or one or more intervening elements may be present therebetween. In a similar sense, when an element (or region, layer, part, etc.) is described as "covering" another element, it can directly cover the other element, or one or more intervening elements may be present therebetween.

In the specification, when an element is "directly on," "directly connected to," or "directly coupled to" another element, there are no intervening elements present. For example, "directly on" may mean that two layers or two elements are disposed without an additional element such as an adhesion element therebetween.

As used herein, the expressions used in the singular such as "a," "an," and "the," are intended to include the plural forms as well, unless the context clearly indicates otherwise.

As used herein, the term "and/or" includes any and all combinations of one or more of the associated listed items. For example, "A and/or B" may be understood to mean "A, B, or A and B." The terms "and" and "or" may be used in the conjunctive or disjunctive sense and may be understood to be equivalent to "and/or".

In the specification and the claims, the term "at least one of" is intended to include the meaning of "at least one selected from the group consisting of" for the purpose of its meaning and interpretation. For example, "at least one of A, B, and C" may be understood to mean A only, B only, C only, or any combination of two or more of A, B, and C, such as ABC, ACC, BC, or CC. When preceding a list of elements, the term, "at least one of," modifies the entire list of elements and does not modify the individual elements of the list.

It will be understood that, although the terms first, second, etc. may be used herein to describe various elements, these elements should not be limited by these terms. These terms are only used to distinguish one element from another element. Thus, a first element could be termed a second element without departing from the teachings of the disclosure. Similarly, a second element could be termed a first element, without departing from the scope of the disclosure.

The spatially relative terms "below", "beneath", "lower", "above", "upper", or the like, may be used herein for ease of description to describe the relations between one element or component and another element or component as illustrated in the drawings. It will be understood that the spatially relative terms are intended to encompass different orientations of the device in use or operation, in addition to the orientation depicted in the drawings. For example, in the case where a device illustrated in the drawing is turned over, the device positioned "below" or "beneath" another device may be placed "above" another device. Accordingly, the illustrative term "below" may include both the lower and upper positions. The device may also be oriented in other directions and thus the spatially relative terms may be interpreted differently depending on the orientations.

The terms "about" or "approximately" as used herein is inclusive of the stated value and means within an acceptable range of deviation for the recited value as determined by one of ordinary skill in the art, considering the measurement in question and the error associated with measurement of the recited quantity (i.e., the limitations of the measurement system). For example, "about" may mean within one or more standard deviations, or within ±20%, ±10%, or ±5% of the stated value.

It should be understood that the terms "comprises," "comprising," "includes," "including," "have," "having," "contains," "containing," and the like are intended to specify the presence of stated features, integers, steps, operations, elements, components, or combinations thereof in the disclosure, but do not preclude the presence or addition of one or more other features, integers, steps, operations, elements, components, or combinations thereof.

Unless otherwise defined or implied herein, all terms (including technical and scientific terms) used have the same meaning as commonly understood by those skilled in the art to which this disclosure pertains. It will be further understood that terms, such as those defined in commonly used dictionaries, should be interpreted as having a meaning that is consistent with their meaning in the context of the relevant art and should not be interpreted in an ideal or excessively formal sense unless clearly defined in the specification.

In the specification, the term "substituted or unsubstituted" may describe a group that is unsubstituted or substituted with at least one substituent selected from the group consisting of a deuterium atom, a halogen atom, a cyano group, a nitro group, an amine group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. Each of the substituents listed above may itself be substituted or unsubstituted. For example, a biphenyl group may be interpreted as an aryl group, or it may be interpreted as a phenyl group substituted with a phenyl group.

In the specification, the term "bonded to an adjacent group to form a ring" may refer to a group that is bonded to an adjacent group to form a substituted or unsubstituted hydrocarbon ring or a substituted or unsubstituted heterocycle. A hydrocarbon ring may be aliphatic or aromatic. A heterocycle may be aliphatic or aromatic. The hydrocarbon ring and the heterocycle may each independently be monocyclic or polycyclic. A ring that is formed by adjacent groups being bonded to each other may itself be connected to another ring to form a spiro structure.

In the specification, the term "adjacent group" may be interpreted as a substituent that is substituted for an atom which is directly linked to an atom substituted with a corresponding substituent, as another substituent that is substituted for an atom which is substituted with a corresponding substituent, or as a substituent that is sterically positioned at the nearest position to a corresponding substituent. For example, two methyl groups in 1,2-dimethylbenzene may be interpreted as "adjacent groups" to each other, and two ethyl groups in 1,1-diethylcyclopentane may be interpreted as "adjacent groups" to each other. For example, two methyl groups in 4,5-dimethylphenanthrene may be interpreted as "adjacent groups" to each other.

In the specification, examples of a halogen atom may include a fluorine atom, a chlorine atom, a bromine atom, and an iodine atom.

In the specification, an alkyl group may be linear or branched. The number of carbon atoms in an alkyl group may be 1 to 50, 1 to 30, 1 to 20, 1 to 10, or 1 to 6. Examples of an alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-heneicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc., but embodiments are not limited thereto.

In the specification, a cycloalkyl group may be a cyclic alkyl group. The number of ring-forming carbon atoms in a cycloalkyl group may be 3 to 50, 3 to 30, 3 to 20, or 3 to 10. Examples of a cycloalkyl group may include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, etc., but embodiments are not limited thereto.

In the specification, an alkenyl group may be a hydrocarbon group that includes at least one carbon-carbon double bond in the middle or at a terminus of an alkyl group having 2 or more carbon atoms. An alkenyl group may be linear or branched. The number of carbon atoms in an alkenyl group is not particularly limited, and may be 2 to 30, 2 to 20, or 2 to 10. Examples of an alkenyl group may include a vinyl group, a 1-butenyl group, a 1-pentenyl group, a 1,3-butadienyl group, a styrenyl group, a styryl vinyl group, *etc.,* but embodiments are not limited thereto.

In the specification, an alkynyl group may be a hydrocarbon group that includes at least one carbon-carbon triple bond in the middle or at a terminus of an alkyl group having 2 or more carbon atoms. An alkynyl group may be linear or branched. The number of carbon atoms in an alkynyl group is not particularly limited, and may be 2 to 30, 2 to 20, or 2 to 10. Examples of an alkynyl group may include an ethynyl group, a propynyl group, etc., but embodiments are not limited thereto.

In the specification, a hydrocarbon ring group may be any functional group or substituent derived from an aliphatic hydrocarbon ring. For example, a hydrocarbon ring group may be a saturated hydrocarbon ring group having 5 to 20 ring-forming carbon atoms.

In the specification, an aryl group may be any functional group or substituent derived from an aromatic hydrocarbon ring. An aryl group may be monocyclic or polycyclic. The number of ring-forming carbon atoms in an aryl group may be 6 to 60, 6 to 30, 6 to 20, or 6 to 15. Examples of an aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, *etc.,* but embodiments are not limited thereto.

In the specification, a fluorenyl group may be substituted, and two substituents may be bonded to each other to form a spiro structure. Examples of a substituted fluorenyl group may include the groups shown below. However, embodiments are not limited thereto.

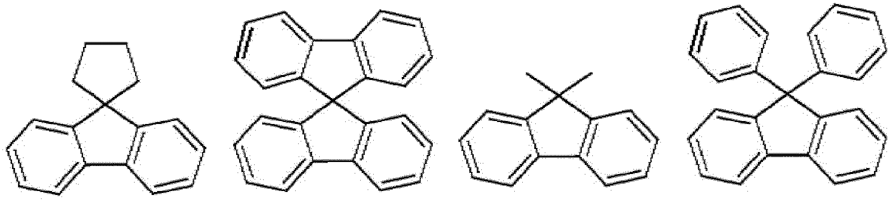

In the specification, a heterocyclic group may be any functional group or substituent derived from a ring that includes at least one of B, O, N, P, Si, and S as a heteroatom. A heterocyclic group may be aliphatic or aromatic. An aromatic heterocyclic group may be a heteroaryl group. An aliphatic heterocycle and an aromatic heterocycle may each independently be monocyclic or polycyclic.

If a heterocyclic group includes two or more heteroatoms, the two or more heteroatoms may be the same as or different from each other. The number of ring-forming carbon atoms in a heterocyclic group may be 2 to 60, 2 to 30, 2 to 20, or 2 to 10.

Examples of an aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, *etc.,* but embodiments are not limited thereto.

Examples of a heteroaryl group may include a thiophene group, a furan group, a pyrrole group, an imidazole group, a pyridine group, a bipyridine group, a pyrimidine group, a triazine group, a triazole group, an acridine group, a pyridazine group, a pyrazine group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, *etc.,* but embodiments are not limited thereto.

In the specification, the above description of an aryl group may be applied to an arylene group, except that an arylene group is a divalent group. In the specification, the above description of a heteroaryl group may be applied to a heteroarylene group, except that a heteroarylene group is a divalent group.

In the specification, a silyl group may be an alkylsilyl group or an arylsilyl group. Examples of a silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, *etc.,* but embodiments are not limited thereto.

In the specification, the number of carbon atoms in a carbonyl group is not particularly limited, and may be 1 to 40, 1 to 30, or 1 to 20. For example, a carbonyl group may have one of the following structures, but embodiments are not limited thereto.

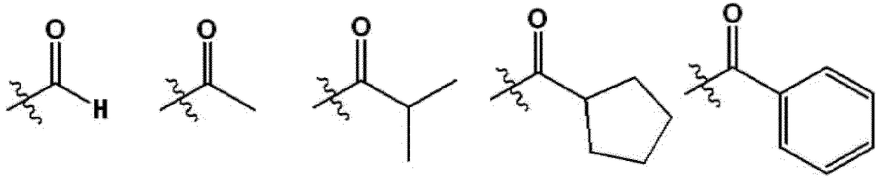

In the specification, the number of carbon atoms in a sulfinyl group or a sulfonyl group is not particularly limited, and may be 1 to 30. A sulfinyl group may be an alkyl sulfinyl group or an aryl sulfinyl group. A sulfonyl group may be an alkyl sulfonyl group or an aryl sulfonyl group.

In the specification, a thio group may be an alkylthio group or an arylthio group. A thio group may be a sulfur atom that is bonded to an alkyl group or an aryl group as defined above. Examples of a thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, but embodiments are not limited thereto.

In the specification, an oxy group may be an oxygen atom that is bonded to an alkyl group or an aryl group as defined above. An oxy group may be an alkoxy group or an aryl oxy group. An alkoxy group may be linear, branched, or cyclic. The number of carbon atoms in an alkoxy group is not particularly limited, and may be, for example, 1 to 20 or 1 to 10. Examples of an oxy group may include a methoxy group, an ethoxy group, an n-propoxy group, an isopropoxy group, a butoxy group, a pentyloxy group, a hexyloxy group, an octyloxy group, a nonyloxy group, a decyloxy group, a benzyloxy group, *etc.,* but embodiments are not limited thereto.

In the specification, a boron group may be a boron atom that is bonded to an alkyl group or an aryl group as defined above. A boron group may be an alkyl boron group or an aryl boron group. Examples of a boron group may include a dimethylboron group, a diethylboron group, a t-butylmethylboron group, a diphenylboron group, a phenylboron group, etc., but embodiments are not limited thereto.

In the specification, the number of carbon atoms in an amine group is not particularly limited, and may be 1 to 30. An amine group may be an alkyl amine group or an aryl amine group. Examples of an amine group may include a methylamine group, a dimethylamine group, a phenylamine group, a diphenylamine group, a naphthylamine group, a 9-methyl-anthracenylamine group, *etc.,* but embodiments are not limited thereto.

In the specification, an alkyl group within an alkylthio group, an alkylsulfoxy group, an alkylaryl group, an alkylamino group, an alkyl boron group, an alkyl silyl group, or an alkyl amine group may be the same as an example of an alkyl group as described above.

In the specification, an aryl group within an aryloxy group, an arylthio group, an arylsulfoxy group, an arylamino group, an arylboron group, an arylsilyl group, or an arylamine group may be the same as an example of an aryl group as described above.

In the specification, the phosphine oxide group may be substituted with, for example, at least one of the alkyl groups or aryl groups described above.

In the specification, the phosphine sulfide group may be substituted with, for example, at least one of the alkyl groups or aryl groups described above.

In the specification, a direct linkage may be a single bond. In the specification, the symbols 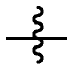 and -* each represent a bond to a neighboring atom in a corresponding formula or moiety.

Hereinafter, embodiments will be described with reference to the accompanying drawings.

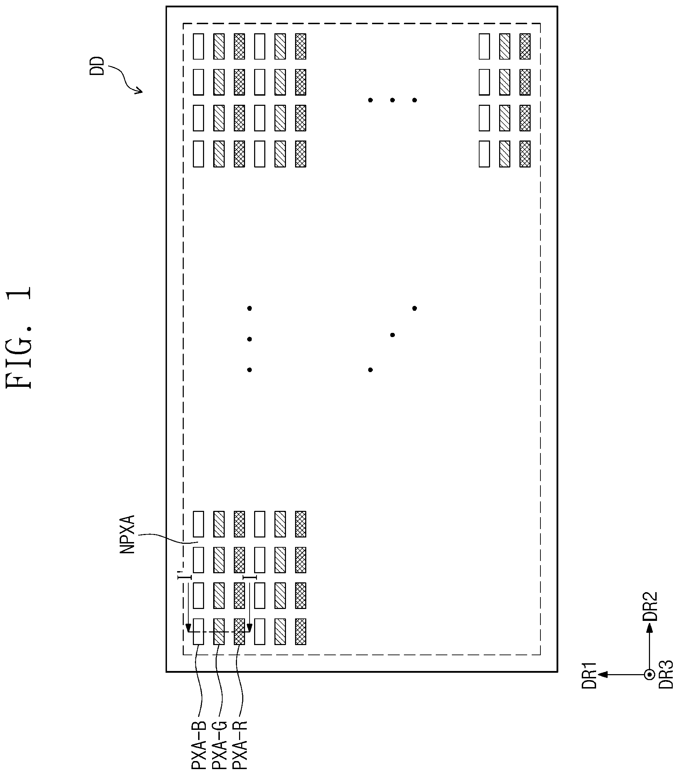

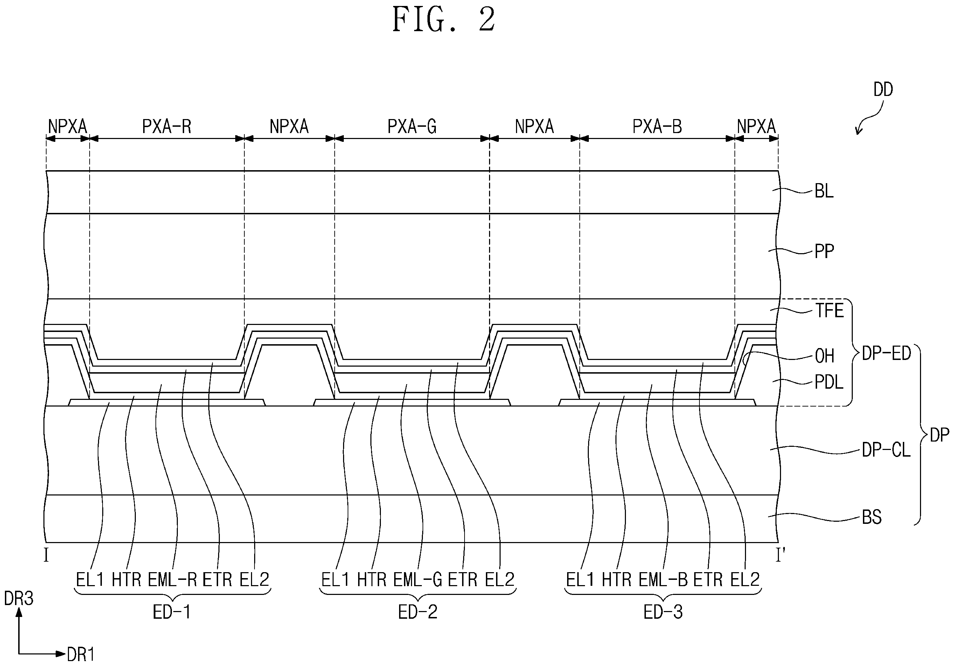

FIG. 1 is a schematic plan view of a display device DD according to an embodiment. FIG. 2 is a cross-sectional view of the display device DD of the embodiment. FIG. 2 is a schematic cross-sectional view of a portion of the display device DD taken along virtual line I-I' of FIG. 1.

The display device DD may include a display panel DP and an optical layer PP disposed on the display panel DP. The display panel DP includes light emitting elements ED-1, ED-2, and ED-3. The display device DD may include multiples of the light emitting elements ED-1, ED-2, and ED-3. The optical layer PP may be disposed on the display panel DP to control light that is reflected at the display panel DP from an external light. The optical layer PP may include, for example, a polarization layer or a color filter layer. Although not shown in the drawing, in an embodiment, the optical layer PP may be omitted from the display device DD.

A base substrate BL may be disposed on the optical layer PP. The base substrate BL may provide a base surface on which the optical layer PP is disposed. The base substrate BL may be a glass substrate, a metal substrate, a plastic substrate, *etc.* However, embodiments are not limited thereto, and the base substrate BL may include an inorganic layer, an organic layer, or a composite material layer. Although not shown in the drawings, in an embodiment, the base substrate BL may be omitted.

The display device DD according to an embodiment may further include a filling layer (not shown). The filling layer (not shown) may be disposed between a display element layer DP-ED and the base substrate BL. The filling layer (not shown) may be an organic material layer. The filling layer (not shown) may include at least one of an acrylic-based resin, a silicone-based resin, and an epoxy-based resin.

The display panel DP may include a base layer BS, a circuit layer DP-CL provided on the base layer BS, and the display element layer DP-ED. The display element layer DP-ED may include a pixel defining film PDL, light emitting elements ED-1, ED-2, and ED-3 disposed between portions of the pixel defining film PDL, and an encapsulation layer TFE disposed on the light emitting elements ED-1, ED-2, and ED-3.

The base layer BS may provide a base surface on which the display element layer DP-ED is disposed. The base layer BS may be a glass substrate, a metal substrate, a plastic substrate, *etc.* However, embodiments are not limited thereto, and the base layer BS may include an inorganic layer, an organic layer, or a composite material layer.

In an embodiment, the circuit layer DP-CL is disposed on the base layer BS, and the circuit layer DP-CL may include transistors (not shown). The transistors (not shown) may each include a control electrode, an input electrode, and an output electrode. For example, the circuit layer DP-CL may include a switching transistor and a driving transistor for driving the light emitting elements ED-1, ED-2, and ED-3 of the display element layer DP-ED.

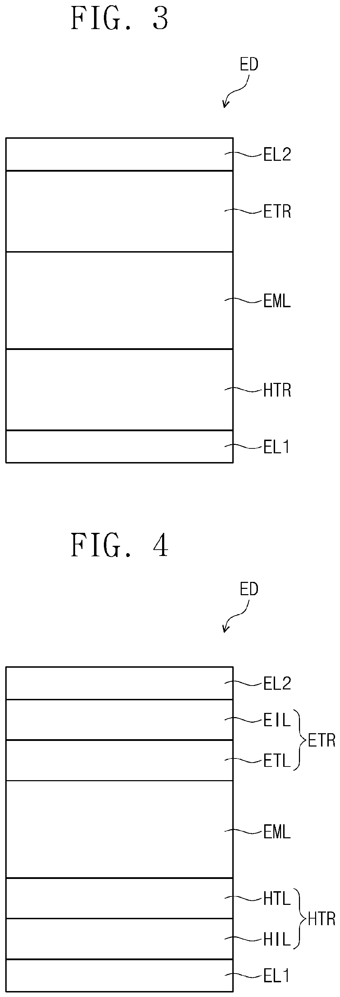

The light emitting elements ED-1, ED-2, and ED-3 may each have a structure of a light emitting element ED of an embodiment according to any of FIGS. 3 to 7, which will be described later. The light emitting elements ED-1, ED-2, and ED-3 may each include a first electrode EL1, a hole transport region HTR, emission layers EML-R, EML-G, and EML-B, an electron transport region ETR, and a second electrode EL2.

FIG. 2 illustrates an embodiment in which the emission layers EML-R, EML-G, and EML-B of the light emitting elements ED-1, ED-2, and ED-3 are disposed in openings OH defined in the pixel defining film PDL, and the hole transport region HTR, the electron transport region ETR, and the second electrode EL2 are each provided as a common layer for the light emitting elements ED-1, ED-2, and ED-3. However, embodiments are not limited thereto. Although not shown in FIG. 2, the hole transport region HTR and the electron transport region ETR may each be provided by being patterned in the openings OH defined in the pixel defining film PDL. For example, in an embodiment, the hole transport region HTR, the emission layers EML-R, EML-G, and EML-B, and the electron transport region ETR of the light emitting elements ED-1, ED-2, and ED-3 may each be provided by being patterned through an inkjet printing method.

The encapsulation layer TFE may cover the light emitting elements ED-1, ED-2, and ED-3. The encapsulation layer TFE may seal the light-emitting elements ED-1, ED-2, and ED-3 in the display element layer DP-ED. The encapsulation layer TFE may be a thin film encapsulation layer. The encapsulation layer TFE may be formed of a single layer or of multiple layers. The encapsulation layer TFE may include at least one insulation layer. The encapsulation layer TFE according to an embodiment may include at least one inorganic film (hereinafter, an encapsulation-inorganic film). In an embodiment, the encapsulation layer TFE may include at least one organic film (hereinafter, an encapsulation-organic film) and at least one encapsulation-inorganic film.

The encapsulation-inorganic film protects the display element layer DP-ED from moisture and/or oxygen, and the encapsulation-organic film protects the display element layer DP-ED from foreign substances such as dust particles. The encapsulation-inorganic film may include silicon nitride, silicon oxynitride, silicon oxide, titanium oxide, aluminium oxide, or the like, but embodiments are not limited thereto. The encapsulation-organic film may include an acrylic-based compound, an epoxy-based compound, or the like. The encapsulation-organic film may include a photopolymerizable organic material, but embodiments are not limited thereto.

The encapsulation layer TFE may be disposed on the second electrode EL2 and may be disposed to fill the openings OH.

Referring to FIGS. 1 and 2, the display device DD may include non-light emitting regions NPXA and light emitting regions PXA-R, PXA-G, and PXA-B. The light emitting regions PXA-R, PXA-G, and PXA-B may each be a region that emits light respectively generated by the light emitting elements ED-1, ED-2, and ED-3. The light emitting regions PXA-R, PXA-G, and PXA-B may be spaced apart from each other in a plan view.

The light emitting regions PXA-R, PXA-G, and PXA-B may be regions that are separated from each other by the pixel defining film PDL. The non-light emitting regions NPXA may be areas between the adjacent light emitting regions PXA-R, PXA-G, and PXA-B, and which may correspond to the pixel defining film PDL. In an embodiment, the light emitting regions PXA-R, PXA-G, and PXA-B may each correspond to a pixel. The pixel defining film PDL may separate the light emitting elements ED-1, ED-2, and ED-3. The emission layers EML-R, EML-G, and EML-B of the light emitting elements ED-1, ED-2, and ED-3 may be disposed in openings OH defined in the pixel defining film PDL and separated from each other.

The light emitting regions PXA-R, PXA-G, and PXA-B may be arranged into groups according to the color of light generated from the light emitting elements ED-1, ED-2, and ED-3. In the display device DD according to an embodiment illustrated in FIGS. 1 and 2, three light emitting regions PXA-R, PXA-G, and PXA-B, which respectively emit red light, green light, and blue light, are illustrated as an example. For example, the display device DD may include a red light emitting region PXA-R, a green light emitting region PXA-G, and a blue light emitting region PXA-B, which are distinct from each other.

In the display device DD according to an embodiment, the light emitting elements ED-1, ED-2, and ED-3 may emit light having wavelengths that are different from each other. For example, in an embodiment, the display device DD may include a first light emitting element ED-1 that emits red light, a second light emitting element ED-2 that emits green light, and a third light emitting element ED-3 that emits blue light. For example, the red light emitting region PXA-R, the green light emitting region PXA-G, and the blue light emitting region PXA-B of the display device DD may respectively correspond to the first light emitting element ED-1, the second light emitting element ED-2, and the third light emitting element ED-3.

However, embodiments are not limited thereto, and the first to third light emitting elements ED-1, ED-2, and ED-3 may emit light in a same wavelength range, or at least one light emitting element may emit light in a wavelength range that is different from the remainder. For example, the first to third light emitting elements ED-1, ED-2, and ED-3 may each emit blue light.

The light emitting regions PXA-R, PXA-G, and PXA-B in the display device DD according to an embodiment may be arranged in a stripe configuration. Referring to FIG. 1, the red light emitting regions PXA-R, the green light emitting regions PXA-G, and the blue light emitting regions PXA-B may be respectively arranged along a second directional axis DR2. In another embodiment, the red light emitting region PXA-R, the green light emitting region PXA-G, and the blue light emitting region PXA-B may be arranged in this repeating order along a first directional axis DR1.

FIGS. 1 and 2 illustrate that the light emitting regions PXA-R, PXA-G, and PXA-B all have a similar area, but embodiments are not limited thereto. In an embodiment, the light emitting regions PXA-R, PXA-G, and PXA-B may be different in size or shape from each other, according to a wavelength range of emitted light. The areas of the light emitting regions PXA-R, PXA-G, and PXA-B may be areas in a plan view that are defined by the first directional axis DR1 and the second directional axis DR2.

An arrangement of the light emitting regions PXA-R, PXA-G, and PXA-B is not limited to the configuration illustrated in FIG. 1, and the order in which the red light emitting region PXA-R, the green light emitting region PXA-G, and the blue light emitting region PXA-B are arranged may be provided in various combinations, according to the display quality characteristics that are required for the display device DD. For example, the light emitting regions PXA-R, PXA-G, and PXA-B may be arranged in a pentile configuration (such as PenTile^{®}) or in a diamond configuration (such as Diamond Pixel^{®}).

The areas of the light emitting regions PXA-R, PXA-G, and PXA-B may be different in size from each other. For example, in an embodiment, an area of a green light emitting region PXA-G may be smaller than an area of a blue light emitting region PXA-B, but embodiments are not limited thereto.

Hereinafter, FIGS. 3 to 7 are each a schematic cross-sectional view of a light emitting element according to an embodiment. Embodiments provide a light emitting element ED that may include a first electrode EL1, a hole transport region HTR, an emission layer EML, an electron transport region ETR, and a second electrode EL2.

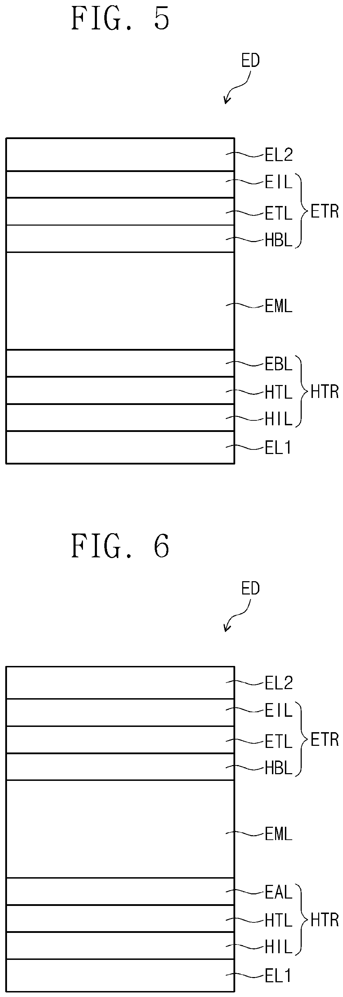

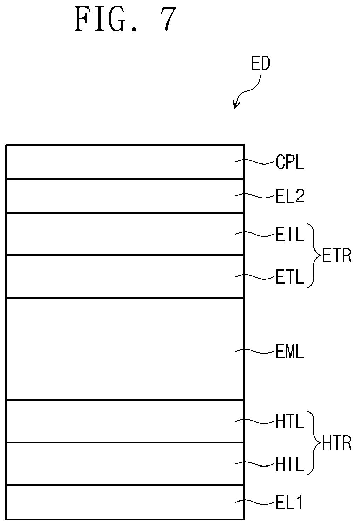

In comparison to FIG. 3, FIG. 4 is a schematic cross-sectional view of a light emitting element ED according to an embodiment, in which the hole transport region HTR includes a hole injection layer HIL and a hole transport layer HTL, and the electron transport region ETR includes an electron injection layer EIL and an electron transport layer ETL. In comparison to FIG. 3, FIG. 5 is a schematic cross-sectional view of a light emitting element ED according to an embodiment, in which the hole transport region HTR includes a hole injection layer HIL, a hole transport layer HTL, and an electron blocking layer EBL, and the electron transport region ETR includes an electron injection layer EIL, an electron transport layer ETL, and a hole blocking layer HBL. In comparison to FIG. 3, FIG. 6 is a schematic cross-sectional view of a light emitting element ED according to an embodiment, in which the hole transport region HTR includes a hole injection layer HIL, a hole transport layer HTL, and an emission auxiliary layer EAL, and the electron transport region ETR includes an electron injection layer EIL, an electron transport layer ETL, and a hole blocking layer HBL. In comparison to FIG. 4, FIG.7 is a schematic cross-sectional view of a light emitting element ED according to an embodiment that further includes a capping layer CPL disposed on the second electrode EL2.

The first electrode EL1 has conductivity. The first electrode EL1 may be formed of a metal material, a metal alloy, or a conductive compound. The first electrode EL1 may be an anode or a cathode. However, embodiments are not limited thereto. In an embodiment, the first electrode EL1 may be a pixel electrode. The first electrode EL1 may be a transmissive electrode, a transflective electrode, or a reflective electrode. The first electrode EL1 may include at least one of Ag, Mg, Cu, Al, Pt, Pd, Au, Ni, Nd, Ir, Cr, Li, Ca, LiF, Mo, Ti, W, In, Sn, and Zn, or may include an oxide thereof, a compound thereof, or a mixture thereof.

If the first electrode EL1 is a transmissive electrode, the first electrode EL1 may include a transparent metal oxide such as indium tin oxide (ITO), indium zinc oxide (IZO), zinc oxide (ZnO), or indium tin zinc oxide (ITZO). If the first electrode EL1 is a transflective electrode or a reflective electrode, the first electrode EL1 may include Ag, Mg, Cu, Al, Pt, Pd, Au, Ni, Nd, Ir, Cr, Li, Ca, LiF/Ca (a stacked structure ofLiF and Ca), LiF/Al (a stacked structure of LiF and Al), Mo, Ti, W, a compound thereof, or a mixture thereof (e.g., a mixture of Ag and Mg). In another embodiment, the first electrode EL1 may have a multilayer structure including a reflective film or a transflective film formed of the above-described materials, and a transparent conductive film formed of ITO, IZO, ZnO, ITZO, *etc.* For example, the first electrode EL1 may have a three-layered structure of ITO/Ag/ITO, but embodiments are not limited thereto. In an embodiment, the first electrode EL1 may include the above-described metal materials, combinations of at least two of the above-described metal materials, oxides of the above-described metal materials, or the like. A thickness of the first electrode EL1 may be in a range of about 700 Å to about 10,000 Å. For example, the thickness of the first electrode EL1 may be in a range of about 1,000 Å to about 3,000 Å.

The light emitting element ED according to an embodiment includes an amine compound according to an embodiment. In an embodiment, the hole transport region HTR includes an amine compound according to an embodiment. At least one of a hole injection layer HIL, a hole transport layer HTL, an emission auxiliary layer EAL, and an electron blocking layer EBL may include the amine compound. For example, the hole transport layer HTL may include the amine compound.

The amine compound according to an embodiment includes three amine groups, two phenyl groups that are each bonded between two amine groups, and a carbazole group bonded to at least one of the amine groups. The amine compound according to an embodiment including three amine groups and at least one carbazole group may exhibit excellent material stability. Accordingly, the light emitting element ED including the amine compound according to an embodiment may exhibit characteristics of low driving voltage, high luminous efficiency, and a long life.

In the following descriptions of the amine compound according to an embodiment, substituents (e.g., aryl groups) that are bonded to the aforementioned amine groups may be bonded together to form a ring (e.g., a carbazole moiety), and such moieties are not considered as being included among the aforementioned the amine groups, but are instead considered as heterocyclic groups. Thus, cyclic groups that include a nitrogen atom as a ring-forming atom are not considered as being included among the aforementioned amine groups, and are instead considered as being included among heterocyclic groups.

The light emitting element ED according to an embodiment includes an amine compound according to an embodiment. The amine compound according to an embodiment is represented by Formula 1:

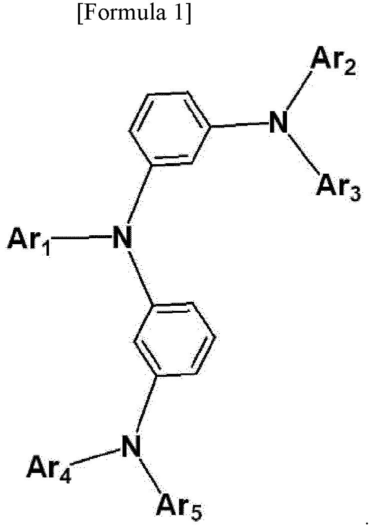

In Formula 1, at least one of Ar₁ to Ar₅ is each independently a group represented by Formula 2, and the remainder of Ar₁ to Ar₅ that are not a group represented by Formula 2 are each independently a substituted or unsubstituted aryl group having 6 to 30 ring-forming carbon atoms or a substituted or unsubstituted heteroaryl group having 2 to 30 ring-forming carbon atoms.

For example, the remainder of Ar₁ to Ar₅ that are not a group represented by Formula 2 may each independently be a substituted or unsubstituted aryl group having 6 to 30 ring-forming carbon atoms. In an embodiment, in Formula 1, the remainder of Ar₁ to Ar₅ that are not a group represented by Formula 2 may each independently be a substituted or unsubstituted phenyl group, an unsubstituted biphenyl group, an unsubstituted naphthyl group, or a substituted or unsubstituted terphenyl group. Among the remainder of Ar₁ to Ar₅ that are not a group represented by Formula 2, when at least one of this remainder is a substituted phenyl group, the substituent of the phenyl group may be a phenyl group, a terphenyl group, a cyclohexyl group, a bicycloheptyl group, an adamantyl group, a methyl group, an ethyl group, an isopropyl group, or a t-butyl group.

In an embodiment, in Formula 1, the remainder of Ar₁ to Ar₅ that are not a group represented by Formula 2 may each independently be a group represented by one of Formula AR1-1 to Formula AR1-20:

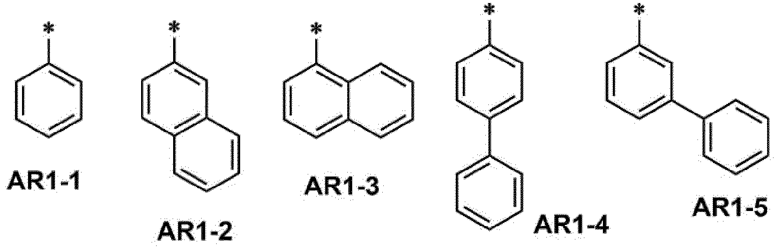

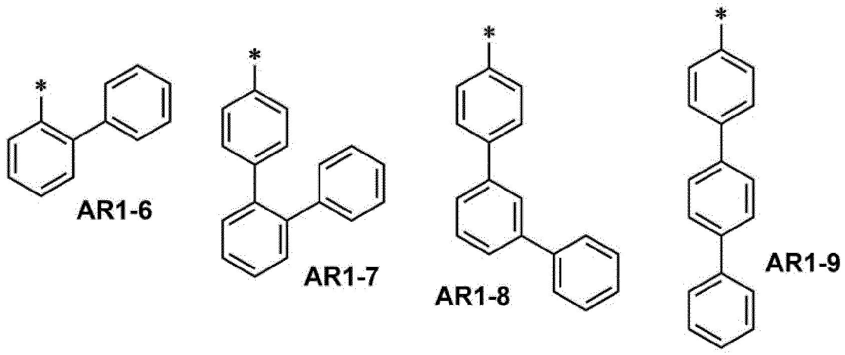

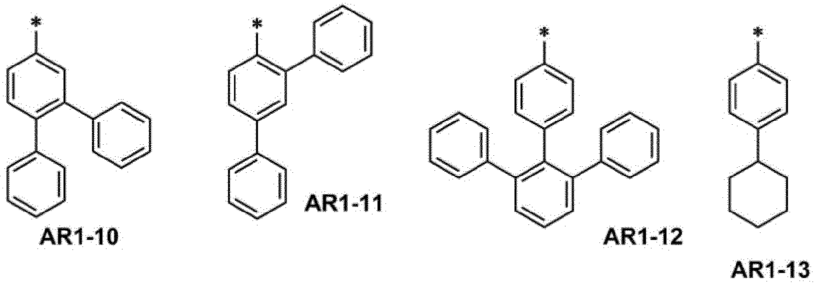

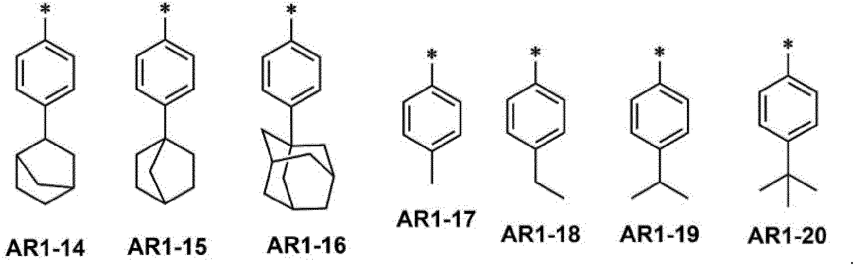

Formula 2 represents a substituted or unsubstituted carbazole group. Among the three rings that constitute a carbazole group, a ring that does not include a ring-forming nitrogen atom may contain a carbon atom that is directly or indirectly bonded to an amine group of Formula 1.

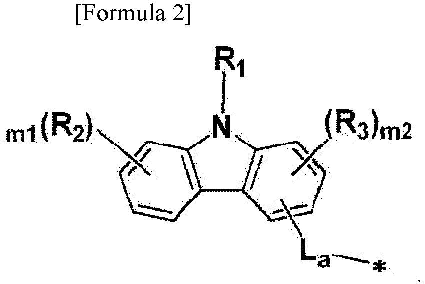

In Formula 2, Lₐ is a direct linkage, a substituted or unsubstituted arylene group having 6 to 30 ring-forming carbon atoms, or a substituted or unsubstituted heteroarylene group having 2 to 30 ring-forming carbon atoms. In an embodiment, in Formula 2, Lₐ may be a direct linkage or an unsubstituted phenylene group.

In Formula 2, m1 is an integer from 0 and 4, and m2 is an integer from 0 and 3. When m1 is 2 or greater, multiple R₂ groups may all be the same or at least one thereof may be different from the remainder. A case when m1 is 0 may be the same as a case in which m1 is 4 and four R₂ groups are all hydrogen atoms. When m2 is 2 or 3, multiple R₃ groups may all be the same or at least one thereof may be different from the remainder. A case when m2 is 0 may be the same as a case in which m2 is 3 and three R₃ groups are all hydrogen atoms.

In Formula 2, R₁ to R₃ are each independently a hydrogen atom, a deuterium atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms. For example, R₂ and R₃ may each independently be a hydrogen atom or a substituted or unsubstituted phenyl group. In an embodiment, R₁ may be an unsubstituted methyl group, an unsubstituted ethyl group, an unsubstituted isopropyl group, an unsubstituted t-butyl group, an unsubstituted phenyl group, or a group represented by one of Formula R1-1 to Formula R1-7.

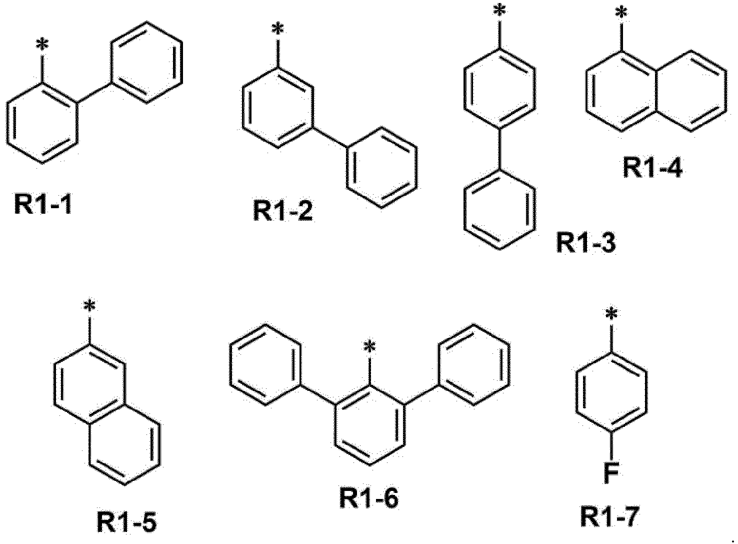

In an embodiment, the group represented by Formula 2 may be a group represented by one of Formula 2-1 to Formula 2-10. Formulas 2-1 to 2-10 each represent a case where R₂ and R₃ in Formula 2 are further defined.

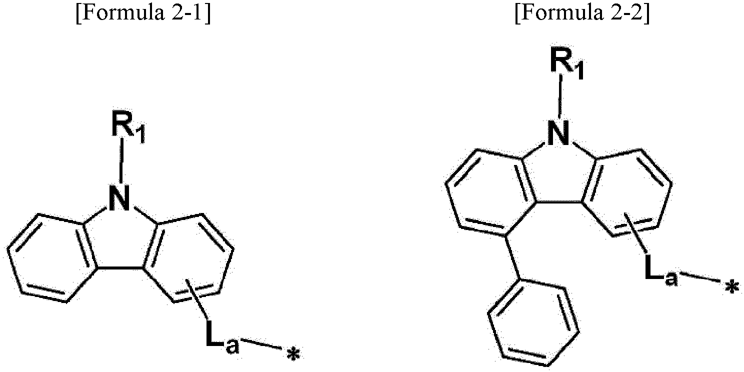

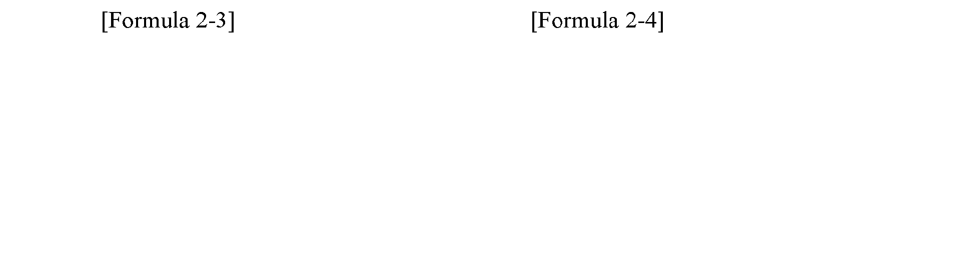

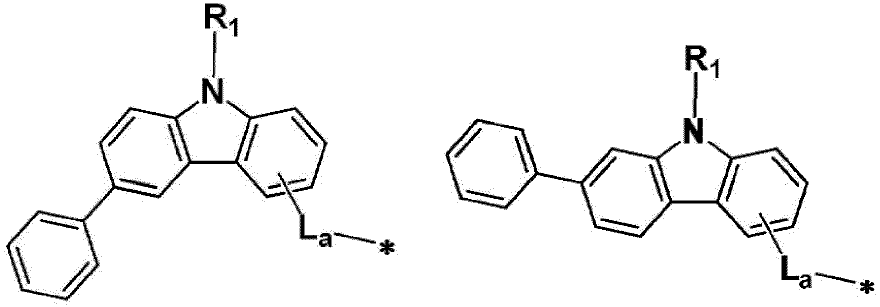

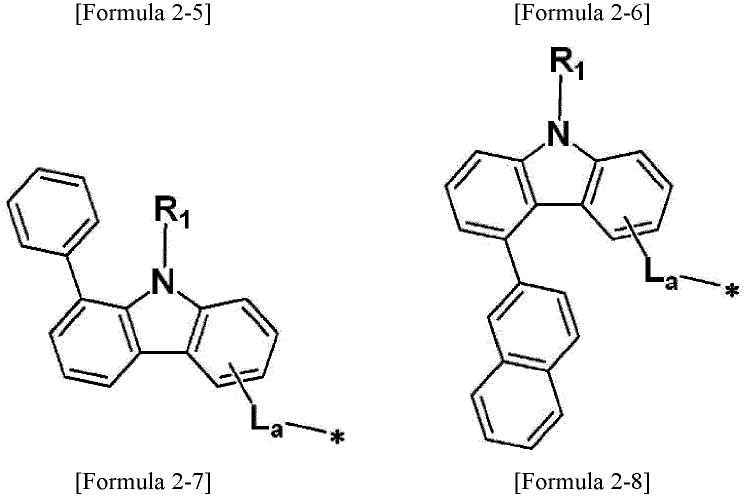

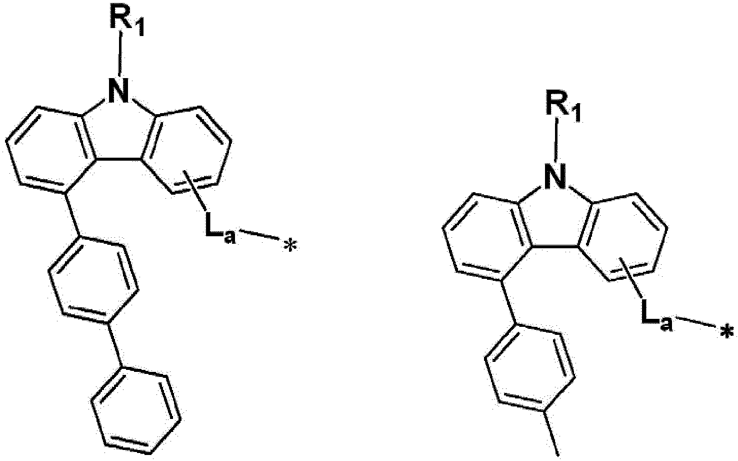

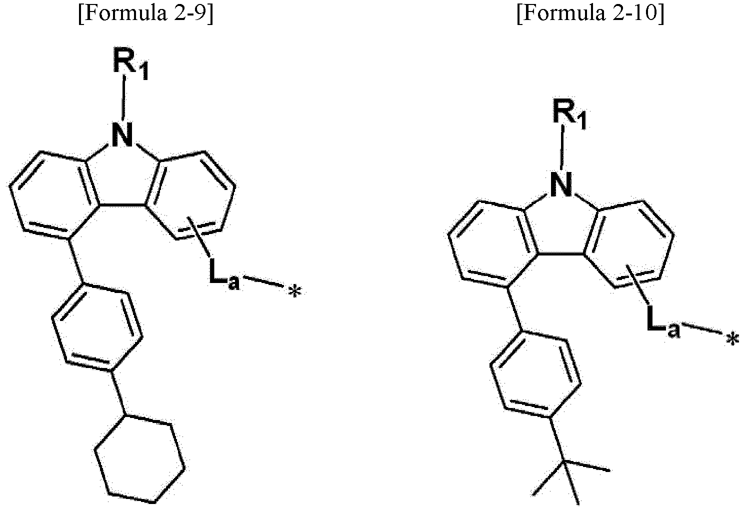

In Formulas 2-1 to 2-10, R₁ and Lₐ are the same as defined in Formula 2. Formulas 2-1 to 2-10 each represent a case in which in Formula 2, R₃ is a hydrogen atom, and R₂ is a hydrogen atom, a substituted or unsubstituted phenyl group, or an unsubstituted naphthyl group.

In an embodiment, the amine compound may be represented by one of Formula 1-A to Formula 1-C. Formula 1-A and Formula 1-B each represent a case in which one of Ar₁ to Ar₅ in Formula 1 is a group represented by Formula 2. Formula 1-C represents a case in which two of Ar₁ to Ar₅ in Formula 1 are each a group represented by Formula 2. In Formula 1-C, a carbazole group containing R₄ to R₆ may correspond to a group represented by Formula 2 as described above.

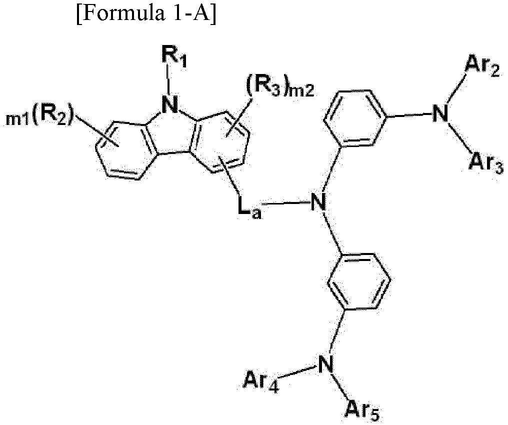

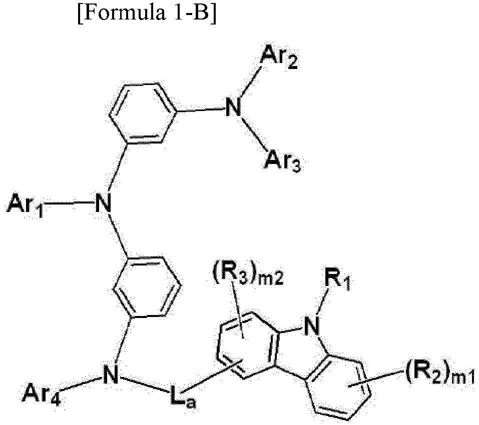

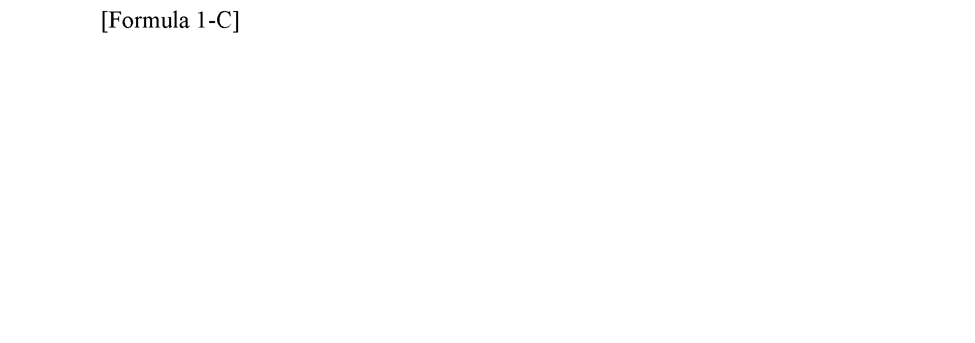

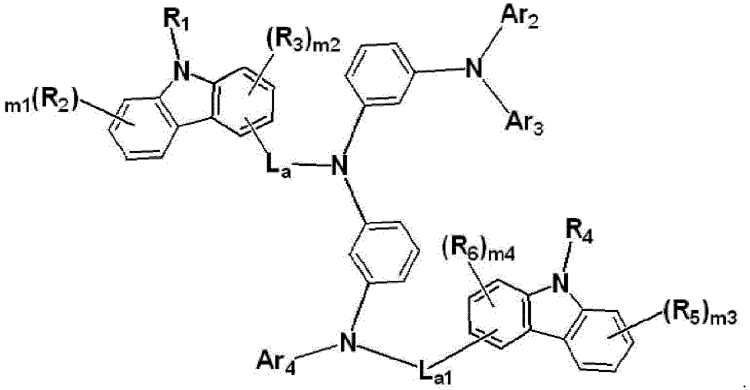

In Formulas 1-A to 1-C, m1, m2, Ar₁ to Ar₅, R₁ to R₃, and Lₐ are the same as defined in Formulas 1 and 2.

In Formula 1-C, Lₐ₁ is a direct linkage, a substituted or unsubstituted arylene group having 6 to 30 ring-forming carbon atoms, or a substituted or unsubstituted heteroarylene group having 2 to 30 ring-forming carbon atoms. For example, Lₐ₁ may be a direct linkage.

In Formula 1-C, m3 is an integer from 0 and 4, and m4 is an integer from 0 and 3. When m3 is 2 or greater, multiple R₅ groups may all be the same or at least one thereof may be different from the remainder. A case when m3 is 0 may be the same as a case where m3 is 4 and four R₅ groups are all hydrogen atoms. When m4 is 2 or 3, multiple R₆ groups may all be the same or at least one thereof may be different from the remainder. A case when m4 is 0 may be the same as a case in which m4 is 3 and three R₆ groups are all hydrogen atoms.

In Formula 1-C, R₄ to R₆ are each independently a hydrogen atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms. For example, R₄ may be an unsubstituted phenyl group. As another example, R₄ may be an unsubstituted methyl group, an unsubstituted ethyl group, an unsubstituted isopropyl group, an unsubstituted t-butyl group, or a group represented by one of Formula R1-1 to Formula R1-7 as described above. In an embodiment, R₅ and R₆ may each independently be a hydrogen atom or a substituted or unsubstituted phenyl group. For example, R₅ and R₆ may each be a hydrogen atom.

In an embodiment, the amine compound represented by Formula 1-A may be represented by one of Formula 1-A1 to Formula 1-A4. Formulas 1-A1 to 1-A4 each represent a case in which a bonding position of a carbazole group in Formula 1-A is further defined and Ar₂ to Ar₅ are further defined.

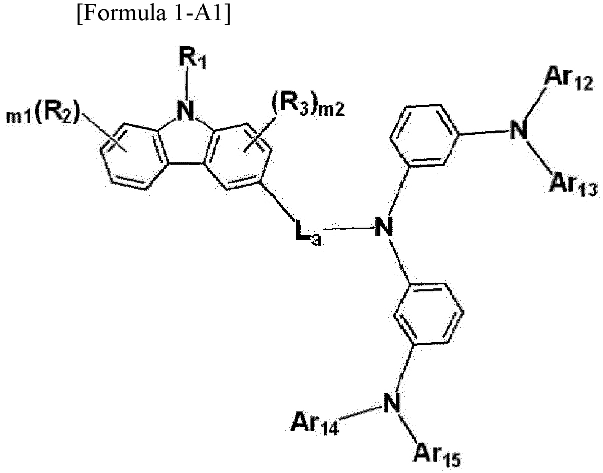

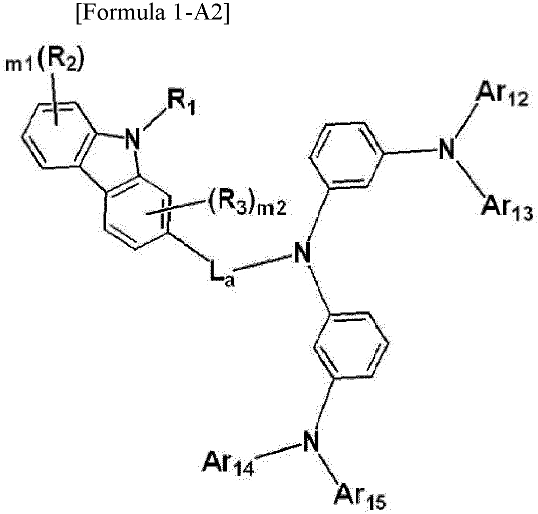

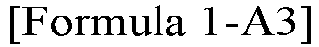

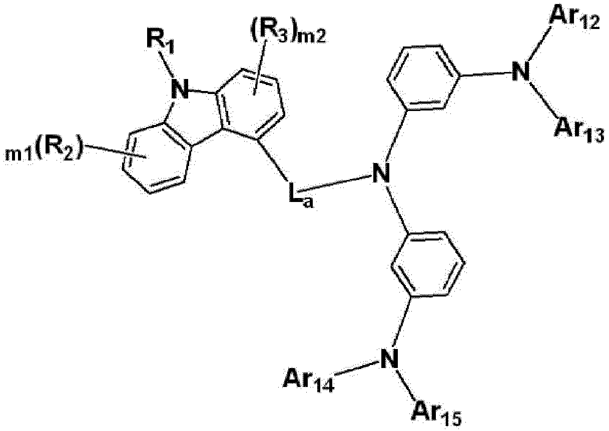

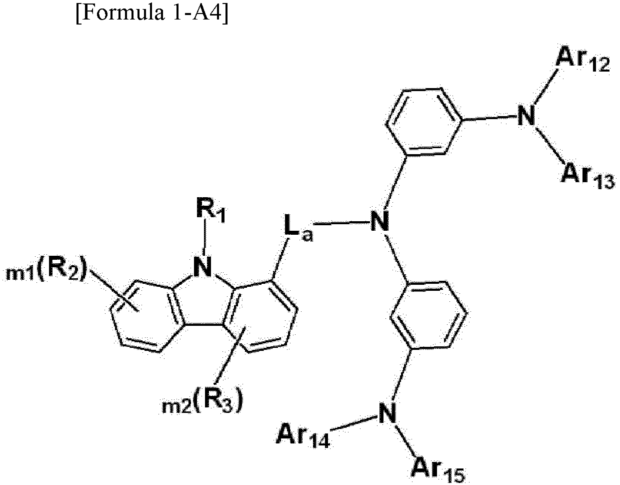

In Formulas 1-A1 to 1-A4, Ar₁₂ to Ar₁₅ are each independently a substituted or unsubstituted aryl group having 6 to 30 ring-forming carbon atoms. For example, Ar₁₂ to Ar₁₅ may each independently be a substituted or unsubstituted phenyl group, an unsubstituted biphenyl group, an unsubstituted naphthyl group, or a substituted or unsubstituted terphenyl group. When at least one of Ar₁₂ to Ar₁₅ is a substituted phenyl group, a substituent of the phenyl group may be a phenyl group, a terphenyl group, a cyclohexyl group, a bicycloheptyl group, an adamantyl group, a methyl group, an ethyl group, an isopropyl group, or a t-butyl group. For example, Ar₁₂ to Ar₁₅ may each independently be a group represented by one of Formula AR1-1 to Formula AR1-20 as described above. In Formulas 1-A1 to 1-A4, m1, m2, R₁ to R₃, and Lₐ are the same as defined in Formulas 1 and 2.

In an embodiment, the amine compound represented by Formula 1-B may be represented by one of Formula 1-B1 to Formula 1-B4. Formulas 1-B1 to 1-B4 each represent a case in which a bonding position of a carbazole group in Formula 1-B is further defined and Lₐ is a direct linkage.

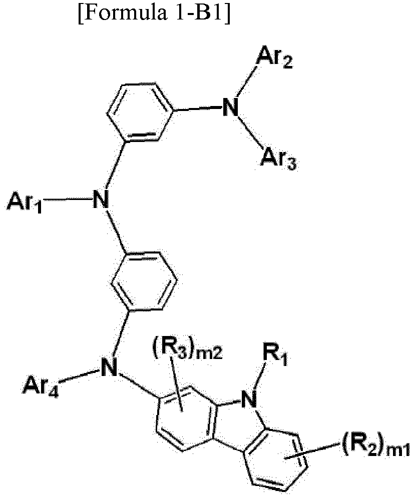

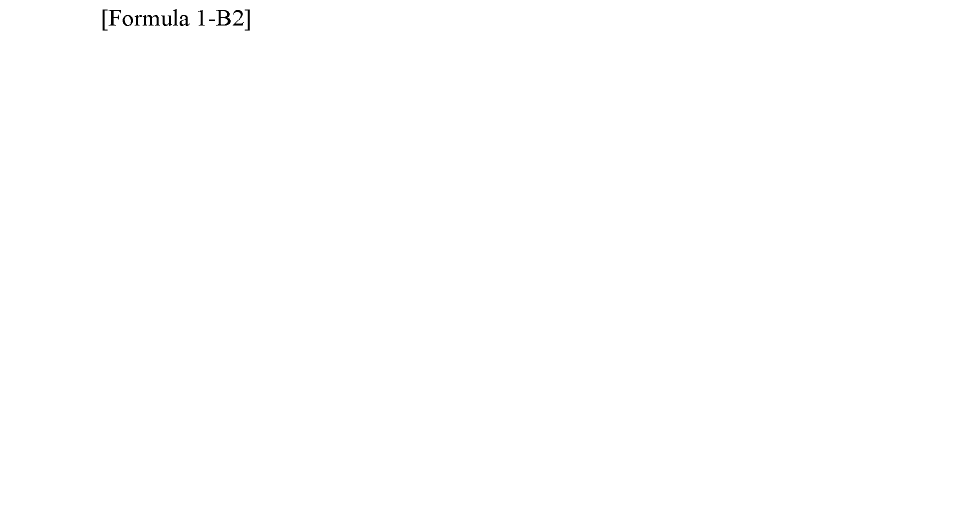

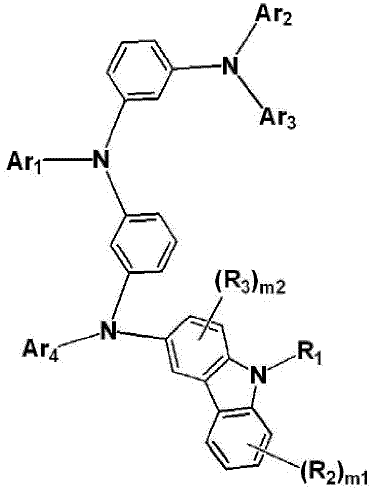

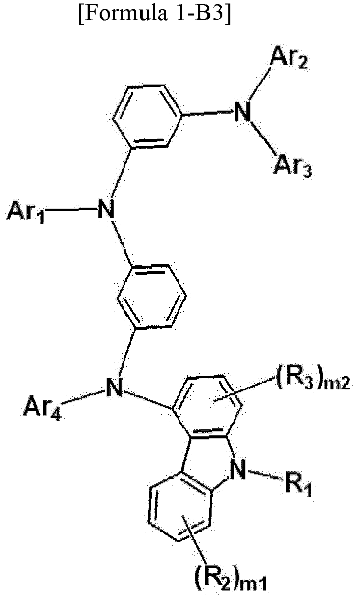

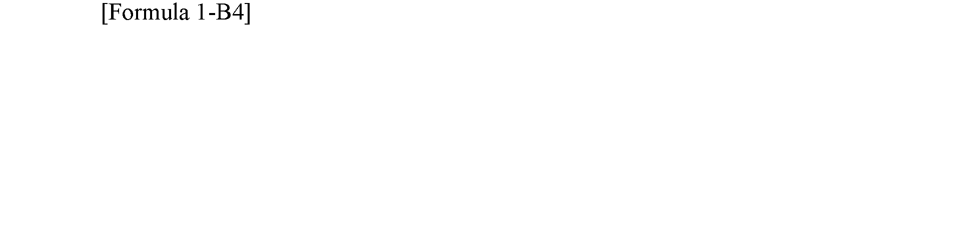

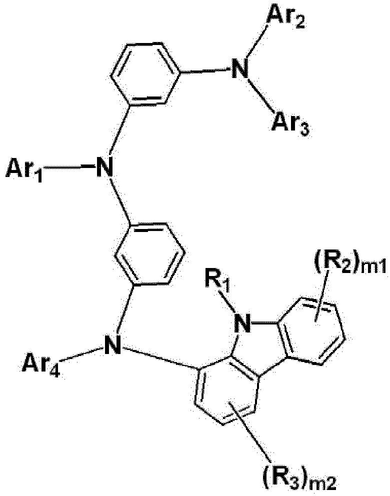

In Formulas 1-B1 to 1-B4, Ar₁ to Ar₄, m1, m2, and R₁ to R₃ are the same as defined in Formulas 1 and 2.

In an embodiment, the amine compound represented by Formula 1 may be any compound selected from Compound Group 1. In an embodiment, the light emitting element ED may include at least one compound selected from Compound Group 1:

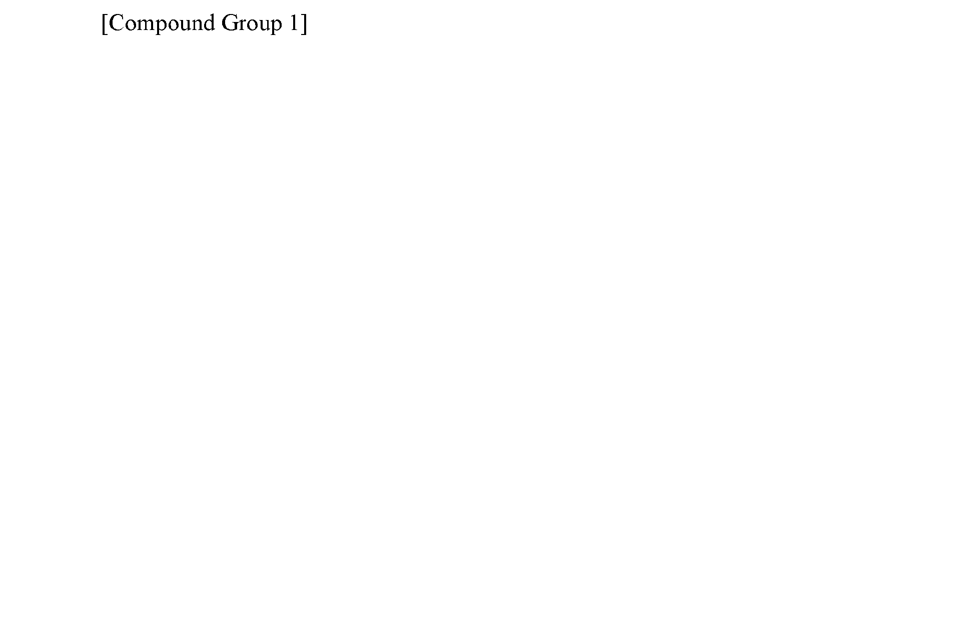

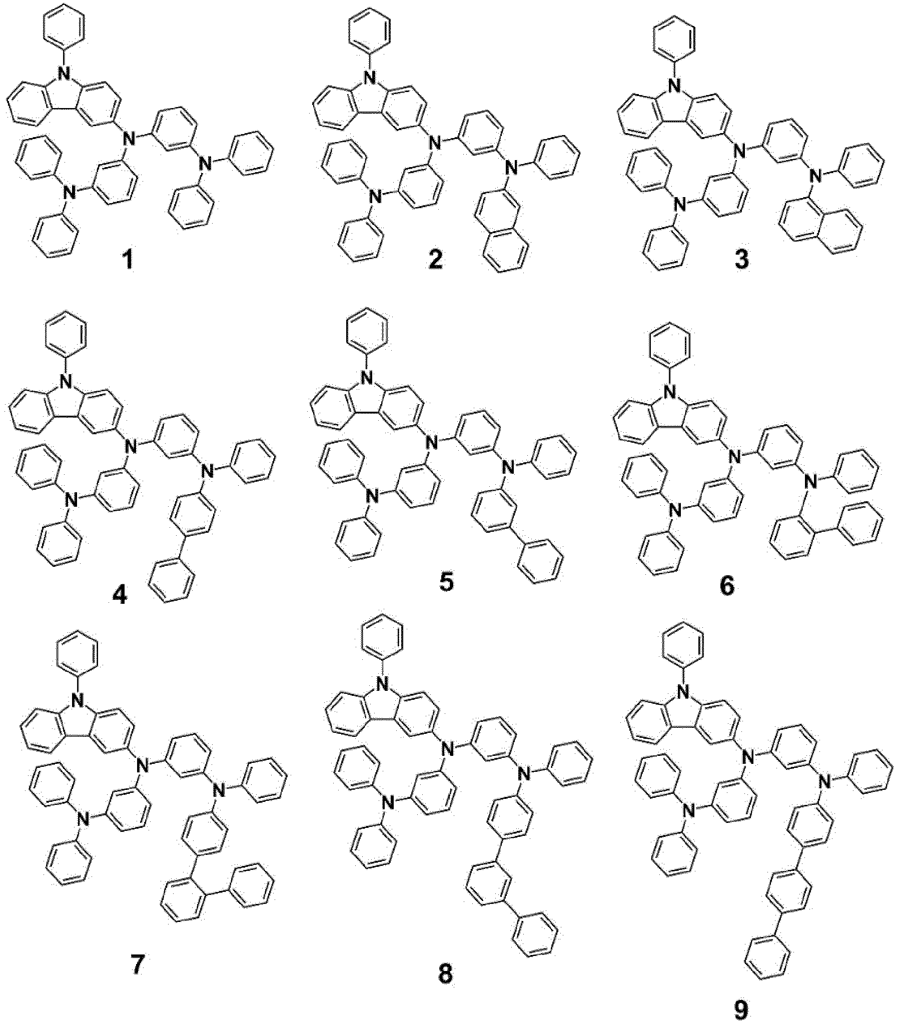

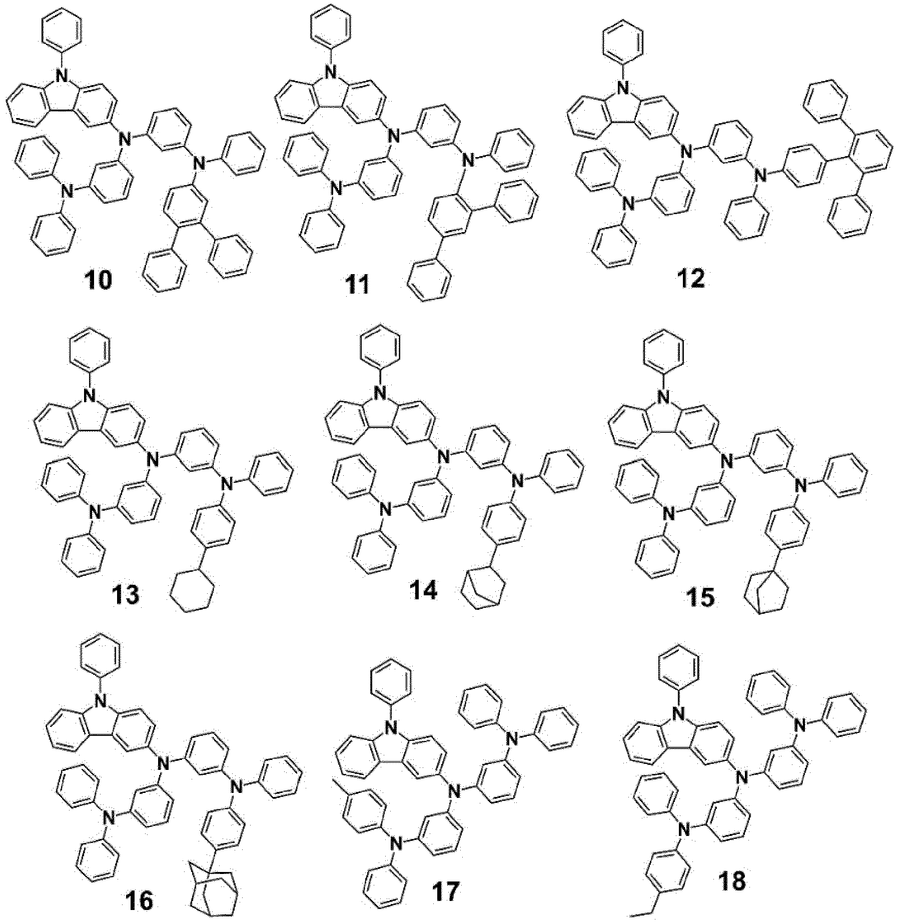

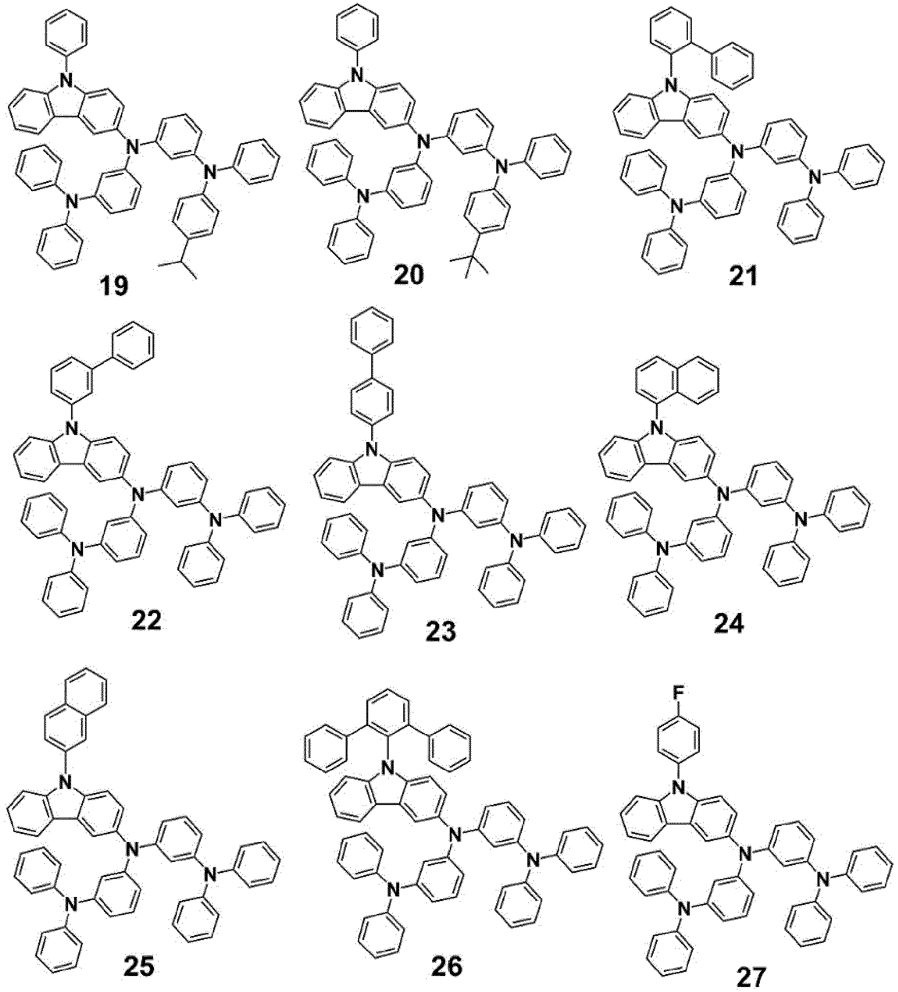

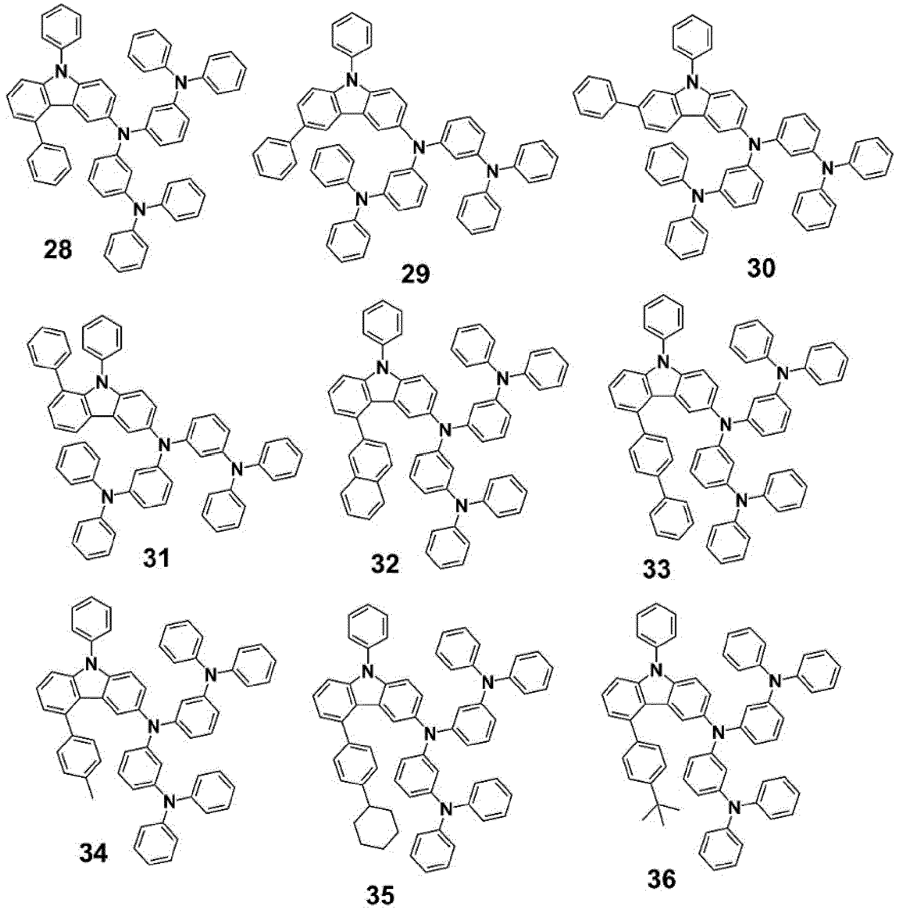

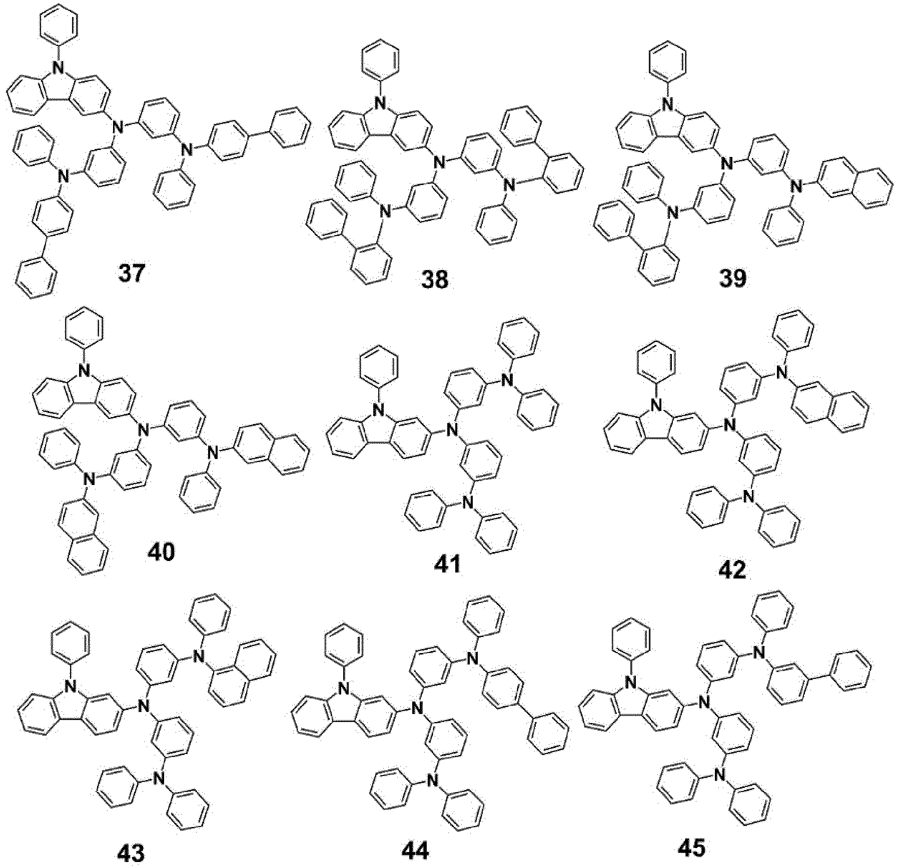

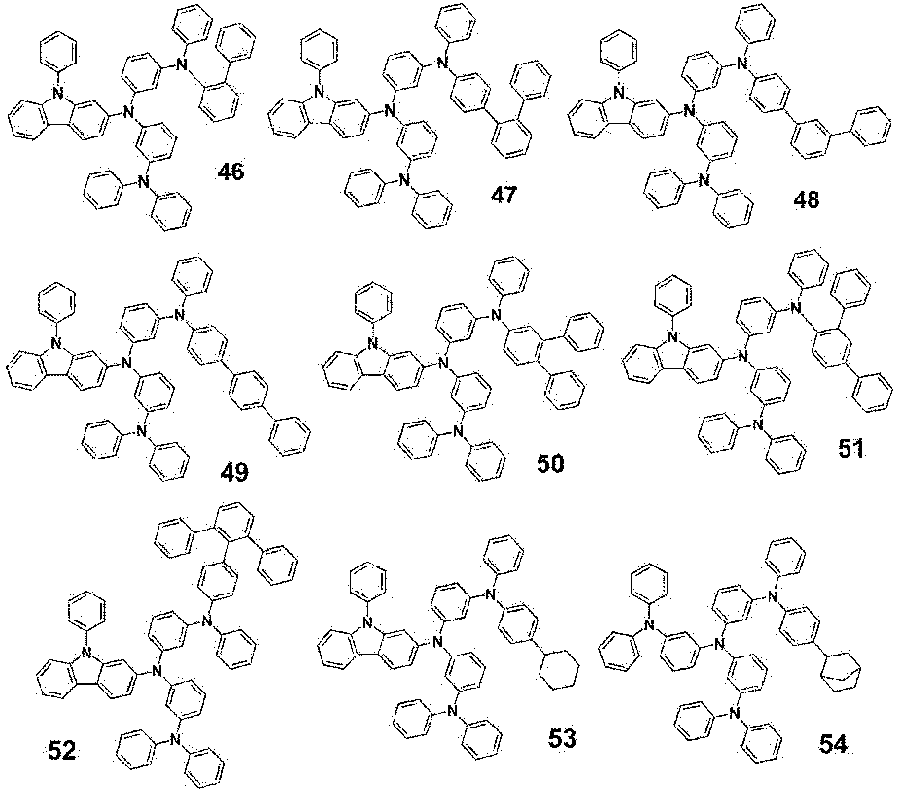

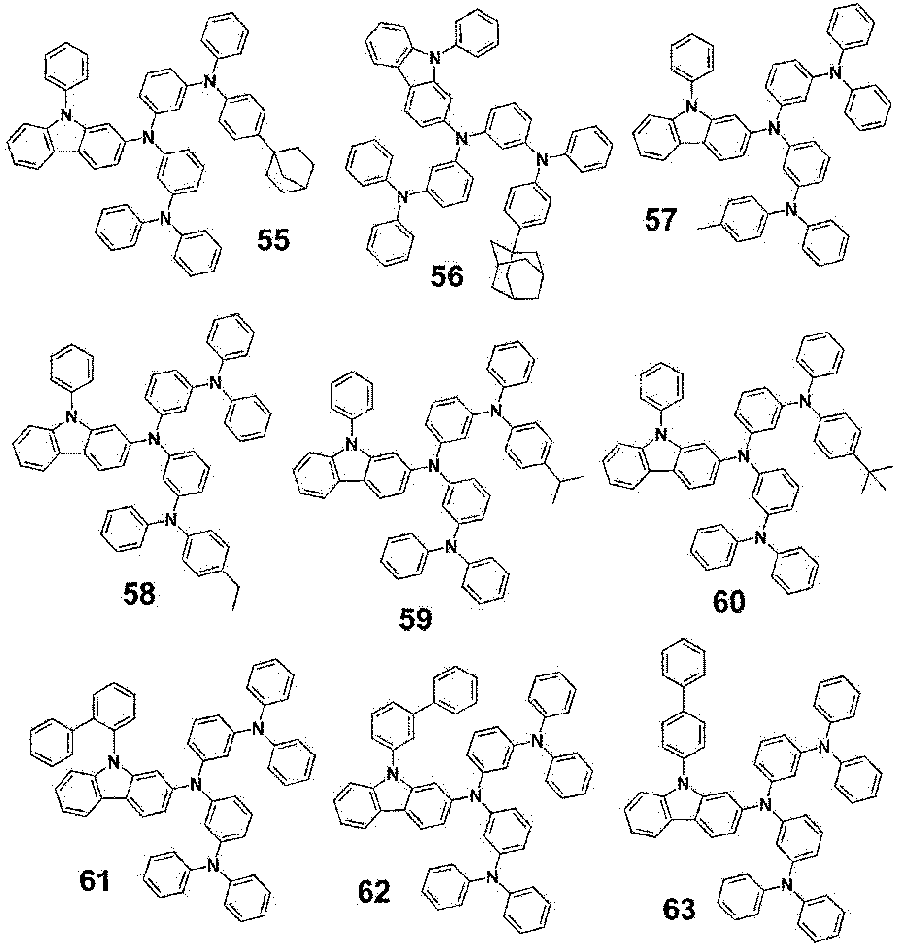

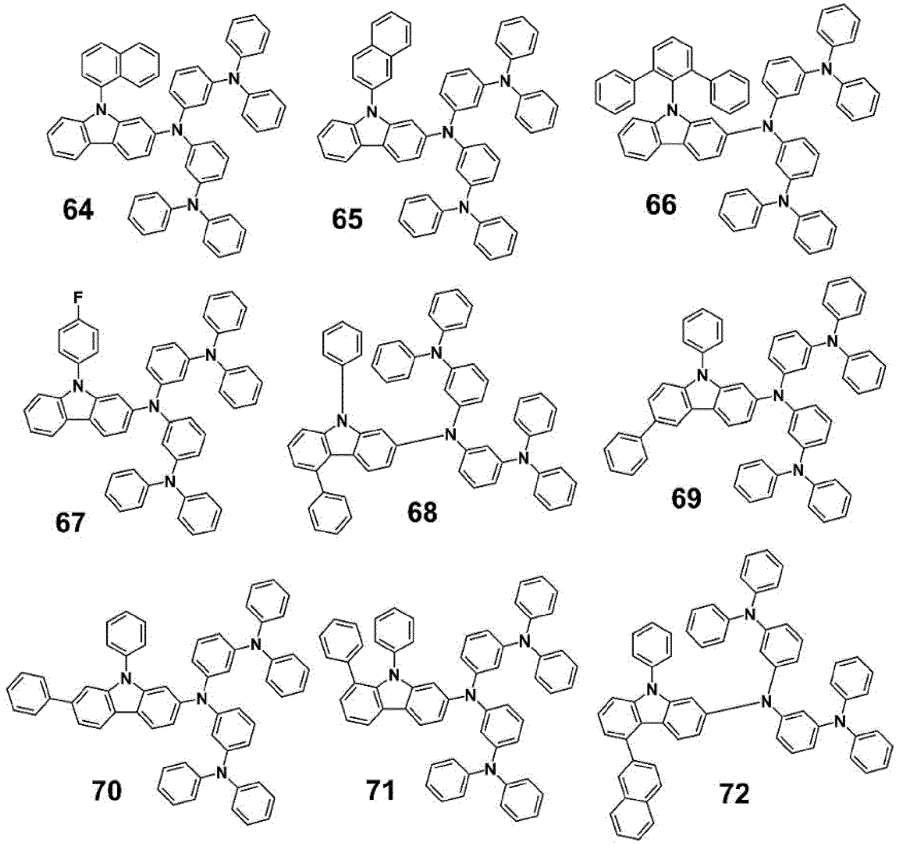

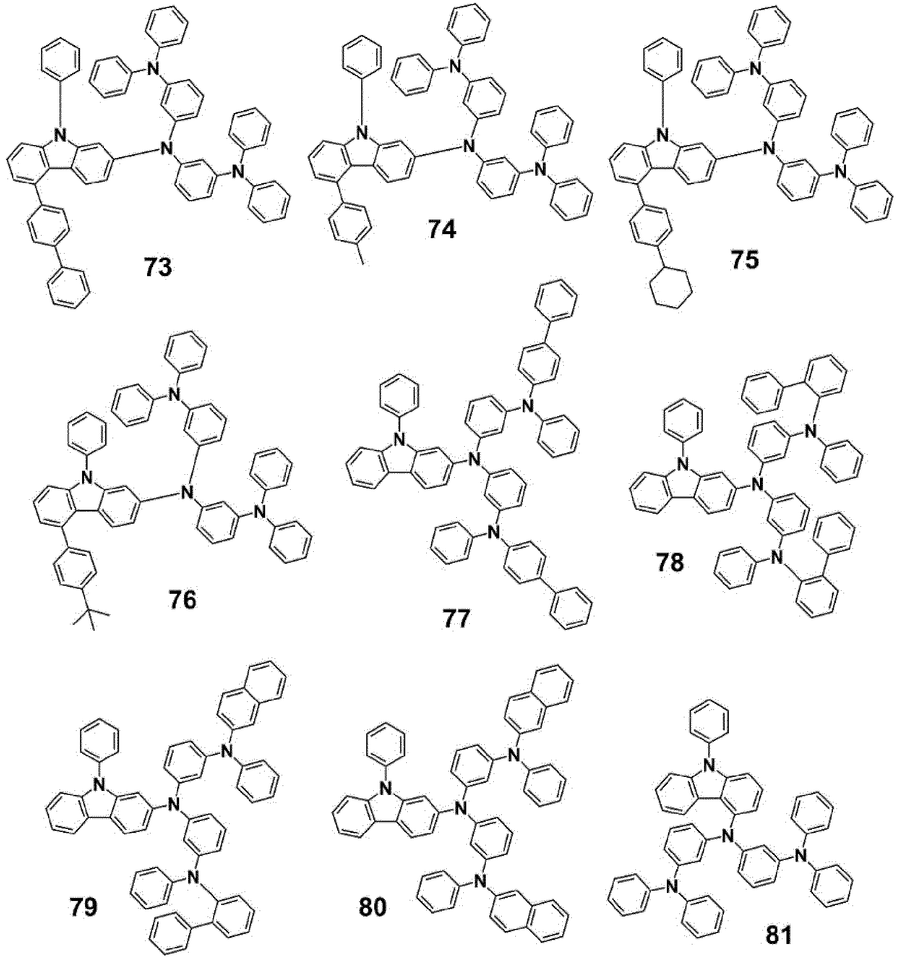

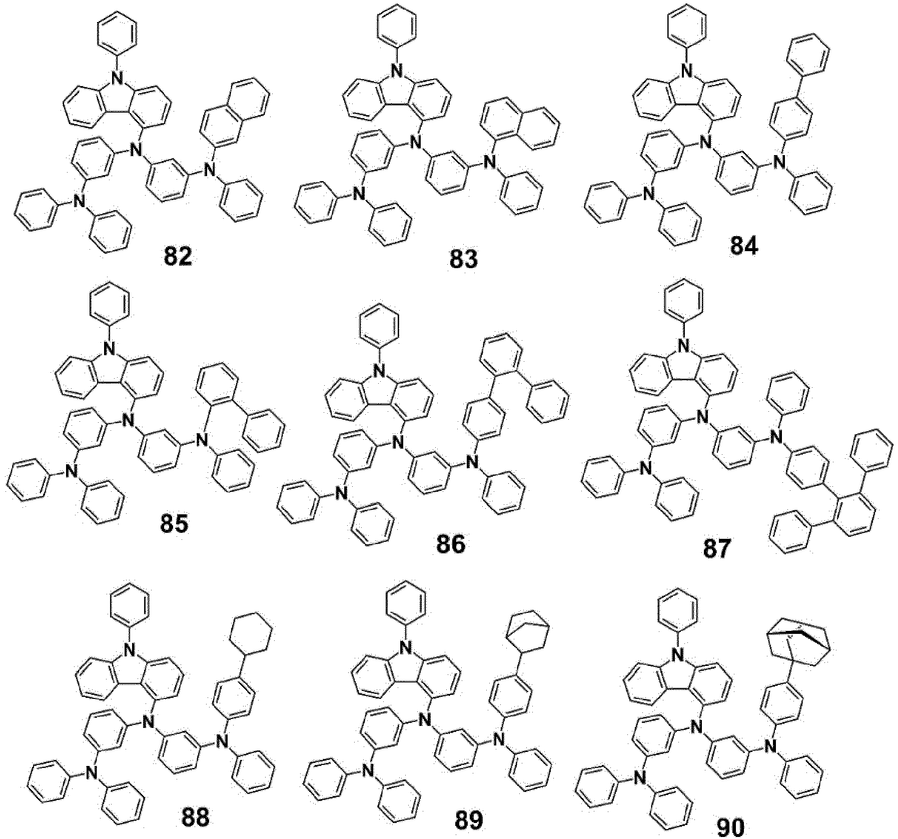

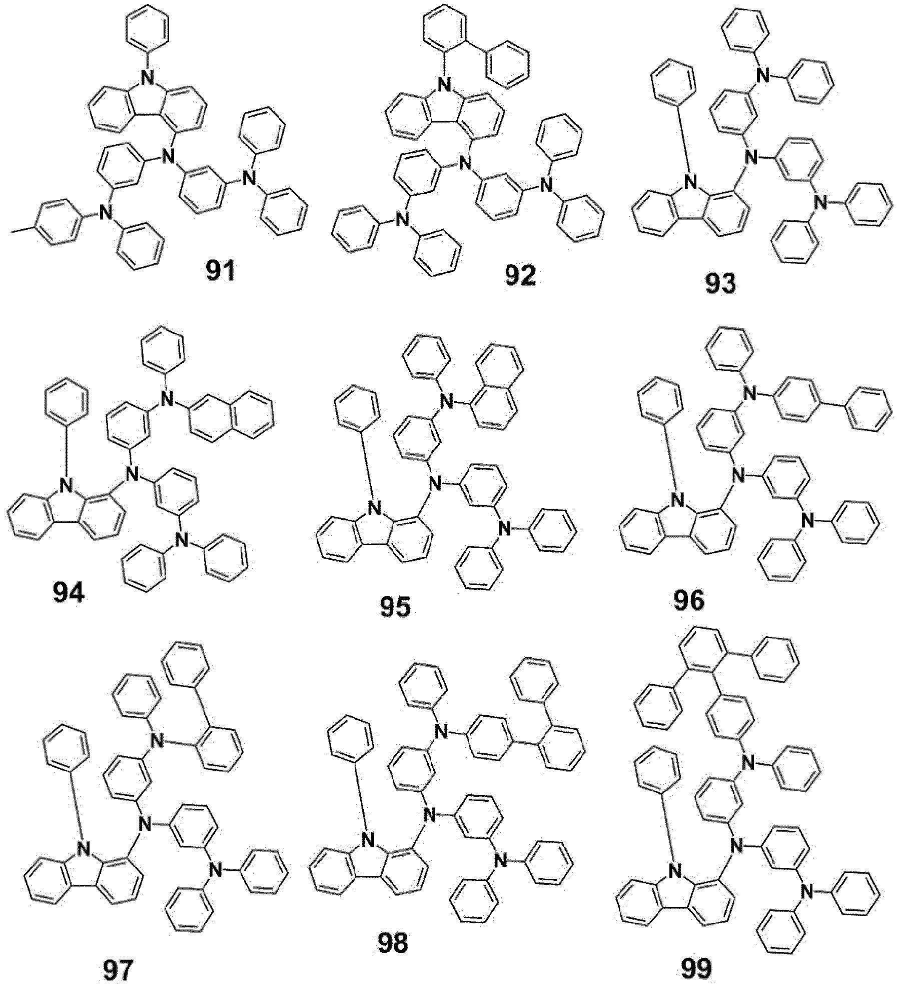

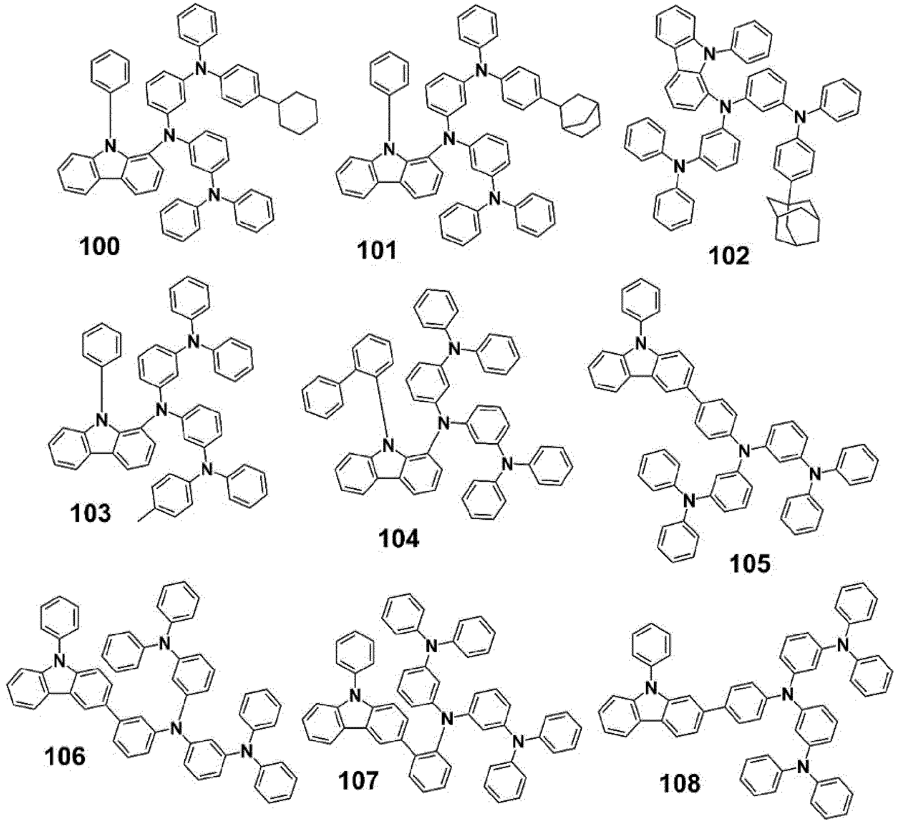

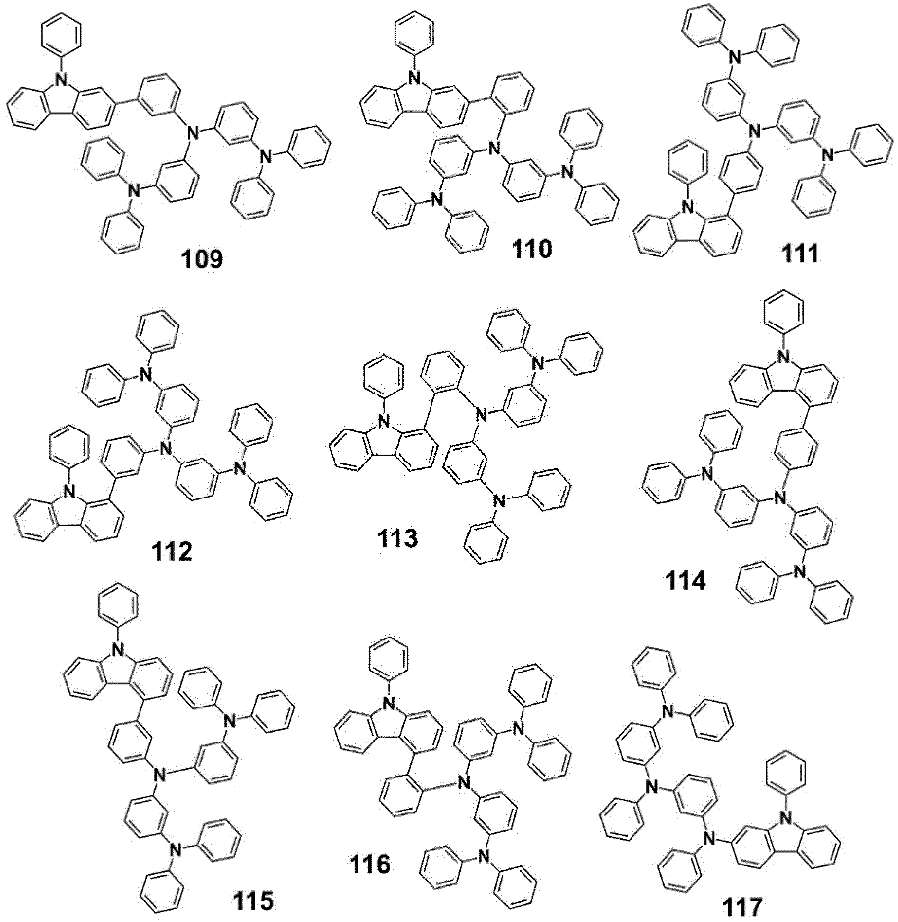

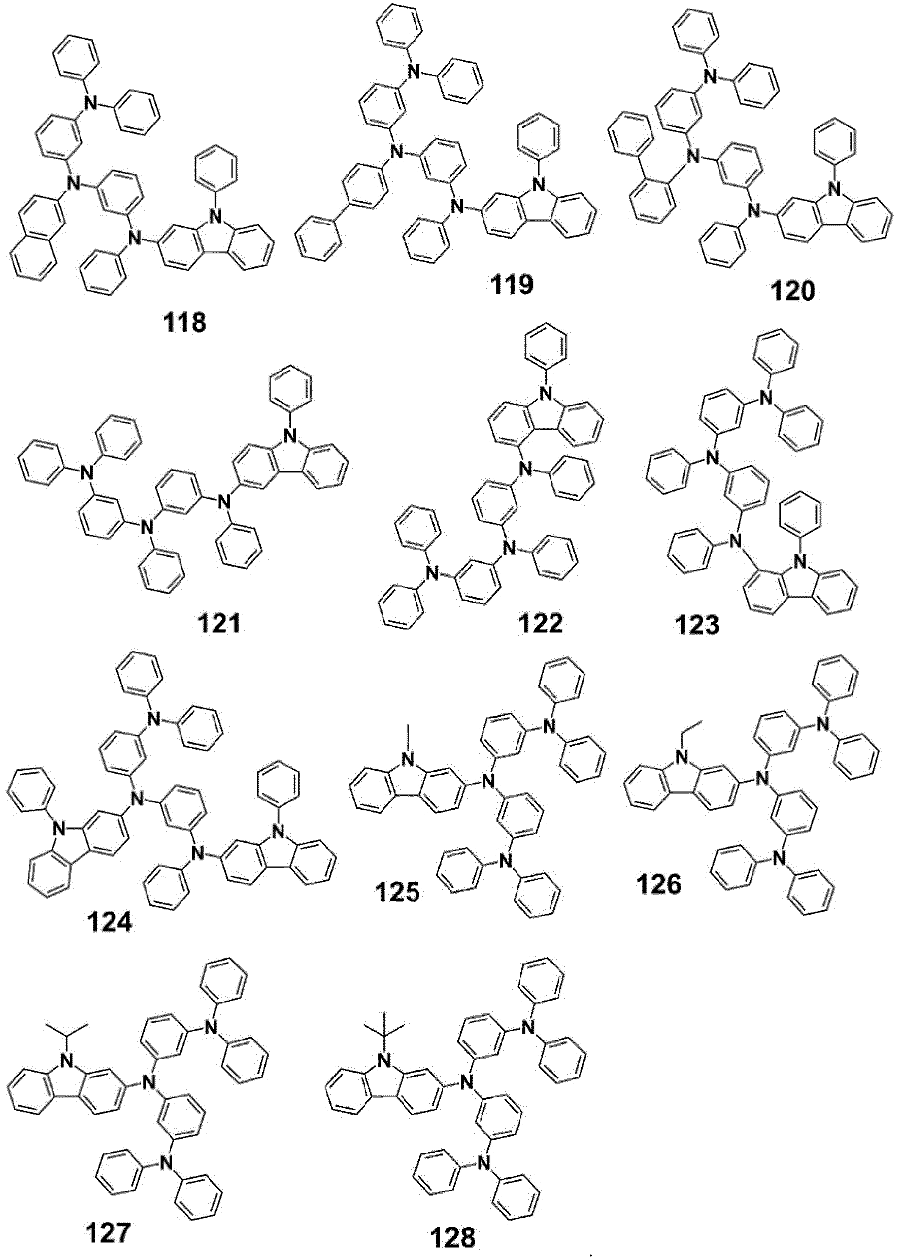

The amine compound according to an embodiment, which is represented by Formula 1, includes three amine groups and at least one carbazole group. A carbazole group may be directly or indirectly bonded to at least one of the three amine groups. The three amine groups include a first amine group (corresponding to an amine group that includes Ar₁ as a substituent), a second amine group (corresponding to an amine group that includes Ar₂ and Ar₃ as a substituent), and a third amine group (corresponding to an amine group that includes Ar₄ and Ar₅ as a substituent). The three amine groups are bonded through two phenyl groups. The first amine group and the second amine group are bonded through a phenyl group, and the second amine group and the third amine group are bonded through another phenyl group. The carbazole group may be directly bonded to an amine group or may be indirectly bonded to an amine group through a linker (corresponding to Lₐ in Formula 1).

The amine compound according to an embodiment includes a carbazole group directly or indirectly bonded to at least one of the three amine groups, and thus a conjugation system expands to achieve stable resonance energy and exhibit excellent material stability. The amine compound according to an embodiment includes a carbazole group directly or indirectly bonded to at least one of the three amine groups, and thus steric distortion may be prevented and excellent thermal stability may be achieved. The amine compound according to an embodiment may contribute to reducing driving voltage and improving luminous efficiency and service life of the light emitting element ED.

The hole transport region HTR may be provided on the first electrode EL1. The hole transport region HTR may include at least one of a hole injection layer HIL, a hole transport layer HTL, an emission auxiliary layer EAL, and an electron blocking layer EBL. A thickness of the hole transport region HTR may be, for example, in a range of about 50 Å to about 15,000 Å. In the specification, the emission auxiliary layer EAL may be referred to as a buffer layer.

The hole transport region HTR may have a structure consisting of a layer consisting of a single material, a structure consisting of a layer including different materials, or a structure including multiple layers including different materials.

In embodiments, the hole transport region HTR may have a single layer structure of a hole injection layer HIL or a hole transport layer HTL, or may have a single layer structure formed of a hole injection material and a hole transport material. In embodiments, the hole transport region HTR may have a single layer structure formed of different materials, or may have a structure in which a hole injection layer HIL/hole transport layer HTL, a hole injection layer HIL/hole transport layer HTL/emission auxiliary layer EAL, a hole injection layer HIL/emission auxiliary layer EAL, a hole transport layer HTL/emission auxiliary layer EAL, or a hole injection layer HIL/hole transport layer HTL/electron blocking layer EBL are stacked in its respective stated order from the first electrode EL1, but embodiments are not limited thereto.

The hole transport region HTR may be formed using various methods such as a vacuum deposition method, a spin coating method, a cast method, a Langmuir-Blodgett (LB) method, an inkjet printing method, a laser printing method, and a laser induced thermal imaging (LITI) method.

The hole transport region HTR may further include compounds as described below, in addition to the amine compound according to an embodiment.

In the light emitting element ED according to an embodiment, the hole transport region HTR may further include a compound represented by Formula H-1:

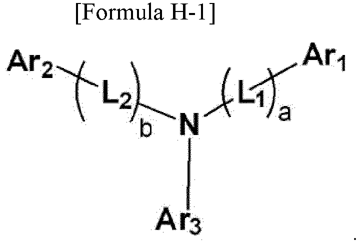

In Formula H-1, L₁ and L₂ may each independently be a direct linkage, a substituted or unsubstituted arylene group having 6 to 30 ring-forming carbon atoms, or a substituted or unsubstituted heteroarylene group having 2 to 30 ring-forming carbon atoms. In Formula H-1, a and b may each independently be an integer from 0 to 10. When a or b is 2 or greater, multiple L₁ groups or multiple L₂ groups may each independently be a substituted or unsubstituted arylene group having 6 to 30 ring-forming carbon atoms or a substituted or unsubstituted heteroarylene group having 2 to 30 ring-forming carbon atoms.

In Formula H-1, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. In Formula H-1, Ar₃ may be a substituted or unsubstituted aryl group having 6 to 30 ring-forming carbon atoms or a substituted or unsubstituted heteroaryl group having 2 to 30 ring-forming carbon atoms.

In an embodiment, the compound represented by Formula H-1 may be a monoamine compound. In another embodiment, the compound represented by Formula H-1 may be a diamine compound in which at least one of Ar₁ to Ar₃ includes an amine group as a substituent. In an embodiment, the compound represented by Formula H-1 may be a carbazole-based compound in which at least one of Ar₁ and Ar₂ includes a substituted or unsubstituted carbazole group, or may be a fluorene-based compound in which at least one of Ar₁ and Ar₂ includes a substituted or unsubstituted fluorene group.

The compound represented by Formula H-1 may be any compound selected from Compound Group H. However, the compounds listed in Compound Group H are only examples, and the compounds represented by Formula H-1 are not limited to Compound Group H:

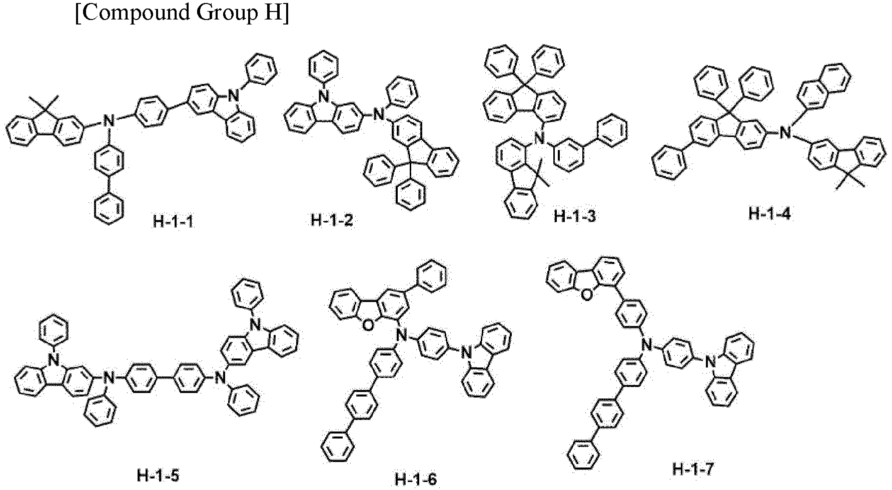

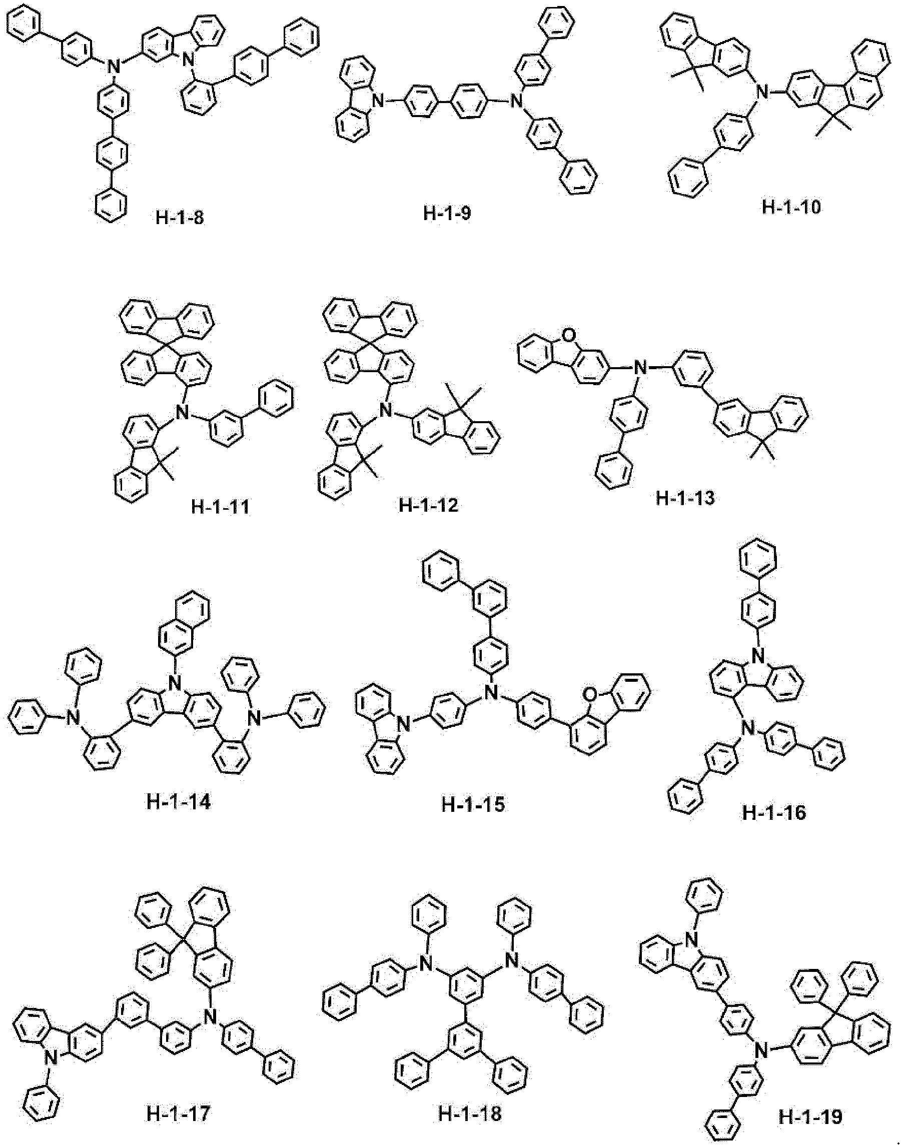

The hole transport region HTR may include a phthalocyanine compound such as copper phthalocyanine, N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N-(2-naphthyl)-N-phenylamino]triphenylamine (2-TNATA), poly(3,4-ethylenedioxythiophene)/poly(4-styrenesulfonate) (PEDOT/PSS), polyaniline/dodecylbenzene sulfonic acid (PANI/DBSA), polyaniline/camphor sulfonic acid (PANI/CSA), polyaniline/poly(4-styrenesulfonate) (PANI/PSS), N,N'-di(naphthalene-1-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HAT-CN), *etc.*

The hole transport region HTR may include a carbazole-based derivative such as N-phenyl carbazole or polyvinyl carbazole, a fluorene-based derivative, a triphenylamine-based derivative such as N,N'-bis(3-methylphenyl)-N,N'-diphenyl-[1,1-biphenyl]-4,4'-diamine (TPD), 4,4',4"-tris(carbazol-9-yl)triphenylamine (TCTA), N,N'-di(naphthalene-1-yl)-N,N'-diphenyl-benzidine (NPB), 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD) or 1,3-bis(carbazol-9-yl)benzene (mCP)), *etc.*

In an embodiment, the hole transport region HTR may include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the above-described compounds of the hole transport region in at least one of a hole injection layer HIL, a hole transport layer HTL, an emission auxiliary layer EAL, and an electron blocking layer EBL.

A thickness of the hole transport region HTR may be in a range of about 100 Å to about 10,000 Å. For example, the thickness of the hole transport region HTR may be in a range of about 100 Å to about 5,000 Å. When the hole transport region HTR includes a hole injection layer HIL, the hole injection layer HIL may have a thickness in a range of about 30 Å to about 1,000 Å. When the hole transport region HTR includes a hole transport layer HTL, the hole transport layer HTL may have a thickness in a range of about 30 Å to about 1,000 Å. When the hole transport region HTR includes an electron blocking layer EBL, the electron blocking layer EBL may have a thickness in a range of about 10 Å to about 1,000 Å. If the thicknesses of the hole transport region HTR, the hole injection layer HIL, the hole transport layer HTL, and the electron blocking layer EBL satisfy the above-described ranges, satisfactory hole transport properties may be achieved without a substantial increase in driving voltage.

The hole transport region HTR may further include a charge generating material to increase conductivity, in addition to the above-described materials. The charge generating material may be dispersed uniformly or non-uniformly in the hole transport region HTR. The charge generating material may be, for example, a p-dopant. The p-dopant may include at least one of a metal halide, a quinone derivative, a metal oxide, and a cyano group-containing compound, but embodiments are not limited thereto. For example, the p-dopant may include a metal halide compound such as CuI or RbI, a quinone derivative such as tetracyanoquinodimethane (TCNQ) or 2,3,5,6-tetrafluoro-7,7'8,8-tetracyanoquinodimethane (F4-TCNQ), a metal oxide such as tungsten oxide or molybdenum oxide, a cyano group-containing compound such as dipyrazino[2,3-f: 2',3'-h] quinoxaline-2,3,6,7,10,11-hexacarbonitrile (HATCN) or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), *etc.,* but embodiments are not limited thereto.

As described above, the hole transport region HTR may further include at least one of an emission auxiliary layer EAL and an electron blocking layer EBL, in addition to a hole injection layer HIL and a hole transport layer HTL. The emission auxiliary layer EAL may compensate for a resonance distance according to a wavelength of light emitted from the emission layer EML and may regulate hole charge balance to increase luminous efficiency. The emission auxiliary layer EAL may also prevent electrons from being injected into the hole transport region HTR. Materials which may be included in the hole transport region HTR may be used as materials for the emission auxiliary layer EAL. The electron blocking layer EBL may prevent electrons from being injected from the electron transport region ETR to the hole transport region HTR.

The emission layer EML may be provided on the hole transport region HTR. The emission layer EML may have a thickness in a range of about 100 Å to about 1,000 Å. For example, the emission layer EML may have a thickness in a range of about 100 Å to about 300 Å. The emission layer EML may have a structure consisting of a layer consisting of a single material, a structure consisting of a layer including different materials, or a structure including multiple layers including different materials.

In an embodiment, the emission layer EML may include at least one of a first compound represented by Formula HT-1, a second compound represented by Formula ET-1, and a third compound represented by Formula D-1.

The emission layer EML may include a first compound represented by Formula HT-1. In an embodiment, the first compound may be used as a hole transporting host material in the emission layer EML:

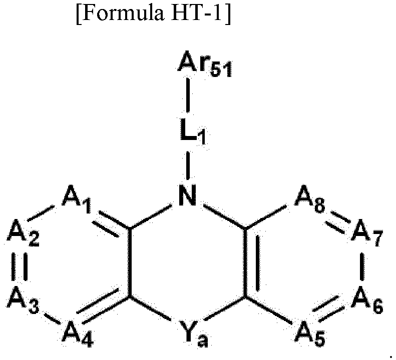

In Formula HT-1, A₁ to As are each independently N or C(R₅₁). For example, A₁ to A₈ may each independently be CR₅₁. As another example, one of A₁ to As may be N, and the remainder of A₁ to As may each independently be C(R₅₁).

In Formula HT-1, L₁ is a direct linkage, a substituted or unsubstituted arylene group having 6 to 30 ring-forming carbon atoms, or a substituted or unsubstituted heteroarylene group having 2 to 30 ring-forming carbon atoms. For example, L₁ may be a direct linkage, a substituted or unsubstituted phenylene group, a substituted or unsubstituted divalent biphenyl group, a substituted or unsubstituted divalent carbazole group, etc., but embodiments are not limited thereto.

In Formula HT-1, Yₐ is a direct linkage, C(R₅₂)(R₅₃), or Si(R₅₄)(R₅₅). For example, the two benzene rings that are linked to the nitrogen atom in Formula HT-1 may be linked to each other via a direct linkage, 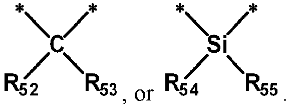 In Formula HT-1, when Yₐ is a direct linkage, the first compound represented by Formula HT-1 may include a carbazole moiety.

In Formula HT-1, Ar₅₁ is a substituted or unsubstituted aryl group having 6 to 30 ring-forming carbon atoms or a substituted or unsubstituted heteroaryl group having 2 to 30 ring-forming carbon atoms. For example, Ar₅₁ may be a substituted or unsubstituted carbazole group, a substituted or unsubstituted dibenzofuran group, a substituted or unsubstituted dibenzothiophene group, a substituted or unsubstituted biphenyl group, *etc.,* but embodiments are not limited thereto.

In Formula HT-1, R₅₁ to R₅₅ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms, or bonded to an adjacent group to form a ring. For example, R₅₁ to R₅₅ may each independently be a hydrogen atom or a deuterium atom. For example, R₅₁ to R₅₅ may each independently be an unsubstituted methyl group or an unsubstituted phenyl group.

In an embodiment, the first compound represented by Formula HT-1 may be selected from Compound Group 2. In an embodiment, in light emitting element ED, the first compound may include at least one compound selected from Compound Group 2:

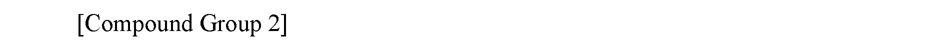

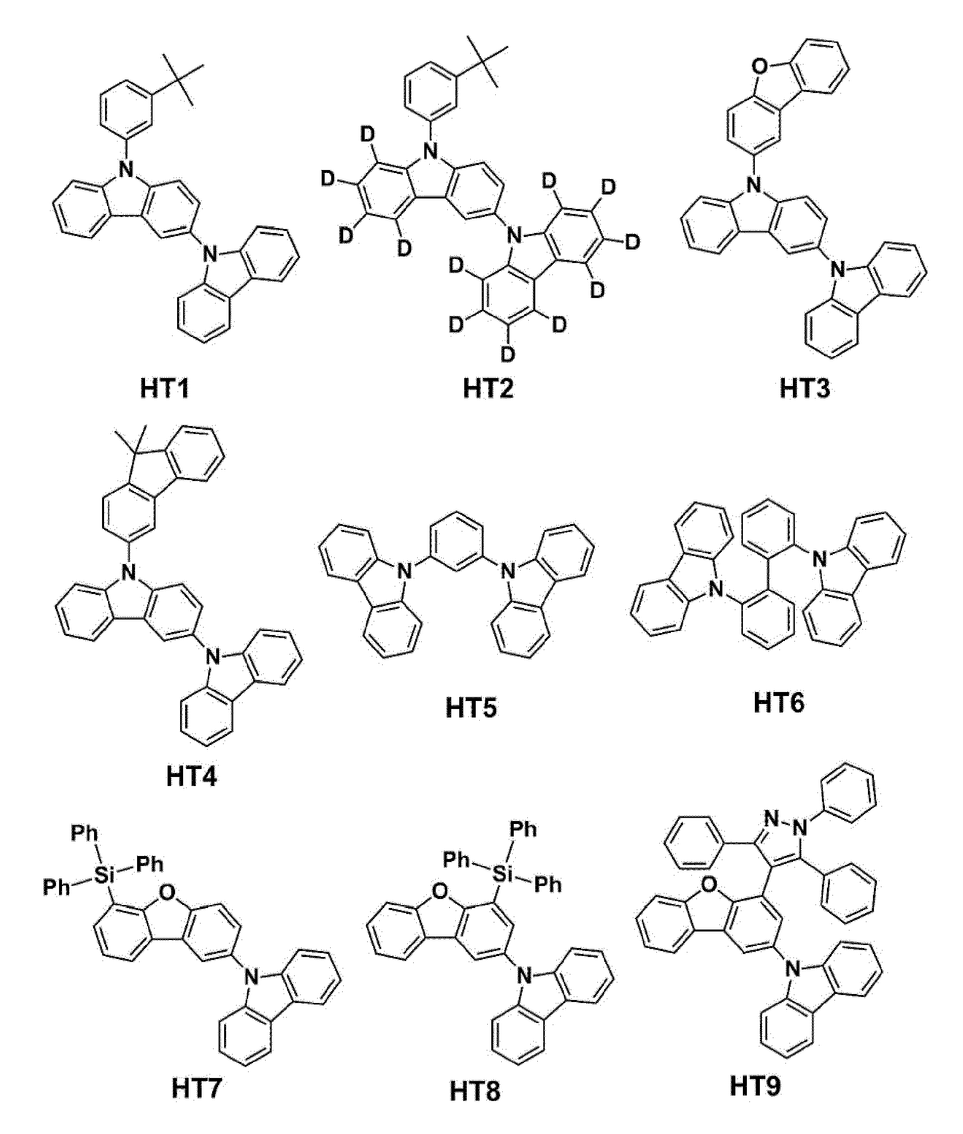

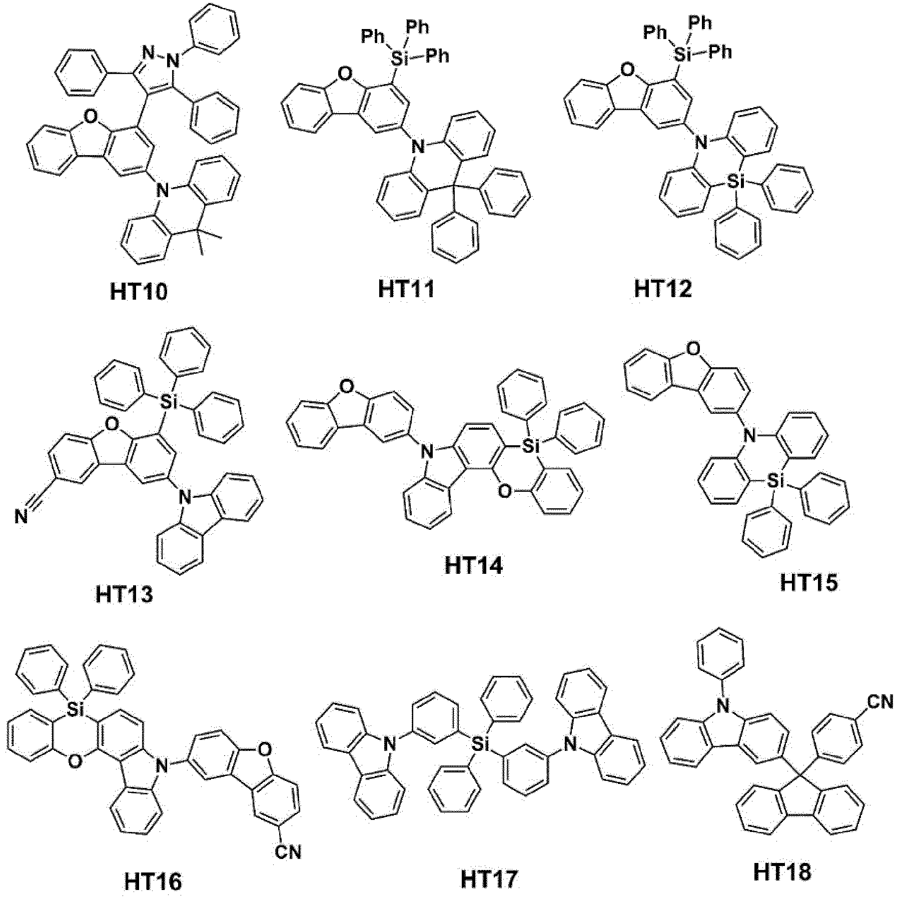

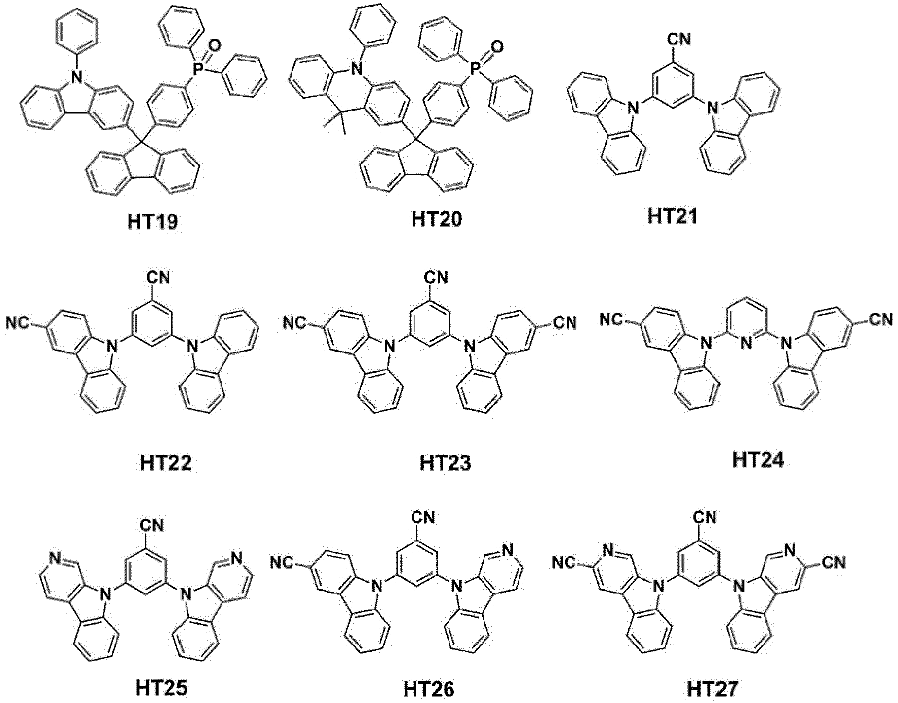

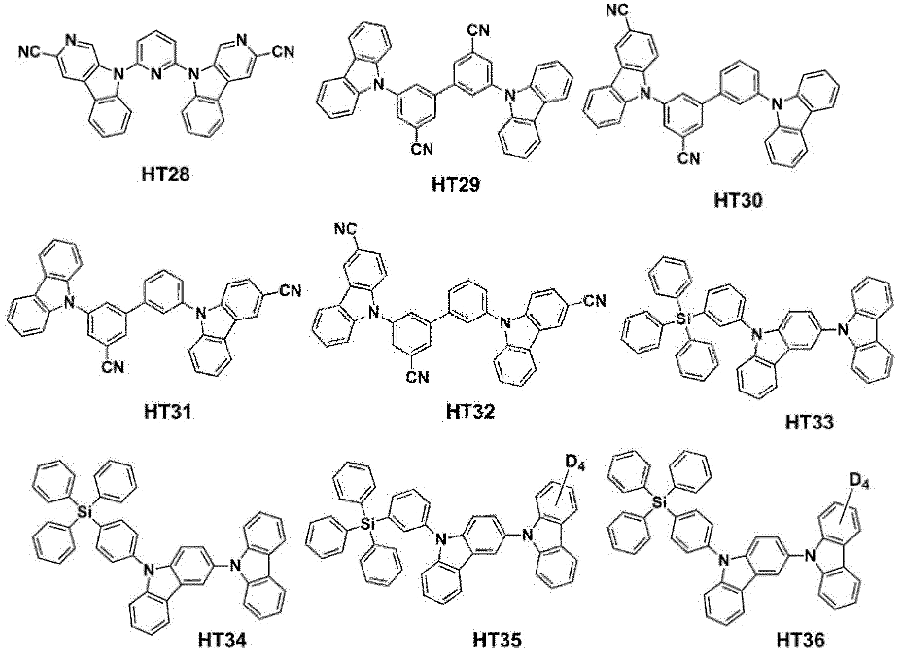

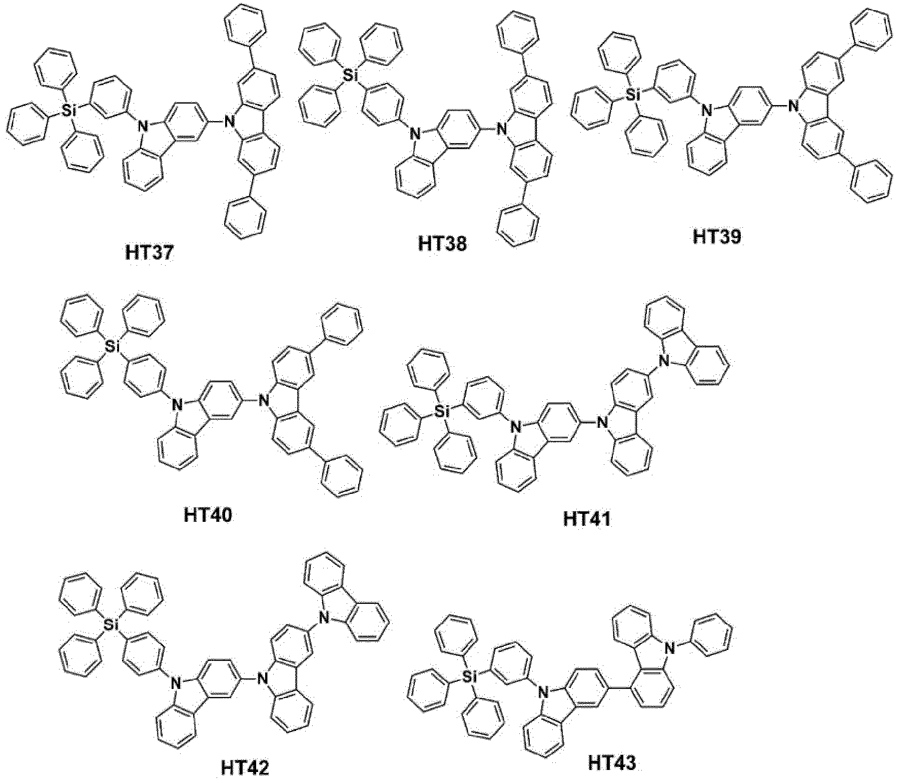

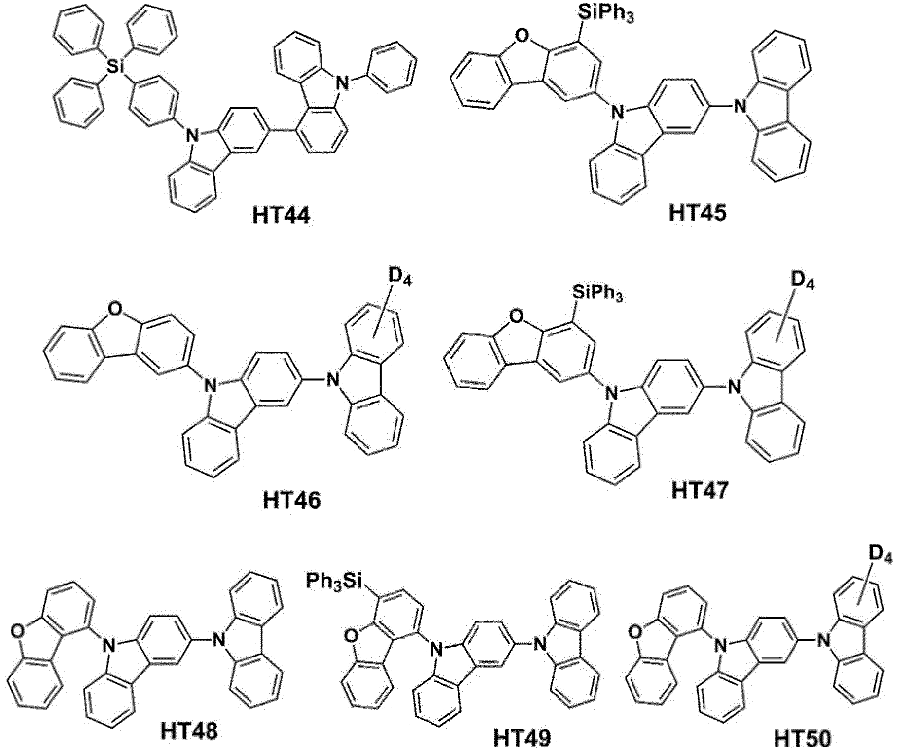

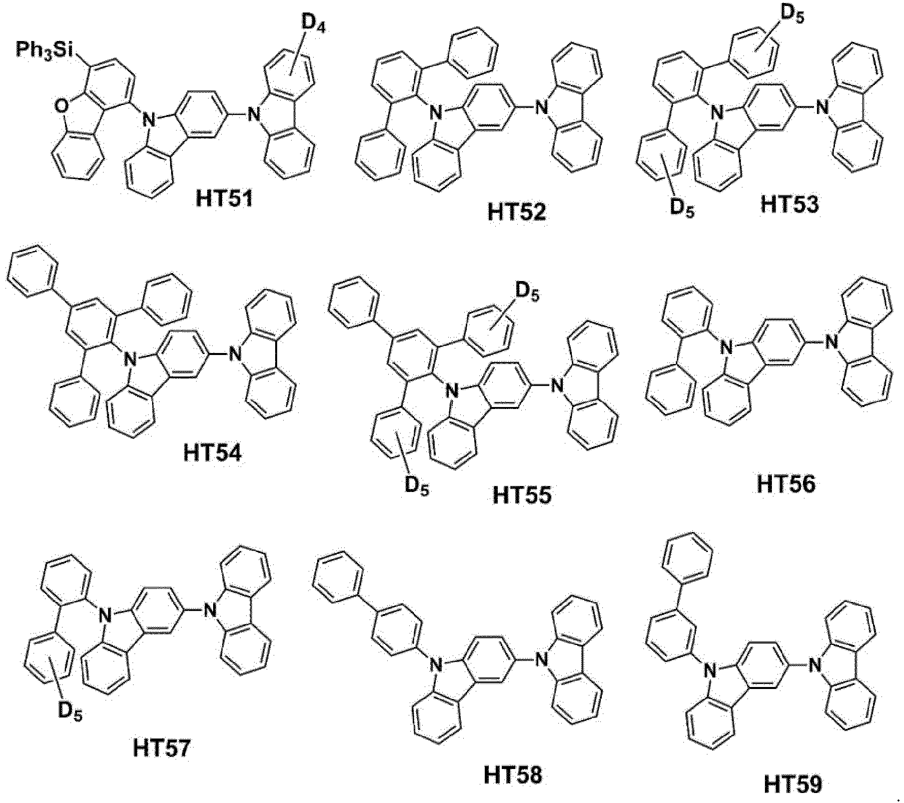

In Compound Group 2, D represents a deuterium atom, and Ph represents a substituted or unsubstituted phenyl group. For example, in Compound Group 2, Ph may represent an unsubstituted phenyl group.

In an embodiment, the emission layer EML may include a second compound represented by Formula ET-1. In an embodiment, the second compound may be used as an electron transport host material in the emission layer EML:

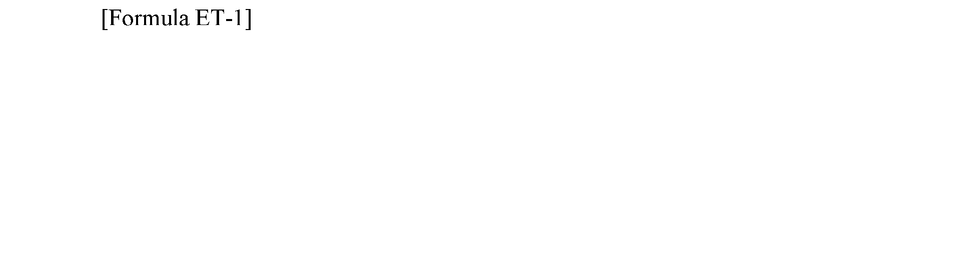

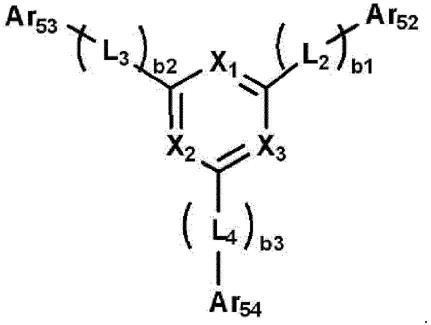

In Formula ET-1, at least one of X₁ to X₃ is N, and the remainder of X₁ to X₃ are each independently C(R₅₆). For example, one of X₁ to X₃ may be N, and the remainder of X₁ to X₃ may each independently be C(R₅₆). Thus, the second compound represented by Formula ET-1 may include a pyridine moiety. As another example, two of X₁ to X₃ may each be N, and the remainder of X₁ to X₃ may be C(R₅₆). Thus, the second compound represented by Formula ET-1 may include a pyrimidine moiety. As yet another example, X₁ to X₃ may each be N. Thus, the second compound represented by Formula ET-1 may include a triazine moiety.

In Formula ET-1, R₅₆ is a hydrogen atom, a deuterium atom, a substituted or unsubstituted alkyl group having 1 to 20 carbon atoms, a substituted or unsubstituted aryl group having 6 to 60 ring-forming carbon atoms, or a substituted or unsubstituted heteroaryl group having 2 to 60 ring-forming carbon atoms.

In Formula ET-1, b1 to b3 are each independently an integer from 0 to 10.

In Formula ET-1, Ar₅₂ to Ar₅₄ are each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. For example, Ar₅₂ to Ar₅₄ may each independently be a substituted or unsubstituted phenyl group or a substituted or unsubstituted carbazole group.

In Formula ET-1, L₂ to L₄ are each independently a direct linkage, a substituted or unsubstituted arylene group having 6 to 30 ring-forming carbon atoms, or a substituted or unsubstituted heteroarylene group having 2 to 30 ring-forming carbon atoms. When b1 to b3 are each 2 or greater, multiple groups of each of L₂ to L₄ may each independently be a substituted or unsubstituted arylene group having 6 to 30 ring-forming carbon atoms or a substituted or unsubstituted heteroarylene group having 2 to 30 ring-forming carbon atoms.

In an embodiment, the second compound represented by Formula ET-1 may be selected from Compound Group 3. In an embodiment, in the light emitting element ED, the second compound may include at least one compound selected from Compound Group 3:

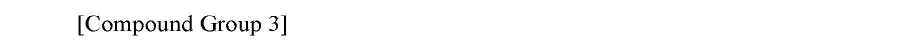

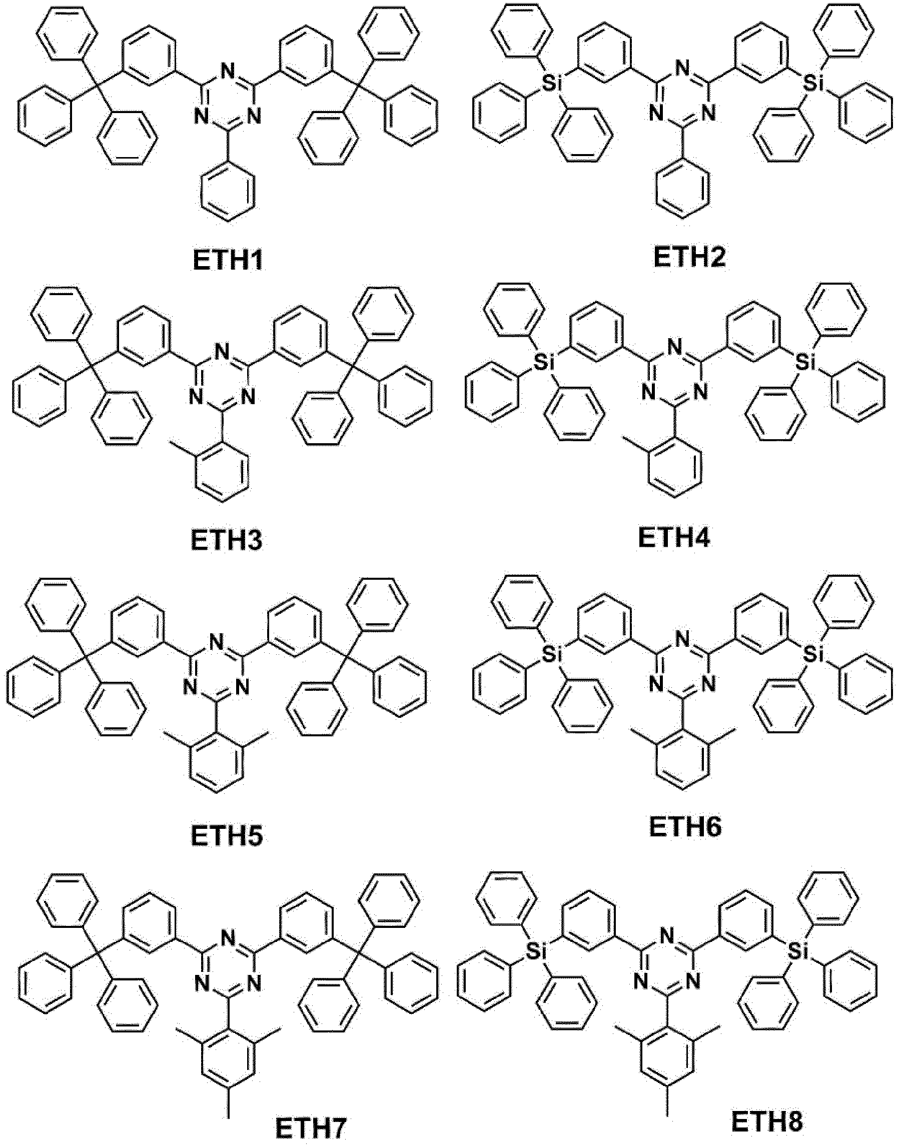

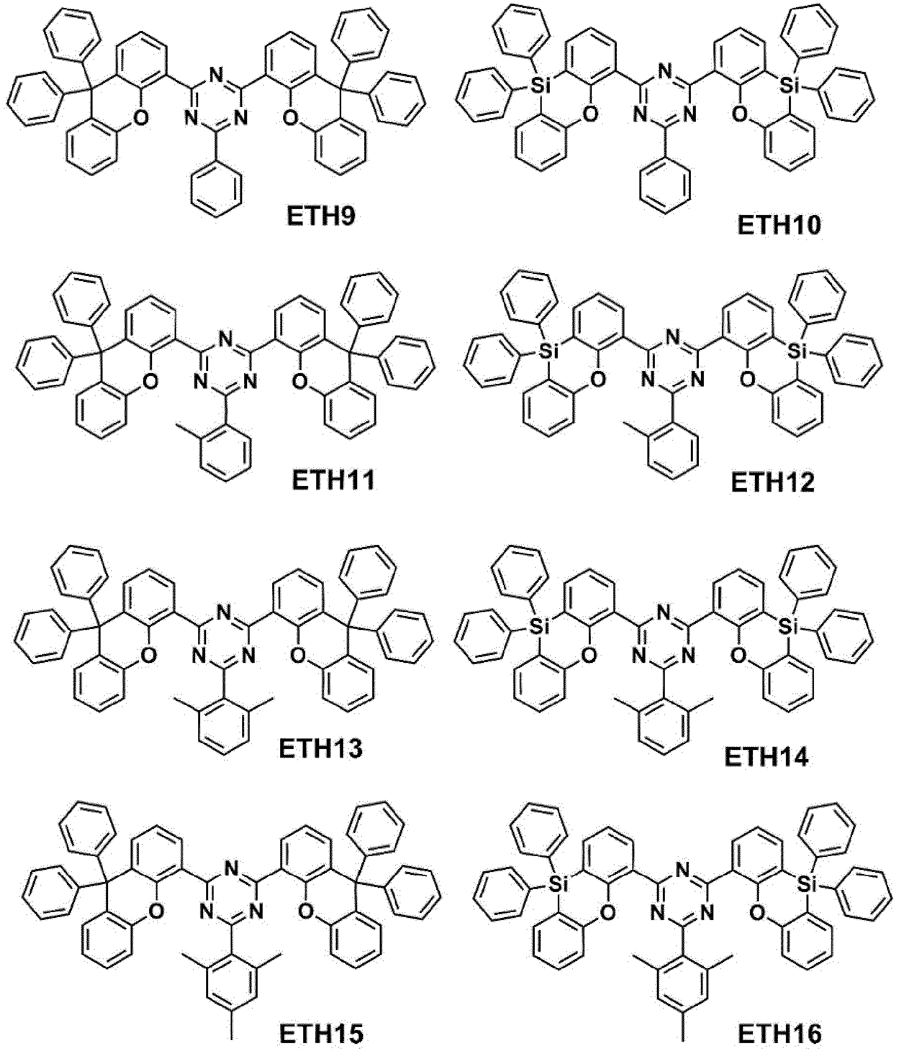

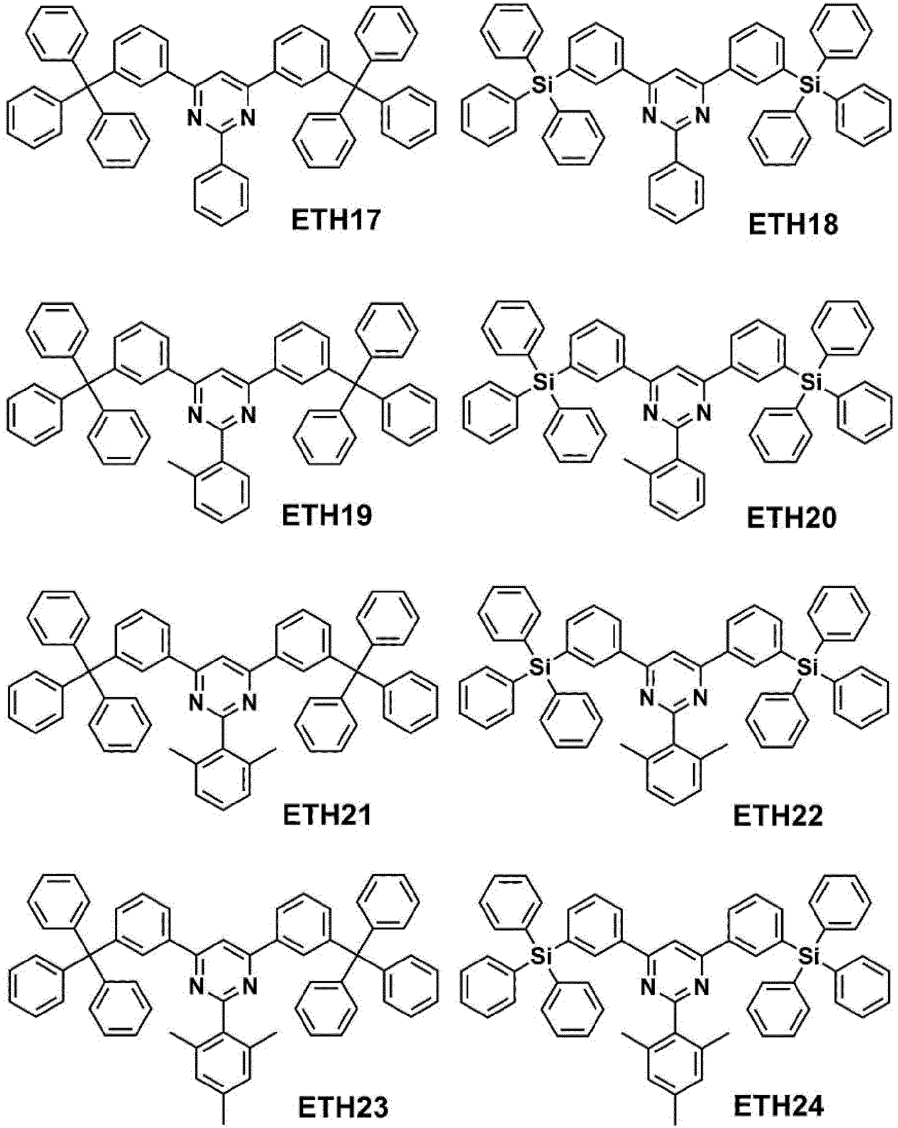

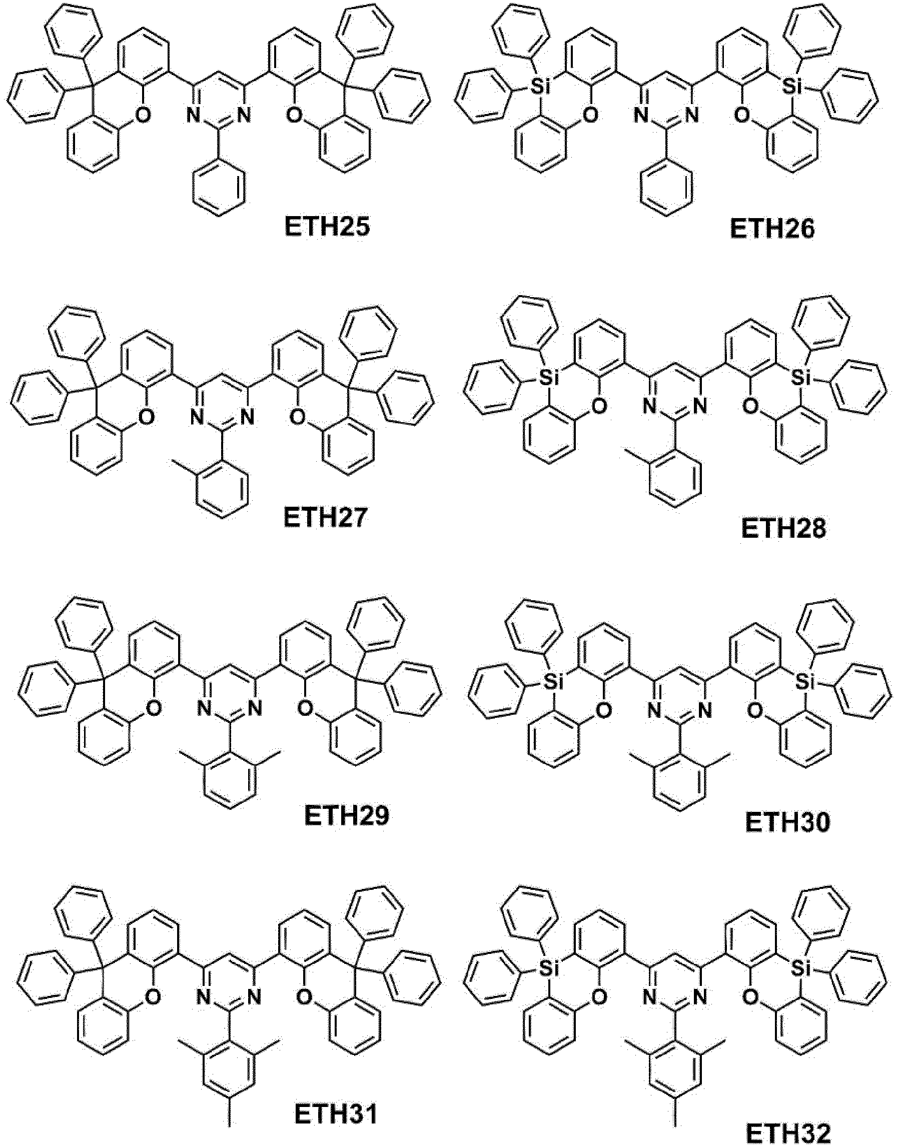

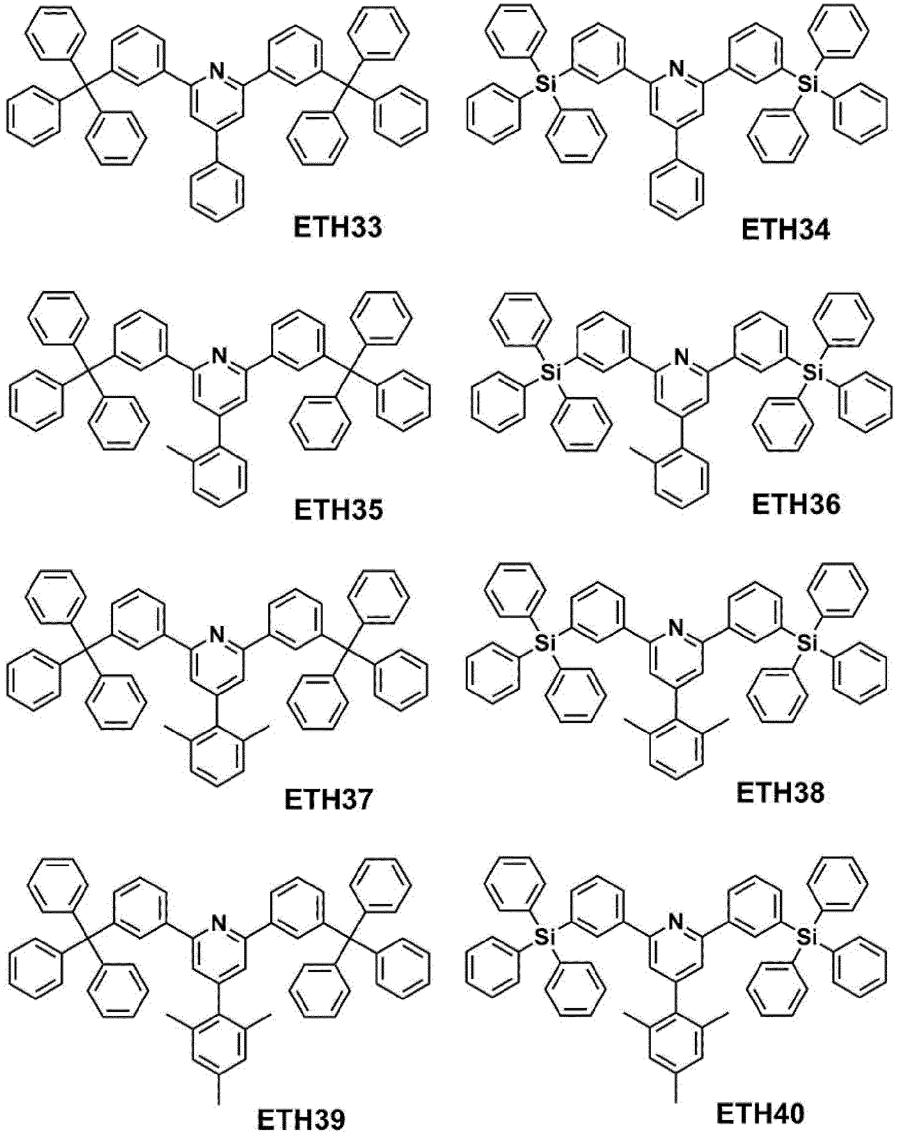

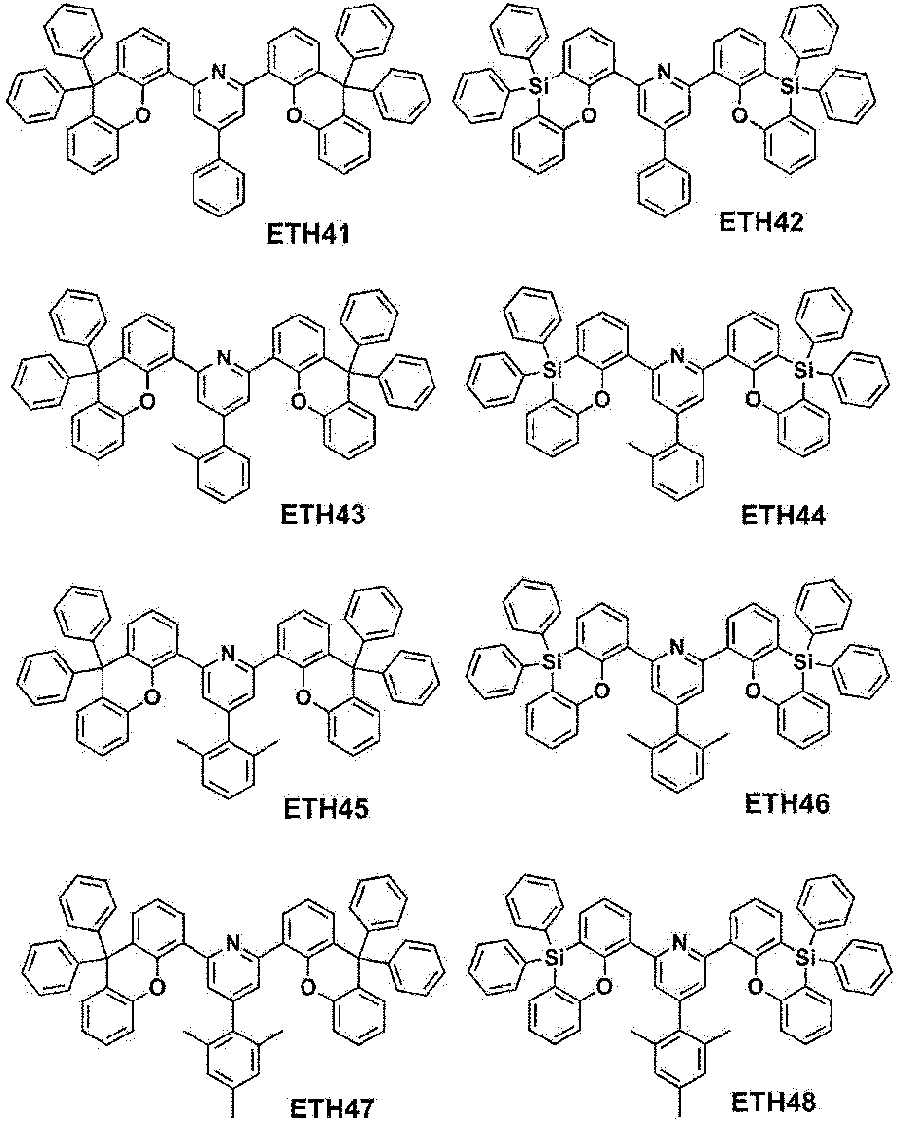

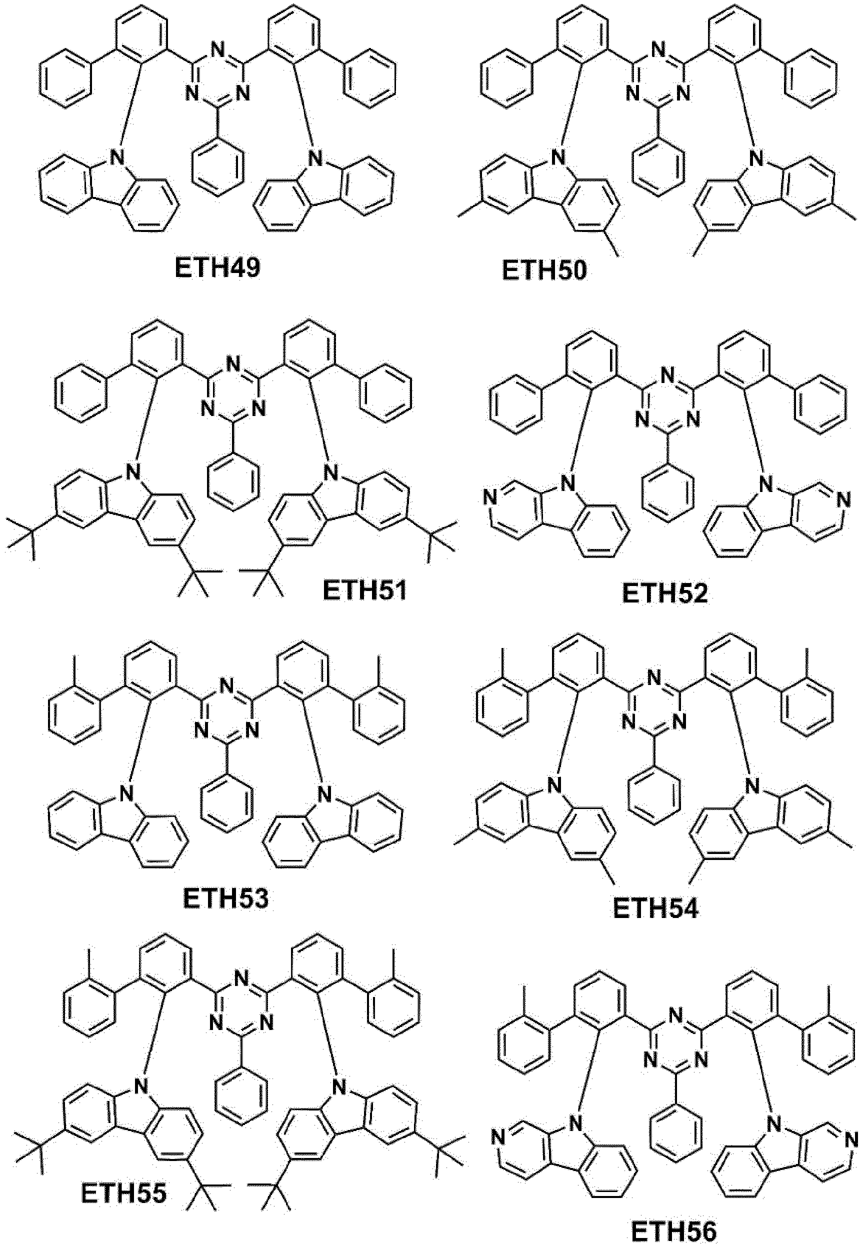

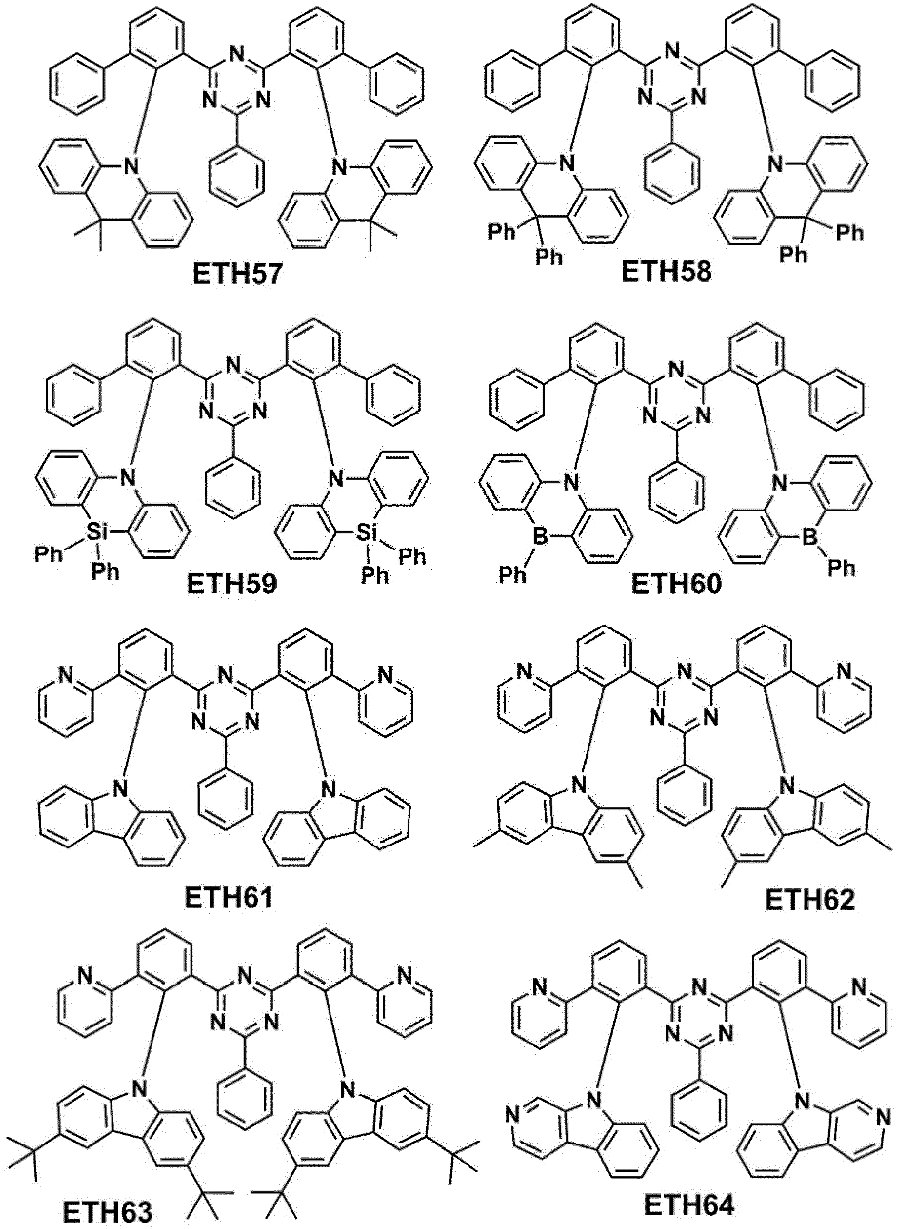

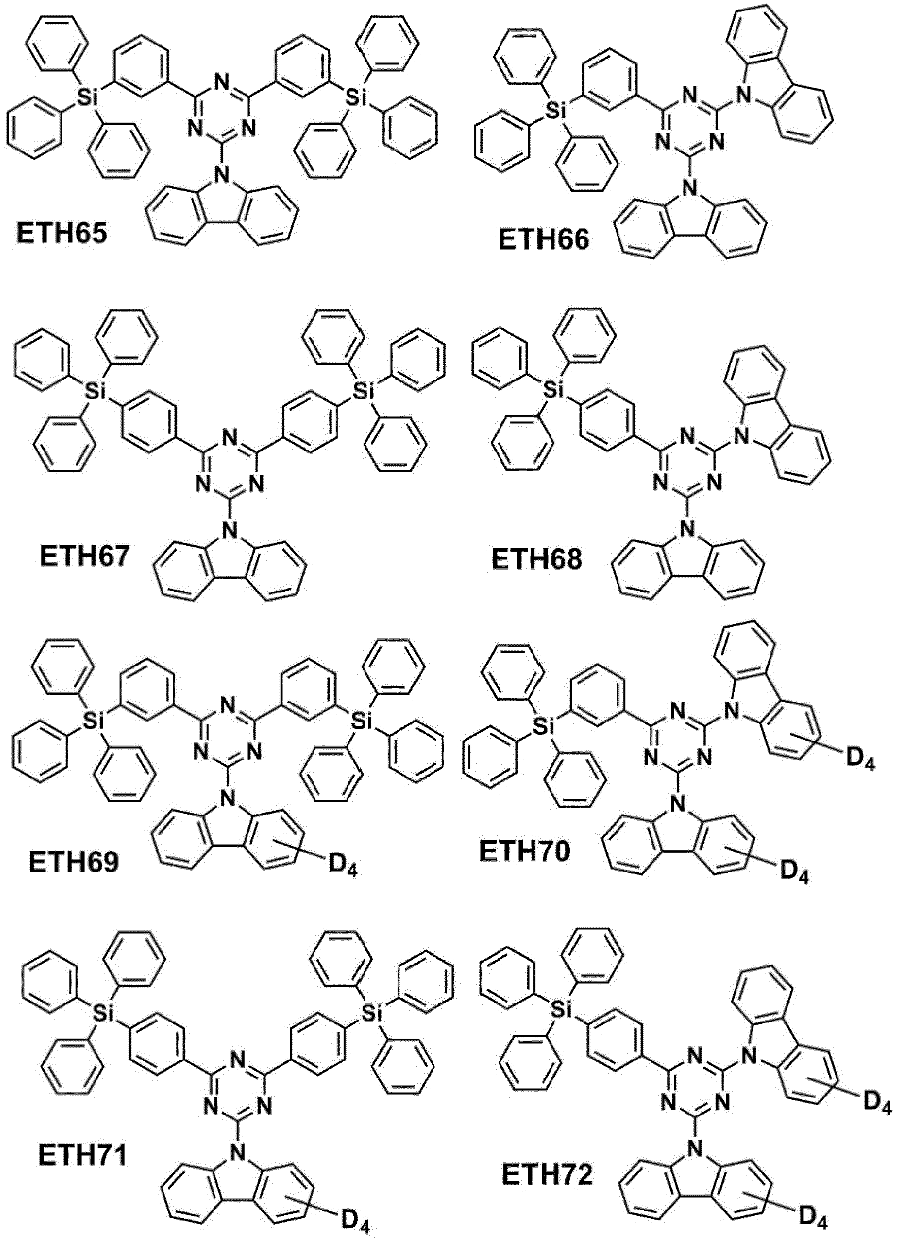

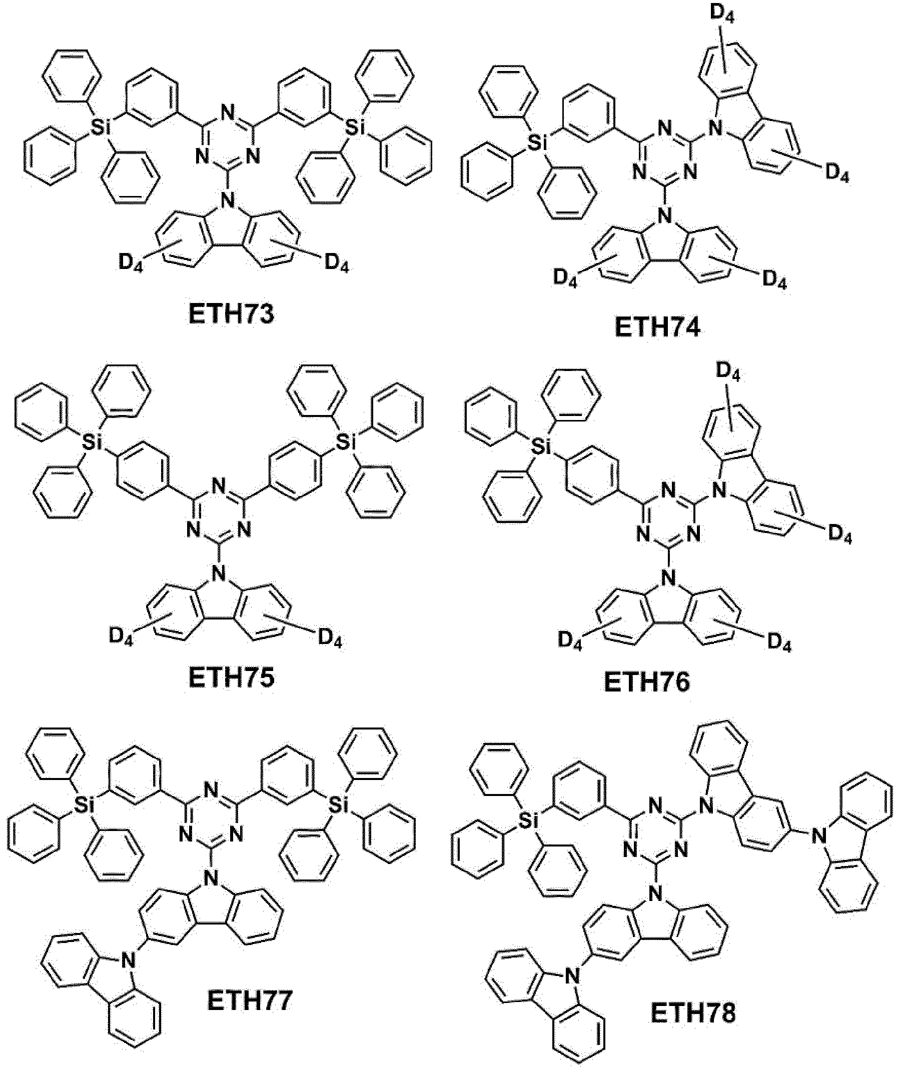

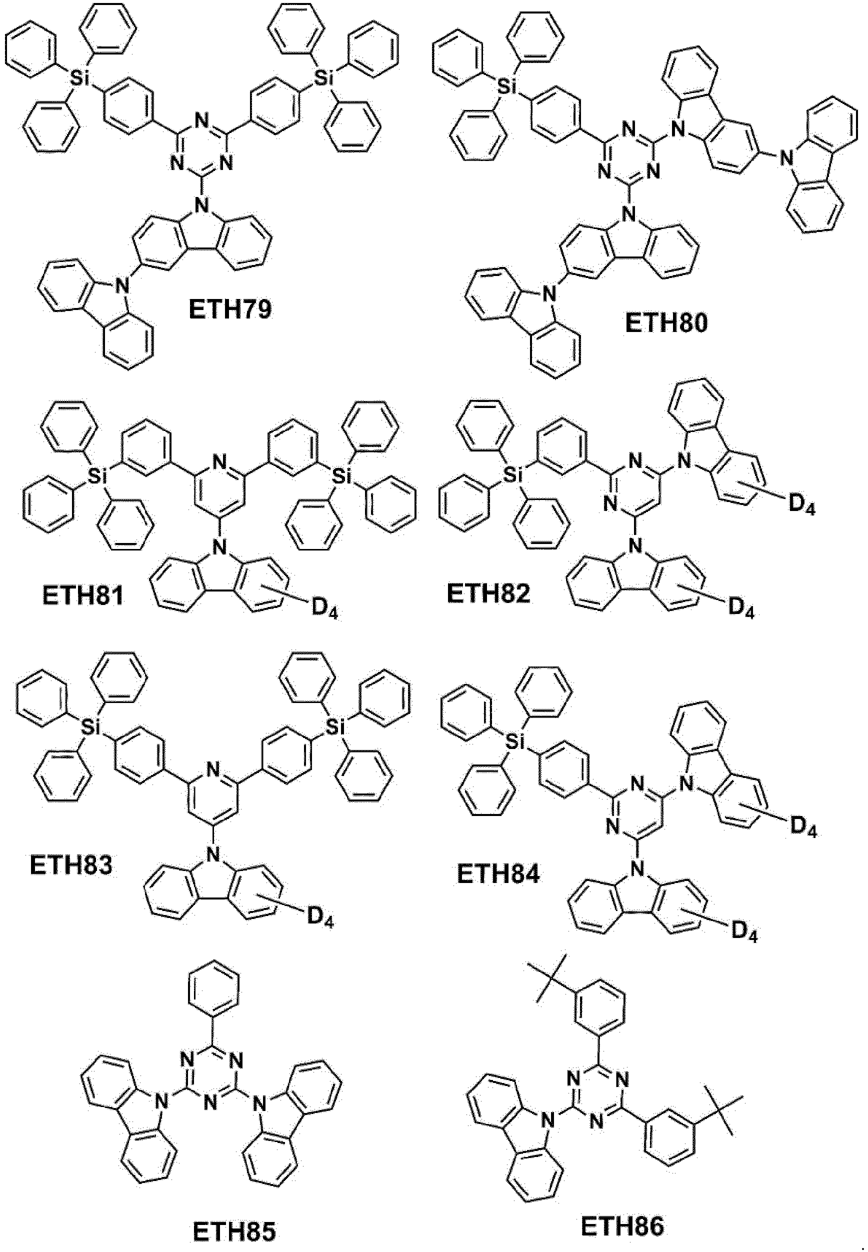

In Compound Group 3, D represents a deuterium atom, and Ph represents an unsubstituted phenyl group.

In an embodiment, the emission layer EML may include, as a third compound, an organometallic complex that includes platinum (Pt) as a central metal atom and ligands linked to the central metal atom. In an embodiment, the emission layer EML may include a third compound represented by Formula D-1. The third compound serve as a sensitizer:

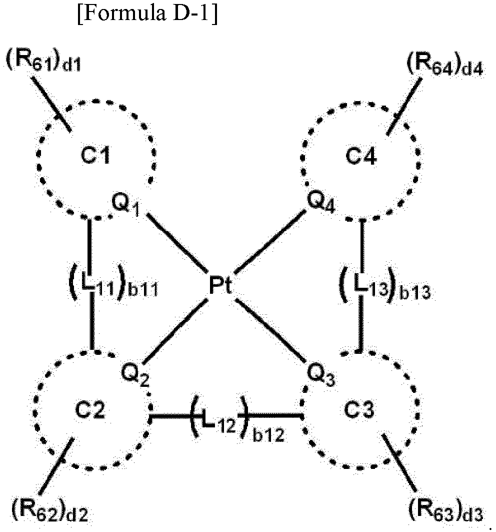

In Formula D-1, Q₁ to Q₄ are each independently C or N. In Formula D-1, C1 to C4 are each independently a substituted or unsubstituted hydrocarbon ring having 5 to 30 ring-forming carbon atoms, a substituted or unsubstituted aryl group having 6 to 30 ring-forming carbon atoms, or a substituted or unsubstituted heterocycle having 2 to 30 ring-forming carbon atoms.

In Formula D-1, L₁₁ to L₁₃ are each independently a direct linkage, *-O-*, *-S-*, 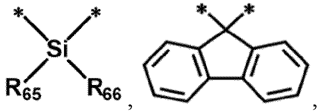 a substituted or unsubstituted alkylene group having 1 to 20 carbon atoms, a substituted or unsubstituted arylene group having 6 to 30 ring-forming carbon atoms, or a substituted or unsubstituted heteroarylene group having 2 to 30 ring-forming carbon atoms. In L₁₁ to L₁₃, -* represents a bond to one of C1 to C4.

In Formula D-1, b11 to b13 are each independently 0 or 1. If b11 is 0, C1 and C2 may not be directly linked to each other. If b12 is 0, C2 and C3 may not be directly linked to each other. If b13 is 0, C3 and C4 may not be directly linked to each other.

In Formula D-1, R₆₁ to R₆₆ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms, or bonded to an adjacent group to form a ring. For example, R₆₁ to R₆₆ may each independently be a substituted or unsubstituted methyl group or a substituted or unsubstituted t-butyl group.

In Formula D-1, d1 to d4 are each independently an integer from 0 to 4. If d1 to d4 are each 0, the third compound may not be substituted with R₆₁ to R₆₄, respectively. A case where d1 to d4 are each 4 and four groups of each of R₆₁ to R₆₄ are all hydrogen atoms may be the same as a case where d1 to d4 are each 0. When d1 to d4 are each 2 or more, multiple groups of each of R₆₁ to R₆₄ may all be the same, or at least one thereof may be different from the remainder.

In an embodiment, in Formula D-1, C1 to C4 may each independently be a substituted or unsubstituted hydrocarbon ring or a substituted or unsubstituted heterocycle that is represented by one of Formula C-1 to Formula C-4:

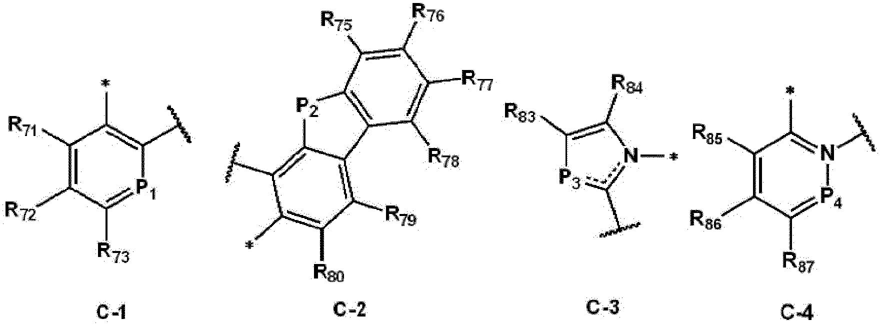

In Formula C-1 to Formula C-4, P₁ may be C-* or C(R₇₄), P₂ may be N-* or N(R₈₁), P₃ may be N-* or N(R₈₂), and P₄ may be **C-*** or C(R₈₈).

In Formula C-1 to Formula C-4, R₇₁ to R₈₈ may each independently be a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

In Formula C-1 to Formula C-4, 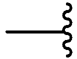 represents a bond to Pt, which is a central metal atom, and -* represents a bond to a neighboring cyclic group (C1 to C4) or to a linking moiety (L₁₁ to L₁₃).

In an embodiment, the third compound represented by Formula D-1 may be selected from Compound Group 4. In an embodiment, in the light emitting element ED, the third compound may include at least one compound selected from Compound Group 4:

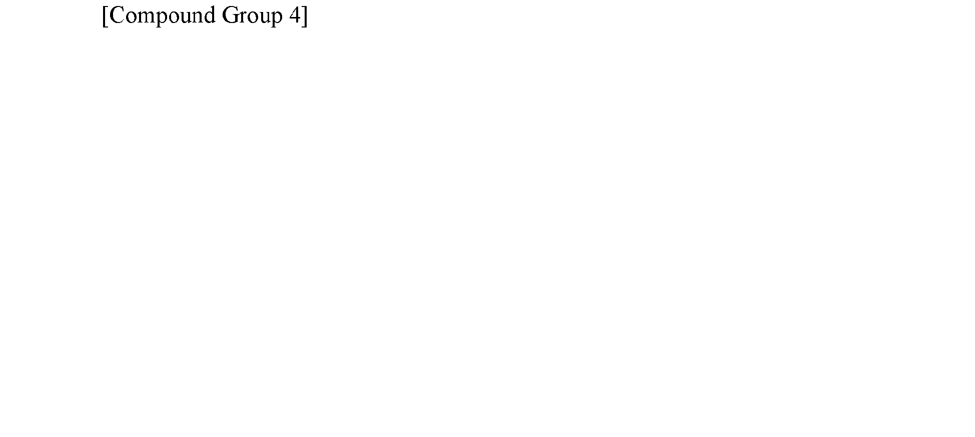

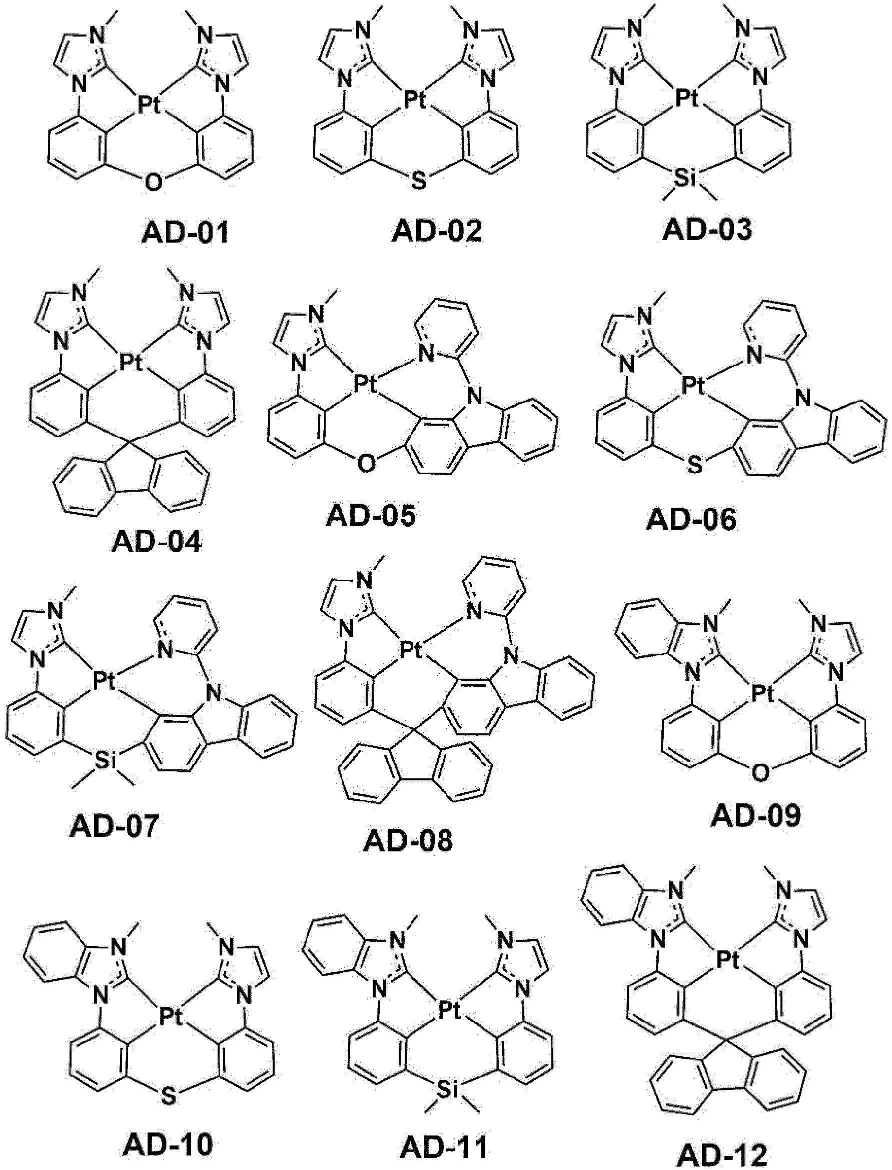

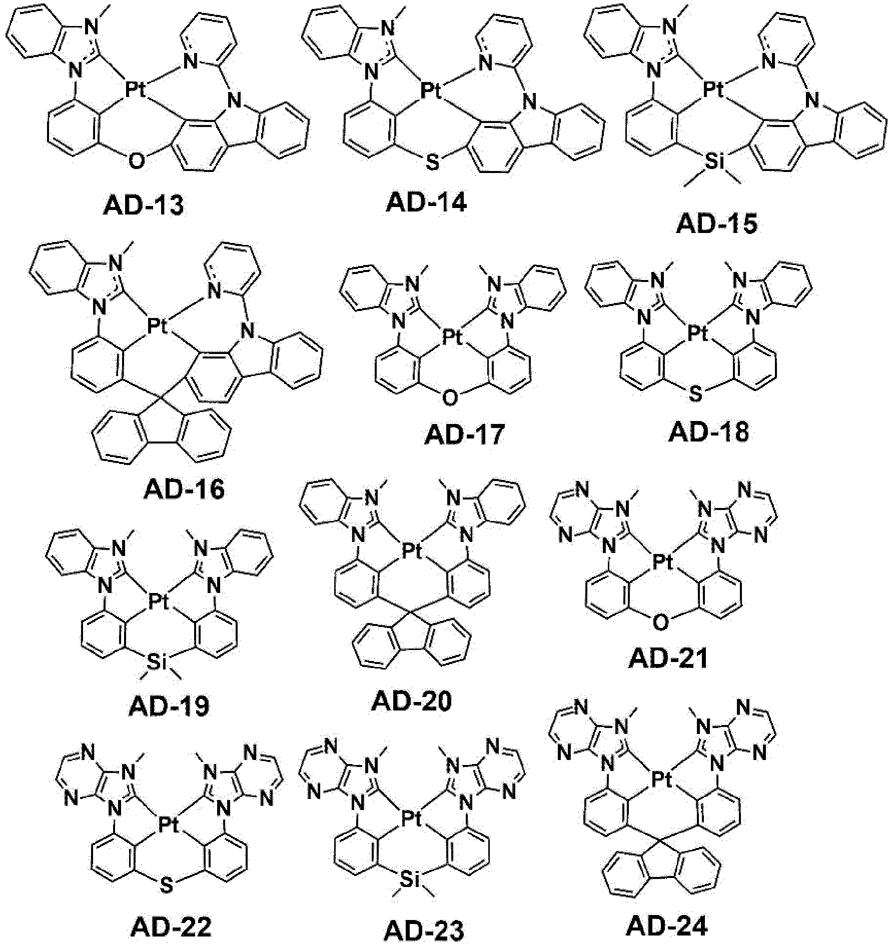

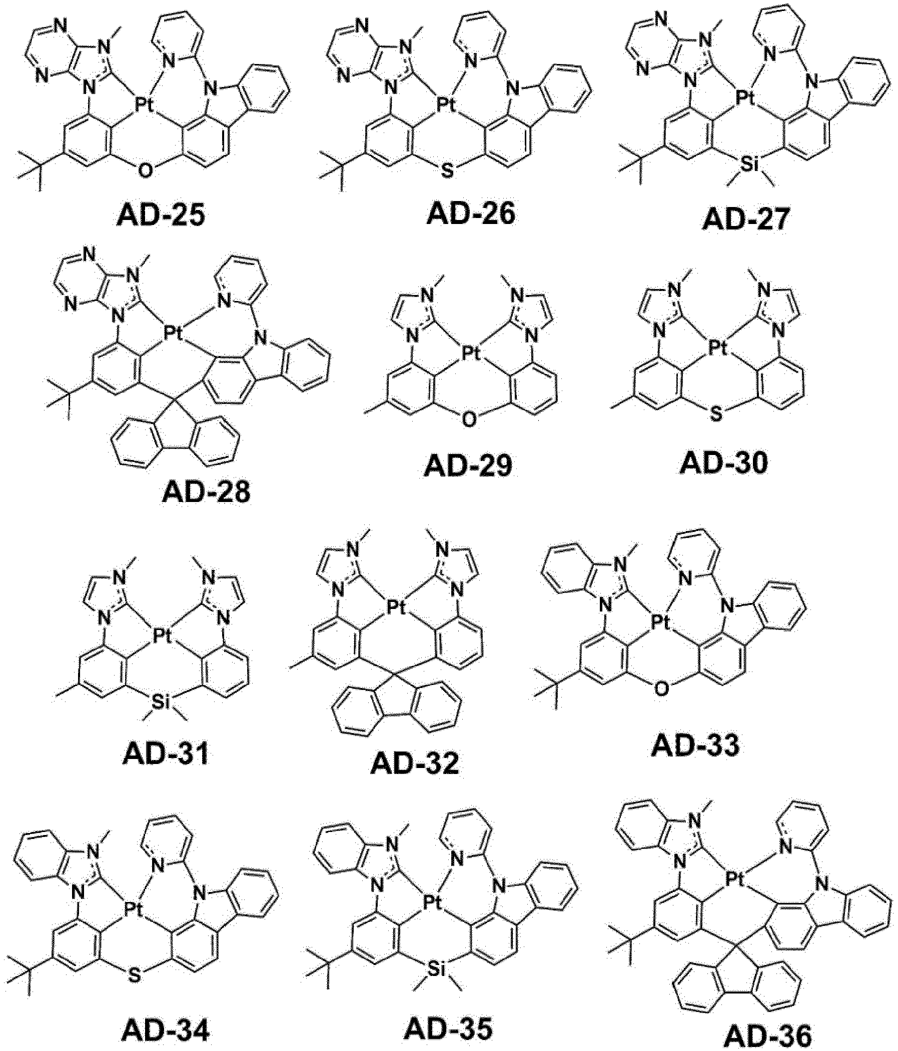

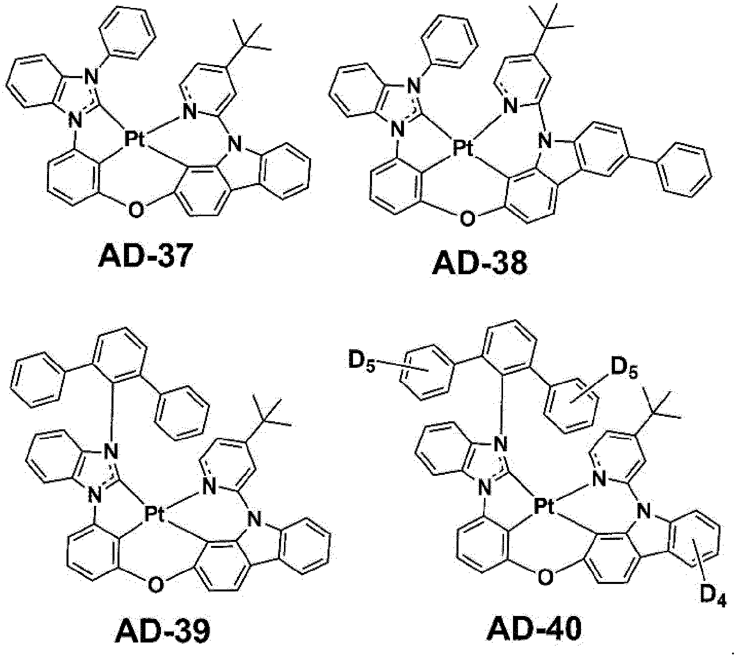

In Compound Group 4, D represents a deuterium atom.

In the light emitting element ED, the emission layer EML may further include an anthracene derivative, a pyrene derivative, a fluoranthene derivative, a chrysene derivative, a dihydrobenzanthracene derivative, or a triphenylene derivative. For example, the emission layer EML may include an anthracene derivative or a pyrene derivative.

In the light emitting element ED according to embodiments as shown in each FIGS. 3 to 7, the emission layer EML may include a host and dopant.

In an embodiment, the emission layer EML may include a compound represented by Formula E-1. The compound represented by Formula E-1 may be used as a fluorescent host material.

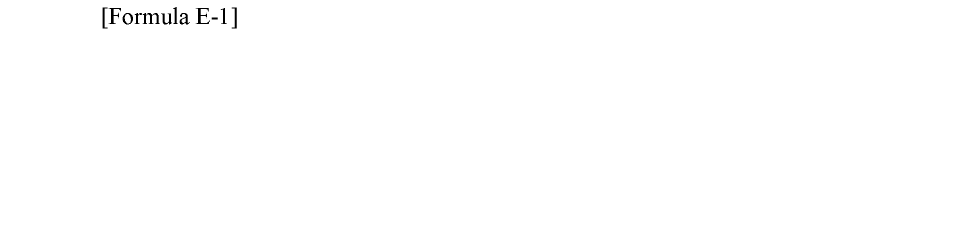

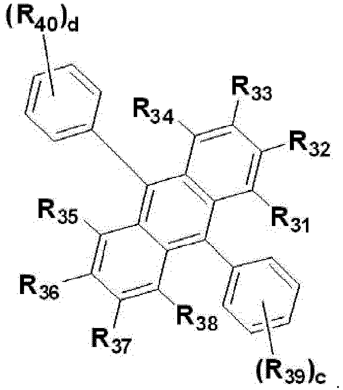

In Formula E-1, R₃₁ to R₄₀ may each independently be a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. For example, R₃₁ to R₄₀ may be bonded to an adjacent group to form a saturated hydrocarbon ring, an unsaturated hydrocarbon ring, a saturated heterocycle, or an unsaturated heterocycle.

In Formula E-1, c and d may each independently be an integer from 0 to 5.

In an embodiment, the compound represented by Formula E-1 may be any compound selected from Compound E1 to Compound E19:

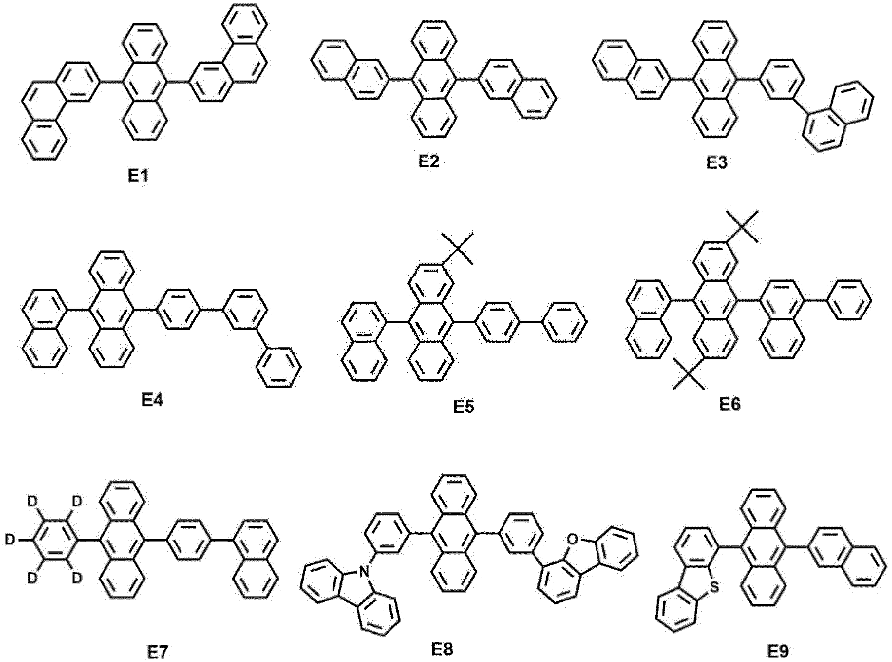

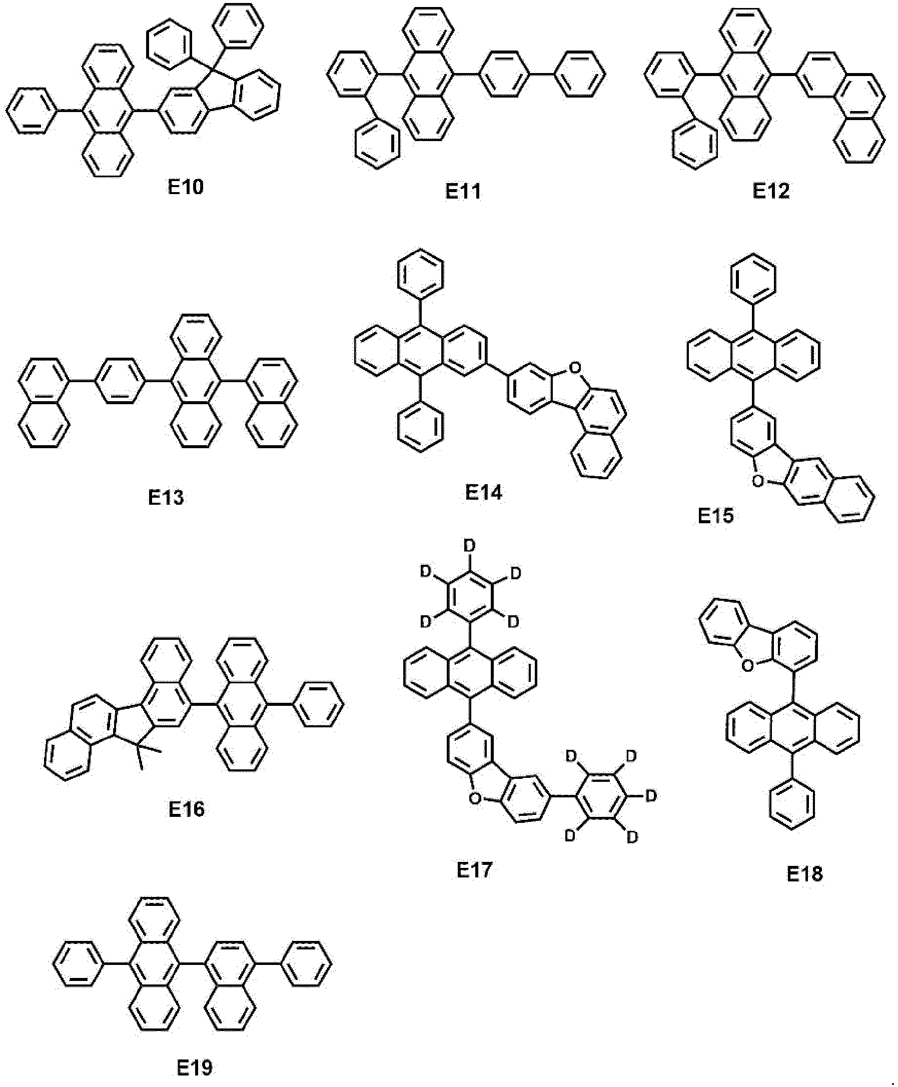

In an embodiment, the emission layer EML may further include a compound represented by Formula E-2a or Formula E-2b. The compound represented by Formula E-2a or Formula E-2b may be used as a host material for phosphorescent element.

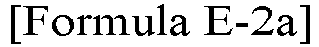

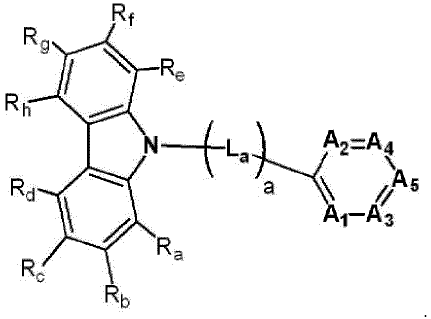

In Formula E-2a, a may be an integer from 0 to 10; and Lₐ may be a direct linkage, a substituted or unsubstituted arylene group having 6 to 30 ring-forming carbon atoms, or a substituted or unsubstituted heteroarylene group having 2 to 30 ring-forming carbon atoms. When a is 2 or greater, multiple Lₐ groups may each independently be a substituted or unsubstituted arylene group having 6 to 30 ring-forming carbon atoms or a substituted or unsubstituted heteroarylene group having 2 to 30 ring-forming carbon atoms.

In Formula E-2a, A₁ to A₅ may each independently be N or C(Rᵢ). In Formula E-2a, Rₐ to Rᵢ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. For example, Rₐ to Rᵢ may be bonded to an adjacent group to form a hydrocarbon ring or a heterocycle containing N, O, *S, etc.,* as a ring-forming atom.

In Formula E-2a, two or three of A₁ to A₅ may each be N, and the remainder of A₁ to A₅ may each independently be C(Rᵢ).

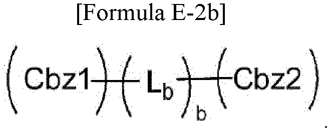

In Formula E-2b, Cbz1 and Cbz2 may each independently be an unsubstituted carbazole group, or a carbazole group substituted with an aryl group having 6 to 30 ring-forming carbon atoms. In Formula E-2b, L_{b} may be a direct linkage, a substituted or unsubstituted arylene group having 6 to 30 ring-forming carbon atoms, or a substituted or unsubstituted heteroarylene group having 2 to 30 ring-forming carbon atoms. In Formula E-2b, b may be an integer from 0 to 10. When b is 2 or more, multiple L_{b} groups may each independently be a substituted or unsubstituted arylene group having 6 to 30 ring-forming carbon atoms or a substituted or unsubstituted heteroarylene group having 2 to 30 ring-forming carbon atoms.

In an embodiment, the compound represented by Formula E-2a or Formula E-2b may be any compound selected from Compound Group E-2. However, the compounds listed in Compound Group E-2 are only examples, and the compound represented by Formula E-2a or Formula E-2b is not limited to Compound Group E-2:

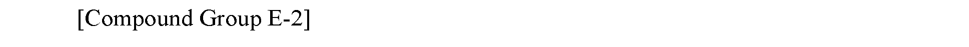

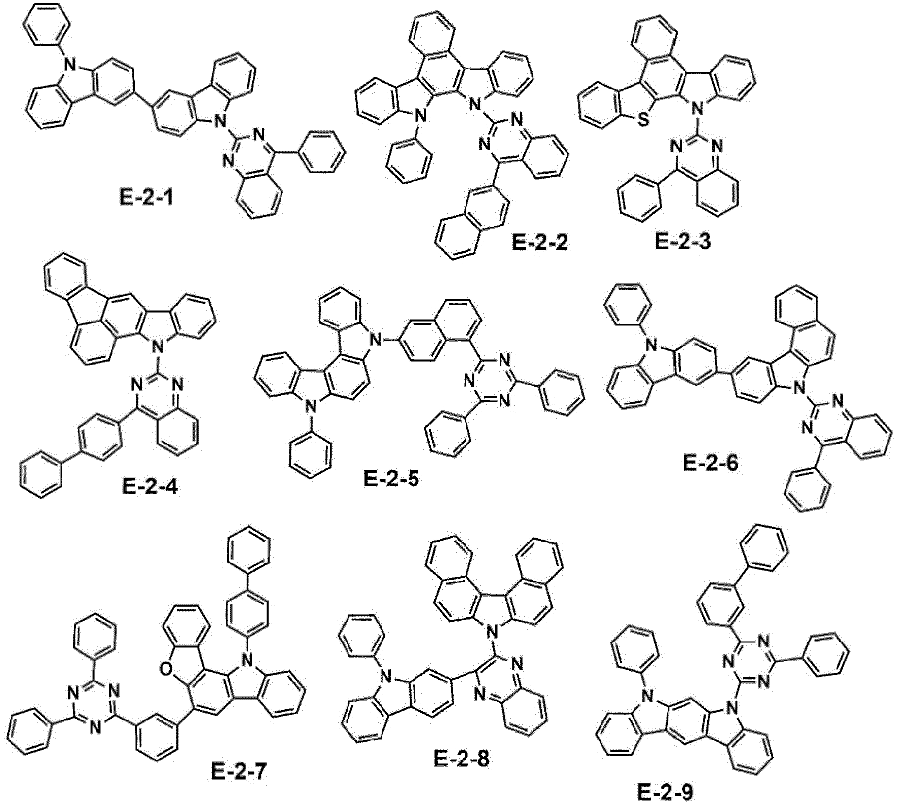

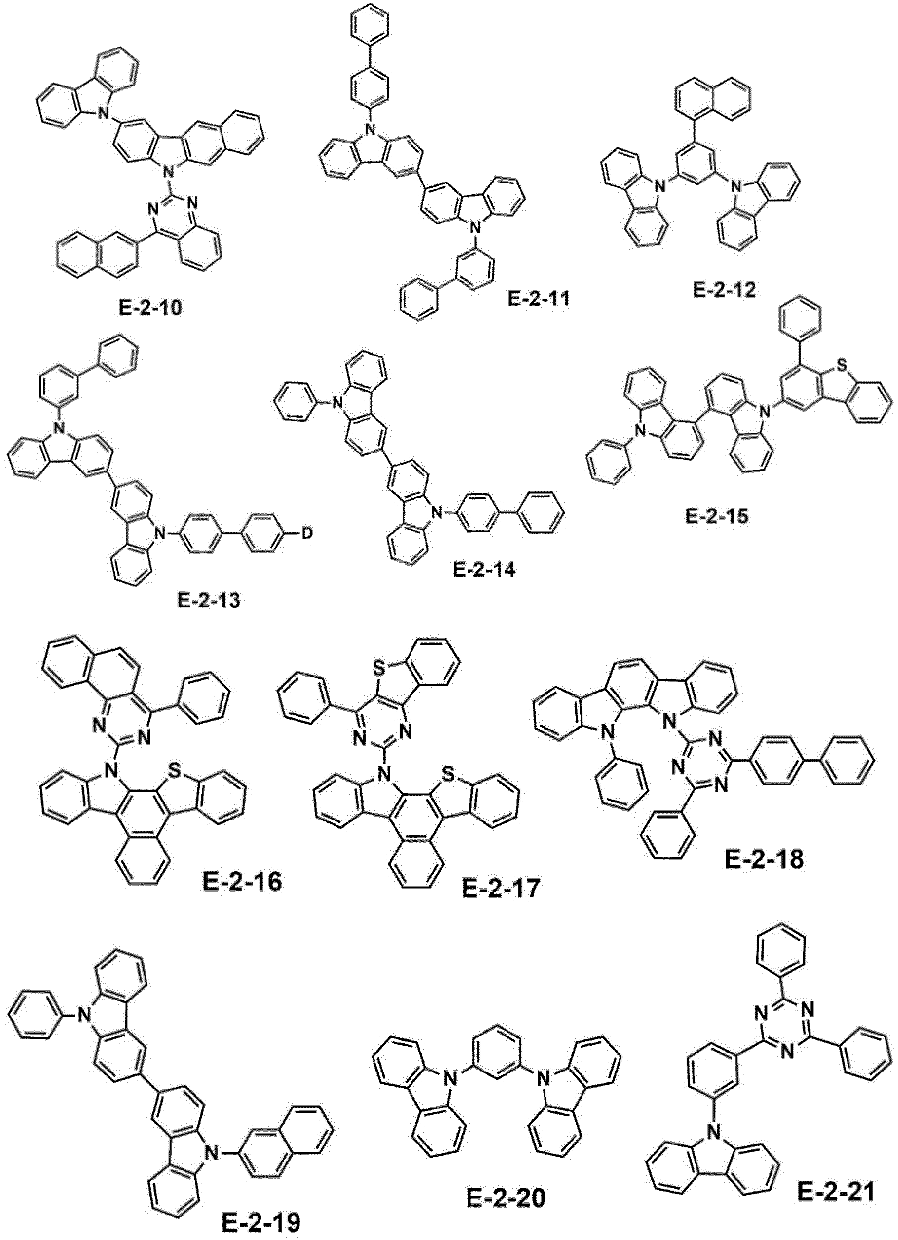

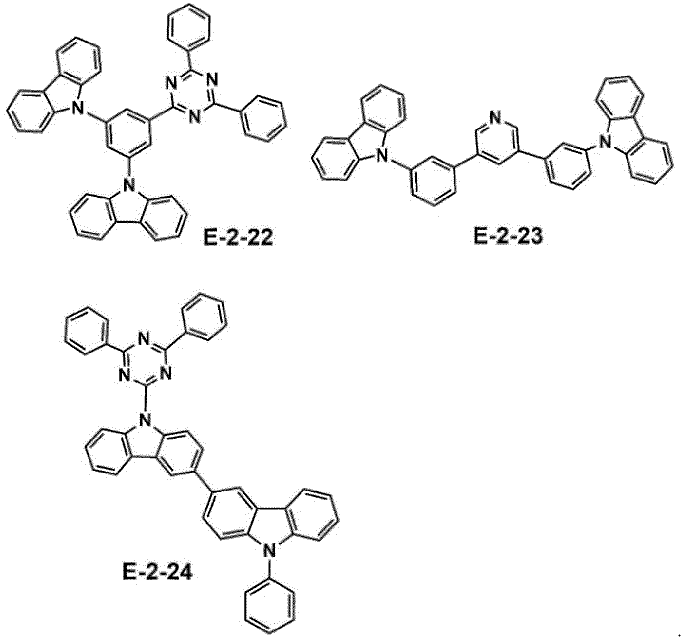

The emission layer EML may further include a material of the related art as a host material. For example, the emission layer EML may include, as a host material, at least one of bis(4-(9H-carbazol-9-yl)phenyl)diphenylsilane (BCPDS), (4-(1-(4-(diphenylamino)phenyl)cyclohexyl)phenyl)diphenyl-phosphine oxide (POPCPA), bis[2-(diphenylphosphino)phenyl]ether oxide (DPEPO), 4,4'-bis(N-carbazolyl)-1,1'-biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), and 1,3,5-tris(1-phenyl-1H-benzo[d]imidazole-2-yl)benzene (TPBi). However, embodiments are not limited thereto. For example, tris(8-hydroxyquinolinato)aluminium (Alq₃), 9,10-di(naphthalen-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetrasiloxane (DPSiO₄), *etc.* may be used as a host material.

In an embodiment, the emission layer EML may include a compound represented by Formula M-a. The compound represented by Formula M-a may be used as a phosphorescent dopant material.

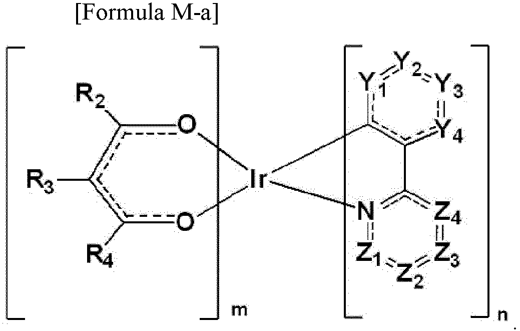

In Formula M-a, Y₁ to Y₄ and Z₁ to Z₄ may each independently be C(R₁) or N; and R₁ to R₄ may each independently be a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. In Formula M-a, m may be 0 or 1, and n may be 2 or 3. In Formula M-a, when m is 0, n may be 3, and when m is 1, n may be 2.

In an embodiment, the compound represented by Formula M-a may be any compound selected from Compound M-a1 to Compound M-a25. However, Compounds M-a1 to M-a25 are only examples, and the compound represented by Formula M-a is not limited to Compounds M-a1 to M-a25:

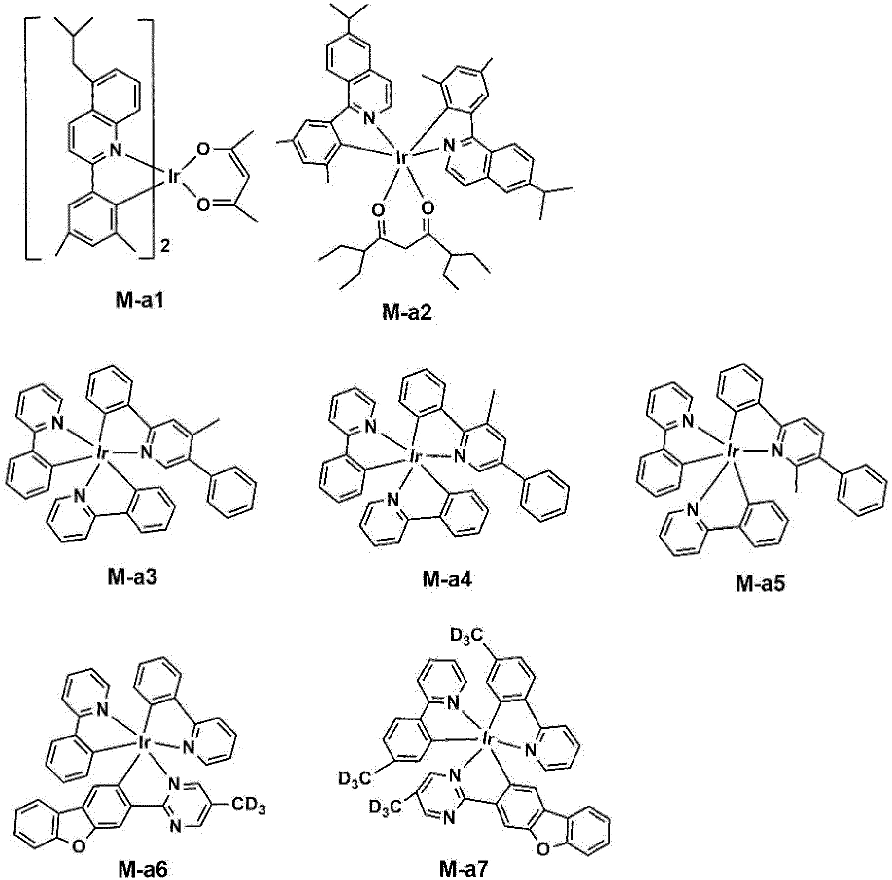

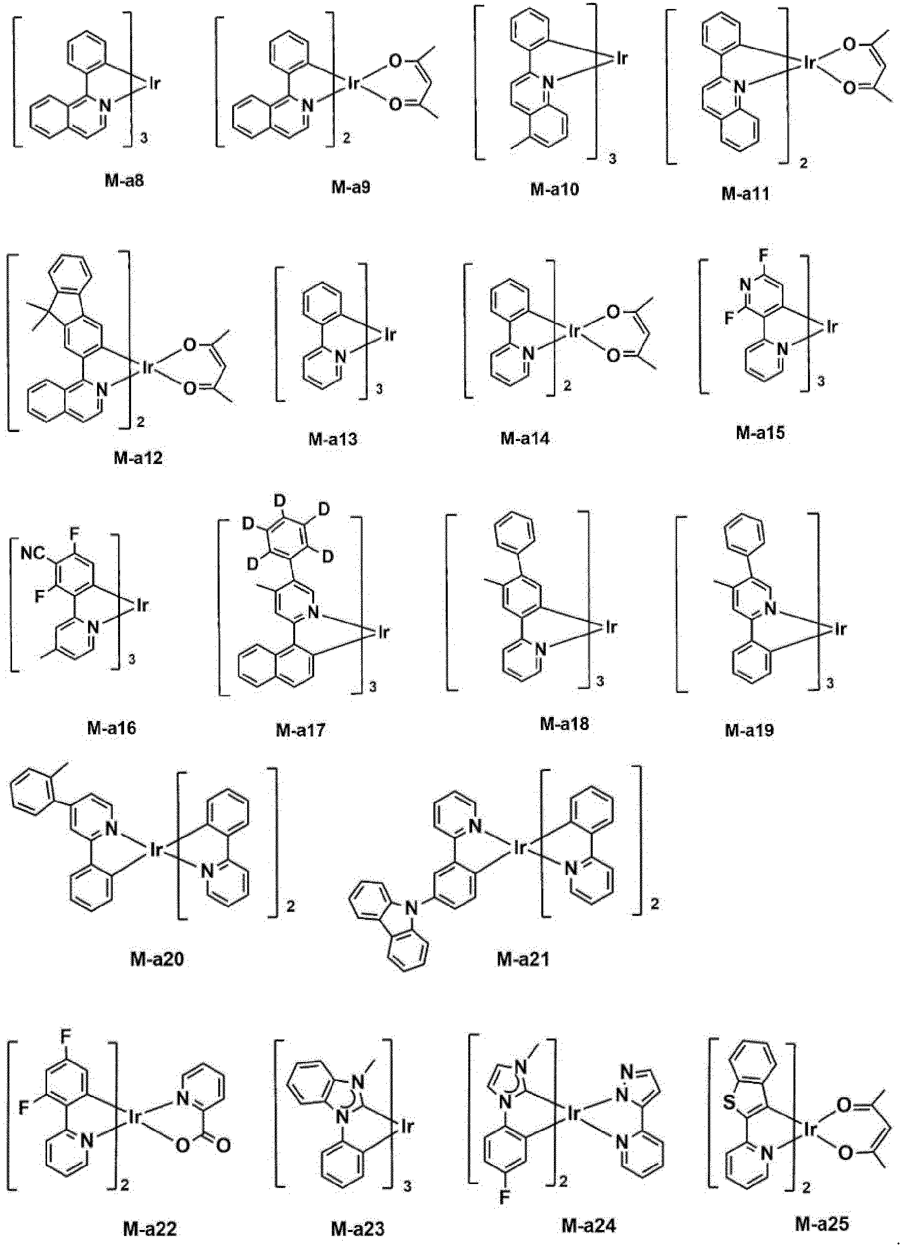

In an embodiment, Compound M-a1 and Compound M-a2 may each be used as a red dopant material, and Compounds M-a3 to M-a5 may each be used as a green dopant material.

In an embodiment, the emission layer EML may include a compound represented by one of Formula F-a to Formula F-c. The compound represented by one of Formula F-a to Formula F-c may be used as a fluorescence dopant material.

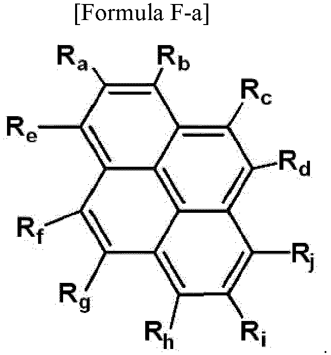

In Formula F-a, two of Rₐ to Rⱼ may each independently be substituted with a group represented by *-NAr₁Ar₂. The remainder of Rₐ to Rⱼ that are not substituted with the group represented by *-NAr₁Ar₂ may each independently be a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In the group represented by +-NAr₁Ar₂, Ar₁ and Ar₂ may each independently be a substituted or unsubstituted aryl group having 6 to 30 ring-forming carbon atoms or a substituted or unsubstituted heteroaryl group having 2 to 30 ring-forming carbon atoms. For example, at least one of Ar₁ and Ar₂ may each independently be a heteroaryl group containing O or S as a ring-forming atom.

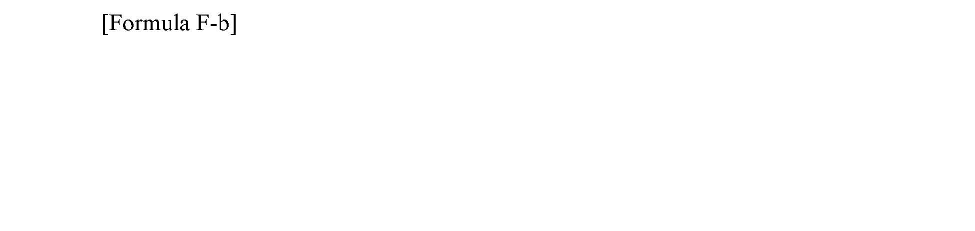

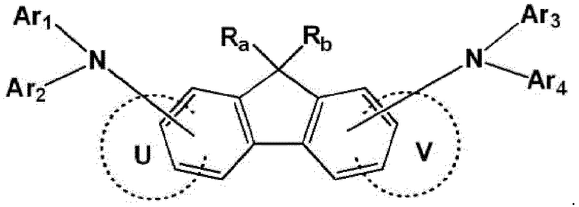

In Formula F-b, Rₐ and R_{b} may each independently be a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring. In Formula F-b, Ar₁ to Ar₄ may each independently be a substituted or unsubstituted aryl group having 6 to 30 ring-forming carbon atoms or a substituted or unsubstituted heteroaryl group having 2 to 30 ring-forming carbon atoms. For example, at least one of Ar₁ to Ar₄ may each independently be a heteroaryl group containing O or S as a ring-forming atom.

In Formula F-b, U and V may each independently be a substituted or unsubstituted hydrocarbon ring having 5 to 30 ring-forming carbon atoms or a substituted or unsubstituted heterocycle having 2 to 30 ring-forming carbon atoms.

In Formula F-b, the number of rings represented by U and V may each independently be 0 or 1. When the number of U or V is 1, a fused ring may be present at a portion respectively indicated by U or V, and when the number of U or V is 0, a fused ring may not be present at the portion respectively indicated by U or V. When the number of U is 0 and the number of V is 1, or when the number of U is 1 and the number of V is 0, a fused ring having a fluorene core of Formula F-b may be a cyclic compound having four rings. When the number of U and V is each 0, a fused ring having a fluorene core of Formula F-b may be a cyclic compound having three rings. When the number of U and V is each 1, a fused ring having a fluorene core of Formula F-b may be a cyclic compound having five rings.

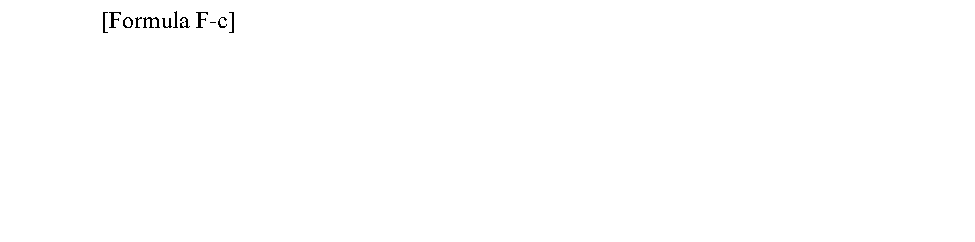

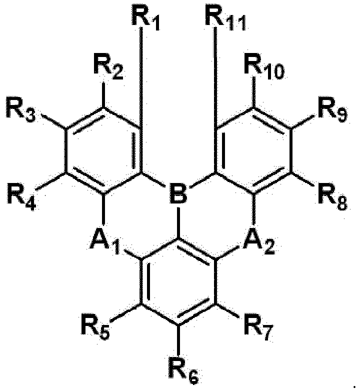

In Formula F-c, A₁ and A₂ may each independently be O, S, Se, or N(Rₘ); and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In Formula F-c, R₁ to R₁₁ may each independently be a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or bonded to an adjacent group to form a ring.

In Formula F-c, A₁ and A₂ may each independently be bonded to a substituent of an adjacent ring to form a fused ring. For example, when A₁ and A₂ are each independently N(Rₘ), A₁ may be bonded to R₄ or R₅ to form a fused ring, and/or A₂ may be bonded to R₇ or R₈ to form a fused ring.

In an embodiment, the emission layer EML may further include, as a dopant material of the related art, a styryl derivative (e.g., 1,4-bis[2-(3-N-ethylcarbazolyl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N-diphenylamino)phenyl)vinyl]biphenyl (DPAVBi), perylene or a derivative thereof (*e.g.,* 2,5,8,11-tetra-t-butylperylene (TBP)), pyrene or a derivative thereof (*e.g.,* 1,1-dipyrene, 1,4-dipyrenylbenzene, 1,4-bis(N,N-diphenylamino)pyrene), *etc.*

The emission layer EML may further include a phosphorescence dopant material of the related art. For example, a metal complex containing iridium (Ir), platinum (Pt), osmium (Os), gold (Au), titanium (Ti), zirconium (Zr), hafnium (Hf), europium (Eu), terbium (Tb), or thulium (Tm) may be used as a phosphorescent dopant. For example, iridium(III) bis(4,6-difluorophenylpyridinato-N, C2')picolinate) (FIrpic), bis(2,4-difluorophenylpyridinato)-tetrakis(1-pyrazolyl)borate iridium(III) (Fir6), or platinum octaethyl porphyrin (PtOEP) may be used as a phosphorescent dopant. However, embodiments are not limited thereto.

In an embodiment, the emission layer EML may include a quantum dot. The quantum dot may include a Group II-VI compound, a Group I-II-VI compound, a Group II-IV-VI compound, a Group I-II-IV-VI compound, a Group II-IV-V compound, a Group III-VI compound, a Group I-III-VI element, a Group III-V compound, a Group III-II-V compound, a Group IV-VI compound, a Group IV element, a Group IV compound, or a combination thereof.

Examples of a Group II-VI compound may include: a binary compound such as CdSe, CdTe, CdS, ZnS, ZnSe, ZnTe, ZnO, HgS, HgSe, HgTe, MgSe, MgS, and a mixture thereof; a ternary compound such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof; a quaternary compound such as HgZnTeS, CdZnSeS, CdZnSeTe, CdZnSTe, CdHgSeS, CdHgSeTe, CdHgSTe, HgZnSeS, HgZnSeTe, and a mixture thereof; and any combination thereof.

In embodiments, a Group II-VI compound may further include a Group I metal and/or a Group IV element. Examples of a Group I-II-VI compound may include CuSnS and CuZnS, and examples of a Group II-IV-VI compound may include ZnSnS and the like. Examples of a Group I-II-IV-VI compound may include quaternary compound such as Cu₂ZnSnS₂, Cu₂ZnSnS₄, Cu₂ZnSnSe₄, Ag₂ZnSnS₂, and a mixture thereof.

Examples of a Group II-IV-V compound may include a ternary compound such as ZnSnP, ZnSnP₂, ZnSnAs₂, ZnGeP₂, ZnGeAs₂, CdSnP₂, and CdGeP₂, and a mixture thereof.

Examples of a Group III-VI compound may include: a binary compound such as GaS, Ga₂S₃, GaSe, Ga₂Se₃, GaTe, InTe, InS, InSe, In₂S₃, and In₂Se₃; a ternary compound such as InGaS₃ and InGaSe₃; and any combination thereof.

Examples of a Group I-III-VI compound may include: a ternary compound such as AgInS, AgInS₂, CuInS, CuInS₂, AgGaS₂, CuGaS₂ CuGaO₂, AgGaO₂, AgAlO₂, and a mixture thereof; a quaternary compound such as AgInGaS₂ and CuInGaS₂; and any combination thereof.

Examples of a Group III-V compound may include: a binary compound such as GaN, GaP, GaAs, GaSb, AlN, AlP, AlAs, AlSb, InN, InP, InAs, InSb, and a mixture thereof; a ternary compound such as GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and a mixture thereof; a quaternary compound such as GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and a mixture thereof; and any combination thereof. In an embodiment, a Group III-V compound may further include a Group II metal. Examples of a Group III-II-V compound may include InZnP, etc.

Examples of a Group IV-VI compound may include: a binary compound such as SnS, SnSe, SnTe, PbS, PbSe, PbTe, and a mixture thereof; a ternary compound such as SnSeS, SnSeTe, SnSTe, PbSeS, PbSeTe, PbSTe, SnPbS, SnPbSe, SnPbTe, and a mixture thereof; a quaternary compound such as SnPbSSe, SnPbSeTe, SnPbSTe, and a mixture thereof; and any combination thereof. Examples of a Group IV element may include Si, Ge, and a mixture thereof. Examples of a Group IV compound may include a binary compound such as SiC, SiGe, and a mixture thereof.

Each element included in a compound, such as a binary compound, a ternary compound, or a quaternary compound, may be present in a particle at a uniform concentration distribution or at a non-uniform concentration distribution. For example, a formula may indicate the elements that are included in a compound, but an elemental ratio of the compound may vary. For example, AgInGaS₂ may indicate AgInₓGa₁₋ₓS₂ (x is a real number between 0 and 1).

In an embodiment, a quantum dot may have a single structure in which the concentration of each element included in the quantum dot is uniform. In another embodiment, a quantum dot may have a core-shell structure in which a quantum dot material surrounds another quantum dot. For example, a material included in the core may be different from a material included in the shell.

The shell of a quantum dot may serve as a protection layer that prevents chemical deformation of a core so as to maintain semiconductor properties, and/or may serve as a charging layer that imparts electrophoretic properties to the quantum dot. The shell may have a single-layered structure or a multilayered structure. An interface between the core and the shell may have a concentration gradient in which the concentration of an element that is present in the shell decreases towards the core.

In embodiments, the quantum dot may have a core/shell structure as described above that includes a core having nano-crystals and a shell surrounding the core. Examples of a shell of a quantum dot may include a metal oxide, a non-metal oxide, a semiconductor compound, or a combination thereof.

Examples of a metal oxide or a non-metal oxide may include: a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO; and a ternary compound such as MgAl₂O₄, CoFe₂O₄, NiFe₂O₄, and CoMn₂O₄, but embodiments are not limited thereto.

Examples of a semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, and the like, but embodiments are not limited thereto.

The quantum dot may have a full width at half maximum (FWHM) of an emission spectrum equal to or less than about 45 nm. For example, the quantum dot may have a FWHM of an emission spectrum equal to or less than about 40 nm. For example, the quantum dot may have a FWHM of an emission spectrum equal to or less than about 30 nm. Color purity or color reproducibility may be improved in any of the above ranges. Light emitted through a quantum dot may be emitted in all directions, so that a wide viewing angle may be improved.

The form of a quantum dot is not particularly limited and may be any form that is used in the related art. For example, a quantum dot may have a spherical shape, a pyramidal shape, a multi-arm shape, or a cubic shape, or the quantum dot may be in the form of nanoparticles, nanotubes, nanowires, nanofibers, nanoplatelets, or the like.

As a size of a quantum dot is adjusted or a ratio of elements in a quantum dot compound is adjusted, an energy band gap may be changed accordingly so that light of various wavelengths may be emitted from a quantum dot emission layer. Therefore, by using quantum dots as described above (for example, using quantum dots of different sizes or having different elemental ratios in a quantum dot compound), a light emitting element that emits light of various wavelengths may be implemented. For example, the size of the quantum dot or a ratio of elements in a quantum dot compound may be adjusted to emit red light, green light, and/or blue light. For example, quantum dots may be configured to emit white light by combining various colors of light.

In the light emitting elements ED according to embodiments as shown in each of FIGS. 3 to 7, the electron transport region ETR may be provided on the emission layer EML. The electron transport region ETR may include at least one of a hole blocking layer HBL, an electron transport layer ETL, and an electron injection layer EIL, but embodiments are not limited thereto.

The electron transport region ETR may have a structure consisting of a layer consisting of a single material, a structure consisting of a layer including different materials, or a structure including multiple layers including different materials.

For example, the electron transport region ETR may have a single layer structure of an electron injection layer EIL or an electron transport layer ETL, or may have a single layer structure formed of an electron injection material and an electron transport material. In an embodiment, the electron transport region ETR may have a single layer structure formed of different materials, or may have a structure in which an electron transport layer ETL/electron injection layer EIL, or a hole blocking layer HBL/electron transport layer ETL/electron injection layer EIL are stacked in its respective stated order from the emission layer EML, but embodiments are not limited thereto. The electron transport region ETR may have a thickness, for example, in a range of about 1,000 Å to about 1,500 Å.

The electron transport region ETR may be formed using various methods such as a vacuum deposition method, a spin coating method, a cast method, a Langmuir-Blodgett (LB) method, an inkjet printing method, a laser printing method, and a laser induced thermal imaging (LITI) method.

In the light emitting element ED according to an embodiment, the electron transport region ETR may include a compound represented by Formula ET-2:

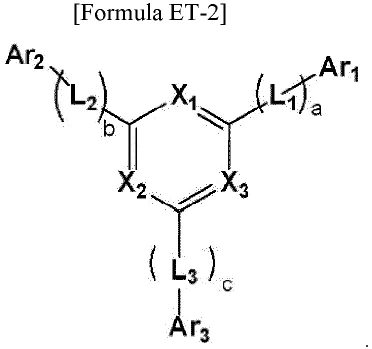

In Formula ET-2, at least one of X₁ to X₃ may be N, and the remainder of X₁ to X₃ may each independently be C(Rₐ). In Formula ET-2, Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. In Formula ET-2, Ar₁ to Ar₃ may each independently be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a to c may each independently be an integer from 0 to 10. In Formula ET-2, L₁ to L₃ may each independently be a direct linkage, a substituted or unsubstituted arylene group having 6 to 30 ring-forming carbon atoms, or a substituted or unsubstituted heteroarylene group having 2 to 30 ring-forming carbon atoms. When a to c are each 2 or more, multiple groups of each of Li to L₃ may each independently be a substituted or unsubstituted arylene group having 6 to 30 ring-forming carbon atoms or a substituted or unsubstituted heteroarylene group having 2 to 30 ring-forming carbon atoms.

The electron transport region ETR may include an anthracene-based compound. However, embodiments are not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), beryllium bis(benzoquinolin-10-olate) (Bebq₂), 9,10-di(naphthalen-2-yl)anthracene (ADN), 1,3-bis[3,5-di(pyridin-3-yl)phenyl]benzene (BmPyPhB), or a mixture thereof.

In an embodiment, the electron transport region ETR may include at least one compound selected from Compound ET1 to Compound ET36:

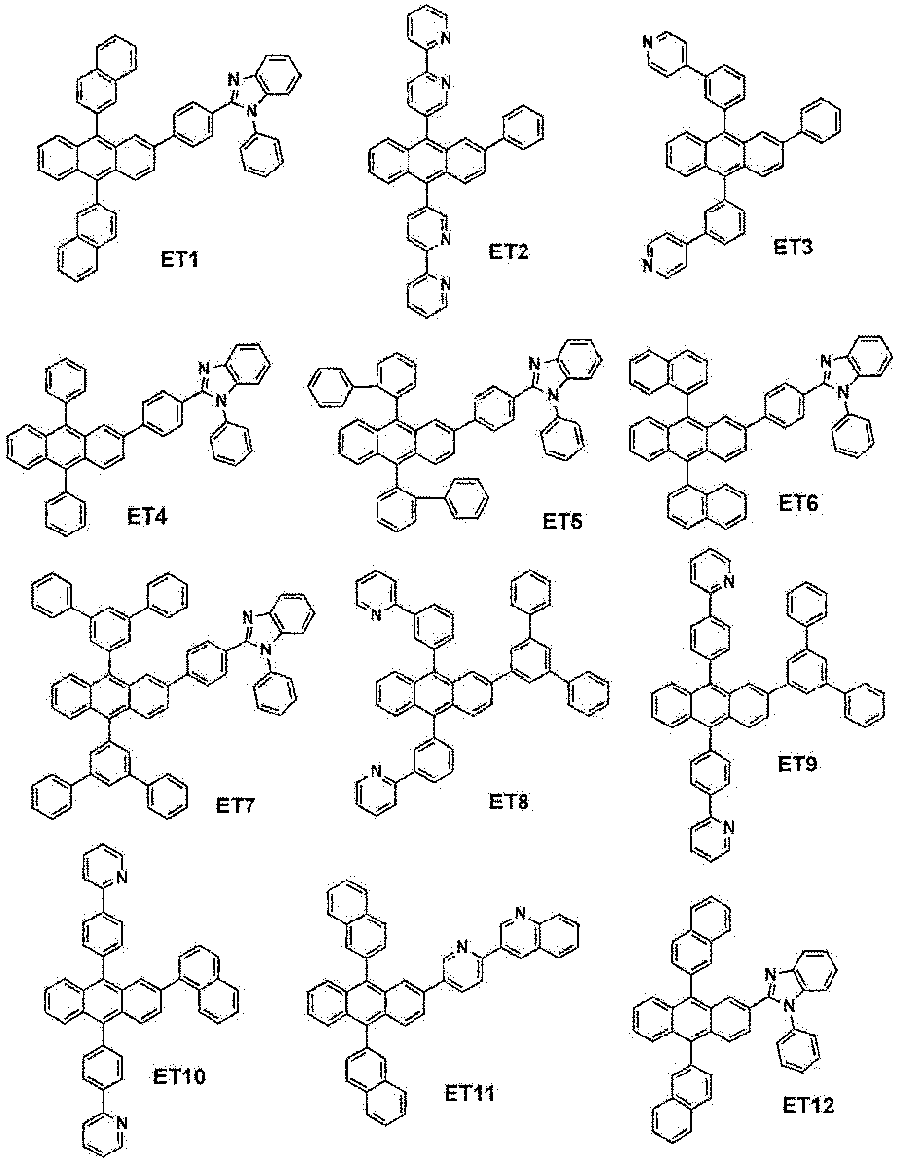

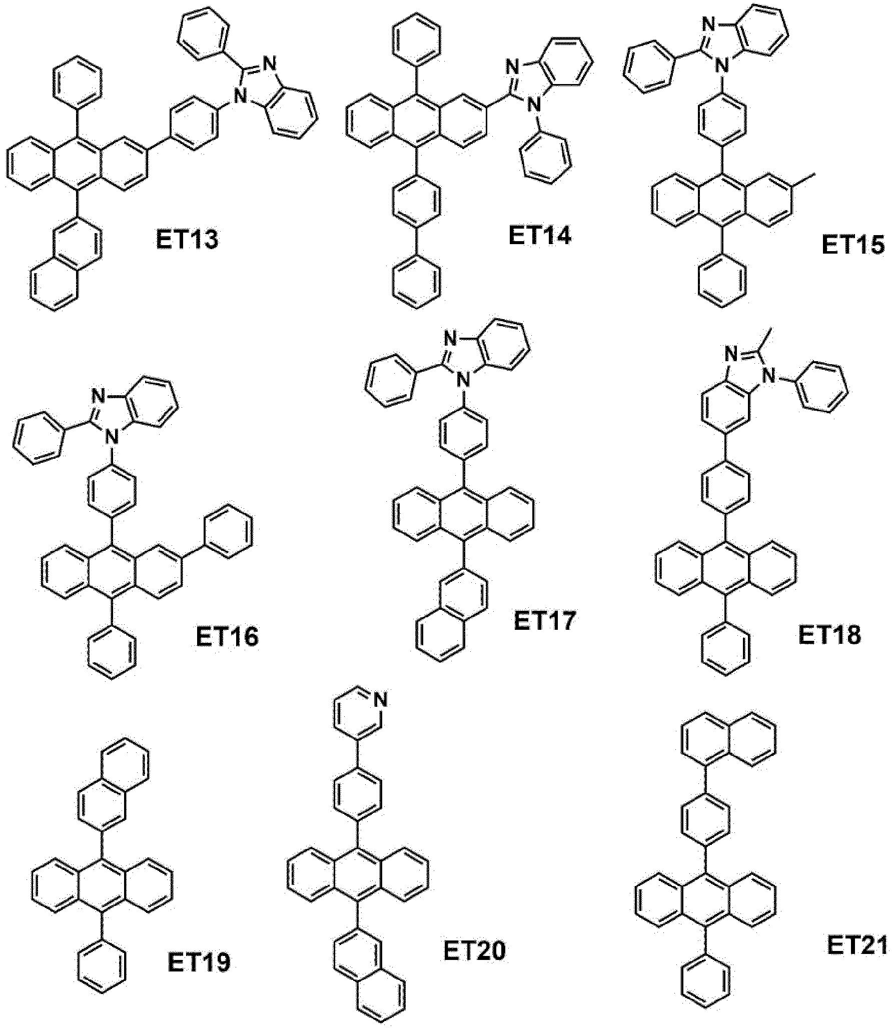

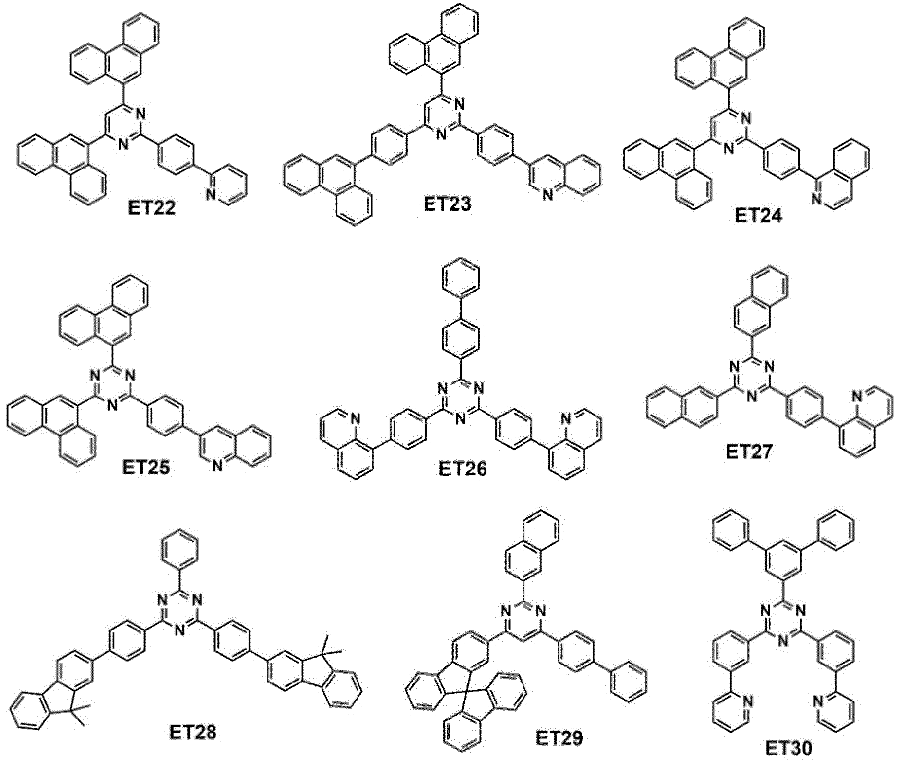

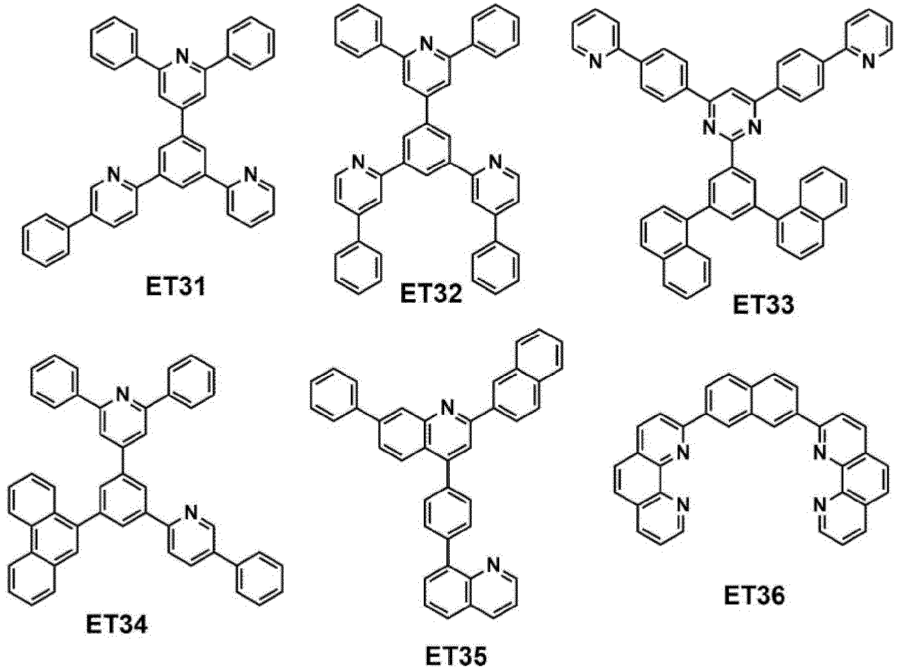

In an embodiment, the electron transport region ETR may include a metal halide such as LiF, NaCl, CsF, RbCl, RbI, CuI, and KI; a lanthanide such as Yb; or a co-deposited material of a metal halide and a lanthanide. For example, the electron transport region ETR may include KI:Yb, RbI:Yb, LiF:Yb, *etc.,* as a co-deposited material. The electron transport region ETR may include a metal oxide such as Li₂O or BaO, or 8-hydroxyl-lithium quinolate (Liq), *etc.,* but embodiments are not limited thereto. The electron transport region ETR may also be formed of a mixture of an electron transport material and an insulating organometallic salt. The organometallic salt may be a material having an energy band gap equal to or greater than about 4 eV. For example, the organometallic salt may include a metal acetate, a metal benzoate, a metal acetoacetate, a metal acetylacetonate, or a metal stearate.

The electron transport region ETR may further include at least one of 2,9-dimethyl-4,7-diphenyl-1,10-phenanthroline (BCP), diphenyl(4-(triphenylsilyl)phenyl)phosphine oxide (TSPO1), and 4,7-diphenyl-1,10-phenanthroline (Bphen) in addition to the above-described materials, but embodiments are not limited thereto.

The electron transport region ETR may include the above-described compounds of the electron transport region in at least one of an electron injection layer EIL, an electron transport layer ETL, and a hole blocking layer HBL.

When the electron transport region ETR includes an electron transport layer ETL, the electron transport layer ETL may have a thickness in a range of about 100 Å to about 1,000 Å. For example, the electron transport layer ETL may have a thickness in a range of about 150 Å to about 500 Å. If the thickness of the electron transport layer ETL satisfies the aforementioned range, satisfactory electron transport characteristics may be obtained without a substantial increase in driving voltage. When the electron transport region ETR includes an electron injection layer EIL, the electron injection layer EIL may have a thickness in a range of about 1 Å to about 100 Å. For example, the electron injection layer EIL may have a thickness in a range of about 3 Å to about 90 Å. If the thickness of the electron injection layer EIL satisfies the above-described range, satisfactory electron injection characteristics may be obtained without a substantial increase in driving voltage.

The second electrode EL2 may be provided on the electron transport region ETR. The second electrode EL2 may be a common electrode. The second electrode EL2 may be a cathode or an anode, but embodiments are not limited thereto. For example, when the first electrode EL1 is an anode, the second electrode EL2 may be a cathode, and when the first electrode EL1 is a cathode, the second electrode EL2 may be an anode.

The second electrode EL2 may be a transmissive electrode, a transflective electrode, or a reflective electrode. When the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), *etc.*

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, a compound thereof, or a mixture thereof (e.g., AgMg, AgYb, or MgYb). In an embodiment, the second electrode EL2 may have a multilayer structure including a reflective film or a transflective film formed of the above-described materials, and a transparent conductive film formed of ITO, IZO, ZnO, ITZO, *etc.* For example, the second electrode EL2 may include the above-described metal materials, combinations of at least two of the above-described metal materials, oxides of the above-described metal materials, or the like.

Although not shown in the drawings, the second electrode EL2 may be electrically connected to an auxiliary electrode. If the second electrode EL2 is electrically connected to an auxiliary electrode, resistance of the second electrode EL2 may decrease.

In an embodiment, the light emitting element ED may further include a capping layer CPL disposed on the second electrode EL2. The capping layer CPL may have a multilayered structure or a single-layered structure.

In an embodiment, the capping layer CPL may include an organic layer or an inorganic layer. For example, when the capping layer CPL contains an inorganic material, the inorganic material may include an alkaline metal compound (e.g., LiF), an alkaline earth metal compound (*e.g.,* MgF₂), SiON, SiNₓ, SiO_{y}, *etc.*

For example, when the capping layer CPL includes an organic material, the organic material may include α-NPD, NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol-9-yl)triphenylamine (TCTA), etc., or may include an epoxy resin, or an acrylate such as methacrylate. However, embodiments are not limited thereto, and the capping layer CPL may include at least one of Compounds P1 to P5:

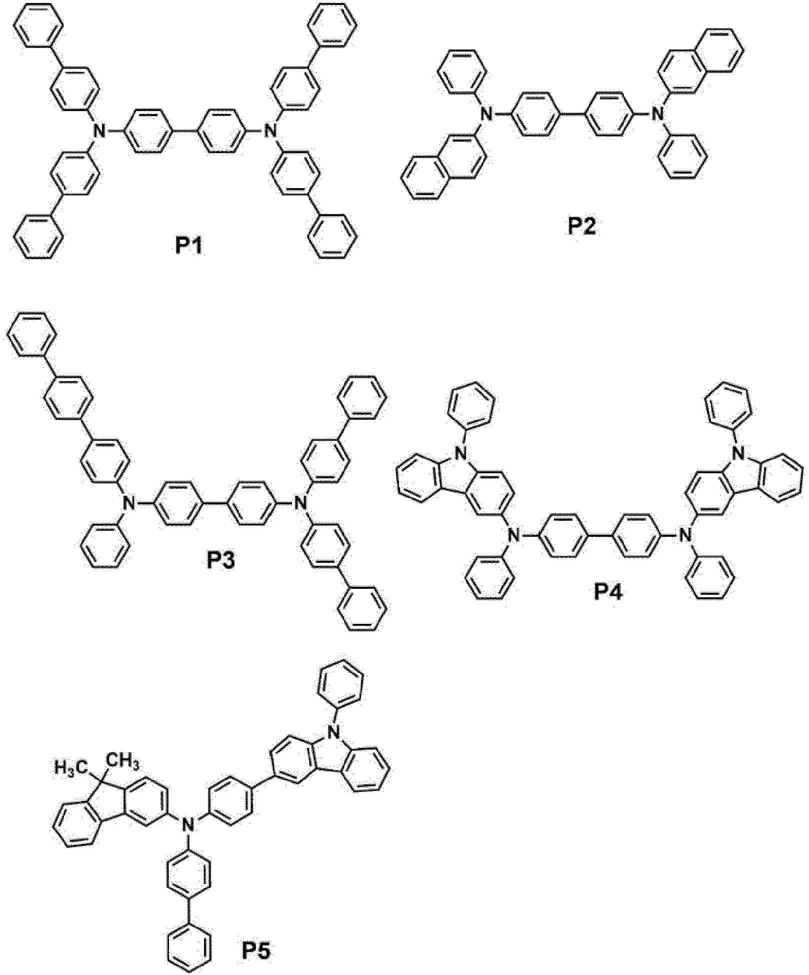

A refractive index of the capping layer CPL may be equal to or greater than about 1.6. For example, the refractive index of the capping layer CPL may be equal to or greater than about 1.6, with respect to light in a wavelength range of about 550 nm to about 660 nm.

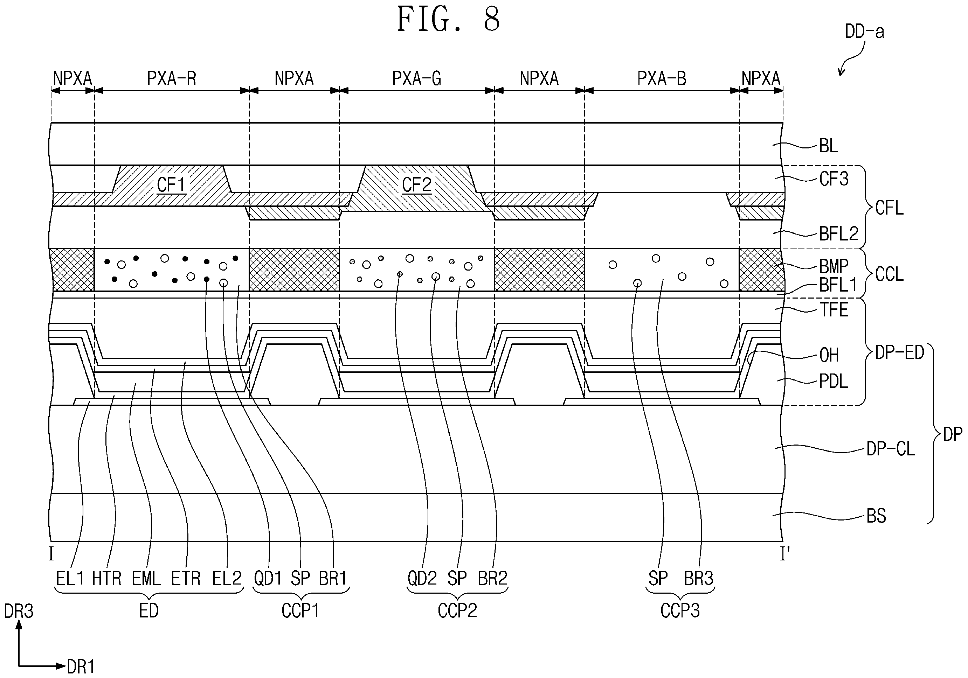

FIGS. 8 to 11 are each a schematic cross-sectional view of a display device according to an embodiment. In the descriptions of the display devices according to embodiments as shown in FIGS. 8 to 11, the features which have been described above with respect to FIGS. 1 to 7 will not be described again, and the differing features will be described.

Referring to FIG. 8, the display device DD-a according to an embodiment may include a display panel DP including a display element layer DP-ED, a light control layer CCL disposed on the display panel DP, and a color filter layer CFL. In an embodiment shown in FIG. 8, the display panel DP may include a base layer BS, a circuit layer DP-CL provided on the base layer BS, and the display element layer DP-ED, and the display element layer DP-ED may include a light emitting element ED.

The light emitting element ED shown in FIG. 8 includes an amine compound according to an embodiment. The light emitting element ED including the amine compound according to an embodiment may exhibit characteristics of low driving voltage, high luminous efficiency, and long life.

The light emitting element ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. In embodiments, a structure of the light emitting element ED shown in FIG. 8 may be same as a structure of light emitting element ED according to one of FIGS. 3 to 7 as described above.

Referring to FIG. 8, the emission layer EML may be disposed in an opening OH defined in a pixel defining film PDL. For example, the emission layer EML, which is separated by the pixel defining film PDL and provided to correspond to each of the light emitting regions PXA-R, PXA-G, and PXA-B, may emit light in a same wavelength range. In the display device DD-a, the emission layer EML may emit blue light. Although not shown in the drawings, in an embodiment, the emission layer EML may be provided as a common layer for all of the light emitting regions PXA-R, PXA-G, and PXA-B.

The light control layer CCL may be disposed on the display panel DP. The light control layer CCL may include a light conversion body. The light conversion body may be a quantum dot, a phosphor, or the like. The light conversion body may convert the wavelength of a provided light and emit the resulting light. For example, the light control layer CCL may be a layer that includes a quantum dot or a layer that includes a phosphor.

The light control layer CCL may include light control parts CCP1, CCP2, and CCP3. The light control parts CCP1, CCP2, and CCP3 may be spaced apart from each other.

Referring to FIG. 8, divided patterns BMP may be disposed between the light control parts CCP1, CCP2, and CCP3, which are spaced apart from each other, but embodiments are not limited thereto. In FIG. 8, it is shown that the divided patterns BMP do not overlap the light control parts CCP1, CCP2, and CCP3, but the edges of the light control parts CCP1, CCP2, and CCP3 may overlap at least a portion of the divided patterns BMP.

The light control layer CCL may include a first light control part CCP1 containing a first quantum dot QD1 that converts first color light provided from the light emitting element ED into second color light, a second light control part CCP2 containing a second quantum dot QD2 that converts the first color light into third color light, and a third light control part CCP3 that transmits the first color light.

In an embodiment, the first light control part CCP1 may provide red light, which is the second color light, and the second light control part CCP2 may provide green light, which is the third color light. The third light control part CCP3 may provide blue light by transmitting the blue light that is the first color light provided from the light emitting element ED. For example, the first quantum dot QD1 may be a red quantum dot, and the second quantum dot QD2 may be a green quantum dot. The quantum dots QD1 and QD2 may each be a quantum dot as described above.

The light control layer CCL may further include a scatterer SP. The first light control part CCP1 may include the first quantum dot QD1 and the scatterer SP, the second light control part CCP2 may include the second quantum dot QD2 and the scatterer SP, and the third light control part CCP3 may not include any quantum dot but may include the scatterer SP.

The scatterer SP may be inorganic particles. For example, the scatterer SP may include at least one of TiO₂, ZnO, Al₂O₃, SiO₂, and hollow silica. The scatterer SP may include one of TiO₂, ZnO, Al₂O₃, SiO₂, and hollow silica, or may be a mixture of at least two materials selected from TiO₂, ZnO, Al₂O₃, SiO₂, and hollow silica.

The first light control part CCP1, the second light control part CCP2, and the third light control part CCP3 may include base resins BR1, BR2, and BR3, in which the quantum dots QD1 and QD2 and the scatterer SP are dispersed. In an embodiment, the first light control part CCP1 may include the first quantum dot QD 1 and the scatterer SP dispersed in a first base resin BR1, the second light control part CCP2 may include the second quantum dot QD2 and the scatterer SP dispersed in a second base resin BR2, and the third light control part CCP3 may include the scatterer SP dispersed in a third base resin BR3.

The base resins BR1, BR2, and BR3 are media in which the quantum dots QD1 and QD2 and the scatterer SP are dispersed, and may include various resin compositions, which may be referred to as a binder. For example, the base resins BR1, BR2, and BR3 may be acrylic-based resins, urethane-based resins, silicone-based resins, epoxy-based resins, *etc.* The base resins BR1, BR2, and BR3 may each be a transparent resin. In an embodiment, the first base resin BR1, the second base resin BR2, and the third base resin BR3 may be the same as or different from each other.

The light control layer CCL may include a barrier layer BFL1. The barrier layer BFL1 may prevent the penetration of moisture and/or oxygen (hereinafter, referred to as 'moisture/oxygen'). The barrier layer BFL1 may block the light control parts CCP1, CCP2, and CCP3 from exposure to moisture/oxygen. The barrier layer BFL1 may cover the light control parts CCP1, CCP2, and CCP3. In an embodiment, the barrier layer BFL2 may be provided between the light control parts CCP1, CCP2, and CCP3, and filters CF1, CF2, and CF3.

The barrier layers BFL1 and BFL2 may each independently include at least one inorganic layer. For example, the barrier layers BFL1 and BFL2 may each independently include an inorganic material. For example, the barrier layers BFL1 and BFL2 may each independently include a silicon nitride, an aluminium nitride, a zirconium nitride, a titanium nitride, a hafnium nitride, a tantalum nitride, a silicon oxide, an aluminium oxide, a titanium oxide, a tin oxide, a cerium oxide, a silicon oxynitride, a metal thin film that secures light transmittance, *etc.* The barrier layers BFL1 and BFL2 may each independently further include an organic film. The barrier layers BFL1 and BFL2 may be formed of a single layer or of multiple layers.

In the display device DD-a, the color filter layer CFL may be disposed on the light control layer CCL. In an embodiment, the color filter layer CFL may be directly disposed on the light control layer CCL. For example, the barrier layer BFL2 may be omitted.

The color filter layer CFL may include filters CF1, CF2, and CF3. The color filter layer CFL may include a first filter CF1 that transmits second color light, a second filter CF2 that transmits third color light, and a third filter CF3 that transmits first color light. For example, the first filter CF1 may be a red filter, the second filter CF2 may be a green filter, and the third filter CF3 may be a blue filter. The filters CF1, CF2, and CF3 may each include a polymeric photosensitive resin and a pigment or dye. The first filter CF1 may include a red pigment or dye, the second filter CF2 may include a green pigment or dye, and the third filter CF3 may include a blue pigment or dye.

However, embodiments are not limited thereto, and the third filter CF3 may not include a pigment or dye. The third filter CF3 may include a polymeric photosensitive resin and may not include a pigment or dye. The third filter CF3 may be transparent. The third filter CF3 may be formed of a transparent photosensitive resin.

In an embodiment, the first filter CF1 and the second filter CF2 may each be a yellow filter. The first filter CF1 and the second filter CF2 may not be provided as separate filters and may be provided as a unitary filter.

Although not shown in the drawings, in an embodiment, the color filter layer CFL may further include a light shielding part (not shown). The light shielding part (not shown) may be a black matrix. The light shielding part (not shown) may include an organic light shielding material or an inorganic light shielding material, each including a black pigment or a black dye. The light shielding part (not shown) may prevent light leakage, and may separate boundaries between adjacent filters CF1, CF2, and CF3. In an embodiment, the light shielding part (not shown) may be formed of a blue filter.

The first filter CF1, the second filter CF2, and the third filter CF3 may be disposed to respectively correspond to the red light emitting region PXA-R, the green light emitting region PXA-G, and the blue light emitting region PXA-B.

A base substrate BL may be disposed on the color filter layer CFL. The base substrate BL may provide a base surface on which the color filter layer CFL, the light control layer CCL, and the like are disposed. The base substrate BL may be a glass substrate, a metal substrate, a plastic substrate, *etc.* However, embodiments are not limited thereto, and the base substrate BL may include an inorganic layer, an organic layer, or a composite material layer. Although not shown in the drawings, in an embodiment, the base substrate BL may be omitted.

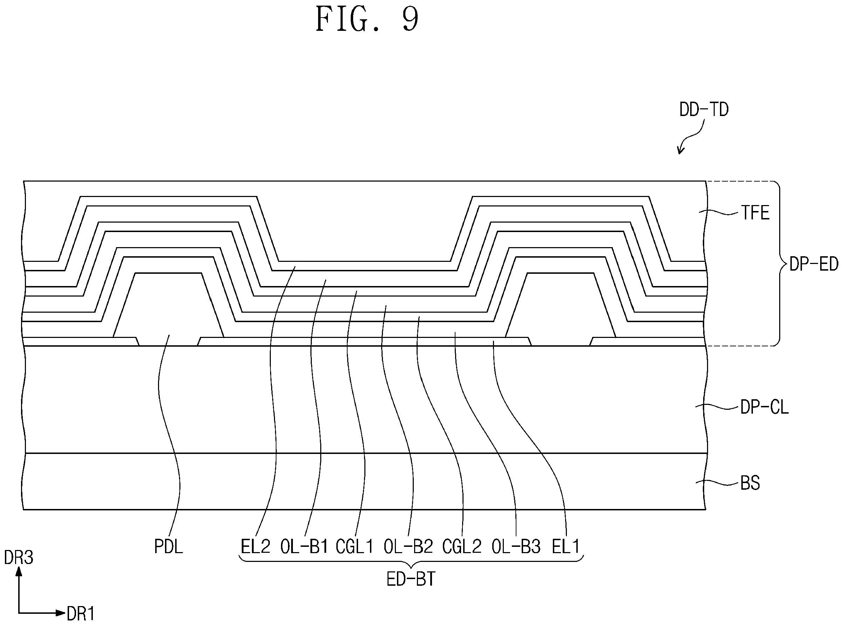

FIG. 9 is a schematic cross-sectional view of a portion of a display device according to an embodiment. In the display device DD-TD according to an embodiment, a light emitting element ED-BT may include light emitting structures OL-B1, OL-B2, and OL-B3. The light emitting element ED-BT may include the amine compound according to an embodiment. The light emitting element ED-BT including the amine compound according to an embodiment may exhibit characteristics of low driving voltage, high luminous efficiency, and long life.

The light emitting element ED-BT may include a first electrode EL1 and a second electrode EL2 that face each other, and light emitting structures OL-B1, OL-B2, and OL-B3 stacked in a thickness direction between the first electrode EL1 and the second electrode EL2. The light emitting structures OL-B1, OL-B2, and OL-B3 may each include a hole transport region HTR, an emission layer EML (FIG. 8), and an electron transport region ETR, which may be disposed in that order between the first electrode EL1 and the second electrode EL2. For example, the light emitting element ED-BT included in the display device DD-TD may be a light emitting element having a tandem structure that includes multiple emission layers.

In an embodiment illustrated in FIG. 9, light emitted from the light emitting structures OL-B1, OL-B2, and OL-B3 may each be blue light. However, embodiments are not limited thereto, and the light emitted from each of the light emitting structures OL-B1, OL-B2, and OL-B3 may have wavelength ranges that are different from each other. For example, the light emitting element ED-BT that includes the light emitting structures OL-B1, OL-B2, and OL-B3, which emit light having wavelength ranges that are different from each other, may emit white light.

Charge generation layers CGL1 and CGL2 may each be disposed between two adjacent light emitting structures among the light emitting structures OL-B1, OL-B2, and OL-B3. Charge generation layers CGL1 and CGL2 may each independently include a p-type charge generation layer and/or an n-type charge generation layer.

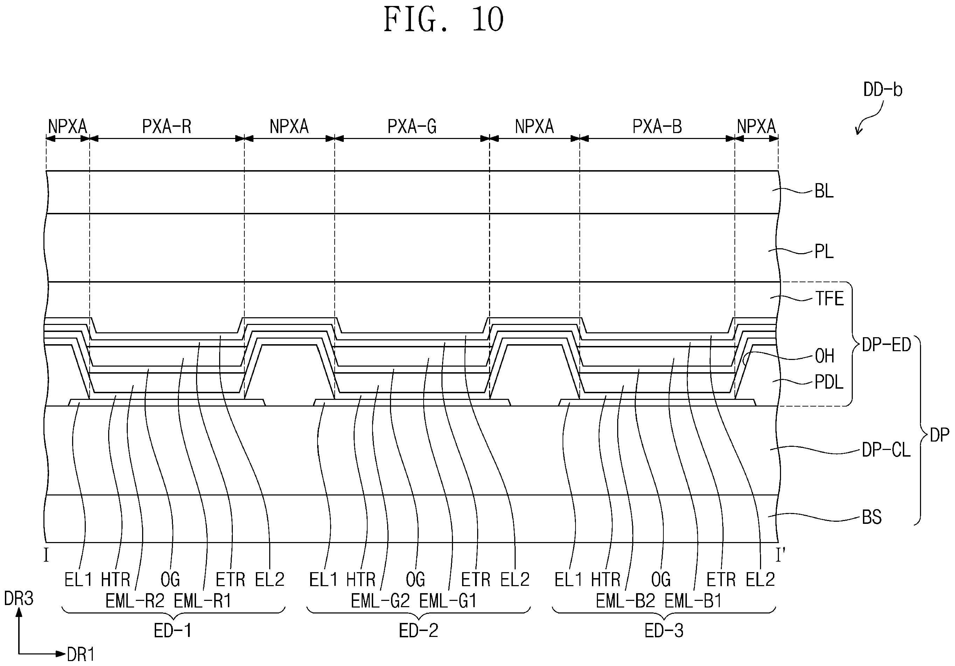

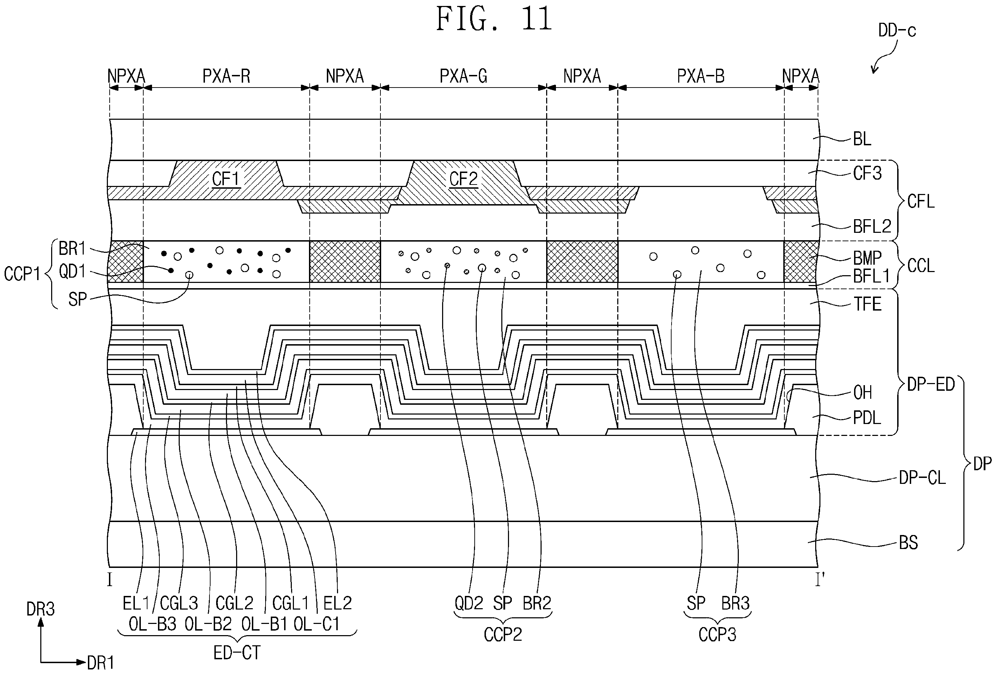

FIG. 10 is a schematic cross-sectional view of a display device DD-b according to an embodiment. FIG. 11 is a schematic cross-sectional view of a display device DD-c according to an embodiment.

Referring to FIG. 10, the display device DD-b according to an embodiment may include light emitting elements ED-1, ED-2, and ED-3 in which two emission layers are stacked. In comparison to the display device DD shown in FIG. 2, the embodiment shown in FIG. 10 is different at least in that the first to third light emitting elements ED-1, ED-2, and ED-3 each include two emission layers that are stacked in a thickness direction. In each of the first to third light emitting elements ED-1, ED-2, and ED-3, the two emission layers may emit light in a same wavelength range. At least one of the first to third light emitting elements ED-1, ED-2, and ED-3 may each independently include an amine compound according to an embodiment, thereby exhibiting characteristics of low driving voltage, high luminous efficiency, and long life.

The first light emitting element ED-1 may include a first red emission layer EML-R1 and a second red emission layer EML-R2. The second light emitting element ED-2 may include a first green emission layer EML-G1 and a second green emission layer EML-G2. The third light emitting element ED-3 may include a first blue emission layer EML-B1 and a second blue emission layer EML-B2. An emission auxiliary part OG may be disposed between the first red emission layer EML-R1 and the second red emission layer EML-R2, between the first green emission layer EML-G1 and the second green emission layer EML-G2, and between the first blue emission layer EML-B1 and the second blue emission layer EML-B2.

The emission auxiliary part OG may have a single-layered structure or a multilayered structure. The emission auxiliary part OG may include a charge generation layer. For example, the emission auxiliary part OG may include an electron transport region, a charge generation layer, and a hole transport region, which may be stacked in that order. The emission auxiliary part OG may be provided as a common layer for the first to third light emitting elements ED-1, ED-2, and ED-3. However, embodiments are not limited thereto, and the emission auxiliary part OG may be provided by being patterned within the openings OH defined in the pixel defining film PDL.

The first red emission layer EML-R1, the first green emission layer EML-G1, and the first blue emission layer EML-B1 may each be disposed between the emission auxiliary part OG and the electron transport region ETR. The second red emission layer EML-R2, the second green emission layer EML-G2, and the second blue emission layer EML-B2 may be disposed between the hole transport region HTR and the emission auxiliary part OG.

For example, the first light emitting element ED-1 may include the first electrode EL1, the hole transport region HTR, the second red emission layer EML-R2, the emission auxiliary part OG, the first red emission layer EML-R1, the electron transport region ETR, and the second electrode EL2, which are stacked in that order. The second light emitting element ED-2 may include the first electrode EL1, the hole transport region HTR, the second green emission layer EML-G2, the emission auxiliary part OG, the first green emission layer EML-G1, the electron transport region ETR, and the second electrode EL2, which are stacked in that order. The third light emitting element ED-3 may include the first electrode EL1, the hole transport region HTR, the second blue emission layer EML-B2, the emission auxiliary part OG, the first blue emission layer EML-B1, the electron transport region ETR, and the second electrode EL2, which are stacked in that order.

An optical auxiliary layer PL may be disposed on the display element layer DP-ED. The optical auxiliary layer PL may include a polarizing layer. The optical auxiliary layer PL may be disposed on the display panel DP and may control light that is reflected at the display panel DP from an external light. Although not shown in the drawings, in an embodiment, the optical auxiliary layer PL may be omitted from the display device DD-b.

In contrast to FIGS. 9 and 10, FIG. 11 shows a display device DD-c that is different at least in that it includes four light emitting structures OL-B1, OL-B2, OL-B3, and OL-C1. A light emitting element ED-CT may include a first electrode EL1 and a second electrode EL2 that face each other, and first to fourth light emitting structures OL-B1, OL-B2, OL-B3, and OL-C1 that are stacked in a thickness direction between the first electrode EL1 and the second electrode EL2. The light emitting element ED-CT may include the amine compound according to an embodiment, thereby exhibiting characteristics of low driving voltage, high luminous efficiency, and long life.

Among the four light emitting structures, the first to third light emitting structures OL-B1, OL-B2, and OL-B3 may each emit blue light, and the fourth light emitting structure OL-C1 may emit green light. However, embodiments are not limited thereto, and the first to fourth light emitting structures OL-B1, OL-B2, OL-B3, and OL-C1 may emit light having wavelength regions that are different from each other.

Light emitting structures OL-C1, OL-B1, OL-B2, and OL-B3 are stacked successively, and the charge generation layer CGL1 is disposed between light emitting structures OL-B1 and OL-C1, the charge generation layer CGL2 is disposed between light emitting structures OL-B1 and OL-B2, and the charge generation layer CGL3 is disposed between light emitting structures OL-B2 and OL-B3. Charge generation layers CGL1, CGL2, and CGL3 may each independently include a p-type charge generation layer and/or an n-type charge generation layer.

In an embodiment, an electronic apparatus may include a display device that includes multiple light emitting elements, and a control part that controls the display device. The electronic apparatus may be a device that is activated by an electrical signal. The electronic apparatus may include display devices according to various embodiments. Examples of an electronic apparatus may include large, medium-sized, and small electronic apparatuses, such as a television set, a monitor, a billboard, a personal computer, a laptop computer, a personal digital terminal, a display device for a vehicle, a game console, a portable electronic apparatus, or a camera. The display device may be a television set, a monitor, a billboard, a personal computer, a laptop computer, a personal digital terminal, a display device for a vehicle, a game console, a portable electronic device, or a camera.

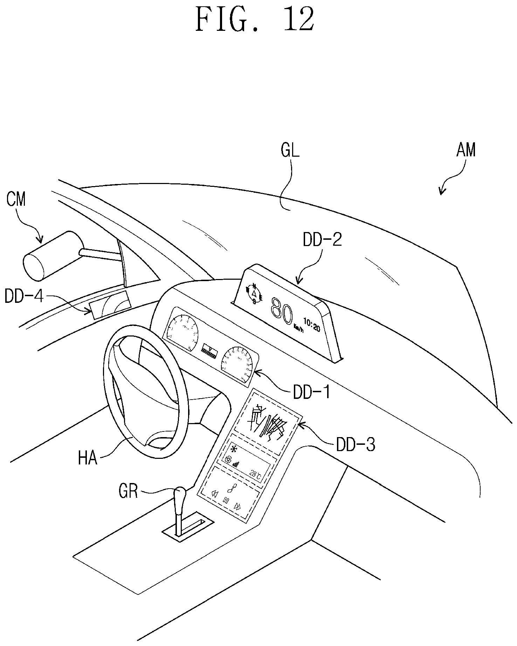

FIG. 12 is a schematic perspective view of a vehicle AM that includes first to fourth display devices DD-1, DD-2, DD-3, and DD-4. At least one of the first to fourth display devices DD-1, DD-2, DD-3, and DD-4 may have a structure according to one of display devices DD, DD-TD, DD-a, DD-b, and DD-c, as described with reference to FIGS. 1, 2, and 8 to 11.

FIG. 12 illustrates a vehicle AM, but this is only an example, and the first to fourth display devices DD-1, DD-2, DD-3, and DD-4 may be disposed in various transportation means such as a bicycle, a motorcycle, a train, a ship, or an airplane. In an embodiment, at least one of the first to fourth display devices DD-1, DD-2, DD-3, and DD-4 having a structure according to one of display devices DD, DD-TD, DD-a, DD-b, and DD-c, may be included in a personal computer, a laptop computer, a personal digital terminal, a game console, a portable electronic device, a television, a monitor, a billboard, or the like. However, these are merely provided as examples, and the display device may be included in other electronic apparatuses.

At least one of the first to fourth display devices DD-1, DD-2, DD-3, and DD-4 may each independently include a light emitting element ED according to an embodiment as described with reference to any of FIGS. 3 to 7. At least one of the first to fourth display devices DD-1, DD-2, DD-3, and DD-4 may each independently include the amine compound according to an embodiment, thereby exhibiting excellent display efficiency and display service life.

Referring to FIG. 12, the vehicle AM may include a steering wheel HA and a gearshift GR for operating the vehicle AM. The vehicle AM may include a front window GL that is disposed so as to face the driver.

The first display device DD-1 may be disposed in a first region that overlaps the steering wheel HA. For example, the first display device DD-1 may be a digital cluster that displays first information of the vehicle AM. The first information may include a first scale that indicates a driving speed of the vehicle AM, a second scale that indicates an engine speed (for example, as revolutions per minute (RPM)), an image that represents a fuel gauge, *etc.* The first scale and the second scale may each be represented by a digital image.

The second display device DD-2 may be disposed in a second region facing the driver's seat that overlaps the front window GL. The driver's seat may be a seat where the steering wheel HA is disposed. For example, the second display device DD-2 may be a head up display (HUD) that shows second information of the vehicle AM. The second display device DD-2 may be optically transparent. The second information may include digital numbers that indicate a driving speed and may further include information such as the current time. Although not shown in the drawings, in an embodiment, the second information of the second display device DD-2 may be displayed by being projected onto the front window GL.

The third display device DD-3 may be disposed in a third region that is adjacent to the gearshift GR. For example, the third display device DD-3 may be disposed between the driver's seat and the passenger seat and may be a center information display (CID) for the vehicle AM that displays third information. The passenger seat may be a seat that is spaced apart from the driver's seat, and the gearshift GR may be disposed between the driver's seat and the passenger seat. The third information may include information about traffic (e.g., navigation information), about music or radio that is playing, about a video (or an image) that is displayed, about temperatures inside the vehicle AM, *etc.*

The fourth display device DD-4 may be disposed in a fourth region that is spaced apart from the steering wheel HA and the gearshift GR and adjacent to a side of the vehicle AM. For example, the fourth display device DD-4 may be a digital side-view mirror that displays fourth information. The fourth display device DD-4 may display an image that is external to the vehicle AM, which may be taken by a camera module CM that is disposed on the exterior of the vehicle AM. The fourth information may include an exterior image of the vehicle AM.

The first to fourth information as described above are only provided as examples, and the first to fourth display devices DD-1, DD-2, DD-3, and DD-4 may further display information about the interior and exterior of the vehicle AM. The first to fourth information may include information that is different from each other. However, embodiments are not limited thereto, and a portion of the first to fourth information may include a same information.

Hereinafter, an amine compound according to an embodiment and a light emitting element according to an embodiment will be described in detail with reference to the Examples and the Comparative Examples. The Examples described below are only provided to assist in understanding the disclosure, and the scope thereof is not limited thereto.

### [Examples]

### 1. Synthesis of amine compounds according to Examples

A process of synthesizing amine compounds according to embodiments will be described in detail by describing synthesis methods for Compounds 1, 13, 17, 21, 41, 53, 61, 81, 93, 105, 109, and 126 as Examples. In the following descriptions, a process of synthesizing amine compounds, which will be described hereinafter, is provided as examples, and the process of synthesizing compounds according to an embodiment is not limited to the Examples below.

### (1) Synthesis of Compound 1

Compound 1 according to an embodiment may be synthesized by, for example, a process according to Reaction Scheme 1 below.

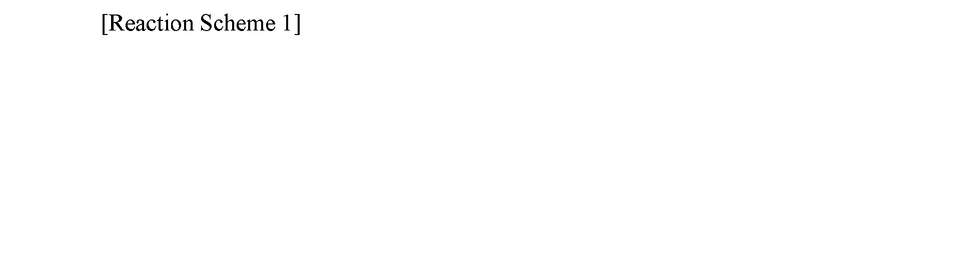

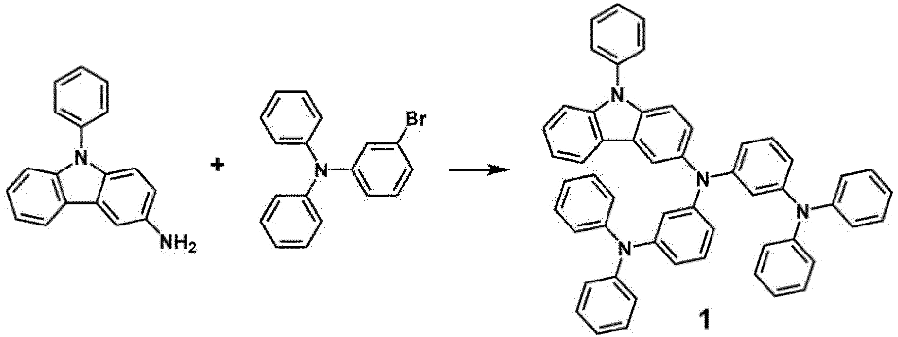

### <Synthesis of Compound 1>

9-phenyl-9H-carbazol-3-amine (2.58 g, 10 mmol), 3-bromo-N,N-diphenylaniline (7.13 g, 22 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over magnesium sulfate (MgSO₄), the solvent was evaporated, and the resulting residue was separated and purified through silica gel chromatography to obtain 5.22 g of Compound 1 (yield: 70%). The obtained compound was determined through LC-MS. C₅₄H₄₀N₄ : M+ 744.9

### (2) Synthesis of Compound 13

Compound 13 according to an embodiment may be synthesized by, for example, a process according to Reaction Scheme 2 below.

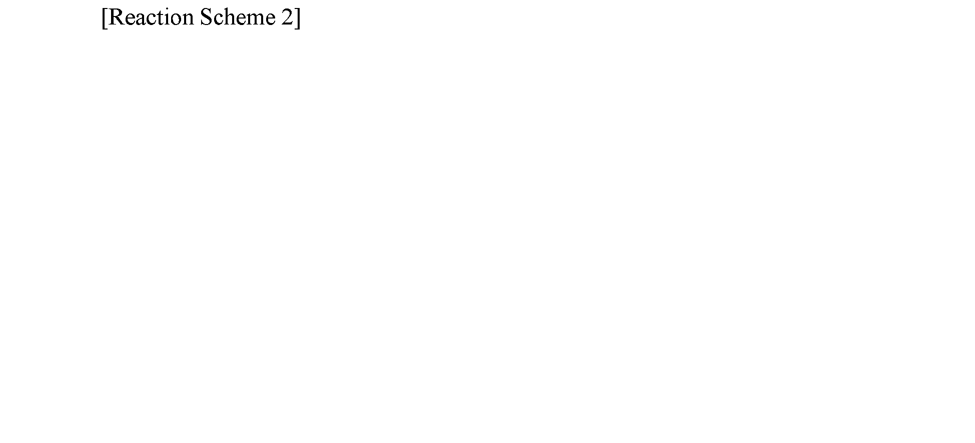

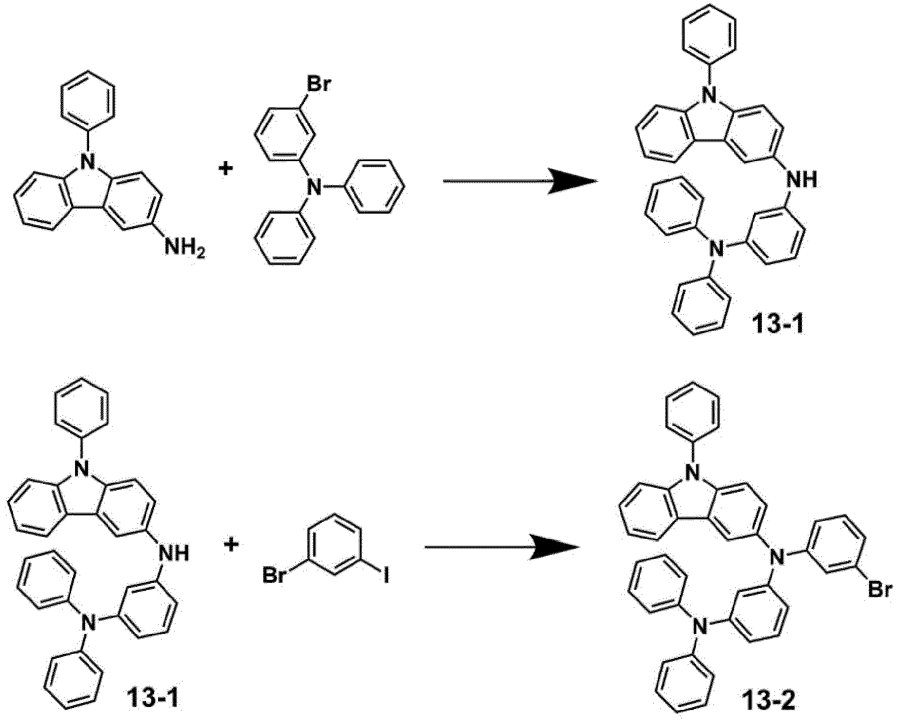

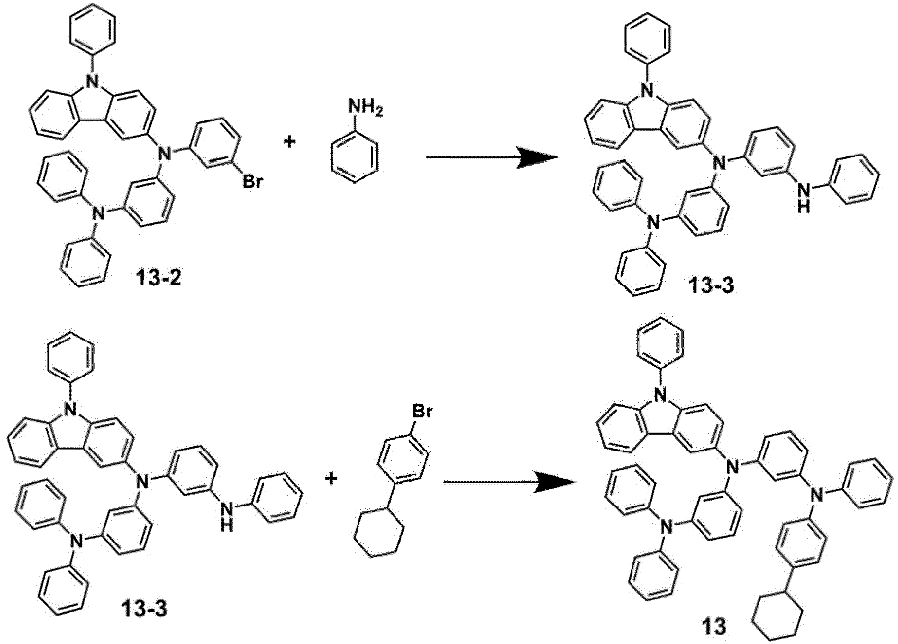

### <Synthesis of Intermediate 13-1>

9-phenyl-9H-carbazol-3-amine (3.10 g, 12 mmol), 3-bromo-N,N-diphenylaniline (3.24 g, 10 mmol), Pd₂(dba)₃ (tris(dibenzylideneacetone)dipalladium(0)) (0.46 g, 0.5 mmol), P(t-Bu)₃ (0.24 g, 1.19 mmol), and sodium tert-butoxide (2.88 g, 30 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 2.91 g of Intermediate 13-1 (yield: 58%). The obtained compound was determined through LC-MS. C₃₆H₂₇N₃: M+ 501.6

### <Synthesis of Intermediate 13-2>

Intermediate 13-1 (5.02 g, 10 mmol), 1-bromo-3-iodobenzene (2.82 g, 10 mmol), Pd₂(dba)₃ (tris(dibenzylideneacetone)dipalladium(0)) (0.46 g, 0.5 mmol), P(t-Bu)₃ (0.24 g, 1.19 mmol), and sodium tert-butoxide (2.88 g, 30 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 3.29g of Intermediate 13-2 (yield: 50%). The obtained compound was determined through LC-MS. C₄₂H₃₀BrN₃: M+ 656.6

### <Synthesis of Intermediate 13-3>

Intermediate 13-2 (6.57 g, 10 mmol), aniline (1.21 g, 13 mmol), Pd₂(dba)₃ (tris(dibenzylideneacetone)dipalladium(0)) (0.46 g, 0.5 mmol), P(t-Bu)₃ (0.24 g, 1.19 mmol), and sodium tert-butoxide (2.88 g, 30 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 4.68 g of Intermediate 13-3 (yield: 70%). The obtained compound was determined through LC-MS. C₄₈H₃₆N₄: M+ 668.8

### <Synthesis of Compound 13>

Intermediate 13-3 (6.69 g, 10 mmol), 1-bromo-4-cyclohexylbenzene (2.39 g, 10 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 6.20 g of Compound 13 (yield: 75%). The obtained compound was determined through LC-MS. C₆₀H₅₀N₄: M+ 827.0

### (3) Synthesis of Compound 17

Compound 17 according to an embodiment may be synthesized by, for example, a process according to Reaction Scheme 3 below.

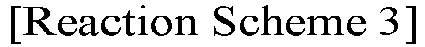

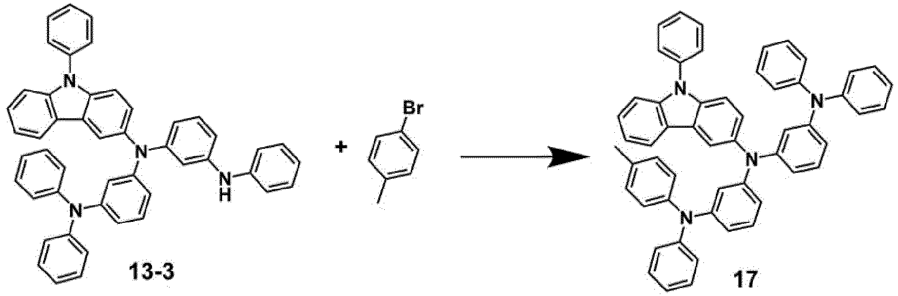

### <Synthesis of Compound 17>

Compound 17 was synthesized in the same manner as in the synthesis of Compound 13, except that 1-bromo-4-methylbenzene was used instead of 1-bromo-4-cyclohexylbenzene. The obtained compound was determined through LC-MS. C₅₅H₄₂N₄: M+ 758.9

### (4) Synthesis of Compound 21

Compound 21 according to an embodiment may be synthesized by, for example, a process according to Reaction Scheme 4 below.

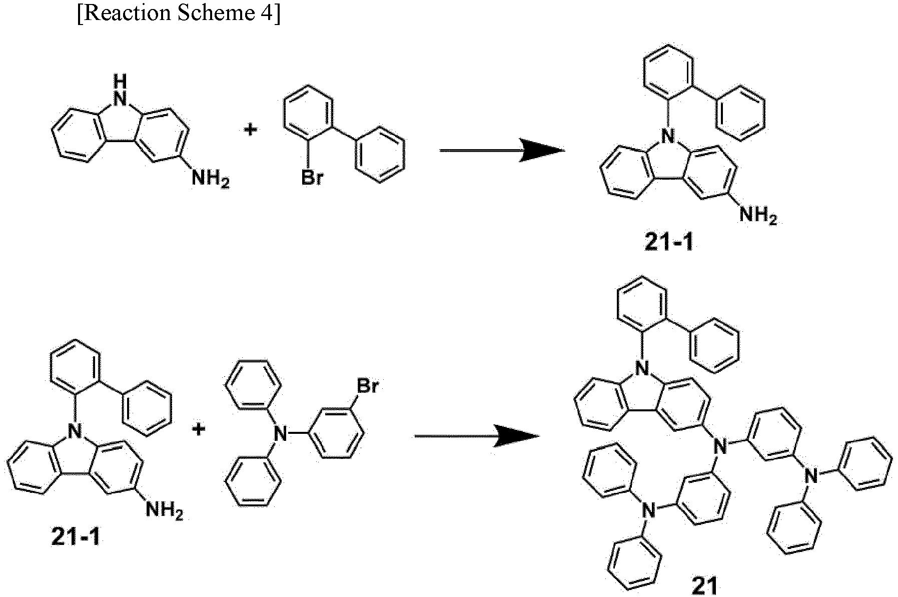

### <Synthesis of Intermediate 21-1>

9H-carbazol-3-amine (2.84 g, 15.59 mmol), 2-bromo-1,1'-biphenyl (2.80 g, 12.0 mmol), CuI (0.1 g, 0.5 mmol), 1,10-phenathroline (0.09 g, 0.5 mmol), and K₂CO₃ (4.14 g, 30.0 mmol) were dissolved in 60 ml of dimethylformamide (DMF) and stirred at 150 °C for 16 hours. The reaction solution was cooled to room temperature and extracted three times with 60 mL of water and 60 mL of diethyl ether. The obtained organic layer was dried over MgSO₄, the solvent was evaporated, and the resulting residue was separated and purified through silica gel chromatography to obtain 2.34 g of Intermediate 21-1 (yield: 70%). The obtained compound was determined through LC-MS. C₂₄H₁₈N₂: M+ 334.4

### <Synthesis of Compound 21>

Intermediate 21-1 (3.34 g, 10 mmol), 3-bromo-N,N-diphenylaniline (7.13 g, 22 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 5.75 g of Compound 21 (yield: 70%). The obtained compound was determined through LC-MS. C₆₀H₄₄N₄: M+ 821.0

### (5) Synthesis of Compound 41

Compound 41 according to an embodiment may be synthesized by, for example, a process according to Reaction Scheme 5 below.

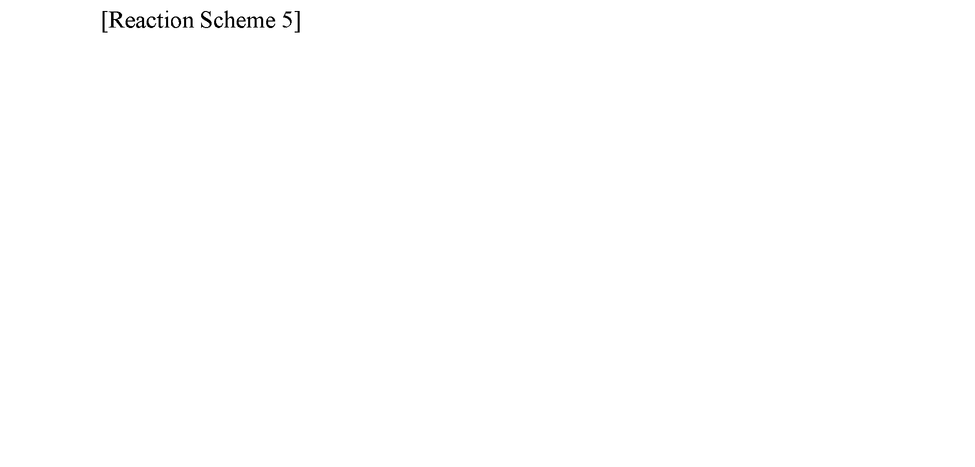

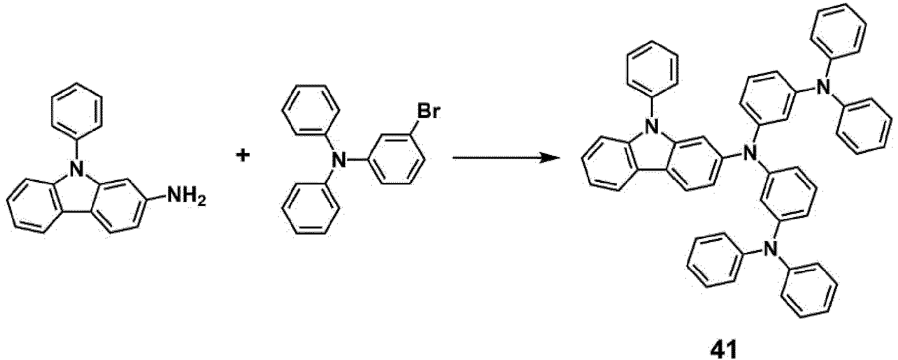

### <Synthesis of Compound 41>

Compound 41 was synthesized in the same manner as in the synthesis of Compound 1, except that 9-phenyl-9H-carbazol-2-amine was used instead of 9-phenyl-9H-carbazol-3-amine. The obtained compound was determined through LC-MS. C₅₄H₄₀N₄: M+ 744.9

### (6) Synthesis of Compound 53

Compound 53 according to an embodiment may be synthesized by, for example, a process according to Reaction Scheme 6 below.

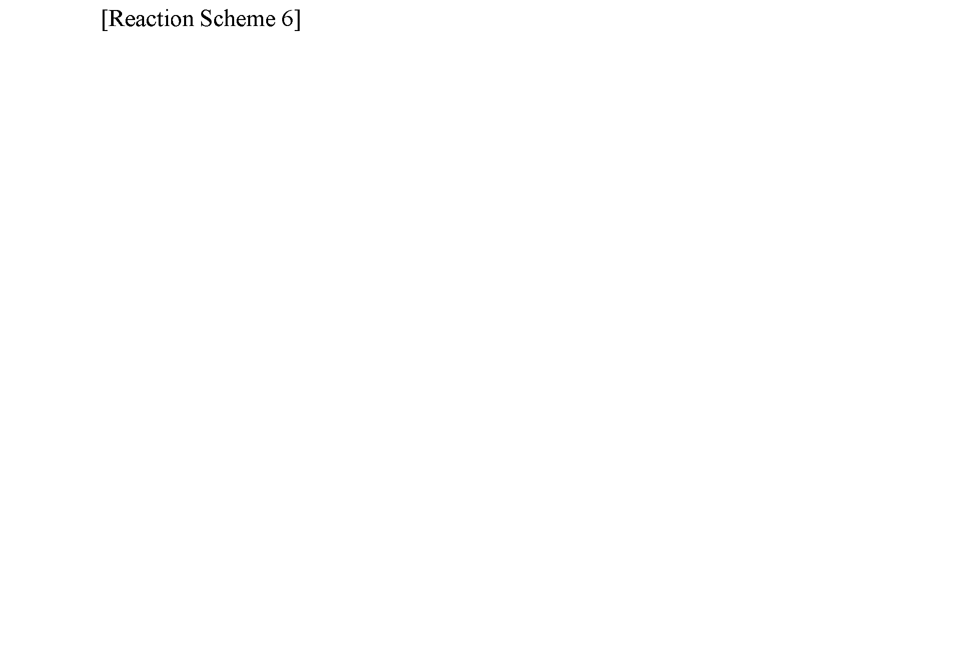

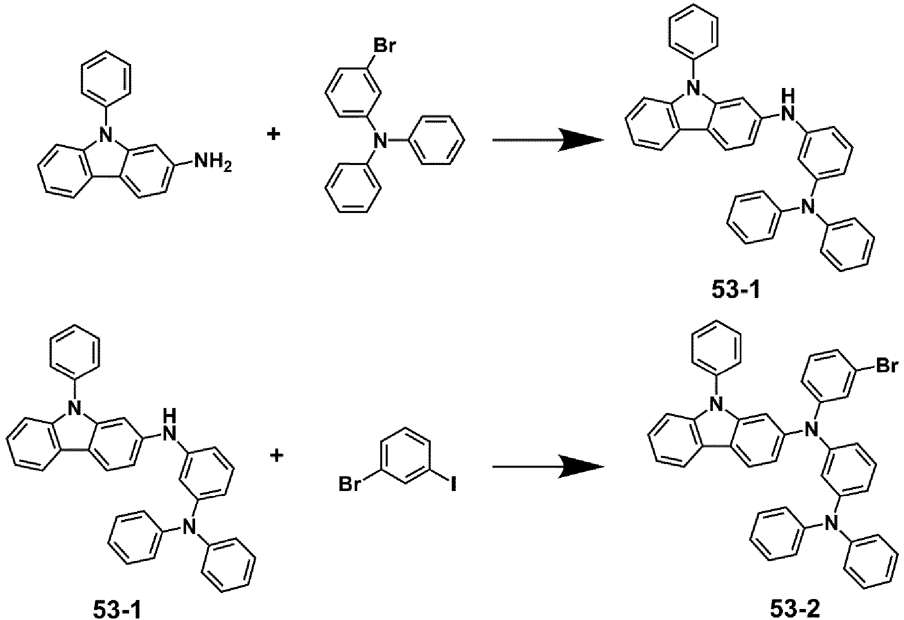

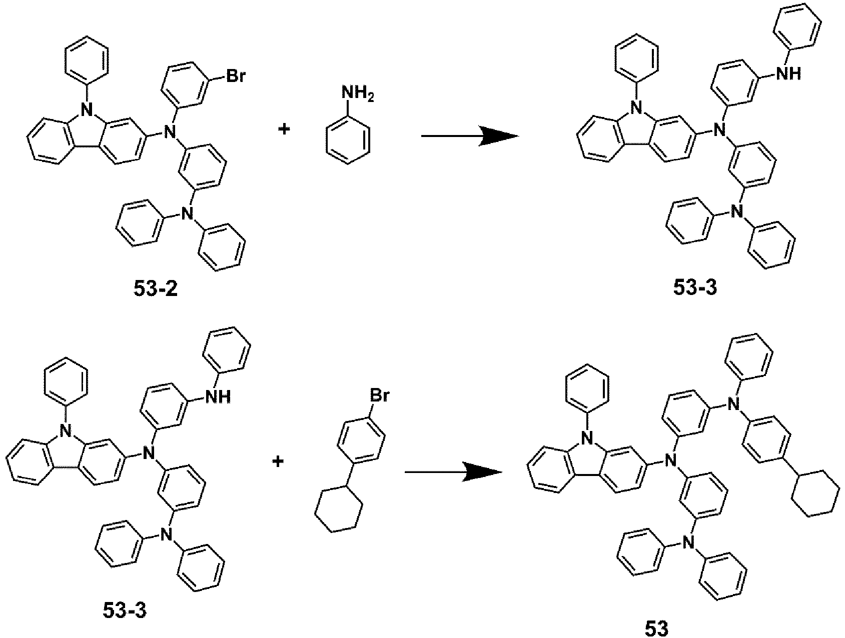

### <Synthesis of Intermediate 53-1>

Compound 53-1 was synthesized in the same manner as in the synthesis of Intermediate 13-1, except that 9-phenyl-9H-carbazol-2-amine was used instead of 9-phenyl-9H-carbazol-3-amine.

### <Synthesis of Intermediate 53-2>

Intermediate 53-2 was synthesized in the same manner as in the synthesis of Intermediate 13-2, except that Intermediate 53-1 was used instead of Intermediate 13-1.

### <Synthesis of Intermediate 53-3>

Intermediate 53-3 was synthesized in the same manner as in the synthesis of Intermediate 13-3, except that Intermediate 53-2 was used instead of Intermediate 13-2.

### <Synthesis of Compound 53>

Compound 53 was synthesized in the same manner as in the synthesis of Compound 13, except that Intermediate 53-3 was used instead of Intermediate 13-3. The obtained compound was determined through LC-MS. C₆₀H₅₀N₄: M+ 827.0

### (7) Synthesis of Compound 61

Compound 61 according to an embodiment may be synthesized by, for example, a process according to Reaction Scheme 7 below.

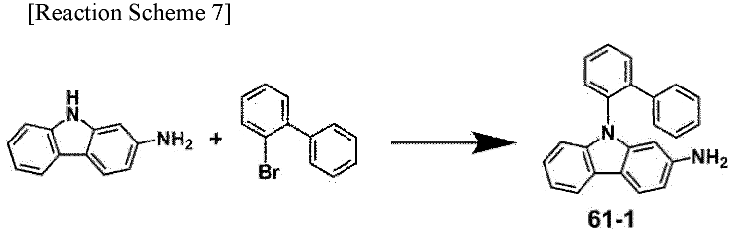

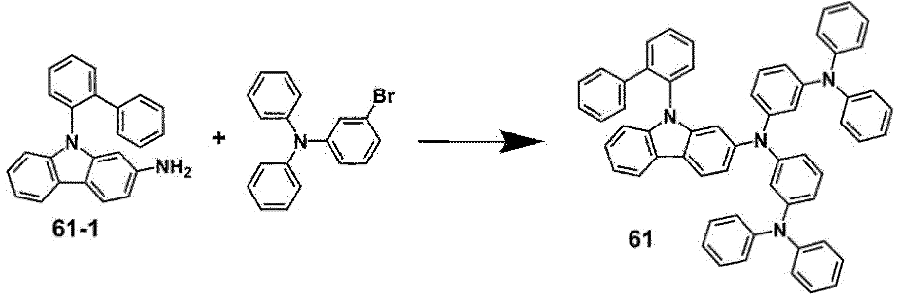

### <Synthesis of Intermediate 61-1>

Compound 61-1 was synthesized in the same manner as in the synthesis of Intermediate 21-1, except that 9H-carbazol-2-amine was used instead of 9H-carbazol-3-amine.

### <Synthesis of Compound 61>

Compound 61 was synthesized in the same manner as in the synthesis of Compound 21, except that Intermediate 61-1 was used instead of Intermediate 21-1. The obtained compound was determined through LC-MS. C₆₀H₄₄N₄: M+ 821.0

### (8) Synthesis of Compound 81

Compound 81 according to an embodiment may be synthesized by, for example, a process according to Reaction Scheme 8 below.

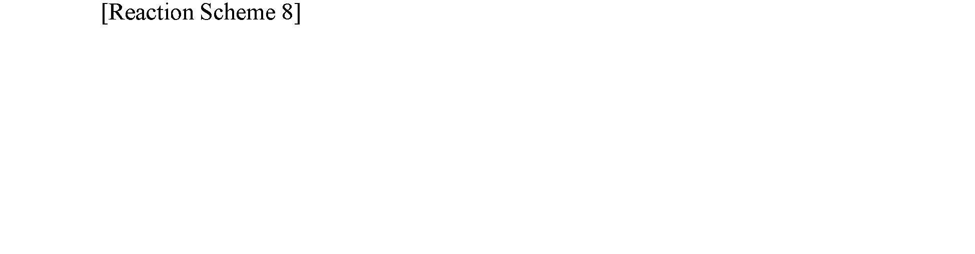

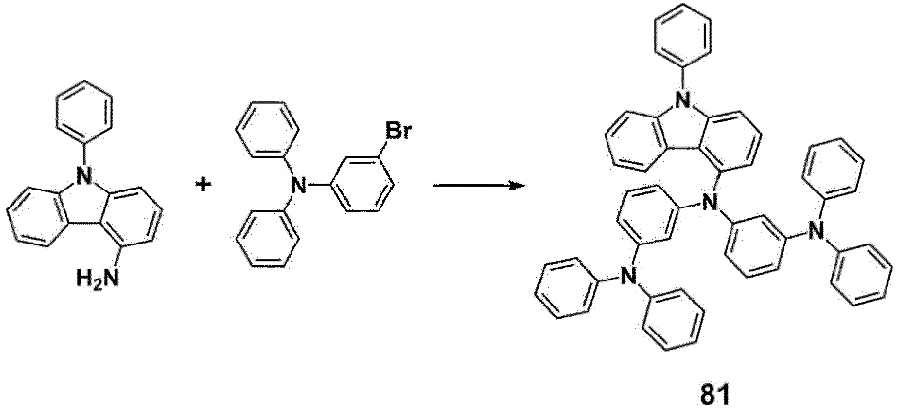

### <Synthesis of Compound 81>

Compound 81 was synthesized in the same manner as in the synthesis of Compound 1, except that 9-phenyl-9H-carbazol-4-amine was used instead of 9-phenyl-9H-carbazol-3-amine. The obtained compound was determined through LC-MS. C₅₄H₄₀N₄: M+ 744.9

### (9) Synthesis of Compound 93

Compound 93 according to an embodiment may be synthesized by, for example, a process according to Reaction Scheme 9 below.

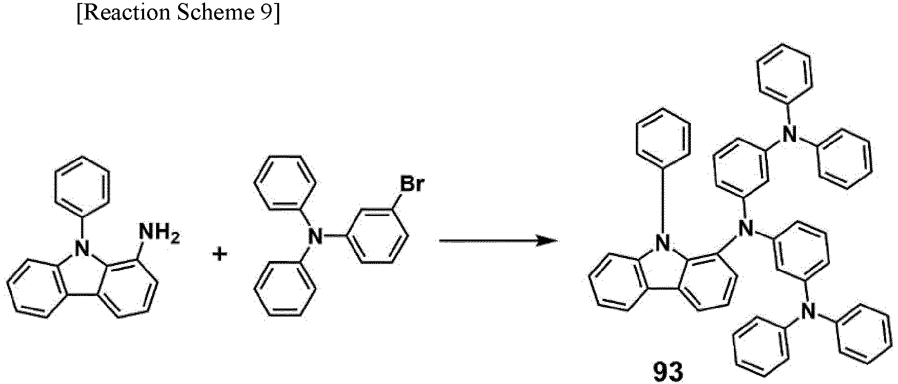

### <Synthesis of Compound 93>

Compound 93 was synthesized in the same manner as in the synthesis of Compound 1, except that 9-phenyl-9H-carbazol-1-amine was used instead of 9-phenyl-9H-carbazol-3-amine. The obtained compound was determined through LC-MS. C₅₄H₄₀N₄: M+ 744.9

### (10) Synthesis of Compound 105

Compound 105 according to an embodiment may be synthesized by, for example, a process according to Reaction Scheme 10 below.

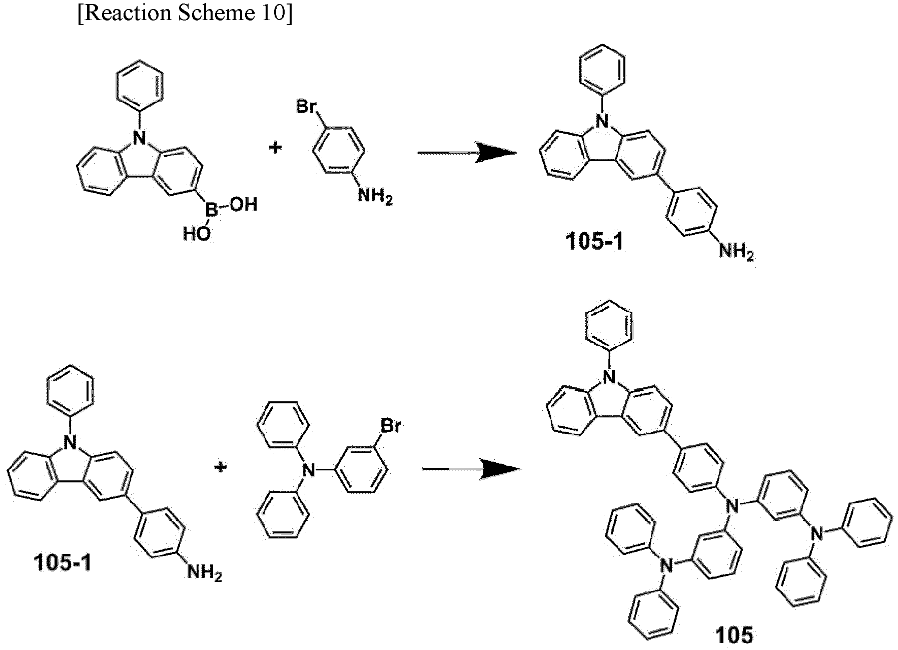

### <Synthesis of Intermediate 105-1>

(9-phenyl-9H-carbazol-3-yl)boronic acid (2.87 g, 10.0 mmol), 4-bromoaniline (1.56 g, 9.07 mmol), Pd(PPh₃)₄ (0.58 g, 0.5 mmol), and K₂CO₃ (4.14 g, 30.0 mmol) were dissolved in 60 ml of a THF/H₂O (volume ratio: 2:1) mixed solution and stirred at 80 °C for 16 hours. The reaction solution was cooled to room temperature and extracted three times with 60 mL of water and 60 mL of diethyl ether. The obtained organic layer was dried over MgSO₄, the solvent was evaporated, and the resulting residue was separated and purified through silica gel chromatography to obtain 1.67 g of Intermediate 105-1 (yield: 50%). The obtained compound was determined through LC-MS. C₂₄H₁₈N₂: M+ 334.4

### <Synthesis of Compound 105>

Intermediate 105-1 (3.34 g, 10 mmol), 3-bromo-N,N-diphenylaniline (7.13 g, 22 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 5.75 g of Compound 105 (yield: 70%). The obtained compound was determined through LC-MS. C₆₀H₄₄N₄: M+ 821.0

### (11) Synthesis of Compound 109

Compound 109 according to an embodiment may be synthesized by, for example, a process according to Reaction Scheme 11 below.

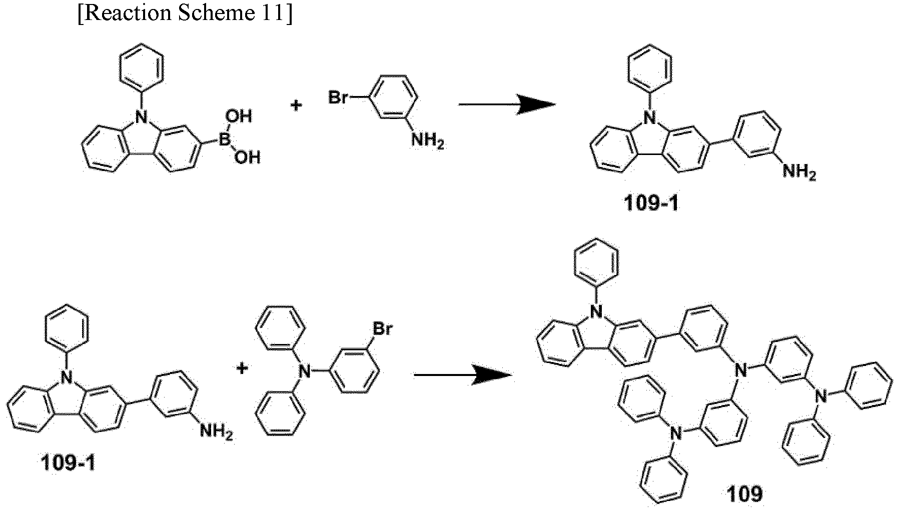

### <Synthesis of Intermediate 109-1>

(9-phenyl-9H-carbazol-2-yl)boronic acid (2.87 g, 10.0 mmol), 3-bromoaniline (1.56 g, 9.07 mmol), Pd(PPh₃)₄ (0.58 g, 0.5 mmol), and K₂CO₃ (4.14 g, 30.0 mmol) were dissolved in 60 ml of a THF/H₂O (volume ratio: 2:1) mixed solution and stirred at 80 °C for 16 hours. The reaction solution was cooled to room temperature and extracted three times with 60 mL of water and 60 mL of diethyl ether. The obtained organic layer was dried over MgSO₄, the solvent was evaporated, and the resulting residue was separated and purified through silica gel chromatography to obtain 1.67 g of Intermediate 109-1 (yield: 50%). The obtained compound was determined through LC-MS. C₂₄H₁₈N₂: M⁺ 334.4

### <Synthesis of Compound 109>

Intermediate 109-1 (3.34 g, 10 mmol), 3-bromo-N,N-diphenylaniline (7.13 g, 22 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 5.75 g of Compound 109 (yield: 70%). The obtained compound was determined through LC-MS. C₆₀H₄₄N₄: M+ 821.0

### (12) Synthesis of Compound 126

Compound 126 according to an embodiment may be synthesized by, for example, a process according to Reaction Scheme 12 below.

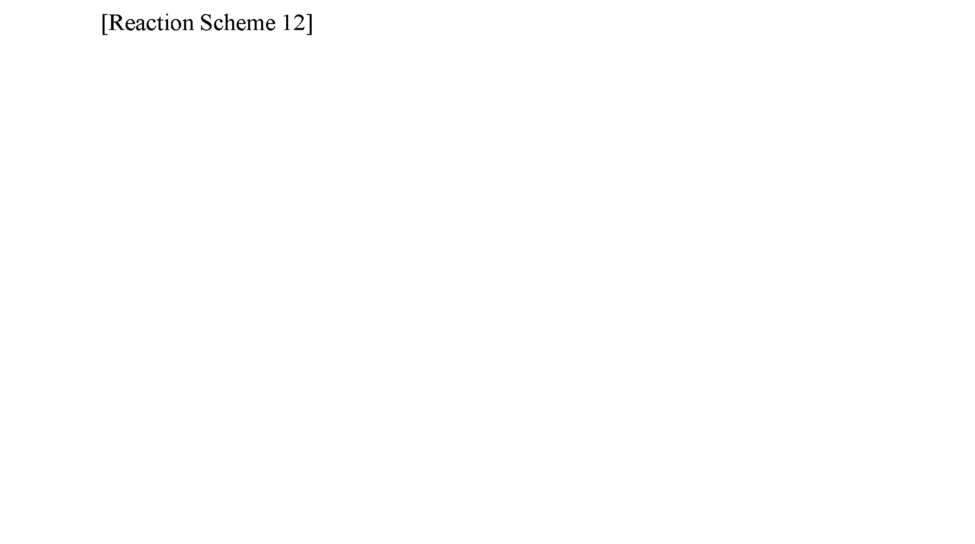

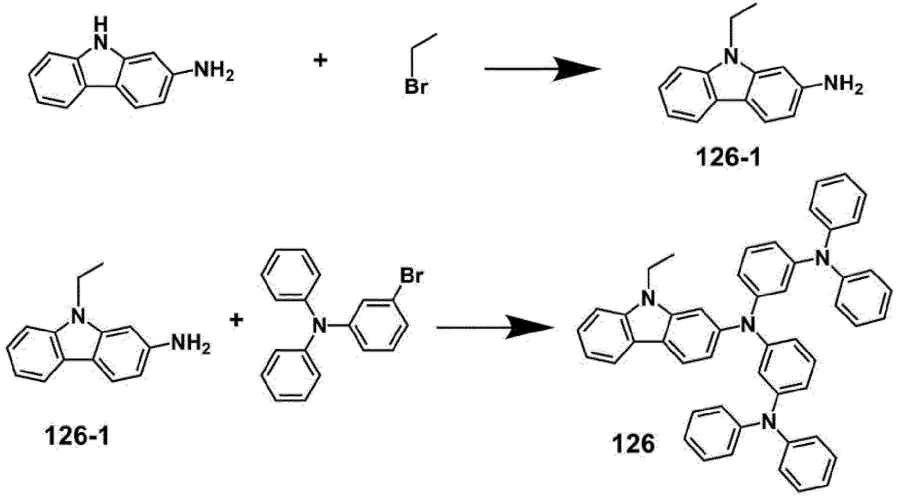

### <Synthesis of Intermediate 126-1>

9H-carbazol-2-amine (2.84 g, 10.0 mmol), bromoethane (2.18 g, 20.0 mmol), tetrabutylammonium iodide (0.37 g, 1.0 mmol), and KOH (4.14 g, 73.78 mmol) were dissolved in 60 ml of toluene/H₂O (volume ratio: 2:1) and stirred at 100 °C for 8 hours. The reaction solution was cooled to room temperature and extracted three times with 60 mL of water and 60 mL of diethyl ether. The obtained organic layer was dried over MgSO₄, the solvent was evaporated, and the resulting residue was separated and purified through silica gel tube chromatography to obtain 1.89 g of Intermediate 126-1 (yield: 90%). The obtained compound was determined through LC-MS. C₁₄H₁₄N₂ : M+ 210.2

### <Synthesis of Compound 126>

Intermediate 126-1 (2.10 g, 10 mmol), 3-bromo-N,N-diphenylaniline (7.13 g, 22 mmol), Pd₂(dba)₃ (tris(dibenzylideneacetone)dipalladium(0)) (0.92 g, 1 mmol), P(t-Bu)₃ (0.48 g, 2.37 mmol), and sodium tert-butoxide (5.76 g, 60 mmol) were dissolved in 60 ml of toluene and stirred at 80 °C for 3 hours. The reaction solution was cooled to room temperature, and 40 ml of water was added thereto, and the mixture was extracted three times with 50 ml of ethyl ether. The collected organic layer was dried over MgSO₄, the solvent was evaporated, and the resulting residue was separated and purified through silica gel chromatography to obtain 4.88 g of Compound 126 (yield: 70%). The obtained compound was determined through LC-MS. C₅₀H₄₀N₄: M+ 696.8

### 2. Preparation and evaluation of light emitting element

### (1) Preparation of light emitting element

Light emitting elements including amine compounds according to an Example or including a Comparative Example Compound in a hole transport layer were prepared through a method described below. Light emitting elements of Examples 1 to 12 were prepared using Compounds 1, 13, 17, 21, 41, 53, 61, 81, 93, 105, 109, and 126, respectively, as a material of the hole transport layer. Light emitting elements of Comparative Examples 1 to 9 were prepared using Comparative Example Compounds CX1 to CX9, respectively, as host materials of the hole transport layer.

As a first electrode, a glass substrate having an ITO electrode (Corning, 15 Ω/cm², 1,200 Å) formed thereon was cut to a size of about 50 mm x 50 mm x 0.7 mm, subjected to ultrasonic cleaning using isopropyl alcohol and pure water for 5 minutes each exposed to ultraviolet radiation for 30 minutes, exposed to ozone, and mounted on a vacuum deposition apparatus.

On the first electrode, a hole injection layer having a thickness of 300 Å was formed through the deposition of NPD, and on the hole injection layer, a hole transport layer having a thickness of 200 Å was formed through the deposition of an Example Compound or a Comparative Example Compound. On the hole transport layer, an emission auxiliary layer having a thickness of 100 Å was formed through the deposition of CzSi.

On the emission auxiliary layer, an emission layer having a thickness of 200 Å was formed through the co-deposition of: a host mixture in which a first host, Compound HT1 and a second host, Compound ETH85 were mixed at a weight ratio of 5:5; a sensitizer; and a dopant at a weight ratio of 85:15:1. Materials listed in Table 1 were used as the host mixture, the sensitizer, and the dopant.

On the emission layer, a hole blocking layer having a thickness of 200 Å was formed through the deposition of TSPO1, and on the hole blocking layer, an electron transport layer having a thickness of 300 Å was formed through the deposition of TPBi. On the electron transport layer, an electron injection layer having a thickness of 10 Å was formed through the deposition of LiF, and on an upper portion of the electron injection layer, a second electrode having a thickness of 3,000 Å was formed through the deposition of Al, thereby preparing a light emitting element.

### <Materials used for preparation of light emitting elements>

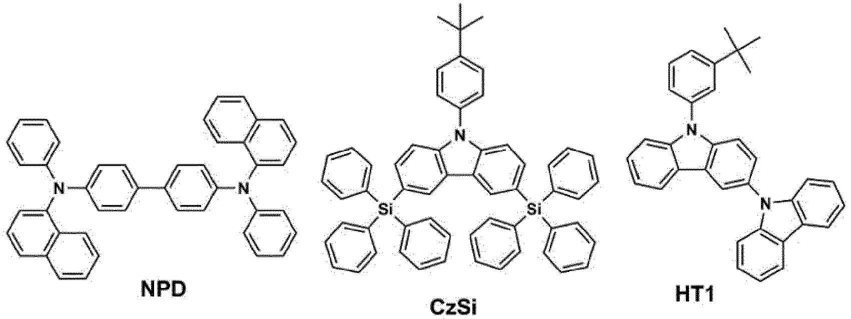

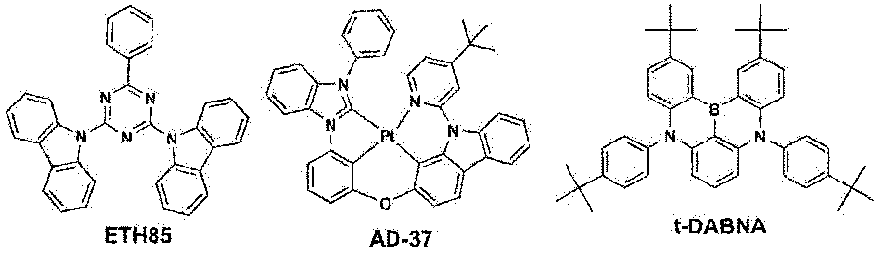

### <Example Compound>

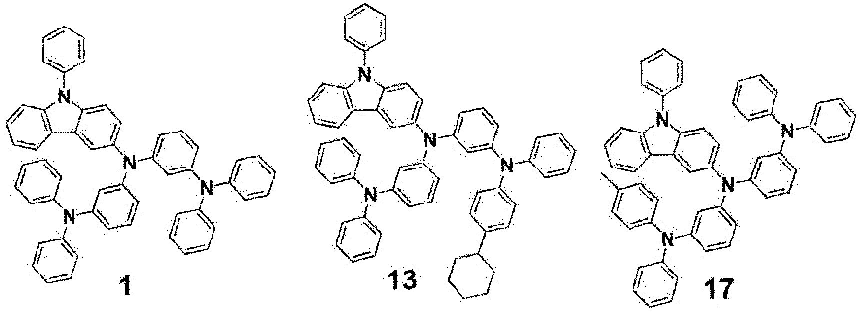

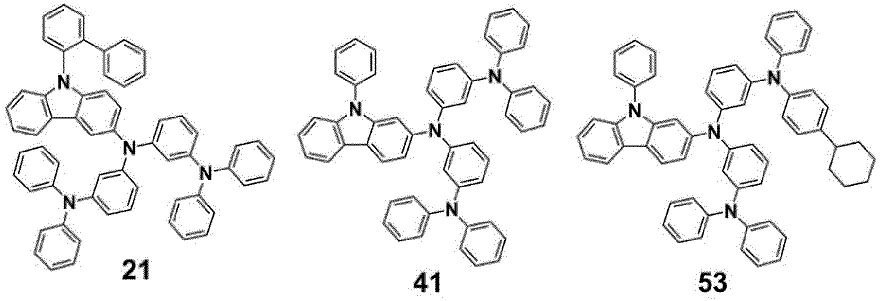

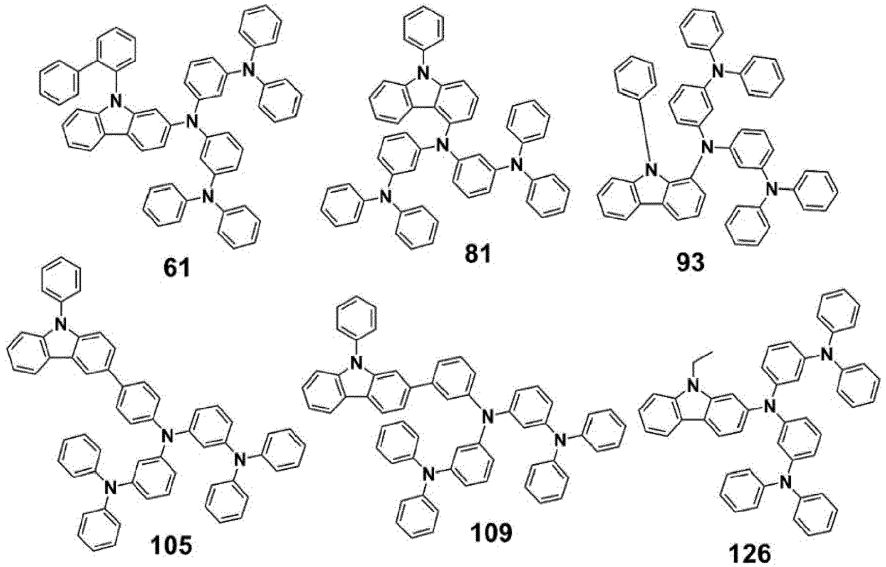

### <Comparative Example Compound>

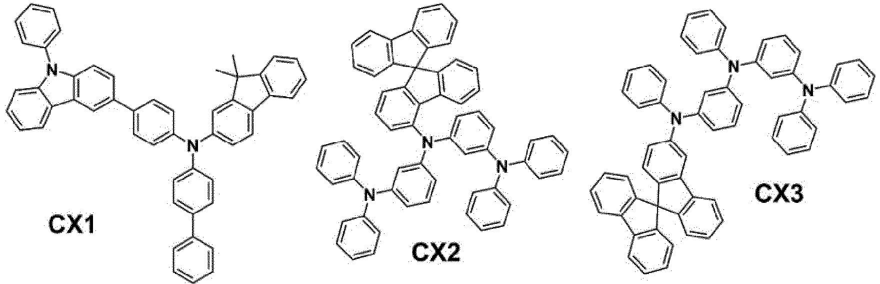

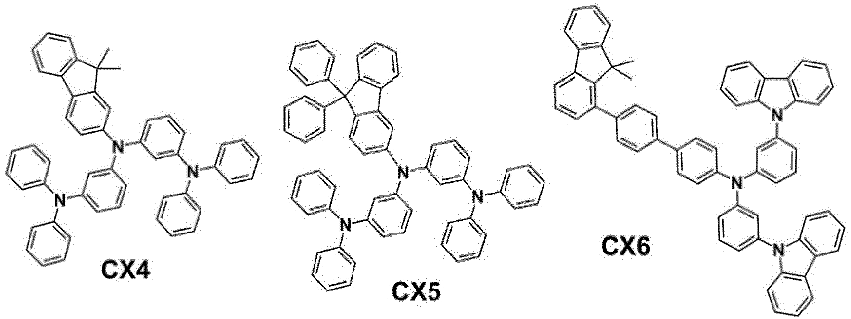

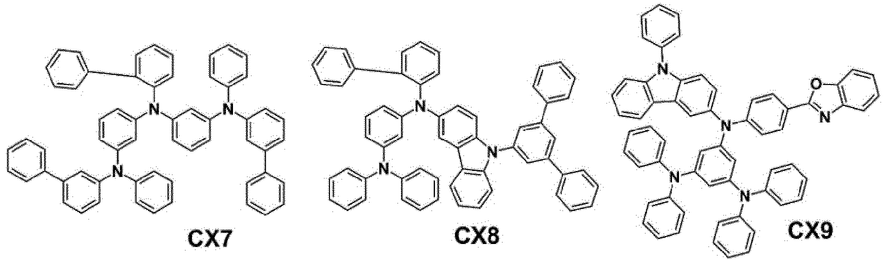

### (2) Evaluation of light emitting element

Table 1 below shows evaluation results of the light emitting elements of the Examples and the Comparative Examples. Table 1 shows driving voltage, luminous efficiency, emission wavelength, and lifetime measured at a current density of 10 mA/cm² using V7000 OLED IVL Test System (Polaronix). Lifetime (T95) indicates the time taken to decrease from an initial luminance of 100% to a luminance of 95%.

**[Table 1]**

| | Hole transport layer | Host (HT:ET =5:5) | Sensi tizer | Dopant | Driving voltage (V) | Luminous efficiency (cd/A) | Emission wavelength (nm) | Lifetime (T95, hr) |
|---|---|---|---|---|---|---|---|---|
| Comparative Example 1 | Comparative Example Compound CX1 | HT1/ET H85 | AD-37 | t-DABNA | 5.6 | 18.8 | 463 | 95 |
| Comparative Example 2 | Comparative Example Compound CX2 | HT1/ET H85 | AD-37 | t-DABNA | 5.5 | 18.5 | 463 | 109 |
| Comparative Example 3 | Comparative Example Compound CX3 | HT1/ET H85 | AD-37 | t-DABNA | 5.9 | 18.4 | 463 | 99 |
| Comparative Example 4 | Comparative Example Compound CX4 | HT1/ET H85 | AD-37 | t-DABNA | 5.2 | 18.0 | 463 | 105 |
| Comparative Example 5 | Comparative Example Compound CX5 | HT1/ET H85 | AD-37 | t-DABNA | 5.8 | 18.1 | 463 | 90 |
| Comparative Example 6 | Comparative Example Compound CX6 | HT1/ET H85 | AD-37 | t-DABNA | 6.5 | 18.8 | 463 | 110 |
| Comparative Example 7 | Comparative Example Compound CX7 | HT1/ET H85 | AD-37 | t-DABNA | 6.3 | 17.5 | 463 | 50 |
| Comparative Example 8 | Comparative Example Compound CX8 | HT1/ET H85 | AD-37 | t-DABNA | 6.0 | 18.0 | 463 | 60 |
| Comparative Example 9 | Comparative Example Compound CX9 | HT1/ET H85 | AD-37 | t-DABNA | 6.8 | 15.2 | 463 | 45 |
| Example 1 | Compound 1 | HT1/ET H85 | AD-37 | t-DABNA | 4.9 | 20.1 | 463 | 201 |
| Example 2 | Compound 13 | HT1/ET H85 | AD-37 | t-DABNA | 5.0 | 20.5 | 463 | 230 |
| Example 3 | Compound 17 | HT1/ET H85 | AD-37 | t-DABNA | 4.8 | 20.4 | 463 | 220 |
| Example 4 | Compound 21 | HT1/ET H85 | AD-37 | t-DABNA | 4.8 | 20.4 | 463 | 225 |
| Example 5 | Compound 41 | HT1/ET H85 | AD-37 | t-DABNA | 4.7 | 20.5 | 463 | 232 |
| Example 6 | Compound 53 | HT1/ET H85 | AD-37 | t-DABNA | 5.0 | 20.4 | 463 | 225 |
| Example 7 | Compound 61 | HT1/ET H85 | AD-37 | t-DABNA | 4.7 | 20.3 | 463 | 219 |
| Example 8 | Compound 81 | HT1/ET H85 | AD-37 | t-DABNA | 4.8 | 20.0 | 463 | 194 |
| Example 9 | Compound 93 | HT1/ET H85 | AD-37 | t-DABNA | 4.8 | 21.1 | 463 | 225 |
| Example 10 | Compound 105 | HT1/ET H85 | AD-37 | t-DABNA | 4.7 | 20.5 | 463 | 233 |
| Example 11 | Compound 109 | HT1/ET H85 | AD-37 | t-DABNA | 4.8 | 21.0 | 463 | 210 |
| Example 12 | Compound 126 | HT1/ET H85 | AD-37 | t-DABNA | 4.8 | 20.5 | 463 | 239 |

Referring to Table 1, it can be seen that the light emitting elements of Examples 1 to 12 and Comparative Examples 1 to 9 emitted blue light in a wavelength range of 450 nm to 470 nm. It can be seen that, as compared to the light emitting elements of Comparative Examples 1 to 9, the light emitting elements of Examples 1 to 12 had lower driving voltage, higher luminous efficiency, and increased lifetime. The light emitting elements of Examples 1 to 12 respectively include Compounds 1, 13, 17, 21, 41, 53, 61, 81, 93, 105, 109, and 126. Compounds 1, 13, 17, 21, 41, 53, 61, 81, 93, 105, 109, and 126 are amine compounds according to embodiments and include three amine groups and at least one carbazole group. Accordingly, it is determined that a light emitting element including the amine compound according to an embodiment may exhibit low driving voltage, high luminous efficiency, and long lifetime.

The light emitting element of Comparative Example 1 includes Comparative Example Compound CX1, and Comparative Example Compound CX1 includes a carbazole group. However, Comparative Example Compound CX1 includes only one amine group, which is different from the amine compound according to an embodiment. Accordingly, the light emitting element of Comparative Example 1 including Comparative Example Compound CX1 exhibits higher driving voltage, lower luminous efficiency, and shorter lifetime than the light emitting elements of the Examples.

The light emitting elements of Comparative Examples 2 to 5 and 7 respectively include Comparative Example Compounds CX2 to CX5 and CX7. Comparative Example Compounds CX2 to CX5 and CX7 each include three amine groups. However, Comparative Example Compounds CX2 to CX5 and CX7 do not include a carbazole group, which is different from the amine compound according to an embodiment. Accordingly, the light emitting elements of Comparative Examples 2 to 5 and 7 exhibit higher driving voltage, lower luminous efficiency, and shorter lifetime than the light emitting elements of the Examples.

The light emitting element of Comparative Example 6 includes Comparative Example Compound CX6, and Comparative Example Compound CX6 includes an amine group and a carbazole group. However, Comparative Example Compound CX6 includes only one amine group, which is different from the amine compound according to an embodiment. Accordingly, the light emitting element of Comparative Example 6 exhibits higher driving voltage, lower luminous efficiency, and shorter lifetime than the light emitting elements of the Examples.

The light emitting element of Comparative Example 8 includes Comparative Example Compound CX8, and Comparative Example Compound CX8 includes an amine group and a carbazole group. However, Comparative Example Compound CX8 includes only two amine group, which is different from the amine compound according to an embodiment. Accordingly, the light emitting element of Comparative Example 8 exhibits higher driving voltage, lower luminous efficiency, and shorter lifetime than the light emitting elements of the Examples.

The light emitting element of Comparative Example 9 includes Comparative Example Compound CX9, and Comparative Example Compound CX9 includes an amine group and a carbazole group. However, Comparative Example Compound CX9 has three amine groups bonded through only one phenyl group, which is different from the amine compound according to an embodiment in which three amine groups are bonded through two phenyl groups. Accordingly, the light emitting element of Comparative Example 9 exhibits higher driving voltage, lower luminous efficiency, and shorter lifetime than the light emitting elements of the Examples.

A display device according to an embodiment includes a light emitting element according to an embodiment. The light emitting element includes the amine compound according to an embodiment in a hole transport region. The amine compound includes three amine groups and at least one carbazole group. The carbazole group may be directly or indirectly bonded to at least one of the three amine groups. Accordingly, the amine compound according to an embodiment may exhibit excellent material stability and thermal stability. The light emitting element including the amine compound according to an embodiment may exhibit low driving voltage, high luminous efficiency, and long lifetime.

A light emitting element according to an embodiment and a display device including the light emitting element include an amine compound according to an embodiment, and may thus exhibit high efficiency and long life.

An amine compound according to an embodiment may contribute to high efficiency and long life of a light emitting element.

Embodiments have been disclosed herein, and although terms are employed, they are used and are to be interpreted in a generic and descriptive sense only and not for the purposes of limitation. In some instances, as would be apparent to one of ordinary skill in the art, features, characteristics, and/or elements described in connection with an embodiment may be used singly or in combination with features, characteristics, and/or elements described in connection with other embodiments unless otherwise specifically indicated. Accordingly, it will be understood by those of ordinary skill in the art that various changes in form and details may be made without departing from the scope of the disclosure as set forth in the claims.

## Claims

1. An amine compound represented by Formula 1: 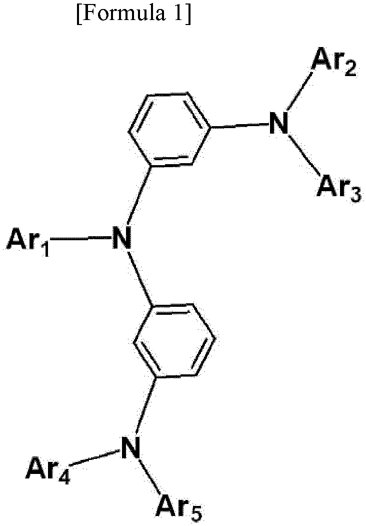 wherein in Formula 1,
at least one of Ar₁ to Ar₅ is each independently a group represented by Formula 2, and
the remainder of Ar₁ to Ar₅ are each independently a substituted or unsubstituted aryl group having 6 to 30 ring-forming carbon atoms or a substituted or unsubstituted heteroaryl group having 2 to 30 ring-forming carbon atoms; 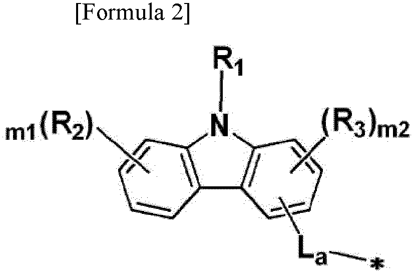 wherein in Formula 2,
m1 is an integer from 0 to 4,
m2 is an integer from 0 to 3,
R₁ to R₃ are each independently a hydrogen atom, a deuterium atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms, and
Lₐ is a direct linkage, a substituted or unsubstituted arylene group having 6 to 30 ring-forming carbon atoms, or a substituted or unsubstituted heteroarylene group having 2 to 30 ring-forming carbon atoms.

2. The amine compound of claim 1, wherein the amine compound is represented by one of Formula 1-A to Formula 1-C: 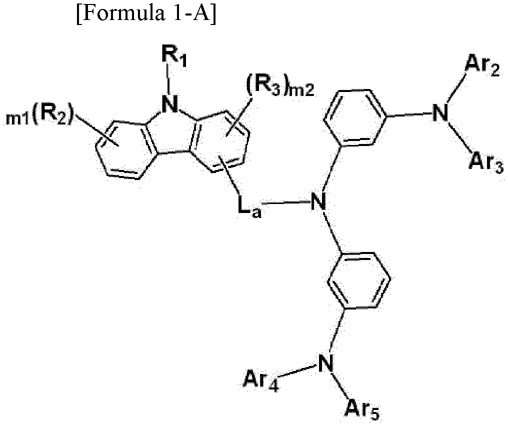 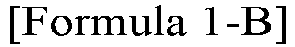 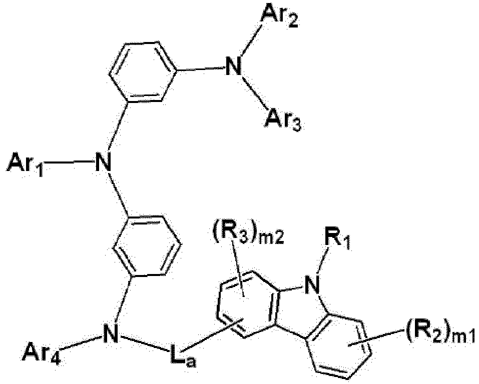 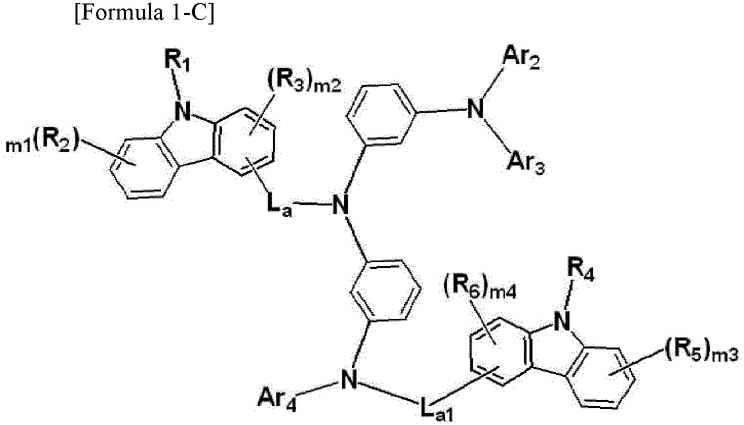 wherein in Formula 1-C,
m3 is an integer from 0 to 4,
m4 is an integer from 0 to 3,
R₄ to R₆ are each independently a hydrogen atom, a substituted or unsubstituted alkyl group having 1 to 30 carbon atoms, a substituted or unsubstituted cycloalkyl group having 3 to 30 ring-forming carbon atoms, or a substituted or unsubstituted aryl group having 6 to 30 ring-forming carbon atoms, and
Lₐ₁ is a direct linkage, a substituted or unsubstituted arylene group having 6 to 30 ring-forming carbon atoms, or a substituted or unsubstituted heteroarylene group having 2 to 30 ring-forming carbon atoms; and
wherein in Formulas 1-A to 1-C,
m1, m2, Ar₁ to Ar₅, R₁ to R₃, and Lₐ are the same as defined in Formulas 1 and 2.

3. The amine compound of claim 2, wherein the amine compound is represented by one of Formula 1-A1 to Formula 1-A4: 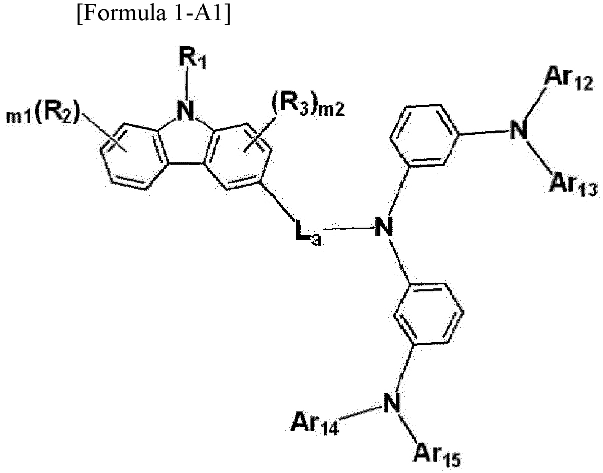 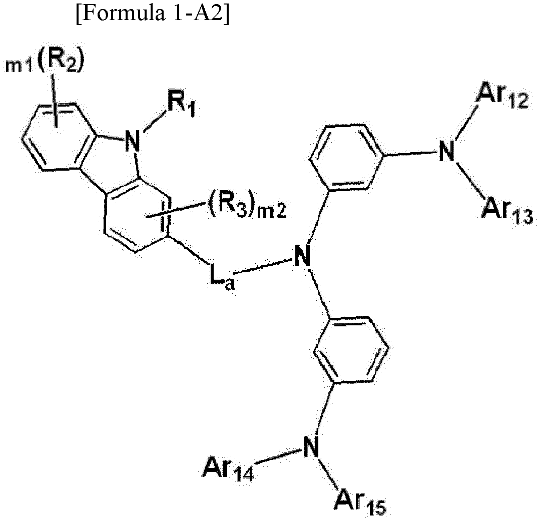 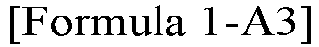 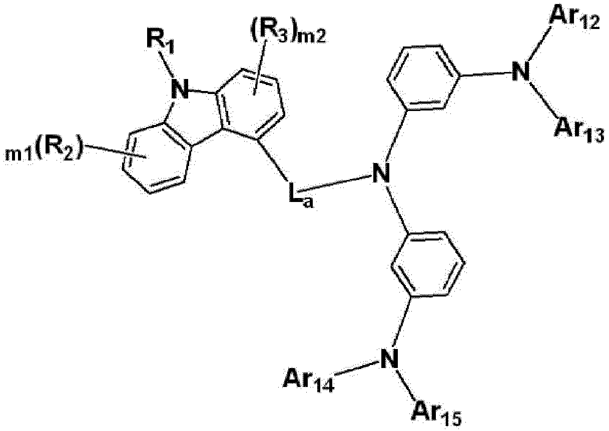 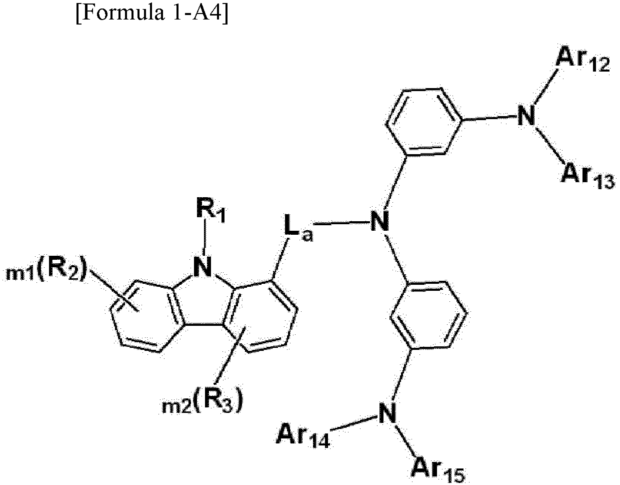 wherein in Formulas 1-A1 to 1-A4,
Ar₁₂ to Ar₁₅ are each independently a substituted or unsubstituted aryl group having 6 to 30 ring-forming carbon atoms, and
m1, m2, R₁ to R₃, and Lₐ are the same as defined in Formulas 1 and 2.

4. The amine compound of any one of claims 1 to 3, wherein in Formula 1, the remainder of Ar₁ to Ar₅ that are not a group represented by Formula 2 are each independently a substituted or unsubstituted phenyl group, an unsubstituted biphenyl group, an unsubstituted naphthyl group, or a substituted or unsubstituted terphenyl group.

5. The amine compound of any one of claims 1 to 4, wherein in Formula 1, the remainder of Ar₁ to Ar₅ that are not a group represented by Formula 2 are each independently a group represented by one of Formula AR1-1 to Formula AR1-20:

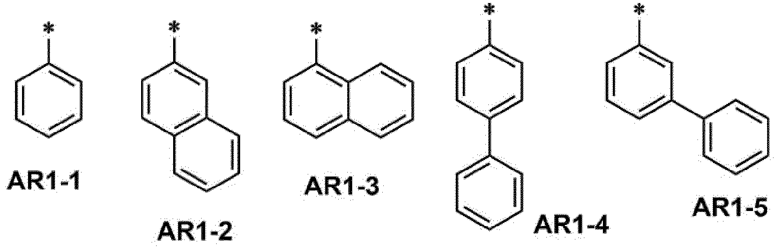

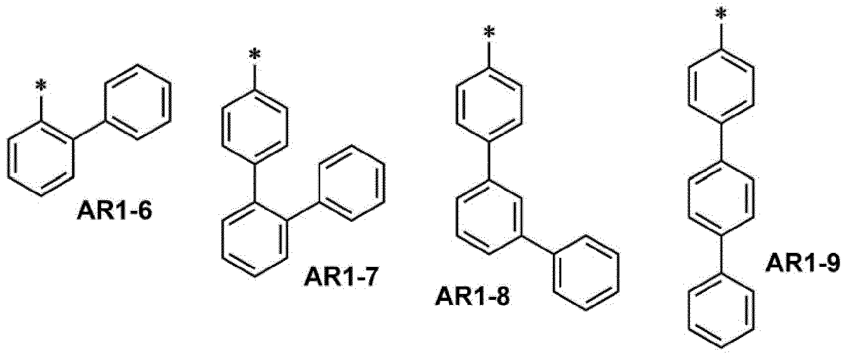

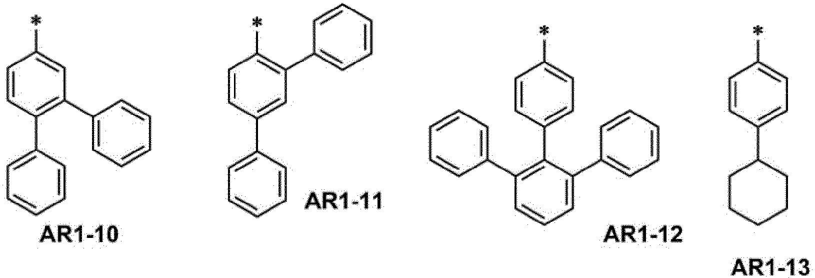

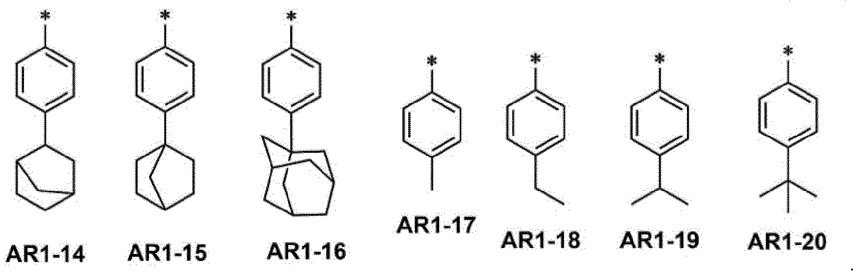

6. The amine compound of any one of claims 1 to 5, wherein in Formula 2, R₁ is an unsubstituted methyl group, an unsubstituted ethyl group, an unsubstituted isopropyl group, an unsubstituted t-butyl group, an unsubstituted phenyl group, or a group represented by one of Formula R1-1 to Formula R1-7:

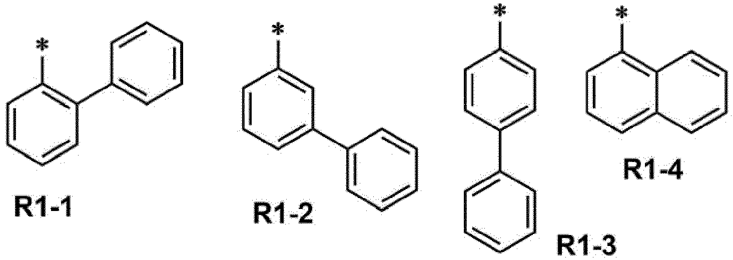

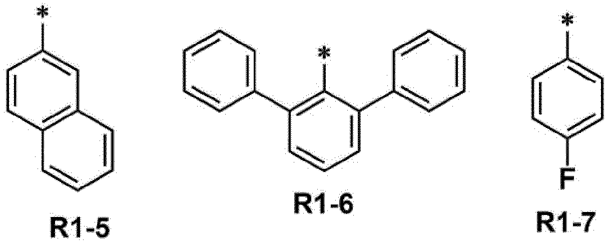

7. The amine compound of any one of claims 1 to 6, wherein the group represented by Formula 2 is a group represented by one of Formula 2-1 to Formula 2-10: 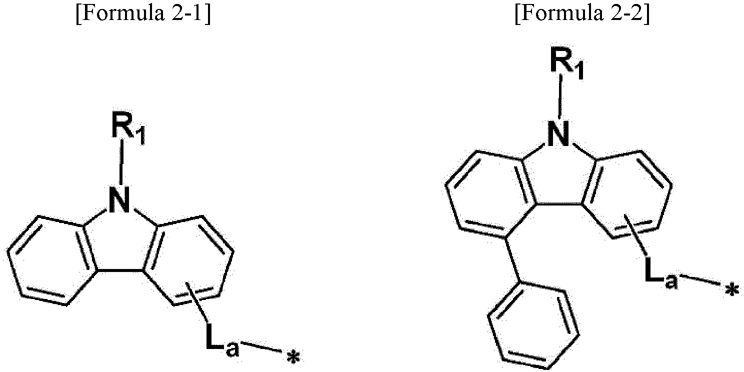 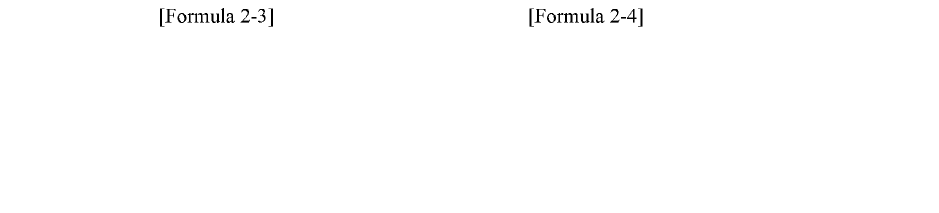 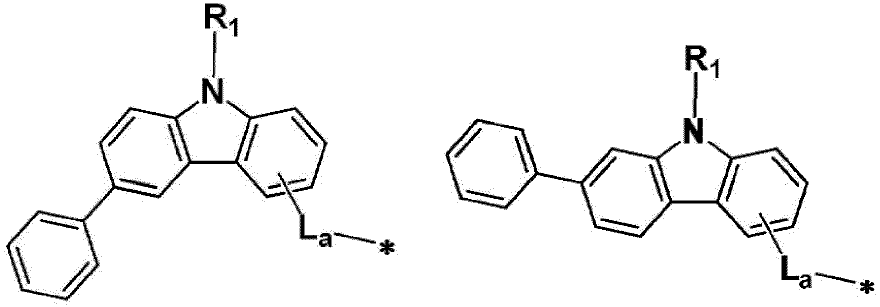 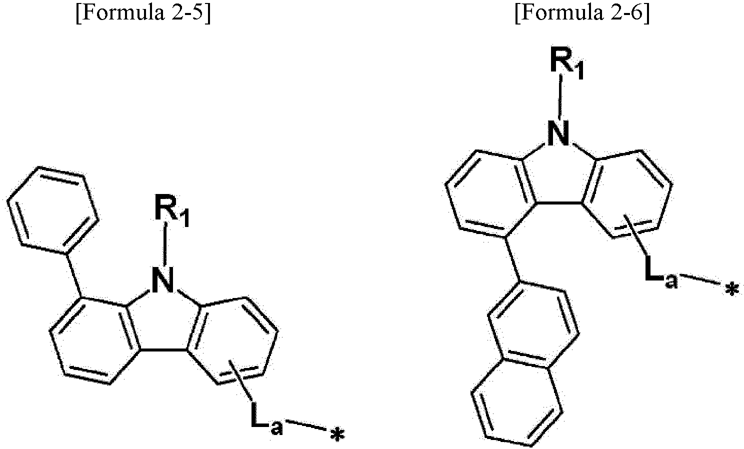 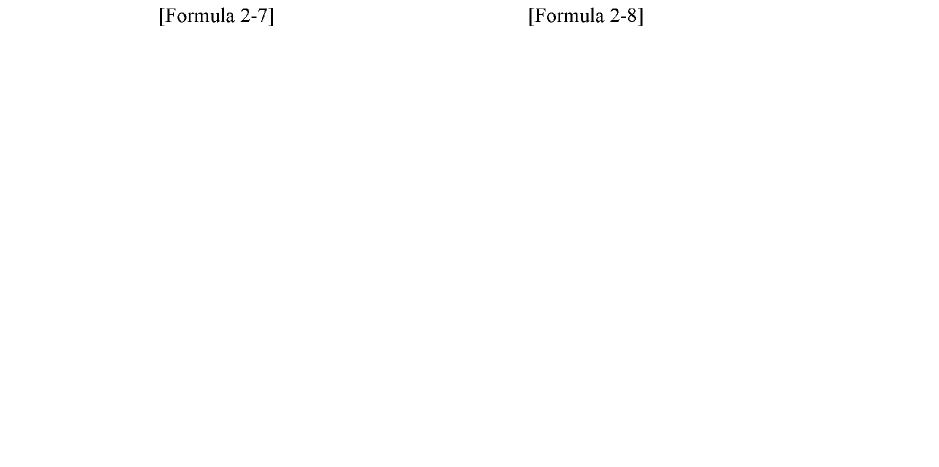 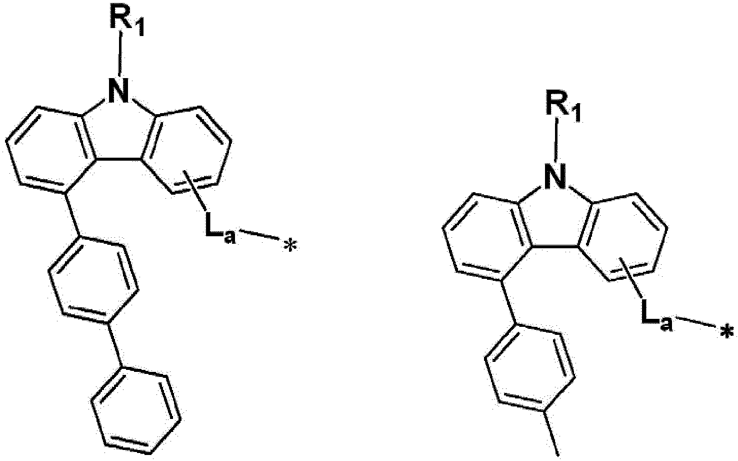 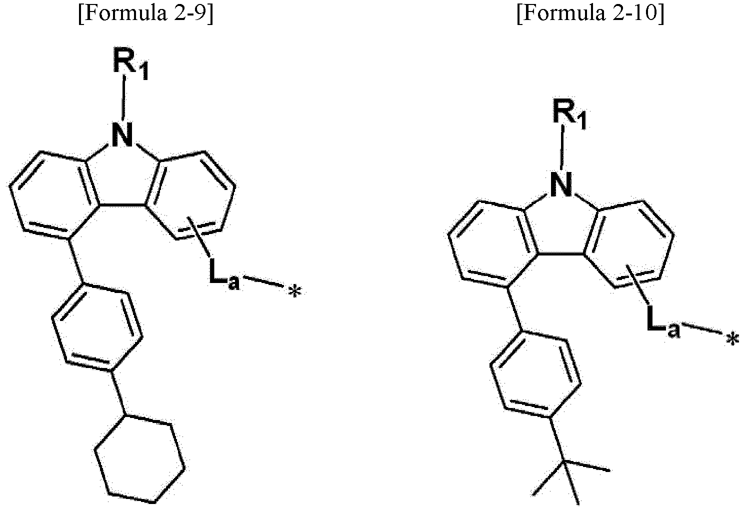 wherein in Formulas 2-1 to 2-10,
R₁ and Lₐ are the same as defined in Formula 2.

8. The amine compound of any one of claims 1 to 7, wherein in Formula 2, Lₐ is a direct linkage or an unsubstituted phenylene group.

9. The amine compound of claim 1, wherein the amine compound is selected from Compound Group 1:

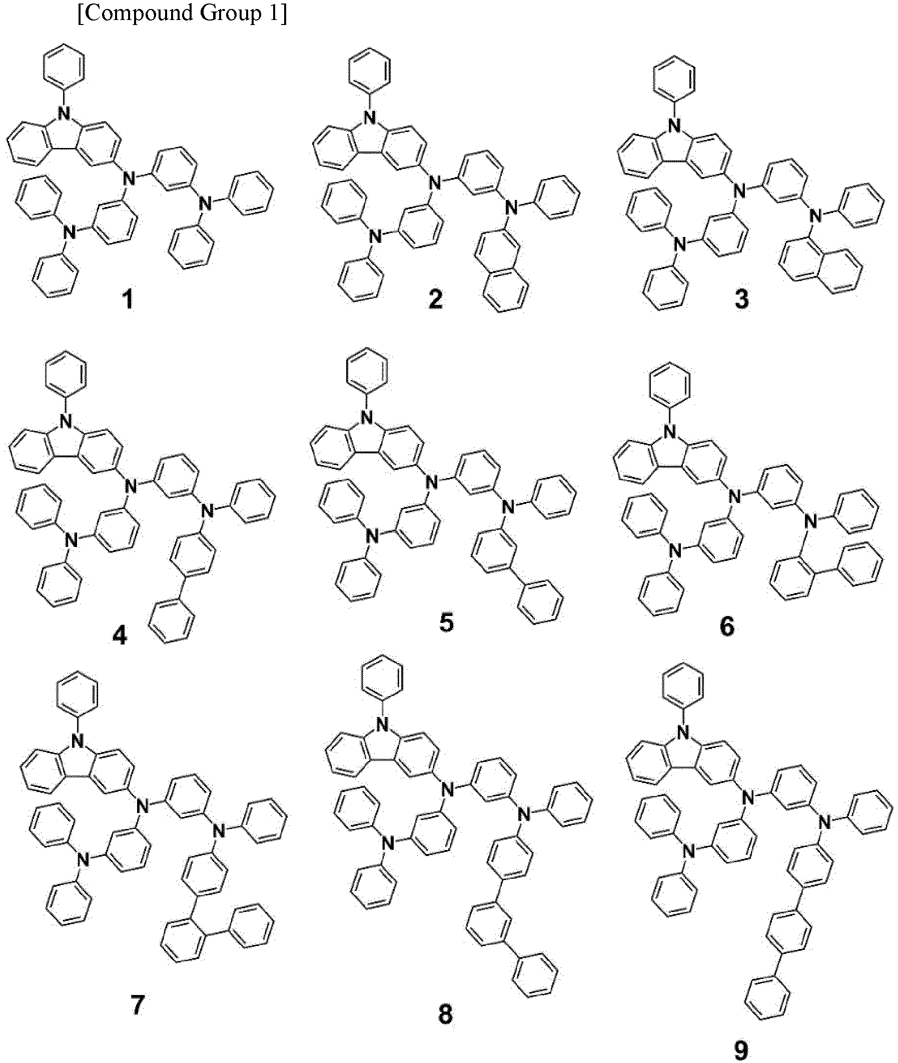

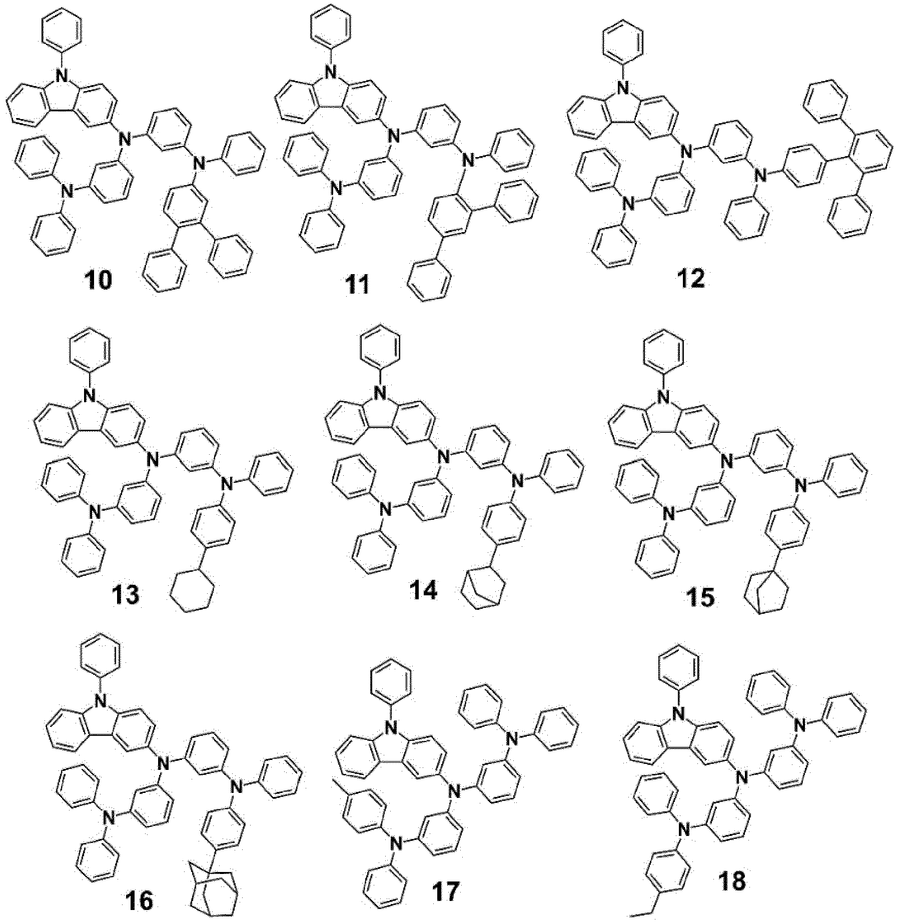

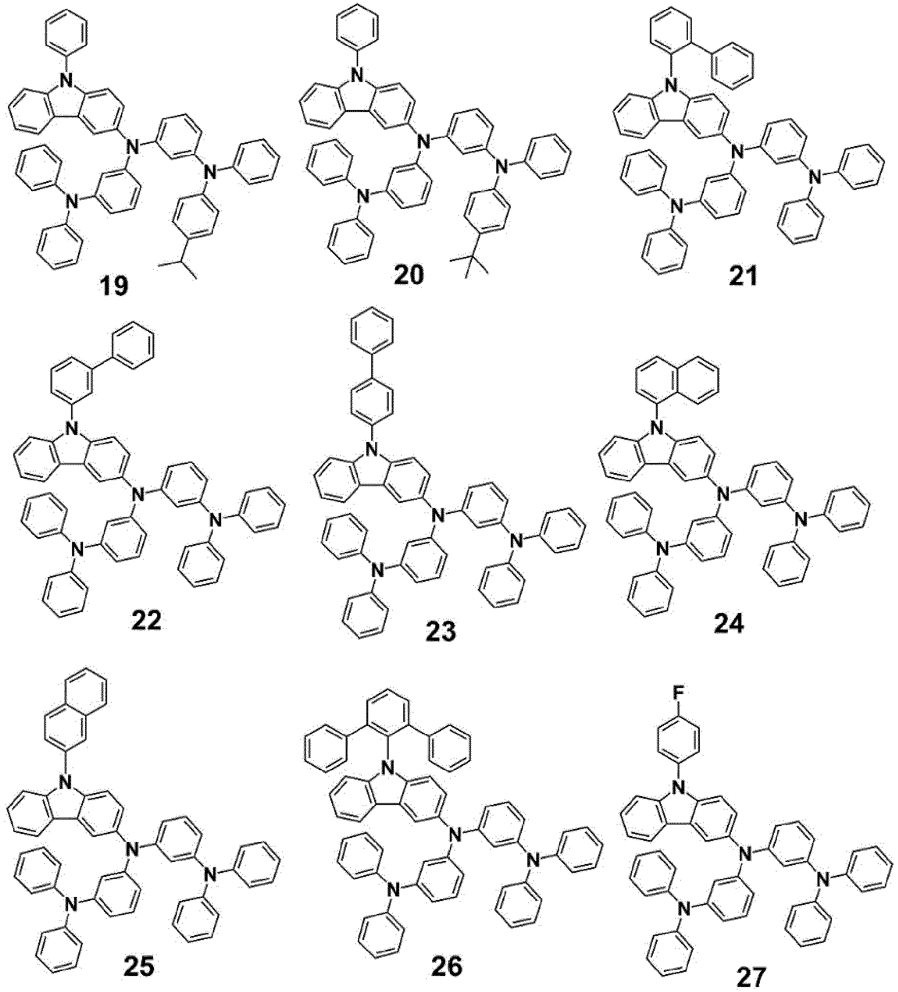

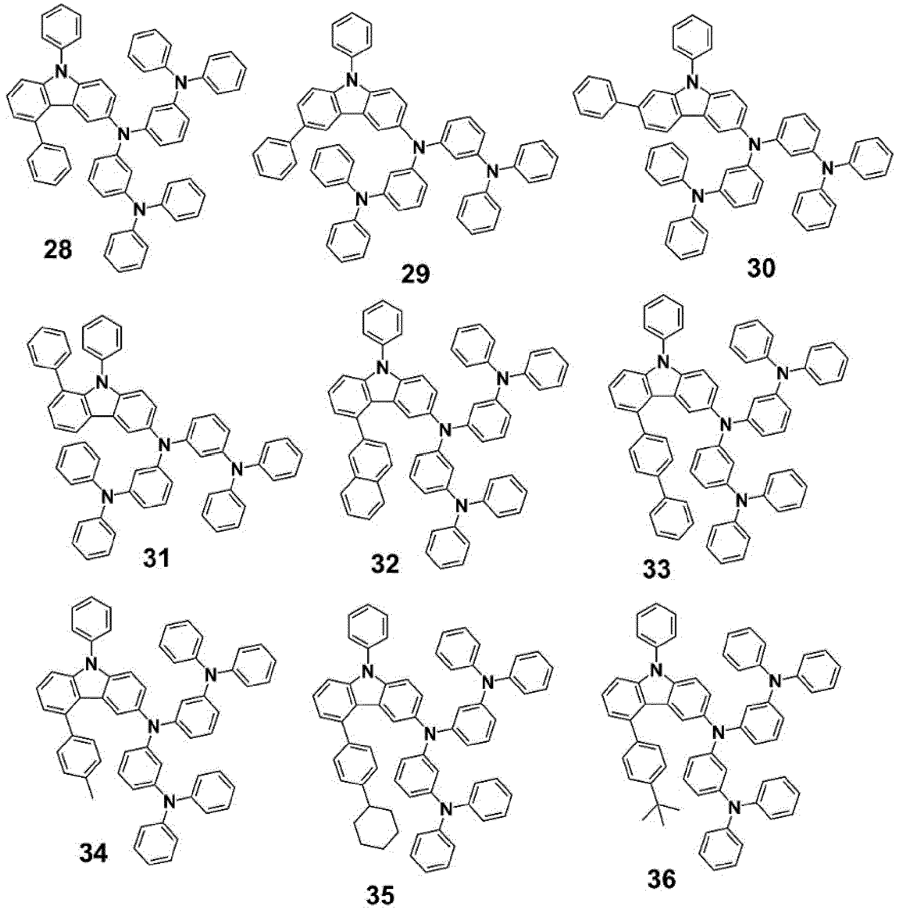

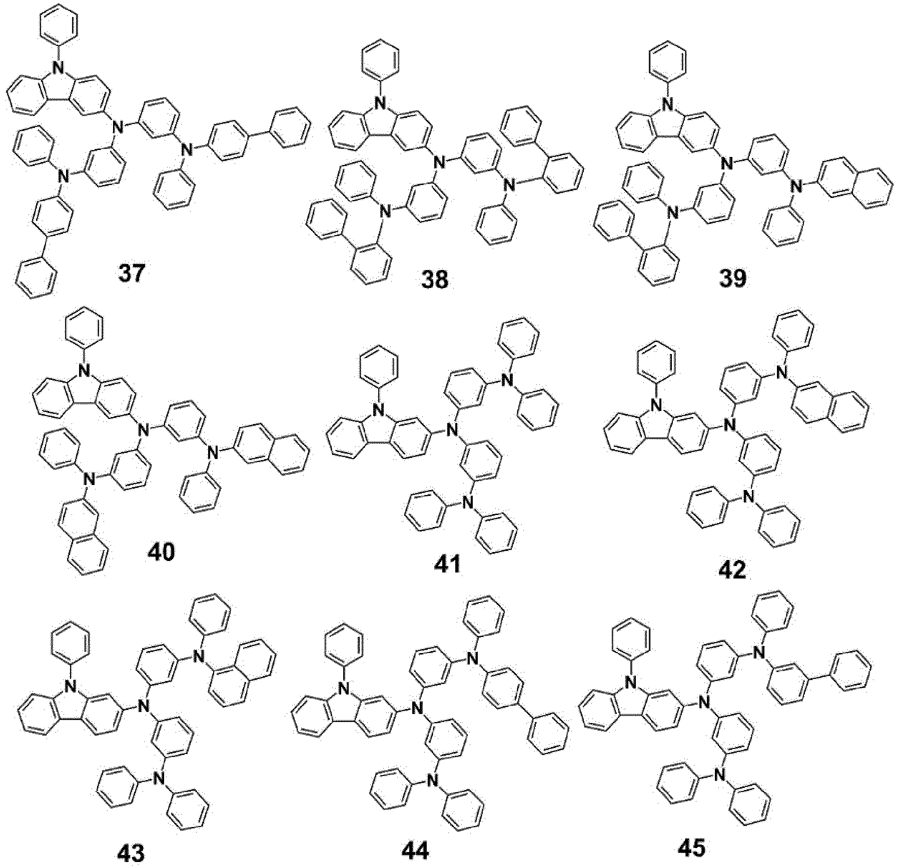

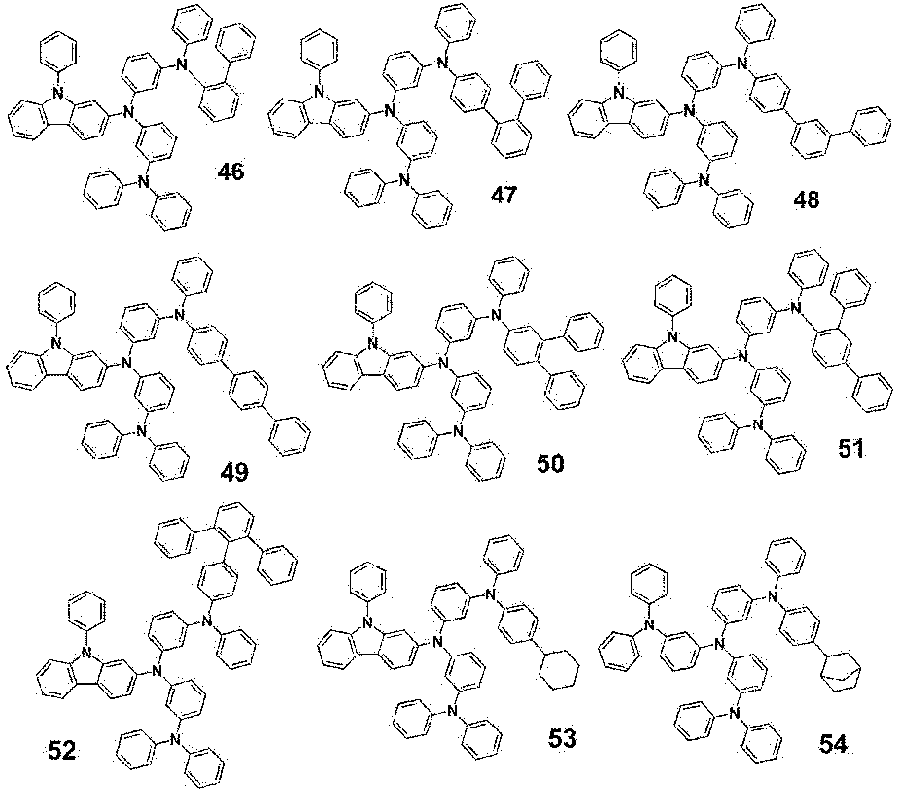

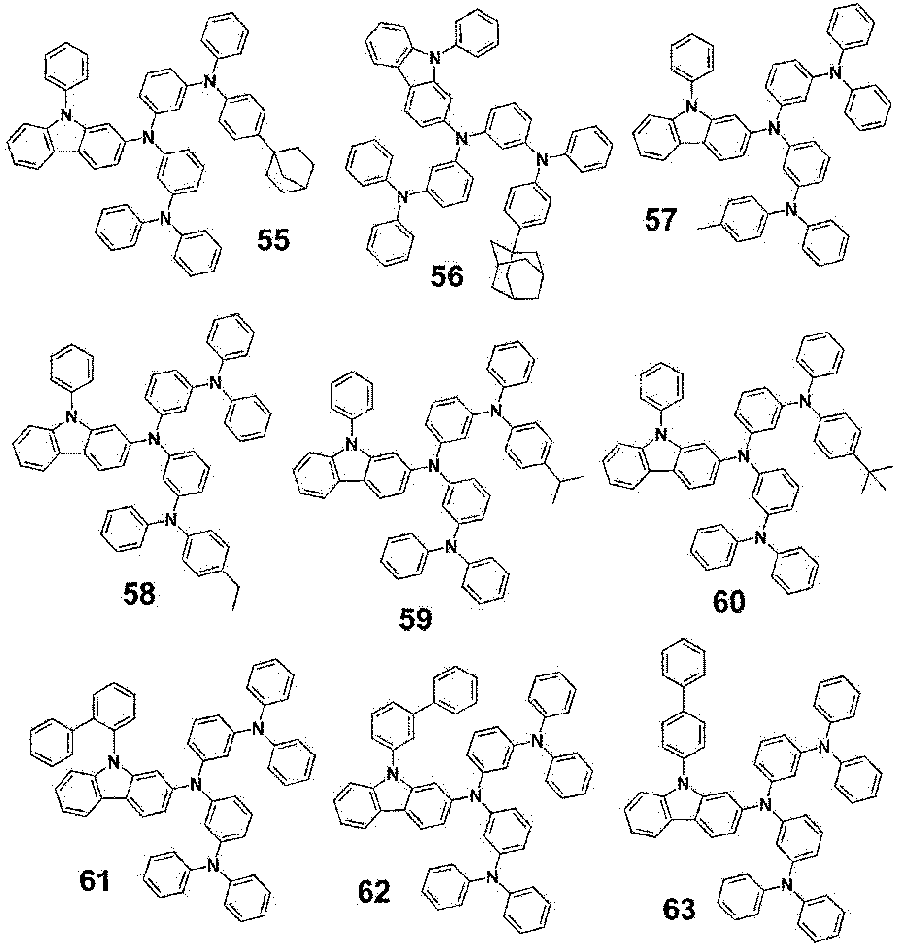

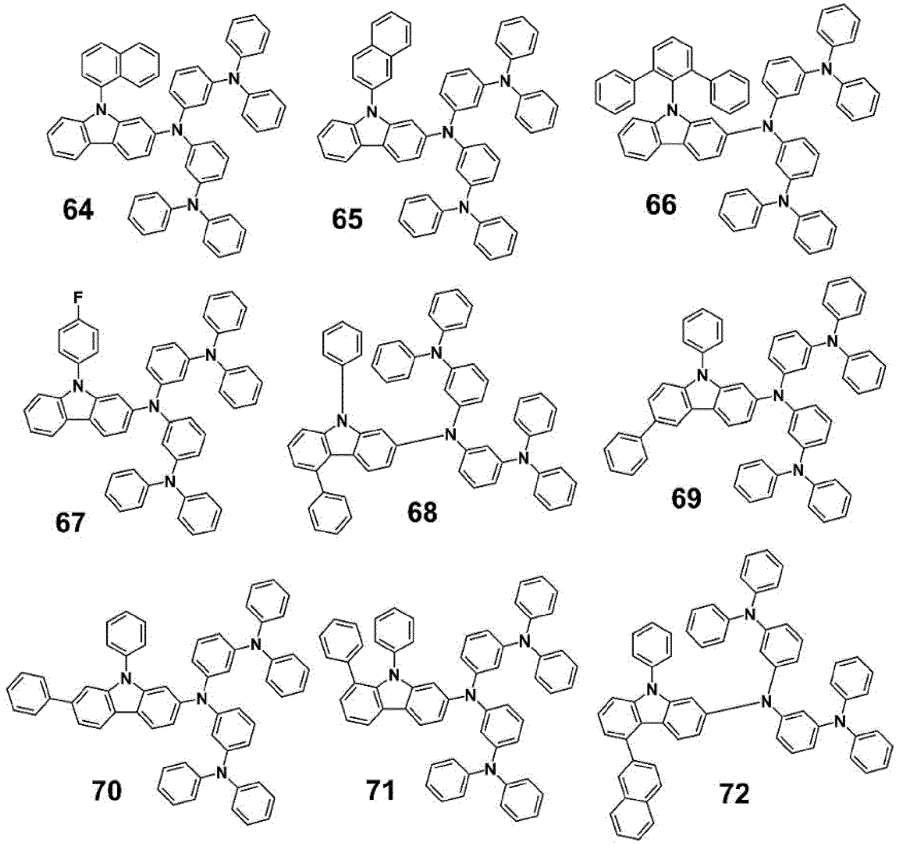

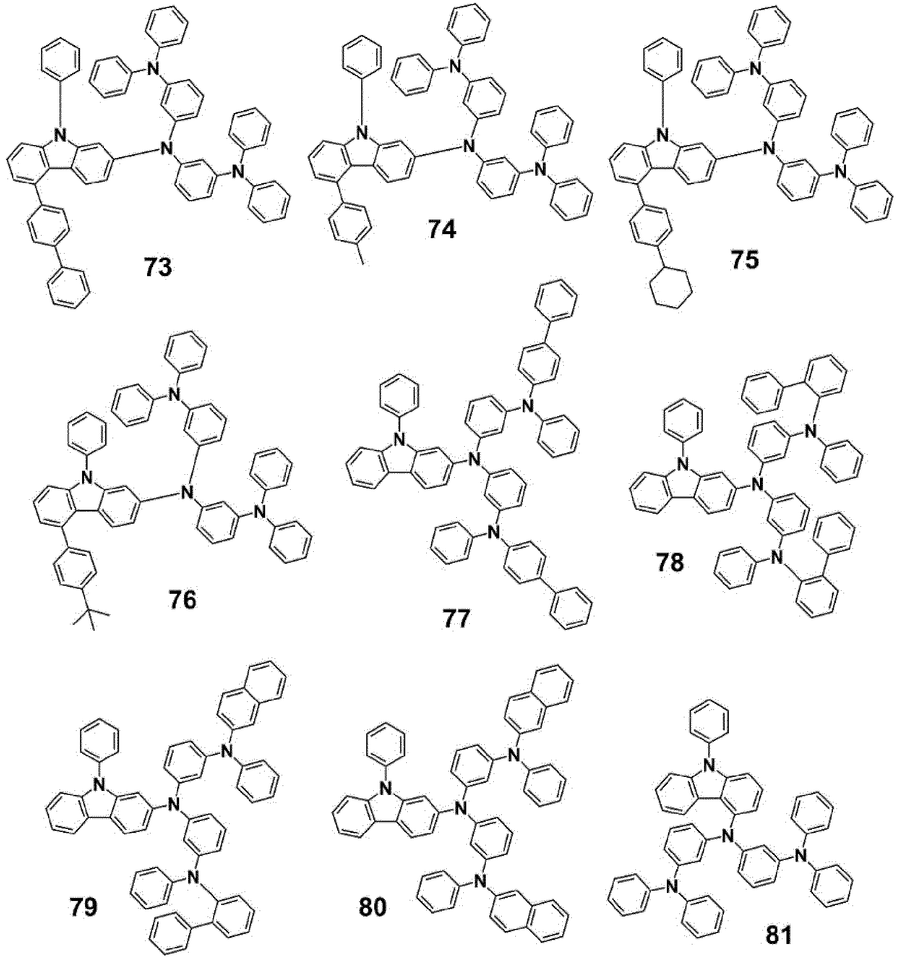

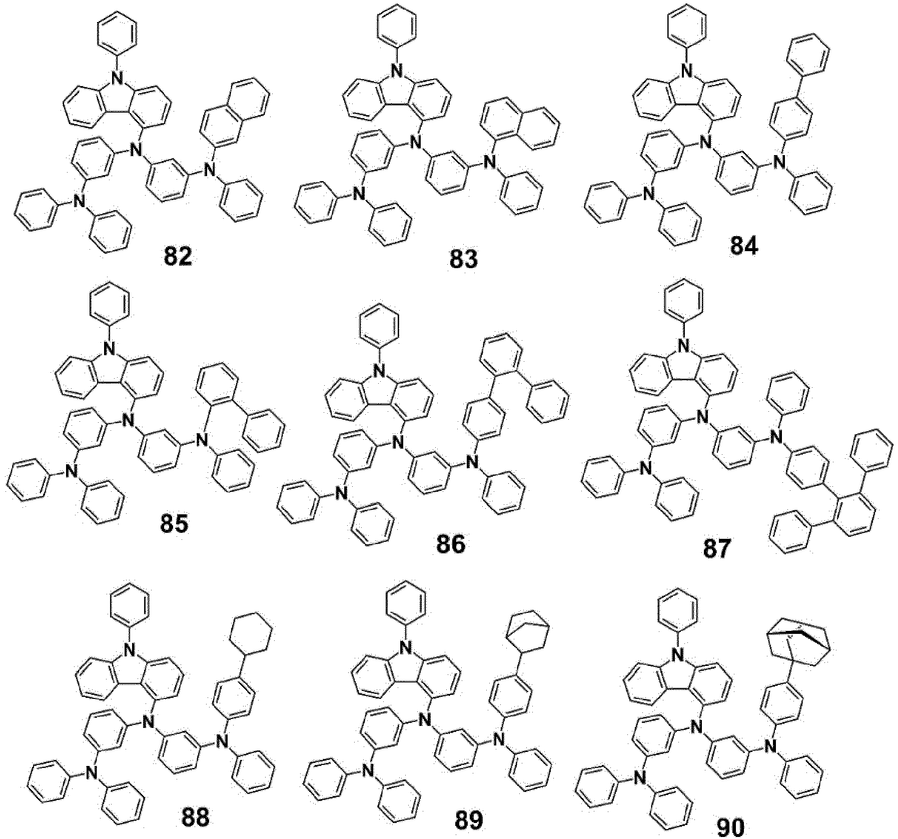

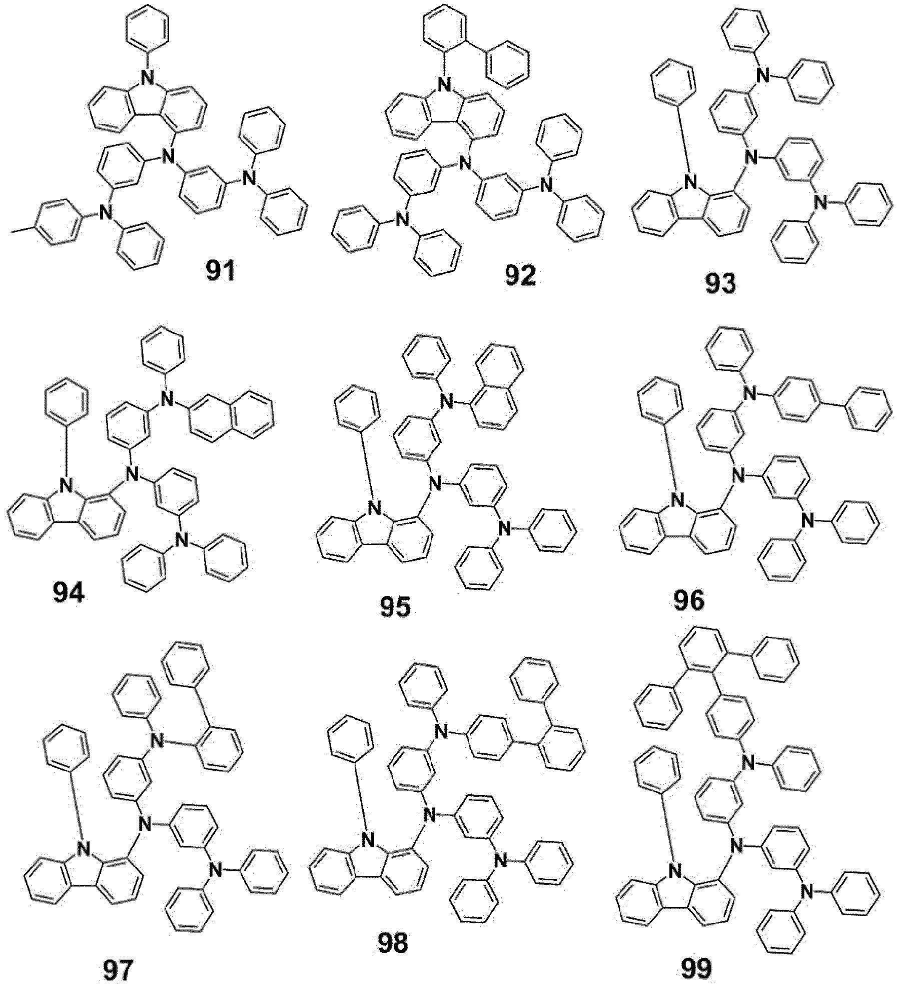

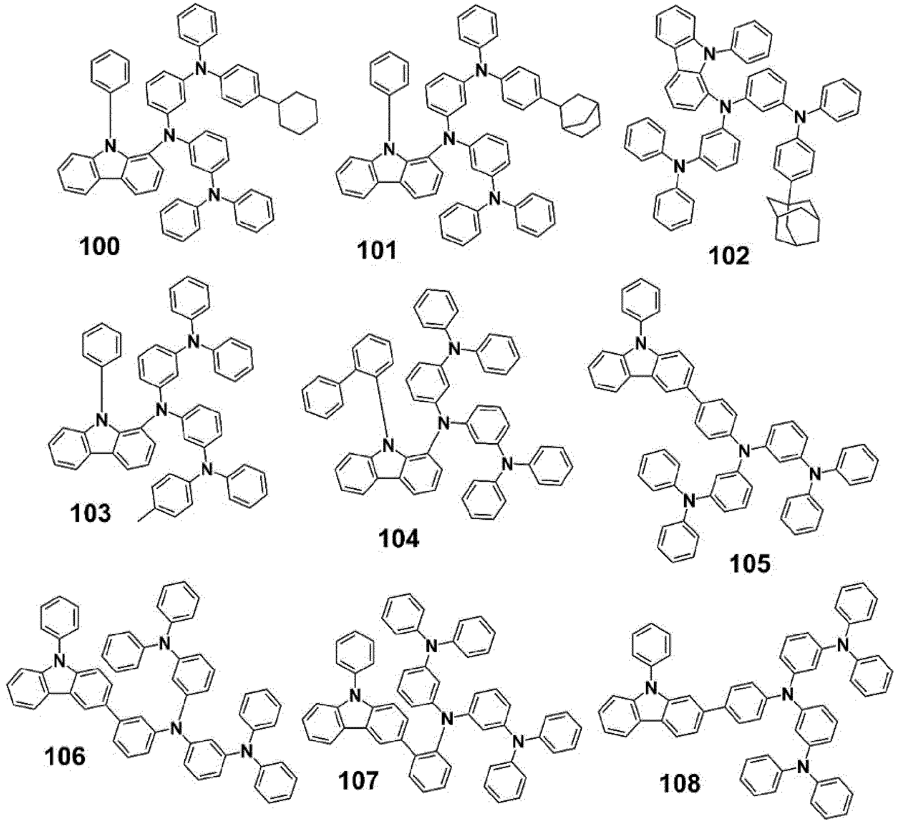

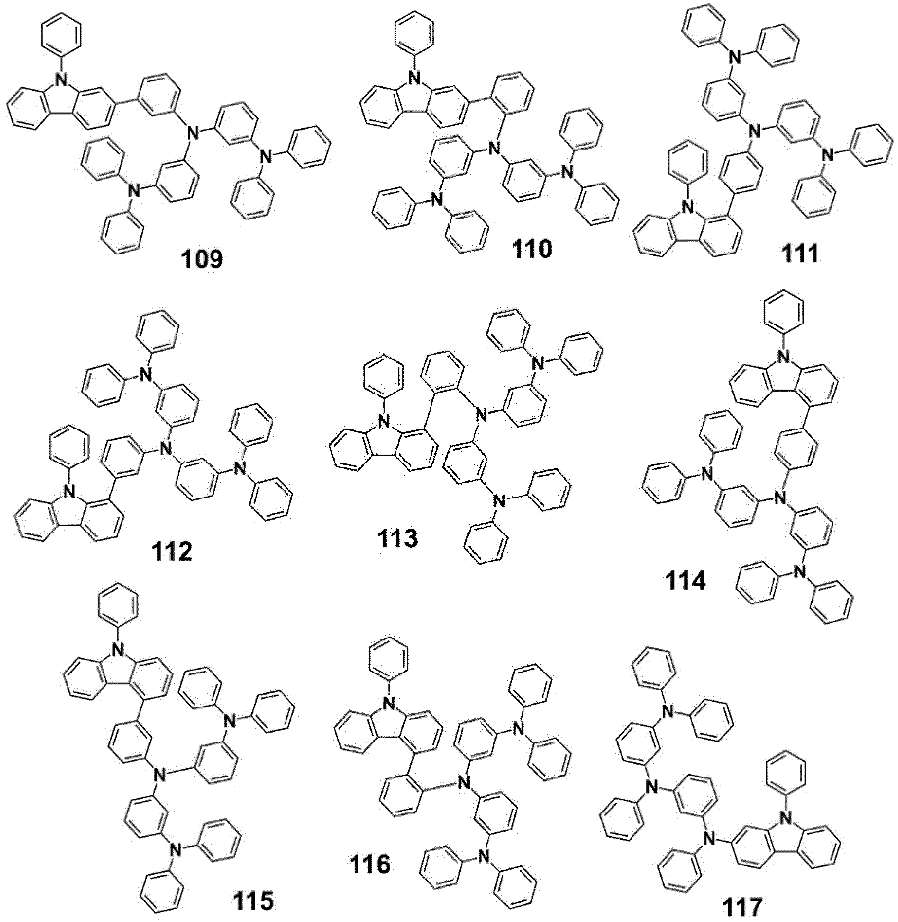

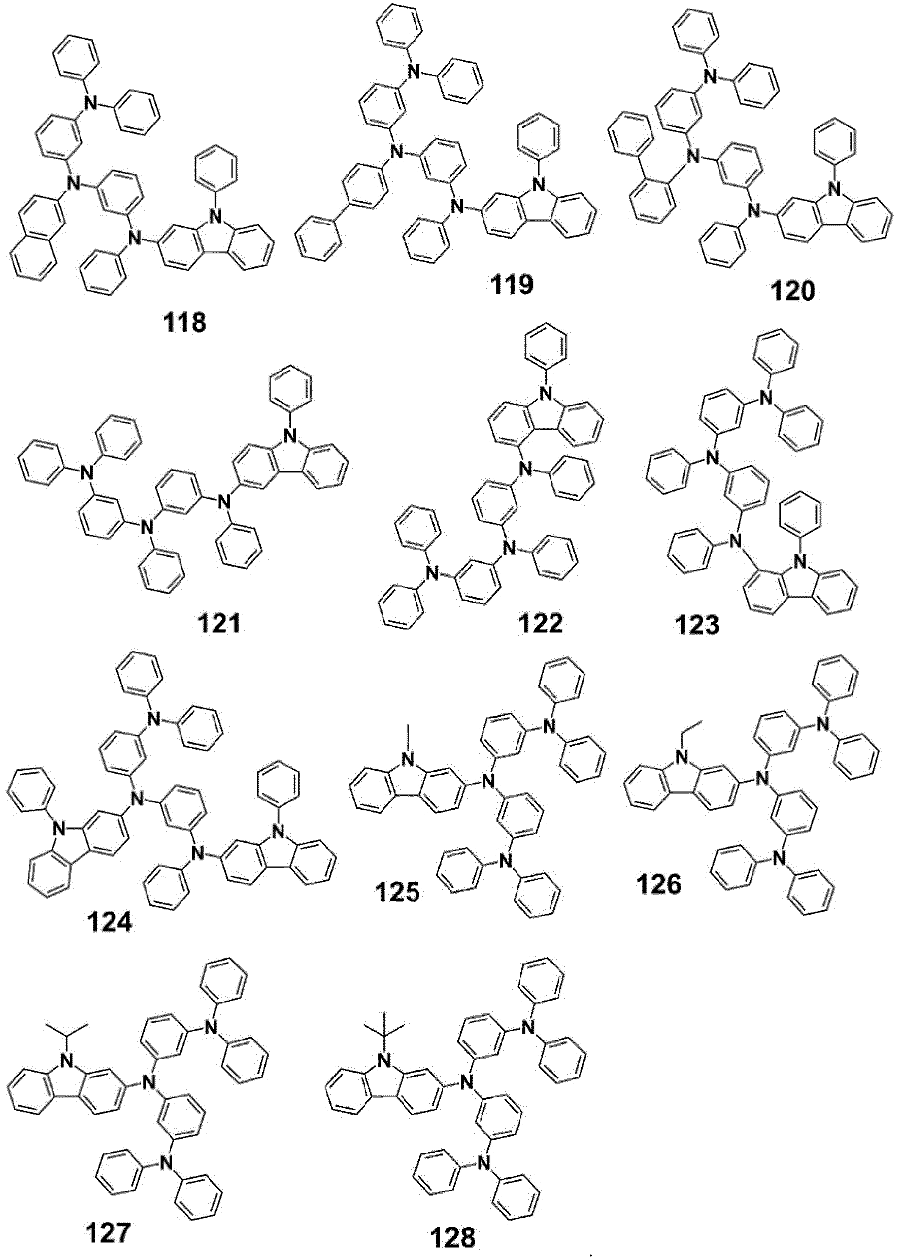

10. A light emitting element comprising:
a first electrode;
a hole transport region disposed on the first electrode;
an emission layer disposed on the hole transport region;
an electron transport region disposed on the emission layer; and
a second electrode disposed on the electron transport region, wherein
the hole transport region includes the amine compound according to any one of claims 1 to 9.

11. The light emitting element of claim 10, wherein the emission layer comprises at least one of a first compound represented by Formula HT-1, a second compound represented by Formula ET-1, and a third compound represented by Formula D-1: 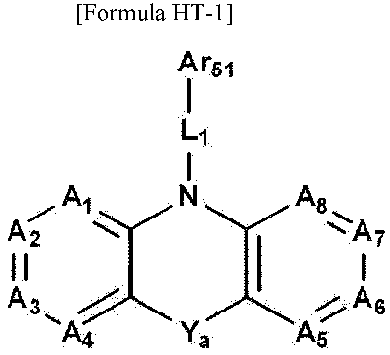 wherein in Formula HT-1,
A₁ to A₈ are each independently N or C(R₅₁),
L₁ is a direct linkage, a substituted or unsubstituted arylene group having 6 to 30 ring-forming carbon atoms, or a substituted or unsubstituted heteroarylene group having 2 to 30 ring-forming carbon atoms,
Yₐ is a direct linkage, C(R₅₂)(R₅₃), or Si(R₅₄)(R₅₅),
Ar₅₁ is a substituted or unsubstituted aryl group having 6 to 30 ring-forming carbon atoms or a substituted or unsubstituted heteroaryl group having 2 to 30 ring-forming carbon atoms, and
R₅₁ to R₅₅ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms, or bonded to an adjacent group to form a ring; 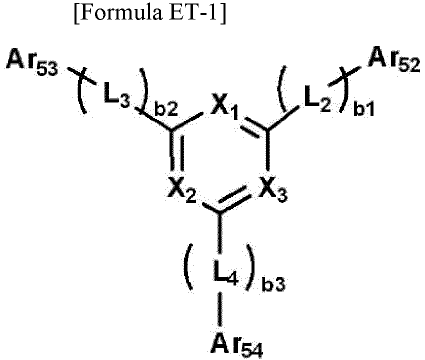
wherein in Formula ET-1,
at least one of X₁ to X₃ is N,
the remainder of X₁ to X₃ are each independently C(R₅₆),
R₅₆ is a hydrogen atom, a deuterium atom, a substituted or unsubstituted alkyl group having 1 to 20 carbon atoms, a substituted or unsubstituted aryl group having 6 to 60 ring-forming carbon atoms, or a substituted or unsubstituted heteroaryl group having 2 to 60 ring-forming carbon atoms,
b1 to b3 are each independently an integer from 0 to 10,
Ar₅₂ to Ar₅₄ are each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and
L₂ to L₄ are each independently a direct linkage, a substituted or unsubstituted arylene group having 6 to 30 ring-forming carbon atoms, or a substituted or unsubstituted heteroarylene group having 2 to 30 ring-forming carbon atoms; and 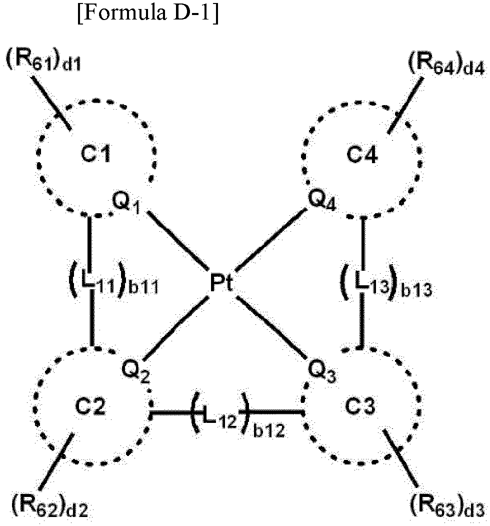
wherein in Formula D-1,
Q₁ to Q₄ are each independently C or N,
C1 to C4 are each independently a substituted or unsubstituted hydrocarbon ring having 5 to 30 ring-forming carbon atoms, a substituted or unsubstituted aryl group having 6 to 30 ring-forming carbon atoms, or a substituted or unsubstituted heterocycle having 2 to 30 ring-forming carbon atoms,
L₁₁ to L₁₃ are each independently a direct linkage, *-O-*, *-S-*, 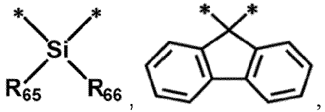 a substituted or unsubstituted alkylene group having 1 to 20 carbon atoms, a substituted or unsubstituted arylene group having 6 to 30 ring-forming carbon atoms, or a substituted or unsubstituted heteroarylene group having 2 to 30 ring-forming carbon atoms,
b11 to b13 are each independently 0 or 1,
R₆₁ to R₆₆ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms, and
d1 to d4 are each independently an integer from 0 to 4.

12. A display device comprising:
a circuit layer disposed on a base layer; and
a display element layer disposed on the circuit layer and including a light emitting element, wherein
the light emitting element includes:
a first electrode;
a hole transport region disposed on the first electrode;
an emission layer disposed on the hole transport region;
an electron transport region disposed on the emission layer; and
a second electrode disposed on the electron transport region, and
the hole transport region includes the amine compound according to any one of claims 1 to 9.

13. The display device of claim 12, further comprising:
at least one of a light control layer and a color filter layer, wherein
the light control layer includes a quantum dot, and
the color filter layer includes a pigment or a dye.

14. The display device of claim 12 or claim 13, wherein the display device is a television set, a monitor, a billboard, a personal computer, a laptop computer, a personal digital terminal, a display device for a vehicle, a game console, a portable electronic device, or a camera.
